# EUROPEAN PATENT APPLICATION

(11) **EP 1 662 255 A2**
(43) Date of publication of application: **31.05.2006**
(21) Application number: 06003521.9
(22) Date of filing: 18.08.1999
(51) Int. Cl.: G01N 33/53, G01N 33/567, C12N 5/00, C12N 5/02, C07K 14/47

(54) **Homer interacting proteins**

(30) Priority: 18.08.1998 US 97334 P; 10.06.1999 US 138493 P; 10.06.1999 US 138426 P; 10.06.1999 US 138494 P
(62) Divisional of application: 99945113.1
(71) Applicant: THE JOHNS HOPKINS UNIVERSITY SCHOOL OF MEDICINE, Baltimore, MD 21205 (US)
(72) Inventor: Worley, Paul F., Baltimore MD 21210 (US); Tu, Jian Cheng, Towson MD 21286 (US); Xiao, Bo c/o The John Hopkins University, Baltimore MD 21205 (US); Leahy, Daniel, Baltimore MD 21218 (US); Beneken, Jutta, Baltimore MD 21201 (US); Lanahan, Anthony A., Baltimore MD 21234 (US)
(74) Representative: Vossius & Partner

(57) **Abstract**

A method is provided for identifying a compound that modulates a cellular response associated with Homer and mediated by a cell-surface or an intracellular receptor. A method is further provided for identifying a compound that modulates receptor activated calcium mobilization associated with Homer. A method is provided for identifying a compound that inhibits Homer protein activity based on the crystal structure coordinates of Homer protein binding domain. A method is also provided for identifying a compound that affects the formation of cell surface receptors into clusters. Also provided are nucleic acids encoding Homer proteins as well as Homer proteins, and Homer interacting proteins.

SEQUENCE LISTING

<110> The Johns Hopkins University
      School of Medicine

<120> HOMER INTERACTING PROTEINS

<130> F 1247 EP/I S3

<140> PCT/US99/18973
<141> 1999-08-18

<150> 60/097,334
<151> 1998-08-18

<150> 60/138,426
<151> 1999-06-09

<150> 60/138,493
<151> 1999-06-09

<150> 60/138,494
<151> 1999-06-09

<160> 58

<170> FastSEQ for Windows Version 4.0

<210> 1
<211> 1084
<212> DNA
<213> Homo sapiens

<220>
<221> CDS
<222> (76)...(789)
<223> Homer 1a

<400> 1
agacggagaa attcctttgg aagttattcc gtagcataag agctgaaact tcagagcaag      60
ttttcattgg gcaaa atg ggg gaa caa cct atc ttc agc act cga gct cat      111
                 Met Gly Glu Gln Pro Ile Phe Ser Thr Arg Ala His
                  1               5                  10

gtc ttc caa att gac cca aac aca aag aag aac tgg gta ccc acc agc      159
Val Phe Gln Ile Asp Pro Asn Thr Lys Lys Asn Trp Val Pro Thr Ser
        15                  20                  25

aag cat gca gtt act gtg tct tat ttc tat gac agc aca aga aat gtg      207
Lys His Ala Val Thr Val Ser Tyr Phe Tyr Asp Ser Thr Arg Asn Val
    30                  35                  40

tat agg ata atc agt tta gat ggc tca aag gca ata ata aat agt acc      255
Tyr Arg Ile Ile Ser Leu Asp Gly Ser Lys Ala Ile Ile Asn Ser Thr
 45                  50                  55                  60

atc acc cca aac atg aca ttt act aaa aca tct cag aag ttt ggc cag      303
Ile Thr Pro Asn Met Thr Phe Thr Lys Thr Ser Gln Lys Phe Gly Gln
                 65                  70                  75

tgg gct gat agc cgg gca aac acc gtt tat gga ttg gga ttc tcc tct      351

Trp Ala Asp Ser Arg Ala Asn Thr Val Tyr Gly Leu Gly Phe Ser Ser
            80                  85                  90

gag cat cat ctt tcg aaa ttt gca gaa aag ttt cag gaa ttt aaa gaa 399
Glu His His Leu Ser Lys Phe Ala Glu Lys Phe Gln Glu Phe Lys Glu
        95                  100                 105

gct gct cga cta gca aag gaa aaa tca caa gag aag atg gaa ctt acc 447
Ala Ala Arg Leu Ala Lys Glu Lys Ser Gln Glu Lys Met Glu Leu Thr
    110                 115                 120

agt aca cct tca cag gaa tcc gca ggc ggg gat ctt cag tct cct tta 495
Ser Thr Pro Ser Gln Glu Ser Ala Gly Gly Asp Leu Gln Ser Pro Leu
125                 130                 135                 140

aca ccg gaa agt atc aac ggg aca gat gat gaa aga aca cct gat gtg 543
Thr Pro Glu Ser Ile Asn Gly Thr Asp Asp Glu Arg Thr Pro Asp Val
                145                 150                 155

aca cag aac tca gag cca agg gct gaa cca act cag aat gca ttg cca 591
Thr Gln Asn Ser Glu Pro Arg Ala Glu Pro Thr Gln Asn Ala Leu Pro
            160                 165                 170

ttt tca cat agt tca gca atc agc aaa cat tgg gag gct gaa ctg gct 639
Phe Ser His Ser Ser Ala Ile Ser Lys His Trp Glu Ala Glu Leu Ala
        175                 180                 185

acc ctc aaa gga aat aat gcc aaa ctc act gca gcc ctg ctg gag tcc 687
Thr Leu Lys Gly Asn Asn Ala Lys Leu Thr Ala Ala Leu Leu Glu Ser
    190                 195                 200

act gcc aat gtg aaa caa tgg aaa cag caa ctt gct gcc tat caa gag 735
Thr Ala Asn Val Lys Gln Trp Lys Gln Gln Leu Ala Ala Tyr Gln Glu
205                 210                 215                 220

gaa gca gaa cgt ctg cac aag cgg gta att tca ggg ctg atg tct ata 783
Glu Ala Glu Arg Leu His Lys Arg Val Ile Ser Gly Leu Met Ser Ile
                225                 230                 235

ggg att tagggctaac aggttttctt gatcagaaga aatttgcatg tagattcagc 839
Gly Ile

acagggatat cttctagttc taggatgtca gaacatagat atgggttgta tgatatgcat 899
ttgtttgatt aagaaaaata ttttccatag tttaatgaga atgaagaata ataccgcctt 959
ttgaagtcaa caaaccatgt tgattcccca tattatccat ggggactagc agtaatgcac 1019
aagtacataa aagcactaat gtattagtgc tagttgatta gtactgacat ggtagttaaa 1079
gtgga 1084

<210> 2
<211> 238
<212> PRT
<213> Homo sapiens

<400> 2
Met Gly Glu Gln Pro Ile Phe Ser Thr Arg Ala His Val Phe Gln Ile
1               5                   10                  15
Asp Pro Asn Thr Lys Lys Asn Trp Val Pro Thr Ser Lys His Ala Val
            20                  25                  30
Thr Val Ser Tyr Phe Tyr Asp Ser Thr Arg Asn Val Tyr Arg Ile Ile
        35                  40                  45
Ser Leu Asp Gly Ser Lys Ala Ile Ile Asn Ser Thr Ile Thr Pro Asn
    50                  55                  60

Met Thr Phe Thr Lys Thr Ser Gln Lys Phe Gly Gln Trp Ala Asp Ser
65                  70                  75                  80
Arg Ala Asn Thr Val Tyr Gly Leu Gly Phe Ser Ser Glu His His Leu
                85                  90                  95
Ser Lys Phe Ala Glu Lys Phe Gln Glu Phe Lys Glu Ala Ala Arg Leu
            100                 105                 110
Ala Lys Glu Lys Ser Gln Glu Lys Met Glu Leu Thr Ser Thr Pro Ser
        115                 120                 125
Gln Glu Ser Ala Gly Gly Asp Leu Gln Ser Pro Leu Thr Pro Glu Ser
    130                 135                 140
Ile Asn Gly Thr Asp Asp Glu Arg Thr Pro Asp Val Thr Gln Asn Ser
145                 150                 155                 160
Glu Pro Arg Ala Glu Pro Thr Gln Asn Ala Leu Pro Phe Ser His Ser
                165                 170                 175
Ser Ala Ile Ser Lys His Trp Glu Ala Glu Leu Ala Thr Leu Lys Gly
            180                 185                 190
Asn Asn Ala Lys Leu Thr Ala Ala Leu Leu Glu Ser Thr Ala Asn Val
        195                 200                 205
Lys Gln Trp Lys Gln Gln Leu Ala Ala Tyr Gln Glu Glu Ala Glu Arg
    210                 215                 220
Leu His Lys Arg Val Ile Ser Gly Leu Met Ser Ile Gly Ile
225                 230                 235

<210> 3
<211> 1166
<212> DNA
<213> Homo sapiens

<220>
<221> CDS
<222> (1)...(1062)
<223> Homer 1b

<400> 3
atg ggg gag cag ccg att ttc agc act cga gct cat gtc ttc caa att      48
Met Gly Glu Gln Pro Ile Phe Ser Thr Arg Ala His Val Phe Gln Ile
 1               5                  10                  15

gac cca aac aca aag aag aac tgg gta ccc acc agc aag cat gca gtt      96
Asp Pro Asn Thr Lys Lys Asn Trp Val Pro Thr Ser Lys His Ala Val
            20                  25                  30

act gtg tct tat ttc tat gac agc aca aga aat gtg tat agg ata atc      144
Thr Val Ser Tyr Phe Tyr Asp Ser Thr Arg Asn Val Tyr Arg Ile Ile
        35                  40                  45

agt tta gat ggc tca aag gca ata ata aat agt acc atc acc cca aac      192
Ser Leu Asp Gly Ser Lys Ala Ile Ile Asn Ser Thr Ile Thr Pro Asn
    50                  55                  60

atg aca ttt act aaa aca tct cag aag ttt ggc cag tgg gct gat agc      240
Met Thr Phe Thr Lys Thr Ser Gln Lys Phe Gly Gln Trp Ala Asp Ser
 65                  70                  75                  80

cgg gca aac acc gtt tat gga ttg gga ttc tcc tct gag cat cat ctt      288
Arg Ala Asn Thr Val Tyr Gly Leu Gly Phe Ser Ser Glu His His Leu
                85                  90                  95

tcg aaa ttt gca gaa aag ttt cag gaa ttt aaa gaa gct gct cga cta      336
Ser Lys Phe Ala Glu Lys Phe Gln Glu Phe Lys Glu Ala Ala Arg Leu
            100                 105                 110

gca aag gaa aaa tca caa gag aag atg gaa ctt acc agt aca cct tca      384

Ala Lys Glu Lys Ser Gln Glu Lys Met Glu Leu Thr Ser Thr Pro Ser
        115                 120                 125

cag gaa tcc gca ggc ggg gat ctt cag tct cct tta aca ccg gaa agt      432
Gln Glu Ser Ala Gly Gly Asp Leu Gln Ser Pro Leu Thr Pro Glu Ser
    130                 135                 140

atc aac ggg aca gat gat gaa aga aca cct gat gtg aca cag aac tca      480
Ile Asn Gly Thr Asp Asp Glu Arg Thr Pro Asp Val Thr Gln Asn Ser
145                 150                 155                 160

gag cca agg gct gaa cca act cag aat gca ttg cca ttt tca cat agt      528
Glu Pro Arg Ala Glu Pro Thr Gln Asn Ala Leu Pro Phe Ser His Ser
                165                 170                 175

tca gca atc agc aaa cat tgg gag gct gaa ctg gct acc ctc aaa gga      576
Ser Ala Ile Ser Lys His Trp Glu Ala Glu Leu Ala Thr Leu Lys Gly
            180                 185                 190

aat aat gcc aaa ctc act gca gcc ctg ctg gag tcc act gcc aat gtg      624
Asn Asn Ala Lys Leu Thr Ala Ala Leu Leu Glu Ser Thr Ala Asn Val
        195                 200                 205

aaa caa tgg aaa cag caa ctt gct gcc tat caa gag gaa gca gaa cgt      672
Lys Gln Trp Lys Gln Gln Leu Ala Ala Tyr Gln Glu Glu Ala Glu Arg
    210                 215                 220

ctg cac aag cgg gtg act gaa ctt gaa tgt gtt agt agc caa gca aat      720
Leu His Lys Arg Val Thr Glu Leu Glu Cys Val Ser Ser Gln Ala Asn
225                 230                 235                 240

gca gta cat act cat aag aca gaa tta aat cag aca ata caa gaa ctg      768
Ala Val His Thr His Lys Thr Glu Leu Asn Gln Thr Ile Gln Glu Leu
                245                 250                 255

gaa gag aca ctg aaa ctg aag gaa gag gaa ata gaa agg tta aaa caa      816
Glu Glu Thr Leu Lys Leu Lys Glu Glu Glu Ile Glu Arg Leu Lys Gln
            260                 265                 270

gaa att gat aat gcc aga gaa cta caa gaa cag agg gat tct ttg act      864
Glu Ile Asp Asn Ala Arg Glu Leu Gln Glu Gln Arg Asp Ser Leu Thr
        275                 280                 285

cag aaa cta cag gaa gta gaa att cgg aac aaa gac ctg gag gga caa      912
Gln Lys Leu Gln Glu Val Glu Ile Arg Asn Lys Asp Leu Glu Gly Gln
    290                 295                 300

ctg tct gac tta gag caa cgt ctg gag aaa agt cag aat gaa caa gaa      960
Leu Ser Asp Leu Glu Gln Arg Leu Glu Lys Ser Gln Asn Glu Gln Glu
305                 310                 315                 320

gct ttt cgc aat aac ctg aag aca ctc tta gaa att ctg gat gga aag     1008
Ala Phe Arg Asn Asn Leu Lys Thr Leu Leu Glu Ile Leu Asp Gly Lys
                325                 330                 335

ata ttt gaa cta aca gaa tta cga gat aac ttg gcc aag cta cta gaa     1056
Ile Phe Glu Leu Thr Glu Leu Arg Asp Asn Leu Ala Lys Leu Leu Glu
            340                 345                 350

tgc agc taaggaaagt gaaatttcag tgccaattaa ttaaaagata cactgtctct     1112
Cys Ser

cttcatagga ctgtttagct ctgcatcaag attgcacaaa aaaaaaaaaa aaaa 1166

<210> 4
<211> 354
<212> PRT
<213> Homo sapiens

<400> 4
Met Gly Glu Gln Pro Ile Phe Ser Thr Arg Ala His Val Phe Gln Ile
1               5                   10                  15
Asp Pro Asn Thr Lys Lys Asn Trp Val Pro Thr Ser Lys His Ala Val
            20                  25                  30
Thr Val Ser Tyr Phe Tyr Asp Ser Thr Arg Asn Val Tyr Arg Ile Ile
        35                  40                  45
Ser Leu Asp Gly Ser Lys Ala Ile Ile Asn Ser Thr Ile Thr Pro Asn
    50                  55                  60
Met Thr Phe Thr Lys Thr Ser Gln Lys Phe Gly Gln Trp Ala Asp Ser
65                  70                  75                  80
Arg Ala Asn Thr Val Tyr Gly Leu Gly Phe Ser Ser Glu His His Leu
                85                  90                  95
Ser Lys Phe Ala Glu Lys Phe Gln Glu Phe Lys Glu Ala Ala Arg Leu
            100                 105                 110
Ala Lys Glu Lys Ser Gln Glu Lys Met Glu Leu Thr Ser Thr Pro Ser
        115                 120                 125
Gln Glu Ser Ala Gly Gly Asp Leu Gln Ser Pro Leu Thr Pro Glu Ser
    130                 135                 140
Ile Asn Gly Thr Asp Asp Glu Arg Thr Pro Asp Val Thr Gln Asn Ser
145                 150                 155                 160
Glu Pro Arg Ala Glu Pro Thr Gln Asn Ala Leu Pro Phe Ser His Ser
                165                 170                 175
Ser Ala Ile Ser Lys His Trp Glu Ala Glu Leu Ala Thr Leu Lys Gly
            180                 185                 190
Asn Asn Ala Lys Leu Thr Ala Ala Leu Leu Glu Ser Thr Ala Asn Val
        195                 200                 205
Lys Gln Trp Lys Gln Gln Leu Ala Ala Tyr Gln Glu Glu Ala Glu Arg
    210                 215                 220
Leu His Lys Arg Val Thr Glu Leu Glu Cys Val Ser Ser Gln Ala Asn
225                 230                 235                 240
Ala Val His Thr His Lys Thr Glu Leu Asn Gln Thr Ile Gln Glu Leu
                245                 250                 255
Glu Glu Thr Leu Lys Leu Lys Glu Glu Glu Ile Glu Arg Leu Lys Gln
            260                 265                 270
Glu Ile Asp Asn Ala Arg Glu Leu Gln Glu Gln Arg Asp Ser Leu Thr
        275                 280                 285
Gln Lys Leu Gln Glu Val Glu Ile Arg Asn Lys Asp Leu Glu Gly Gln
    290                 295                 300
Leu Ser Asp Leu Glu Gln Arg Leu Glu Lys Ser Gln Asn Glu Gln Glu
305                 310                 315                 320
Ala Phe Arg Asn Asn Leu Lys Thr Leu Leu Glu Ile Leu Asp Gly Lys
                325                 330                 335
Ile Phe Glu Leu Thr Glu Leu Arg Asp Asn Leu Ala Lys Leu Leu Glu
            340                 345                 350
Cys Ser

<210> 5
<211> 1767
<212> DNA
<213> Homo sapiens

<220>
<221> CDS
<222> (1)...(1029)

<223> Homer 2a

<400> 5
atg ggg gag cag ccg atc ttc acc acc cga gcg cat gtc ttc cag att 48
gac ccc aac acc aag aag aac tgg atg cct gcg agc aag cag gcg gtc 96
acc gtt tcc tac ttc tat gat gtc aca agg aac agc tat cgg atc atc 144
agt gtg gac gga gcc aag gtg atc ata aac agc aca atc aca ccg aat 192
atg acc ttc acc aaa acg tca cag aag ttt ggg cag tgg gcc gac agc 240
aga gcc aac aca gtg ttt ggt ttg ggg ttt tcc tct gag cag cag ctg 288
aca aag ttt gca gag aaa ttc cag gag gtg aaa gaa gct gcc aag ata 336
gcc aaa gac aag acg cag gag aaa atc gag acc tca agt aat cat tcc 384
caa gca tcc agt gtc aac ggg acg gac gag gaa aag gcc tct cac gcc 432
ggt cca gcc aac aca caa ctg aag tct gag aat gac aag ctg aag att 480
gcc ttg acg cag agc gca gcc aac gtg aag aag tgg gag atc gag ctg 528
cag acc ctt cgg gag agc aat gca cgg ctg acc aca gca ctg cag gag 576
tcg gca gcc agt gtg gag cag tgg aag agg cag ttc tcc atc tgc cgt 624
gat gag aat gac cgg ctc cgc aac aag att gat gag ctg gaa gaa caa 672
tgc agt gag atc aac aga gag aag gag aag aac acg cag ctg aag agg 720
agg atc gag gag ctg gag gca gag ctc cga gaa aag gag aca gag ctg 768
aaa gat ctc cga aaa caa agt gaa atc ata cct cag ctc atg tca gag 816
tgc gaa tat gtc tct gag aag cta gag gcg gca gag aga gac aat caa 864
aac ctg gaa gac aaa gtg cgt tcc tta aag aca gac att gag gag agc 912
aaa tac cga cag cgc cac ctg aag gtg gag ttg aag agc ttc ctg gag 960
gtg ctg gac ggg aag att gac gac ctg cat gac ttc cgc cga ggg ctc 1008
tcc aag ctg ggc acc gat aac tagggctggc cgaggcccag gccccgcccg 1059
tgagtcccaa gcgtgtgtgc gagaccagat agctctagga cgttcttctg tgtgcattgc 1119
ttctgtaaat gcaggcgcag tttgtcgtgt ttccaaacca gttgtgccgt ccactcactc 1179
cttttcagaa tagaaatctc ctctcgcttc tctggccttg tgaggttgtg gacaactgga 1239
agattctgac tcaggaatcc agaactaggt ctaccttcaa catttatgca gtcagggcag 1299
ggatgtttat atctttcata agggctgttg caaccatatg aactgaaaaa acacgcattt 1359
tgtaatccaa atattgatat tctttacacc aagccatcag gctcctttta tcaaatagca 1419
ttcagagtat ttgaatgtcc accagacacc agccccgggg ggcacagaga gaacaacatt 1479
cctctctgtc aacatcgaga ggctttaaaa caactgttta gtggaaactt tctgagagat 1539
ggaaaacaag cttctggtgg gtgcattttc tggcccggag ttgcctgcat ccacgctact 1599
gccccctgcc ccccgccccc ccagtttgta cggttgcaac agtgttcctt ttcttggttt 1659
taatttctga gcagatgatt tgctgtggga acagcacaca gtgagggtgc ctagcacaat 1719
gtctggcaca aagtaggtgc ttaataaata tttgttcaat taaaaaaa 1767

<210> 6
<211> 343
<212> PRT
<213> Homo sapiens

<400> 6

<210> 7
<211> 1800
<212> DNA
<213> Homo sapiens

<220>
<221> CDS
<222> (1)...(1062)
<223> Homer 2b

<400> 7
atg ggg gag cag ccg atc ttc acc acc cga gcg cat gtc ttc cag att      48
Met Gly Glu Gln Pro Ile Phe Thr Thr Arg Ala His Val Phe Gln Ile
 1               5                  10                  15

gac ccc aac acc aag aag aac tgg atg cct gcg agc aag cag gcg gtc      96
Asp Pro Asn Thr Lys Lys Asn Trp Met Pro Ala Ser Lys Gln Ala Val
            20                  25                  30

acc gtt tcc tac ttc tat gat gtc aca agg aac agc tat cgg atc atc      144
Thr Val Ser Tyr Phe Tyr Asp Val Thr Arg Asn Ser Tyr Arg Ile Ile
        35                  40                  45

agt gtg gac gga gcc aag gtg atc ata aac agc aca atc aca ccg aat      192
Ser Val Asp Gly Ala Lys Val Ile Ile Asn Ser Thr Ile Thr Pro Asn
    50                  55                  60

atg acc ttc acc aaa acg tca cag aag ttt ggg cag tgg gcc gac agc      240
Met Thr Phe Thr Lys Thr Ser Gln Lys Phe Gly Gln Trp Ala Asp Ser
 65                  70                  75                  80

aga gcc aac aca gtg ttt ggt ttg ggg ttt tcc tct gag cag cag ctg      288
Arg Ala Asn Thr Val Phe Gly Leu Gly Phe Ser Ser Glu Gln Gln Leu
                85                  90                  95

aca aag ttt gca gag aaa ttc cag gag gtg aaa gaa gct gcc aag ata 336
Thr Lys Phe Ala Glu Lys Phe Gln Glu Val Lys Glu Ala Ala Lys Ile
            100                 105                 110

gcc aaa gac aag acg cag gag aaa atc gag acc tca agt aat cat tcc 384
Ala Lys Asp Lys Thr Gln Glu Lys Ile Glu Thr Ser Ser Asn His Ser
        115                 120                 125

caa gaa tct ggg cgt gaa acc cca tct tct act cag gca tcc agt gtc 432
Gln Glu Ser Gly Arg Glu Thr Pro Ser Ser Thr Gln Ala Ser Ser Val
    130                 135                 140

aac ggg acg gac gag gaa aag gcc tct cac gcc ggt cca gcc aac aca 480
Asn Gly Thr Asp Glu Glu Lys Ala Ser His Ala Gly Pro Ala Asn Thr
145                 150                 155                 160

caa ctg aag tct gag aat gac aag ctg aag att gcc ttg acg cag agc 528
Gln Leu Lys Ser Glu Asn Asp Lys Leu Lys Ile Ala Leu Thr Gln Ser
                165                 170                 175

gca gcc aac gtg aag aag tgg gag atc gag ctg cag acc ctt cgg gag 576
Ala Ala Asn Val Lys Lys Trp Glu Ile Glu Leu Gln Thr Leu Arg Glu
            180                 185                 190

agc aat gca cgg ctg acc aca gca ctg cag gag tcg gca gcc agt gtg 624
Ser Asn Ala Arg Leu Thr Thr Ala Leu Gln Glu Ser Ala Ala Ser Val
        195                 200                 205

gag cag tgg aag agg cag ttc tcc atc tgc cgt gat gag aat gac cgg 672
Glu Gln Trp Lys Arg Gln Phe Ser Ile Cys Arg Asp Glu Asn Asp Arg
    210                 215                 220

ctc cgc aac aag att gat gag ctg gaa gaa caa tgc agt gag atc aac 720
Leu Arg Asn Lys Ile Asp Glu Leu Glu Glu Gln Cys Ser Glu Ile Asn
225                 230                 235                 240

aga gag aag gag aag aac acg cag ctg aag agg agg atc gag gag ctg 768
Arg Glu Lys Glu Lys Asn Thr Gln Leu Lys Arg Arg Ile Glu Glu Leu
                245                 250                 255

gag gca gag ctc cga gaa aag gag aca gag ctg aaa gat ctc cga aaa 816
Glu Ala Glu Leu Arg Glu Lys Glu Thr Glu Leu Lys Asp Leu Arg Lys
            260                 265                 270

caa agt gaa atc ata cct cag ctc atg tca gag tgc gaa tat gtc tct 864
Gln Ser Glu Ile Ile Pro Gln Leu Met Ser Glu Cys Glu Tyr Val Ser
        275                 280                 285

gag aag cta gag gcg gca gag aga gac aat caa aac ctg gaa gac aaa 912
Glu Lys Leu Glu Ala Ala Glu Arg Asp Asn Gln Asn Leu Glu Asp Lys
    290                 295                 300

gtg cgt tcc tta aag aca gac att gag gag agc aaa tac cga cag cgc 960
Val Arg Ser Leu Lys Thr Asp Ile Glu Glu Ser Lys Tyr Arg Gln Arg
305                 310                 315                 320

cac ctg aag gtg gag ttg aag agc ttc ctg gag gtg ctg gac ggg aag 1008
His Leu Lys Val Glu Leu Lys Ser Phe Leu Glu Val Leu Asp Gly Lys
                325                 330                 335

att gac gac ctg cat gac ttc cgc cga ggg ctc tcc aag ctg ggc acc 1056
Ile Asp Asp Leu His Asp Phe Arg Arg Gly Leu Ser Lys Leu Gly Thr

            340                 345                 350

gat aac tagggctggc cgaggcccag gccccgcccg tgagtcccaa gcgtgtgtgc 1112
Asp Asn

gagaccagat agctctagga cgttcttctg tgtgcattgc ttctgtaaat gcaggcgcag 1172
tttgtcgtgt ttccaaacca gttgtgccgt ccactcactc cttttcagaa tagaaatctc 1232
ctctcgcttc tctggccttg tgaggttgtg gacaactgga agattctgac tcaggaatcc 1292
agaactaggt ctaccttcaa catttatgca gtcagggcag ggatgtttat atctttcata 1352
agggctgttg caaccatatg aactgaaaaa acacgcattt tgtaatccaa atattgatat 1412
tctttacacc aagccatcag gctcctttta tcaaatagca ttcagagtat ttgaatgtcc 1472
accagacacc agccccgggg ggcacagaga gaacaacatt cctctctgtc aacatcgaga 1532
ggctttaaaa caactgttta gtggaaactt tctgagagat ggaaaacaag cttctggtgg 1592
gtgcattttc tggcccggag ttgcctgcat ccacgctact gccccctgcc ccccgccccc 1652
ccagtttgta cggttgcaac agtgttcctt ttcttggttt taatttctga gcagatgatt 1712
tgctgtggga acagcacaca gtgagggtgc ctagcacaat gtctggcaca aagtaggtgc 1772
ttaataaata tttgttcaat taaaaaaa 1800

<210> 8
<211> 354
<212> PRT
<213> Homo sapiens

<400> 8
Met Gly Glu Gln Pro Ile Phe Thr Thr Arg Ala His Val Phe Gln Ile
1               5                   10                  15
Asp Pro Asn Thr Lys Lys Asn Trp Met Pro Ala Ser Lys Gln Ala Val
            20                  25                  30
Thr Val Ser Tyr Phe Tyr Asp Val Thr Arg Asn Ser Tyr Arg Ile Ile
        35                  40                  45
Ser Val Asp Gly Ala Lys Val Ile Ile Asn Ser Thr Ile Thr Pro Asn
    50                  55                  60
Met Thr Phe Thr Lys Thr Ser Gln Lys Phe Gly Gln Trp Ala Asp Ser
65                  70                  75                  80
Arg Ala Asn Thr Val Phe Gly Leu Gly Phe Ser Ser Glu Gln Gln Leu
                85                  90                  95
Thr Lys Phe Ala Glu Lys Phe Gln Glu Val Lys Glu Ala Ala Lys Ile
            100                 105                 110
Ala Lys Asp Lys Thr Gln Glu Lys Ile Glu Thr Ser Ser Asn His Ser
        115                 120                 125
Gln Glu Ser Gly Arg Glu Thr Pro Ser Ser Thr Gln Ala Ser Ser Val
    130                 135                 140
Asn Gly Thr Asp Glu Glu Lys Ala Ser His Ala Gly Pro Ala Asn Thr
145                 150                 155                 160
Gln Leu Lys Ser Glu Asn Asp Lys Leu Lys Ile Ala Leu Thr Gln Ser
                165                 170                 175
Ala Ala Asn Val Lys Lys Trp Glu Ile Glu Leu Gln Thr Leu Arg Glu
            180                 185                 190
Ser Asn Ala Arg Leu Thr Thr Ala Leu Gln Glu Ser Ala Ala Ser Val
        195                 200                 205
Glu Gln Trp Lys Arg Gln Phe Ser Ile Cys Arg Asp Glu Asn Asp Arg
    210                 215                 220
Leu Arg Asn Lys Ile Asp Glu Leu Glu Glu Gln Cys Ser Glu Ile Asn
225                 230                 235                 240
Arg Glu Lys Glu Lys Asn Thr Gln Leu Lys Arg Arg Ile Glu Glu Leu
                245                 250                 255
Glu Ala Glu Leu Arg Glu Lys Glu Thr Glu Leu Lys Asp Leu Arg Lys
            260                 265                 270
Gln Ser Glu Ile Ile Pro Gln Leu Met Ser Glu Cys Glu Tyr Val Ser
        275                 280                 285
Glu Lys Leu Glu Ala Ala Glu Arg Asp Asn Gln Asn Leu Glu Asp Lys
    290                 295                 300

Val Arg Ser Leu Lys Thr Asp Ile Glu Glu Ser Lys Tyr Arg Gln Arg
305                 310                 315                 320
His Leu Lys Val Glu Leu Lys Ser Phe Leu Glu Val Leu Asp Gly Lys
                325                 330                 335
Ile Asp Asp Leu His Asp Phe Arg Arg Gly Leu Ser Lys Leu Gly Thr
            340                 345                 350
Asp Asn

<210> 9
<211> 1429
<212> DNA
<213> Homo sapiens

<220>
<221> CDS
<222> (91)...(1165)
<223> Homer 3

<221> misc_feature
<222> (1)...(1429)
<223> n = A,T,C or G

<400> 9
   gcacgagggc gcatgactag ttggggccaa accagtgctc ctgccacctc tctggctgcc      60
   ccctagagcc tgcccatccc agcctgacca atg tcc aca gcc agg gag cag cca      114
                                    Met Ser Thr Ala Arg Glu Gln Pro
                                     1               5
   atc ttc agc aca cgg gcg cac gtg ttc caa att gac cca gcc acc aag      162
   Ile Phe Ser Thr Arg Ala His Val Phe Gln Ile Asp Pro Ala Thr Lys
       10                  15                  20
   cga aac tgg atc cca gcg ggc aag cac gca ctc act gtc tcc tat ttc      210
   Arg Asn Trp Ile Pro Ala Gly Lys His Ala Leu Thr Val Ser Tyr Phe
    25                  30                  35                  40
   tac gat gcc acc cgc aat gtg tac cgc atc atc agc atc gga ggc gcc      258
   Tyr Asp Ala Thr Arg Asn Val Tyr Arg Ile Ile Ser Ile Gly Gly Ala
                   45                  50                  55
   aag gcc atc atc aac agc act gtc act ccc aac atg acc ttc acc aaa      306
   Lys Ala Ile Ile Asn Ser Thr Val Thr Pro Asn Met Thr Phe Thr Lys
               60                  65                  70
   act tcc cag aag ttc ggg cag tgg gcc gac agt cgc gcc aac aca gtc      354
   Thr Ser Gln Lys Phe Gly Gln Trp Ala Asp Ser Arg Ala Asn Thr Val
           75                  80                  85
   tac ggc ctg ggc ttt gcc tct gaa cag cat ctg aca cag ttt gcc gag      402
   Tyr Gly Leu Gly Phe Ala Ser Glu Gln His Leu Thr Gln Phe Ala Glu
       90                  95                  100
   aag ttc cag gaa gtg aag gaa gca gcc agg ctg gcc agg gag aaa tct      450
   Lys Phe Gln Glu Val Lys Glu Ala Ala Arg Leu Ala Arg Glu Lys Ser
   105                 110                 115                 120
   cag gat ggc ggg gag ctc acc agt cca gcc ctg ggg ctc gcc tcc cac      498
   Gln Asp Gly Gly Glu Leu Thr Ser Pro Ala Leu Gly Leu Ala Ser His
                   125                 130                 135
   cag gtc ccc ccg agc cct ctc gtc agt gcc aac ggc ccc ggc gag gaa      546
   Gln Val Pro Pro Ser Pro Leu Val Ser Ala Asn Gly Pro Gly Glu Glu
   140 145 150
   aaa ctg ttc cgc agc cag agc gct gat gcc ccc ggc ccc aca gag cgc 594
   Lys Leu Phe Arg Ser Gln Ser Ala Asp Ala Pro Gly Pro Thr Glu Arg
   155 160 165
   gag cgg cta aag aag atg ttg tct gag ggc tcc gtg ggc gag gta cag 642
   Glu Arg Leu Lys Lys Met Leu Ser Glu Gly Ser Val Gly Glu Val Gln
   170 175 180
   tgg gag gcc gag ttt ttc gca ctg cag gac agc aac aac aag ctg gca 690
   Trp Glu Ala Glu Phe Phe Ala Leu Gln Asp Ser Asn Asn Lys Leu Ala
   185 190 195 200
   ggc gcc ctg cga gag gcc aac gcc gcc gca gcc cag tgg agg cag cag 738
   Gly Ala Leu Arg Glu Ala Asn Ala Ala Ala Ala Gln Trp Arg Gln Gln
   205 210 215
   ctg gag gct cag cgt gca gag gcc gag cgg ctg cgg cag cgg gtg gct 786
   Leu Glu Ala Gln Arg Ala Glu Ala Glu Arg Leu Arg Gln Arg Val Ala
   220 225 230
   gag ctg gag gct cag gca gct tca gag gtg acc ccc acc ggt gag aag 834
   Glu Leu Glu Ala Gln Ala Ala Ser Glu Val Thr Pro Thr Gly Glu Lys
   235 240 245
   gag ggg ctg ggc cag ggc cag tcg ctg gaa cag ctg gaa gct ctg gtg 882
   Glu Gly Leu Gly Gln Gly Gln Ser Leu Glu Gln Leu Glu Ala Leu Val
   250 255 260
   caa acc aag gac cag gag att cag acc ctg aag agt cag act ggg ggg 930
   Gln Thr Lys Asp Gln Glu Ile Gln Thr Leu Lys Ser Gln Thr Gly Gly
   265 270 275 280
   ccc cgc gag gcc ctg gag gct gcc gag cgt gag gag act cag cag aag 978
   Pro Arg Glu Ala Leu Glu Ala Ala Glu Arg Glu Glu Thr Gln Gln Lys
   285 290 295
   gtg cag acc cgc aat gcg gag ttg gag cac cag ctg cgg gcg atg gag 1026
   Val Gln Thr Arg Asn Ala Glu Leu Glu His Gln Leu Arg Ala Met Glu
   300 305 310
   cgc agc ctg gag gag gca cgg gca gag cgg gag cgg gcg cgg gct gag 1074
   Arg Ser Leu Glu Glu Ala Arg Ala Glu Arg Glu Arg Ala Arg Ala Glu
   315 320 325
   gtg ggc cgg gca gcg cag ctg ctg gac gtc agc ctg ttt gag ctg agt 1122
   Val Gly Arg Ala Ala Gln Leu Leu Asp Val Ser Leu Phe Glu Leu Ser
   330 335 340
   gag ctg cgt gag ggc ctg gcc cgc ctg gct gag gct gcg ccc t 1165
   Glu Leu Arg Glu Gly Leu Ala Arg Leu Ala Glu Ala Ala Pro
   345 350 355
   gagccggggc tggttttcta tgaacgattc cggcctggga tgcgggccag gctgcaggcg 1225
   gcatagttgg gcccattcgt cctggaaagg gactgggggg tcccaactta gccctgggtg 1285
   ggccgggccg ggntgggctg gggtgggccc cagtcggctc tggttgttgg cagctttggg 1345
   gctgtttttg agcttctcat tgtgtagaat ttctagatcc cccgattaca tttctaagcg 1405
   tgaaaaaaaa aaaaaaaaaa aaaa 1429

<210> 10
<211> 358
<212> PRT

<213> Homo sapiens

<400> 10
Met Ser Thr Ala Arg Glu Gln Pro Ile Phe Ser Thr Arg Ala His Val
1               5                   10                  15
Phe Gln Ile Asp Pro Ala Thr Lys Arg Asn Trp Ile Pro Ala Gly Lys
            20                  25                  30
His Ala Leu Thr Val Ser Tyr Phe Tyr Asp Ala Thr Arg Asn Val Tyr
        35                  40                  45
Arg Ile Ile Ser Ile Gly Gly Ala Lys Ala Ile Ile Asn Ser Thr Val
    50                  55                  60
Thr Pro Asn Met Thr Phe Thr Lys Thr Ser Gln Lys Phe Gly Gln Trp
65                  70                  75                  80
Ala Asp Ser Arg Ala Asn Thr Val Tyr Gly Leu Gly Phe Ala Ser Glu
                85                  90                  95
Gln His Leu Thr Gln Phe Ala Glu Lys Phe Gln Glu Val Lys Glu Ala
            100                 105                 110
Ala Arg Leu Ala Arg Glu Lys Ser Gln Asp Gly Gly Glu Leu Thr Ser
        115                 120                 125
Pro Ala Leu Gly Leu Ala Ser His Gln Val Pro Pro Ser Pro Leu Val
    130                 135                 140
Ser Ala Asn Gly Pro Gly Glu Glu Lys Leu Phe Arg Ser Gln Ser Ala
145                 150                 155                 160
Asp Ala Pro Gly Pro Thr Glu Arg Glu Arg Leu Lys Lys Met Leu Ser
                165                 170                 175
Glu Gly Ser Val Gly Glu Val Gln Trp Glu Ala Glu Phe Phe Ala Leu
            180                 185                 190
Gln Asp Ser Asn Asn Lys Leu Ala Gly Ala Leu Arg Glu Ala Asn Ala
        195                 200                 205
Ala Ala Ala Gln Trp Arg Gln Gln Leu Glu Ala Gln Arg Ala Glu Ala
    210                 215                 220
Glu Arg Leu Arg Gln Arg Val Ala Glu Leu Glu Ala Gln Ala Ala Ser
225                 230                 235                 240
Glu Val Thr Pro Thr Gly Glu Lys Glu Gly Leu Gly Gln Gly Gln Ser
                245                 250                 255
Leu Glu Gln Leu Glu Ala Leu Val Gln Thr Lys Asp Gln Glu Ile Gln
            260                 265                 270
Thr Leu Lys Ser Gln Thr Gly Gly Pro Arg Glu Ala Leu Glu Ala Ala
        275                 280                 285
Glu Arg Glu Glu Thr Gln Gln Lys Val Gln Thr Arg Asn Ala Glu Leu
    290                 295                 300
Glu His Gln Leu Arg Ala Met Glu Arg Ser Leu Glu Glu Ala Arg Ala
305                 310                 315                 320
Glu Arg Glu Arg Ala Arg Ala Glu Val Gly Arg Ala Ala Gln Leu Leu
                325                 330                 335
Asp Val Ser Leu Phe Glu Leu Ser Glu Leu Arg Glu Gly Leu Ala Arg
            340                 345                 350
Leu Ala Glu Ala Ala Pro
        355

<210> 11
<211> 6
<212> PRT
<213> Artificial Sequence

<220>
<223> core region for peptide binding

<221> VARIANT
<222> (1)...(6)
<223> Xaa = Any Amino Acid

<400> 11

Pro Pro Xaa Xaa Phe Arg
1               5

<210> 12
<211> 10
<212> PRT
<213> Artificial Sequence

<220>
<223> extended region for peptide binding

<400> 12
Ala Leu Thr Pro Pro Ser Pro Phe Arg Asp
1               5                   10

<210> 13
<211> 1415
<212> DNA
<213> Rattus norvegicus

<220>
<221> CDS
<222> (1)...(1110)
<223> I30

<400> 13
cac gcg tcc gtg gcg gag ctg cag cag ctg cag cag ttg cag gag ttc      48
His Ala Ser Val Ala Glu Leu Gln Gln Leu Gln Gln Leu Gln Glu Phe
1               5                   10                  15

gat atc ccc acg ggc cgg gag gct ctg cgg ggc aac cac agc gcc ctg      96
Asp Ile Pro Thr Gly Arg Glu Ala Leu Arg Gly Asn His Ser Ala Leu
            20                  25                  30

cta cgg gtg gcc aac tac tgt gag gat aac tac ttg cag gcc aca gac      144
Leu Arg Val Ala Asn Tyr Cys Glu Asp Asn Tyr Leu Gln Ala Thr Asp
        35                  40                  45

aag cgg aag gcg ctg gaa gag acg atg gct ttc acc acc cag gcc ctg      192
Lys Arg Lys Ala Leu Glu Glu Thr Met Ala Phe Thr Thr Gln Ala Leu
    50                  55                  60

gcc agt gta gcc tat caa gtg ggt aac ctg gcg ggg cac acg ctt cga      240
Ala Ser Val Ala Tyr Gln Val Gly Asn Leu Ala Gly His Thr Leu Arg
65                  70                  75                  80

atg ctg gat cta cag ggt gct gcc ctg cgg cag gtg gaa gcc aag atg      288
Met Leu Asp Leu Gln Gly Ala Ala Leu Arg Gln Val Glu Ala Lys Met
                85                  90                  95

agc aca ctg ggc cag atg gtg aac atg cac ctg gag aaa gta gcc aga      336
Ser Thr Leu Gly Gln Met Val Asn Met His Leu Glu Lys Val Ala Arg
            100                 105                 110

agg gag att ggc acg ttg gcc act gtc gtg cgg ctg ccc cct agc cag      384
Arg Glu Ile Gly Thr Leu Ala Thr Val Val Arg Leu Pro Pro Ser Gln
        115                 120                 125

aag gtc atc cct cct gag agc ctg cct ccc ctc act ccc tac tgc aga      432
Lys Val Ile Pro Pro Glu Ser Leu Pro Pro Leu Thr Pro Tyr Cys Arg
    130                 135                 140

aaa ccc ctc aac ttt gcc tgc ttg gat gat gtt ggc cat gga gtc aag 480
Lys Pro Leu Asn Phe Ala Cys Leu Asp Asp Val Gly His Gly Val Lys
145                 150                 155                 160

gac ttg agc aca cag ctg tca cgg acc ggg acc ctg tct cgc aag agc 528
Asp Leu Ser Thr Gln Leu Ser Arg Thr Gly Thr Leu Ser Arg Lys Ser
                165                 170                 175

ata aag gcg ccc gct aca cct gcc tct gcc acg ctg ggg aga cca ccc 576
Ile Lys Ala Pro Ala Thr Pro Ala Ser Ala Thr Leu Gly Arg Pro Pro
            180                 185                 190

cgg atc cct gag ccg gtg cag ctc cca gcg gtg cca gac ggc aag ctc 624
Arg Ile Pro Glu Pro Val Gln Leu Pro Ala Val Pro Asp Gly Lys Leu
        195                 200                 205

tcc gct gcc tcc tct gtg tct tcc ttg gcc tcc gca ggc agt gca gaa 672
Ser Ala Ala Ser Ser Val Ser Ser Leu Ala Ser Ala Gly Ser Ala Glu
    210                 215                 220

ggt gcc agt ggg atc ccc cag tcc aag gga cag gta gca cct gca acc 720
Gly Ala Ser Gly Ile Pro Gln Ser Lys Gly Gln Val Ala Pro Ala Thr
225                 230                 235                 240

ccg cct cct cca cct ata gcg cct gta act cca cct cct cca cca ttg 768
Pro Pro Pro Pro Pro Ile Ala Pro Val Thr Pro Pro Pro Pro Pro Leu
                245                 250                 255

cct gct gag atc ttc ttg ctg ccc cct ccg atg gag gag tcc cag ccc 816
Pro Ala Glu Ile Phe Leu Leu Pro Pro Pro Met Glu Glu Ser Gln Pro
            260                 265                 270

cct ccg gaa aca gag ttg ccc ctg cct cct cct ccg gct cta cag ggg 864
Pro Pro Glu Thr Glu Leu Pro Leu Pro Pro Pro Pro Ala Leu Gln Gly
        275                 280                 285

gat gaa ctg ggg ctg ctg cct ccg cct cca cca ggt ttt gga ccg gat 912
Asp Glu Leu Gly Leu Leu Pro Pro Pro Pro Pro Gly Phe Gly Pro Asp
    290                 295                 300

gag ccc agc tgg gtc cct gct gcc tac ttg gag aaa gtg gtg acg ctg 960
Glu Pro Ser Trp Val Pro Ala Ala Tyr Leu Glu Lys Val Val Thr Leu
305                 310                 315                 320

tac cca tac acc cgg cag aag gac aat gag ctc tcc ttt tct gaa gga 1008
Tyr Pro Tyr Thr Arg Gln Lys Asp Asn Glu Leu Ser Phe Ser Glu Gly
                325                 330                 335

acc gtc atc tgt gtc act cga cgc tac tca gat ggc tgg tgt gag ggt 1056
Thr Val Ile Cys Val Thr Arg Arg Tyr Ser Asp Gly Trp Cys Glu Gly
            340                 345                 350

gtc agc tca gag ggc act gga ttc ttc cca ggg aac tat gtg gag ccc 1104
Val Ser Ser Glu Gly Thr Gly Phe Phe Pro Gly Asn Tyr Val Glu Pro
        355                 360                 365

agc tgc tgacagccca gatctgtccc tgcctctttg gtgggcctct tgagccccaa 1160
Ser Cys
    370

gaagccacct tccactcaaa gctggactaa ggacctgtct acctcttggg ctgtgaactg 1220
tgttcagtcc cacacagcag taggaagggg tatgggatgg gctagagagt ggtggtactg 1280

aggacgattg ctccagatgg caagaacaaa acaaaacaaa ccaagaagtt aagtttaagc 1340
accttgccca gaggaccccc tagctcatgc accgatcgcc agcattgaat aaaactgttg 1400
acctccagga ttgtt 1415

<210> 14
<211> 370
<212> PRT
<213> Rattus norvegicus

<400> 14
His Ala Ser Val Ala Glu Leu Gln Gln Leu Gln Gln Leu Gln Glu Phe
1               5                   10                  15
Asp Ile Pro Thr Gly Arg Glu Ala Leu Arg Gly Asn His Ser Ala Leu
            20                  25                  30
Leu Arg Val Ala Asn Tyr Cys Glu Asp Asn Tyr Leu Gln Ala Thr Asp
        35                  40                  45
Lys Arg Lys Ala Leu Glu Glu Thr Met Ala Phe Thr Thr Gln Ala Leu
    50                  55                  60
Ala Ser Val Ala Tyr Gln Val Gly Asn Leu Ala Gly His Thr Leu Arg
65                  70                  75                  80
Met Leu Asp Leu Gln Gly Ala Ala Leu Arg Gln Val Glu Ala Lys Met
                85                  90                  95
Ser Thr Leu Gly Gln Met Val Asn Met His Leu Glu Lys Val Ala Arg
            100                 105                 110
Arg Glu Ile Gly Thr Leu Ala Thr Val Val Arg Leu Pro Pro Ser Gln
        115                 120                 125
Lys Val Ile Pro Pro Glu Ser Leu Pro Pro Leu Thr Pro Tyr Cys Arg
    130                 135                 140
Lys Pro Leu Asn Phe Ala Cys Leu Asp Asp Val Gly His Gly Val Lys
145                 150                 155                 160
Asp Leu Ser Thr Gln Leu Ser Arg Thr Gly Thr Leu Ser Arg Lys Ser
                165                 170                 175
Ile Lys Ala Pro Ala Thr Pro Ala Ser Ala Thr Leu Gly Arg Pro Pro
            180                 185                 190
Arg Ile Pro Glu Pro Val Gln Leu Pro Ala Val Pro Asp Gly Lys Leu
        195                 200                 205
Ser Ala Ala Ser Ser Val Ser Ser Leu Ala Ser Ala Gly Ser Ala Glu
    210                 215                 220
Gly Ala Ser Gly Ile Pro Gln Ser Lys Gly Gln Val Ala Pro Ala Thr
225                 230                 235                 240
Pro Pro Pro Pro Pro Ile Ala Pro Val Thr Pro Pro Pro Pro Pro Leu
                245                 250                 255
Pro Ala Glu Ile Phe Leu Leu Pro Pro Pro Met Glu Glu Ser Gln Pro
            260                 265                 270
Pro Pro Glu Thr Glu Leu Pro Leu Pro Pro Pro Pro Ala Leu Gln Gly
        275                 280                 285
Asp Glu Leu Gly Leu Leu Pro Pro Pro Pro Pro Gly Phe Gly Pro Asp
    290                 295                 300
Glu Pro Ser Trp Val Pro Ala Ala Tyr Leu Glu Lys Val Val Thr Leu
305                 310                 315                 320
Tyr Pro Tyr Thr Arg Gln Lys Asp Asn Glu Leu Ser Phe Ser Glu Gly
                325                 330                 335
Thr Val Ile Cys Val Thr Arg Arg Tyr Ser Asp Gly Trp Cys Glu Gly
            340                 345                 350
Val Ser Ser Glu Gly Thr Gly Phe Phe Pro Gly Asn Tyr Val Glu Pro
        355                 360                 365
Ser Cys
    370

<210> 15
<211> 3843
<212> DNA
<213> Rattus norvegicus

<220>
<221> CDS
<222> (1)...(3840)
<223> I42

<400> 15
atg atg aca aac cga gat gga cgt gac tac ttc atc aat cac atg aca      48
Met Met Thr Asn Arg Asp Gly Arg Asp Tyr Phe Ile Asn His Met Thr
 1               5                  10                  15

cag gca atc cca ttt gat gac cct cgg ttt gac agc tgc caa atc att      96
Gln Ala Ile Pro Phe Asp Asp Pro Arg Phe Asp Ser Cys Gln Ile Ile
            20                  25                  30

ccc cca gct cca cgg aag gtg gag atg agg agg gac cct gtg ctg ggc     144
Pro Pro Ala Pro Arg Lys Val Glu Met Arg Arg Asp Pro Val Leu Gly
        35                  40                  45

ttt ggg ttc gtg gca ggg agt gaa aag cca gtg gtc gtt cga tcg gta     192
Phe Gly Phe Val Ala Gly Ser Glu Lys Pro Val Val Val Arg Ser Val
    50                  55                  60

aca cca ggt ggc cct tca gaa ggc aag ctg atc ccg gga gat caa att     240
Thr Pro Gly Gly Pro Ser Glu Gly Lys Leu Ile Pro Gly Asp Gln Ile
65                  70                  75                  80

gta atg att aat gat gaa cca gtc agc gct gcg cca aga gag agg gtc     288
Val Met Ile Asn Asp Glu Pro Val Ser Ala Ala Pro Arg Glu Arg Val
                85                  90                  95

atc gac ctg gtc agg agc tgc aaa gaa tcg att ctg ttc act gtc atc     336
Ile Asp Leu Val Arg Ser Cys Lys Glu Ser Ile Leu Phe Thr Val Ile
            100                 105                 110

cag cct tat cct tct ccc aaa tca gca ttt att agt gct gct aaa aag     384
Gln Pro Tyr Pro Ser Pro Lys Ser Ala Phe Ile Ser Ala Ala Lys Lys
        115                 120                 125

gca aga ttg aag tcc aat cca gtc aaa gta cgc ttt tcc gaa gag gtc     432
Ala Arg Leu Lys Ser Asn Pro Val Lys Val Arg Phe Ser Glu Glu Val
    130                 135                 140

atc atc aat ggt cag gtg tcg gaa act gtt aaa gac aat tca ctt ctt     480
Ile Ile Asn Gly Gln Val Ser Glu Thr Val Lys Asp Asn Ser Leu Leu
145                 150                 155                 160

ttt atg cca aat gtt ttg aaa gtc tac ttg gaa aat gga cag acc aaa     528
Phe Met Pro Asn Val Leu Lys Val Tyr Leu Glu Asn Gly Gln Thr Lys
                165                 170                 175

tcc ttt cgc ttt gac tgc agc act tcc att aag gat gtc atc tta act     576
Ser Phe Arg Phe Asp Cys Ser Thr Ser Ile Lys Asp Val Ile Leu Thr
            180                 185                 190

ctg caa gag aag ctg tct atc aaa ggc att gag cac ttc tct ctc atg     624
Leu Gln Glu Lys Leu Ser Ile Lys Gly Ile Glu His Phe Ser Leu Met
        195                 200                 205

ctg gag cag aga act gaa ggg gcc ggc acc aag ctg ctc tta ctt cat     672
Leu Glu Gln Arg Thr Glu Gly Ala Gly Thr Lys Leu Leu Leu Leu His
    210                 215                 220

gaa cag gag aca ctc act cag gtg aca cag agg ccg agt tcc cat aag 720
Glu Gln Glu Thr Leu Thr Gln Val Thr Gln Arg Pro Ser Ser His Lys
225                 230                 235                 240

atg agg tgt ctt ttc cga atc agt ttt gtt ccc aag gat ccc att gac 768
Met Arg Cys Leu Phe Arg Ile Ser Phe Val Pro Lys Asp Pro Ile Asp
                245                 250                 255

ctg tta agg aga gat cca gtt gct ttc gag tat ctc tat gtt cag agc 816
Leu Leu Arg Arg Asp Pro Val Ala Phe Glu Tyr Leu Tyr Val Gln Ser
            260                 265                 270

tgt aac gat gtc gtt cag gag cga ttt gga cca gag ctg aaa tac gac 864
Cys Asn Asp Val Val Gln Glu Arg Phe Gly Pro Glu Leu Lys Tyr Asp
        275                 280                 285

att gcc ttg cgg ctg gcc gct tta caa atg tac att gct act gtc acc 912
Ile Ala Leu Arg Leu Ala Ala Leu Gln Met Tyr Ile Ala Thr Val Thr
    290                 295                 300

acc aaa cag acg cag aaa atc tcc ctc aag tac att gag aaa gaa tgg 960
Thr Lys Gln Thr Gln Lys Ile Ser Leu Lys Tyr Ile Glu Lys Glu Trp
305                 310                 315                 320

gga cta gag act ttc ctt cca tct gct gta ctt cag agc atg aaa gag 1008
Gly Leu Glu Thr Phe Leu Pro Ser Ala Val Leu Gln Ser Met Lys Glu
                325                 330                 335

aag aac atc aag aaa gcg ctc tcc cac ctt gtc aaa gca aat caa aac 1056
Lys Asn Ile Lys Lys Ala Leu Ser His Leu Val Lys Ala Asn Gln Asn
            340                 345                 350

ttg gta cca ccg ggt aaa aag ctc tct gca cta caa gct aag gtc cac 1104
Leu Val Pro Pro Gly Lys Lys Leu Ser Ala Leu Gln Ala Lys Val His
        355                 360                 365

tat ctc aag ttc ctc agt gac ctg cga cta tac ggg ggc cgt gtg ttc 1152
Tyr Leu Lys Phe Leu Ser Asp Leu Arg Leu Tyr Gly Gly Arg Val Phe
    370                 375                 380

aag gca aca tta gtg cag gca gag aag cgc tca gaa gtg act ctt ctg 1200
Lys Ala Thr Leu Val Gln Ala Glu Lys Arg Ser Glu Val Thr Leu Leu
385                 390                 395                 400

gtg ggt ccc cgg tat ggc ata agc cat gtc ata aac acc aaa acc aac 1248
Val Gly Pro Arg Tyr Gly Ile Ser His Val Ile Asn Thr Lys Thr Asn
                405                 410                 415

ctg gtg gct ctt tta gct gac ttc agc cat gtc aac agg att gaa atg 1296
Leu Val Ala Leu Leu Ala Asp Phe Ser His Val Asn Arg Ile Glu Met
            420                 425                 430

ttt act gaa gag gag agt ttg gtg agg gtg gag ttg cat gtg ctc gat 1344
Phe Thr Glu Glu Glu Ser Leu Val Arg Val Glu Leu His Val Leu Asp
        435                 440                 445

gtg aag ccc att aca ctc ctt atg gag tca tca gat gcc atg aac ctg 1392
Val Lys Pro Ile Thr Leu Leu Met Glu Ser Ser Asp Ala Met Asn Leu
    450                 455                 460

gcc tgt ctg aca gct gga tac tac cgg ttg ctc gtg gac tcc agg agg 1440
Ala Cys Leu Thr Ala Gly Tyr Tyr Arg Leu Leu Val Asp Ser Arg Arg
465                 470                 475                 480

tca ata ttt aac atg gcc aac aag aaa aat gca ggc aca cag gac aca 1488
Ser Ile Phe Asn Met Ala Asn Lys Lys Asn Ala Gly Thr Gln Asp Thr
                485                 490                 495

gga acg gaa aat aaa ggc aag cat aat ctc ctt ggt cct gac tgg aac 1536
Gly Thr Glu Asn Lys Gly Lys His Asn Leu Leu Gly Pro Asp Trp Asn
            500                 505                 510

tgt atg ccc cag atg acg acc ttc att ggc gaa ggg gaa caa gaa gcc 1584
Cys Met Pro Gln Met Thr Thr Phe Ile Gly Glu Gly Glu Gln Glu Ala
        515                 520                 525

caa atc act tat ata gat tct aag cag aag gca gtt gag atg aca gac 1632
Gln Ile Thr Tyr Ile Asp Ser Lys Gln Lys Ala Val Glu Met Thr Asp
    530                 535                 540

agc acc ttg tgt ccc aaa gag cac cgg cac tta tat atc gac aac aca 1680
Ser Thr Leu Cys Pro Lys Glu His Arg His Leu Tyr Ile Asp Asn Thr
545                 550                 555                 560

tac agt tca gat gaa ctt agc cag ccg ctg act cag cca ggt gat gca 1728
Tyr Ser Ser Asp Glu Leu Ser Gln Pro Leu Thr Gln Pro Gly Asp Ala
                565                 570                 575

ccc tgt gag gcc gac tat aga agc cta gct cag cgg tcc ctt ttg acc 1776
Pro Cys Glu Ala Asp Tyr Arg Ser Leu Ala Gln Arg Ser Leu Leu Thr
            580                 585                 590

ctc tca gga cca gac act ctg aag aaa gca cag gaa tct ccg cga gga 1824
Leu Ser Gly Pro Asp Thr Leu Lys Lys Ala Gln Glu Ser Pro Arg Gly
        595                 600                 605

gct aaa gtg tcc ttt att ttt gga gat ctt gcc tta gat gat ggc atg 1872
Ala Lys Val Ser Phe Ile Phe Gly Asp Leu Ala Leu Asp Asp Gly Met
    610                 615                 620

agt ccc cca act cta ggc tat gaa aga atg tta gat gag aat cca gaa 1920
Ser Pro Pro Thr Leu Gly Tyr Glu Arg Met Leu Asp Glu Asn Pro Glu
625                 630                 635                 640

atg ctg gag aag cag agg aat ctc tac atc agc agt gcc aat gat atg 1968
Met Leu Glu Lys Gln Arg Asn Leu Tyr Ile Ser Ser Ala Asn Asp Met
                645                 650                 655

aaa aac ctg gac ctc act cca gac aca gac agc atc cag ttt gtg gca 2016
Lys Asn Leu Asp Leu Thr Pro Asp Thr Asp Ser Ile Gln Phe Val Ala
            660                 665                 670

aat tca gta tat gca aac ata ggt gat gtg aag aac ttt gaa gcc cct 2064
Asn Ser Val Tyr Ala Asn Ile Gly Asp Val Lys Asn Phe Glu Ala Pro
        675                 680                 685

gag gga ata gag gag ccc ctc tta cat gac atc tgt tat gct gaa aac 2112
Glu Gly Ile Glu Glu Pro Leu Leu His Asp Ile Cys Tyr Ala Glu Asn
    690                 695                 700

aca gat gat gca gaa gat gaa gat gag gtg agc tgc gag gag gat ctc 2160
Thr Asp Asp Ala Glu Asp Glu Asp Glu Val Ser Cys Glu Glu Asp Leu
705                 710                 715                 720

gtg gtg agt gaa atc aac caa cca gcc atc ctt gac ctg tct ggg tca 2208
Val Val Ser Glu Ile Asn Gln Pro Ala Ile Leu Asp Leu Ser Gly Ser

                725                 730                 735

agt gat gat att att gac ctt aca aca ctg cct cct cca gaa gga gat 2256
Ser Asp Asp Ile Ile Asp Leu Thr Thr Leu Pro Pro Pro Glu Gly Asp
            740                 745                 750

gac aat gag gat gac ttc ctc ctg cgt tct ctg aac atg gcc att gct 2304
Asp Asn Glu Asp Asp Phe Leu Leu Arg Ser Leu Asn Met Ala Ile Ala
        755                 760                 765

gct ccc cca cct ggt ttt aga gac agt tct gat gaa gag gac act cag 2352
Ala Pro Pro Pro Gly Phe Arg Asp Ser Ser Asp Glu Glu Asp Thr Gln
    770                 775                 780

agc cag gca aca tcc ttc cat gag aac aaa gaa caa ggc agc agc ctg 2400
Ser Gln Ala Thr Ser Phe His Glu Asn Lys Glu Gln Gly Ser Ser Leu
785                 790                 795                 800

cag aat gag gag atc cct gtg tcc ctc att gat gct gtg ccc acc agt 2448
Gln Asn Glu Glu Ile Pro Val Ser Leu Ile Asp Ala Val Pro Thr Ser
                805                 810                 815

gca gag ggc aag tgt gag aag gga ctg gac cct acc gtc gtt tcc aca 2496
Ala Glu Gly Lys Cys Glu Lys Gly Leu Asp Pro Thr Val Val Ser Thr
            820                 825                 830

cta gaa gcc cta gaa gct ctt tca gaa gaa cag cag aag agt gaa aat 2544
Leu Glu Ala Leu Glu Ala Leu Ser Glu Glu Gln Gln Lys Ser Glu Asn
        835                 840                 845

tca ggt gta gcc atc ttg cgg gct tat agt ccc gag tct tcc tca gac 2592
Ser Gly Val Ala Ile Leu Arg Ala Tyr Ser Pro Glu Ser Ser Ser Asp
    850                 855                 860

tcg ggc aat gag act aac tct tct gaa atg aca gag ggt tct gaa cta 2640
Ser Gly Asn Glu Thr Asn Ser Ser Glu Met Thr Glu Gly Ser Glu Leu
865                 870                 875                 880

gct gca gca cag aag cag tcg gaa agc ctc tcc cgc atg ttc ttg gcc 2688
Ala Ala Ala Gln Lys Gln Ser Glu Ser Leu Ser Arg Met Phe Leu Ala
                885                 890                 895

act cat gaa ggt tat cac cct ctg gca gaa gaa cag aca gag ttc ccc 2736
Thr His Glu Gly Tyr His Pro Leu Ala Glu Glu Gln Thr Glu Phe Pro
            900                 905                 910

acc tcc aaa acc ccc tct gtg ggc ttg cct cca aag tcc tct cat ggc 2784
Thr Ser Lys Thr Pro Ser Val Gly Leu Pro Pro Lys Ser Ser His Gly
        915                 920                 925

ctg gct gct cgc cca gcg acc gac ctc cca ccc aaa gtt gtg cct tcc 2832
Leu Ala Ala Arg Pro Ala Thr Asp Leu Pro Pro Lys Val Val Pro Ser
    930                 935                 940

aag cag atc ctt cac tca gat cac atg gaa atg gag cca gaa acc atg 2880
Lys Gln Ile Leu His Ser Asp His Met Glu Met Glu Pro Glu Thr Met
945                 950                 955                 960

gag acc aag tca gtc act gac tat ttt agc aaa ctg cac atg ggg tca 2928
Glu Thr Lys Ser Val Thr Asp Tyr Phe Ser Lys Leu His Met Gly Ser
                965                 970                 975

gtg gca tat tcc tgt acc agc aaa agg aaa agc aag ctt gct gag gga 2976

Val Ala Tyr Ser Cys Thr Ser Lys Arg Lys Ser Lys Leu Ala Glu Gly
            980                 985                 990

gag ggg aaa tgc ccc ctg agt ggg aat gta cca ggg aaa aaa cag caa      3024
Glu Gly Lys Cys Pro Leu Ser Gly Asn Val Pro Gly Lys Lys Gln Gln
        995                 1000                1005

gga acc aaa ata gca gag acg gag gag gac acc aaa ggc aaa gtt ggc      3072
Gly Thr Lys Ile Ala Glu Thr Glu Glu Asp Thr Lys Gly Lys Val Gly
    1010                1015                1020

act gta tct tca aga gac aat cca cac ctc agc act ttt aac ctg gag      3120
Thr Val Ser Ser Arg Asp Asn Pro His Leu Ser Thr Phe Asn Leu Glu
1025                1030                1035                1040

aga act gcc ttt cgc aag gac agc caa aga tgg tat gtg gcc tct gat      3168
Arg Thr Ala Phe Arg Lys Asp Ser Gln Arg Trp Tyr Val Ala Ser Asp
                1045                1050                1055

ggt ggg gtg gta gag aaa agt gga gtg gaa gca cca gcc atg aaa gcc      3216
Gly Gly Val Val Glu Lys Ser Gly Val Glu Ala Pro Ala Met Lys Ala
            1060                1065                1070

ttt ccc aga ggt cct ggt ctg ggg aac aga gag gct gaa ggg aaa gag      3264
Phe Pro Arg Gly Pro Gly Leu Gly Asn Arg Glu Ala Glu Gly Lys Glu
        1075                1080                1085

gat ggc act atg gaa gga gag gct gat gat gct tca gga ctt ggt caa      3312
Asp Gly Thr Met Glu Gly Glu Ala Asp Asp Ala Ser Gly Leu Gly Gln
    1090                1095                1100

ggg gaa cgc ttc ctg tca gat atg gcc tgt gta gcc tca gcc aaa gac      3360
Gly Glu Arg Phe Leu Ser Asp Met Ala Cys Val Ala Ser Ala Lys Asp
1105                1110                1115                1120

tta gac aac cct gaa gac act gac tct ccc act tgt gac cat gcc act      3408
Leu Asp Asn Pro Glu Asp Thr Asp Ser Pro Thr Cys Asp His Ala Thr
                1125                1130                1135

aag ctt cct gag gct gaa gac aat gtg gcc cgc ctt tgt gac tac cat      3456
Lys Leu Pro Glu Ala Glu Asp Asn Val Ala Arg Leu Cys Asp Tyr His
            1140                1145                1150

ttg gcc aag cga atg tca tcc ctg cag agt gag ggc cat ttt tct cta      3504
Leu Ala Lys Arg Met Ser Ser Leu Gln Ser Glu Gly His Phe Ser Leu
        1155                1160                1165

cag agc tct caa ggc tct tca gtg gac aca ggc tgt ggc cca ggc agc      3552
Gln Ser Ser Gln Gly Ser Ser Val Asp Thr Gly Cys Gly Pro Gly Ser
    1170                1175                1180

agt agc agt gcc tgt gcc act cct gtg gaa tcg ccc ctc tgc cca tcc      3600
Ser Ser Ser Ala Cys Ala Thr Pro Val Glu Ser Pro Leu Cys Pro Ser
1185                1190                1195                1200

atg gga aag cac ctg att cca gat gct tct ggg aaa ggt ggg agt tac      3648
Met Gly Lys His Leu Ile Pro Asp Ala Ser Gly Lys Gly Gly Ser Tyr
                1205                1210                1215

att tca cca gag gag aga gtc gct ggt cat ccc aac cat gga gcc acc      3696
Ile Ser Pro Glu Glu Arg Val Ala Gly His Pro Asn His Gly Ala Thr
            1220                1225                1230

ttc aag gaa ctg cac cca cag aca gaa ggg atg tgt cca cgc atg aca     3744
Phe Lys Glu Leu His Pro Gln Thr Glu Gly Met Cys Pro Arg Met Thr
        1235                1240                1245

gtg cct gct ctg cac aca gcc att aat gcc gac ccc ctg ttt ggc act     3792
Val Pro Ala Leu His Thr Ala Ile Asn Ala Asp Pro Leu Phe Gly Thr
    1250                1255                1260

ttg aga gat gga tgc cat cga ctg ccc aag att aag gaa acc aca gtg     3840
Leu Arg Asp Gly Cys His Arg Leu Pro Lys Ile Lys Glu Thr Thr Val
1265                1270                1275                1280

tag                                                                 3843

<210> 16
<211> 1280
<212> PRT
<213> Rattus norvegicus

<400> 16
Met Met Thr Asn Arg Asp Gly Arg Asp Tyr Phe Ile Asn His Met Thr
 1               5                  10                  15
Gln Ala Ile Pro Phe Asp Asp Pro Arg Phe Asp Ser Cys Gln Ile Ile
            20                  25                  30
Pro Pro Ala Pro Arg Lys Val Glu Met Arg Arg Asp Pro Val Leu Gly
        35                  40                  45
Phe Gly Phe Val Ala Gly Ser Glu Lys Pro Val Val Val Arg Ser Val
    50                  55                  60
Thr Pro Gly Gly Pro Ser Glu Gly Lys Leu Ile Pro Gly Asp Gln Ile
65                  70                  75                  80
Val Met Ile Asn Asp Glu Pro Val Ser Ala Ala Pro Arg Glu Arg Val
                85                  90                  95
Ile Asp Leu Val Arg Ser Cys Lys Glu Ser Ile Leu Phe Thr Val Ile
            100                 105                 110
Gln Pro Tyr Pro Ser Pro Lys Ser Ala Phe Ile Ser Ala Ala Lys Lys
        115                 120                 125
Ala Arg Leu Lys Ser Asn Pro Val Lys Val Arg Phe Ser Glu Glu Val
    130                 135                 140
Ile Ile Asn Gly Gln Val Ser Glu Thr Val Lys Asp Asn Ser Leu Leu
145                 150                 155                 160
Phe Met Pro Asn Val Leu Lys Val Tyr Leu Glu Asn Gly Gln Thr Lys
                165                 170                 175
Ser Phe Arg Phe Asp Cys Ser Thr Ser Ile Lys Asp Val Ile Leu Thr
            180                 185                 190
Leu Gln Glu Lys Leu Ser Ile Lys Gly Ile Glu His Phe Ser Leu Met
        195                 200                 205
Leu Glu Gln Arg Thr Glu Gly Ala Gly Thr Lys Leu Leu Leu Leu His
    210                 215                 220
Glu Gln Glu Thr Leu Thr Gln Val Thr Gln Arg Pro Ser Ser His Lys
225                 230                 235                 240
Met Arg Cys Leu Phe Arg Ile Ser Phe Val Pro Lys Asp Pro Ile Asp
                245                 250                 255
Leu Leu Arg Arg Asp Pro Val Ala Phe Glu Tyr Leu Tyr Val Gln Ser
            260                 265                 270
Cys Asn Asp Val Val Gln Glu Arg Phe Gly Pro Glu Leu Lys Tyr Asp
        275                 280                 285
Ile Ala Leu Arg Leu Ala Ala Leu Gln Met Tyr Ile Ala Thr Val Thr
    290                 295                 300
Thr Lys Gln Thr Gln Lys Ile Ser Leu Lys Tyr Ile Glu Lys Glu Trp
305                 310                 315                 320
Gly Leu Glu Thr Phe Leu Pro Ser Ala Val Leu Gln Ser Met Lys Glu
                325                 330                 335
Lys Asn Ile Lys Lys Ala Leu Ser His Leu Val Lys Ala Asn Gln Asn

            340                 345                 350
Leu Val Pro Pro Gly Lys Lys Leu Ser Ala Leu Gln Ala Lys Val His
        355                 360                 365
Tyr Leu Lys Phe Leu Ser Asp Leu Arg Leu Tyr Gly Gly Arg Val Phe
    370                 375                 380
Lys Ala Thr Leu Val Gln Ala Glu Lys Arg Ser Glu Val Thr Leu Leu
385                 390                 395                 400
Val Gly Pro Arg Tyr Gly Ile Ser His Val Ile Asn Thr Lys Thr Asn
                405                 410                 415
Leu Val Ala Leu Leu Ala Asp Phe Ser His Val Asn Arg Ile Glu Met
            420                 425                 430
Phe Thr Glu Glu Glu Ser Leu Val Arg Val Glu Leu His Val Leu Asp
        435                 440                 445
Val Lys Pro Ile Thr Leu Leu Met Glu Ser Ser Asp Ala Met Asn Leu
    450                 455                 460
Ala Cys Leu Thr Ala Gly Tyr Tyr Arg Leu Leu Val Asp Ser Arg Arg
465                 470                 475                 480
Ser Ile Phe Asn Met Ala Asn Lys Lys Asn Ala Gly Thr Gln Asp Thr
                485                 490                 495
Gly Thr Glu Asn Lys Gly Lys His Asn Leu Leu Gly Pro Asp Trp Asn
            500                 505                 510
Cys Met Pro Gln Met Thr Thr Phe Ile Gly Glu Gly Glu Gln Glu Ala
        515                 520                 525
Gln Ile Thr Tyr Ile Asp Ser Lys Gln Lys Ala Val Glu Met Thr Asp
    530                 535                 540
Ser Thr Leu Cys Pro Lys Glu His Arg His Leu Tyr Ile Asp Asn Thr
545                 550                 555                 560
Tyr Ser Ser Asp Glu Leu Ser Gln Pro Leu Thr Gln Pro Gly Asp Ala
                565                 570                 575
Pro Cys Glu Ala Asp Tyr Arg Ser Leu Ala Gln Arg Ser Leu Leu Thr
            580                 585                 590
Leu Ser Gly Pro Asp Thr Leu Lys Lys Ala Gln Glu Ser Pro Arg Gly
        595                 600                 605
Ala Lys Val Ser Phe Ile Phe Gly Asp Leu Ala Leu Asp Asp Gly Met
    610                 615                 620
Ser Pro Pro Thr Leu Gly Tyr Glu Arg Met Leu Asp Glu Asn Pro Glu
625                 630                 635                 640
Met Leu Glu Lys Gln Arg Asn Leu Tyr Ile Ser Ser Ala Asn Asp Met
                645                 650                 655
Lys Asn Leu Asp Leu Thr Pro Asp Thr Asp Ser Ile Gln Phe Val Ala
            660                 665                 670
Asn Ser Val Tyr Ala Asn Ile Gly Asp Val Lys Asn Phe Glu Ala Pro
        675                 680                 685
Glu Gly Ile Glu Glu Pro Leu Leu His Asp Ile Cys Tyr Ala Glu Asn
    690                 695                 700
Thr Asp Asp Ala Glu Asp Glu Asp Glu Val Ser Cys Glu Glu Asp Leu
705                 710                 715                 720
Val Val Ser Glu Ile Asn Gln Pro Ala Ile Leu Asp Leu Ser Gly Ser
                725                 730                 735
Ser Asp Asp Ile Ile Asp Leu Thr Thr Leu Pro Pro Pro Glu Gly Asp
            740                 745                 750
Asp Asn Glu Asp Asp Phe Leu Leu Arg Ser Leu Asn Met Ala Ile Ala
        755                 760                 765
Ala Pro Pro Pro Gly Phe Arg Asp Ser Ser Asp Glu Glu Asp Thr Gln
    770                 775                 780
Ser Gln Ala Thr Ser Phe His Glu Asn Lys Glu Gln Gly Ser Ser Leu
785                 790                 795                 800
Gln Asn Glu Glu Ile Pro Val Ser Leu Ile Asp Ala Val Pro Thr Ser
                805                 810                 815
Ala Glu Gly Lys Cys Glu Lys Gly Leu Asp Pro Thr Val Val Ser Thr
            820                 825                 830
Leu Glu Ala Leu Glu Ala Leu Ser Glu Glu Gln Gln Lys Ser Glu Asn
        835                 840                 845

Ser Gly Val Ala Ile Leu Arg Ala Tyr Ser Pro Glu Ser Ser Ser Asp
    850                 855                 860
Ser Gly Asn Glu Thr Asn Ser Ser Glu Met Thr Glu Gly Ser Glu Leu
865                 870                 875                 880
Ala Ala Ala Gln Lys Gln Ser Glu Ser Leu Ser Arg Met Phe Leu Ala
                885                 890                 895
Thr His Glu Gly Tyr His Pro Leu Ala Glu Glu Gln Thr Glu Phe Pro
            900                 905                 910
Thr Ser Lys Thr Pro Ser Val Gly Leu Pro Pro Lys Ser Ser His Gly
        915                 920                 925
Leu Ala Ala Arg Pro Ala Thr Asp Leu Pro Pro Lys Val Val Pro Ser
    930                 935                 940
Lys Gln Ile Leu His Ser Asp His Met Glu Met Glu Pro Glu Thr Met
945                 950                 955                 960
Glu Thr Lys Ser Val Thr Asp Tyr Phe Ser Lys Leu His Met Gly Ser
                965                 970                 975
Val Ala Tyr Ser Cys Thr Ser Lys Arg Lys Ser Lys Leu Ala Glu Gly
            980                 985                 990
Glu Gly Lys Cys Pro Leu Ser Gly Asn Val Pro Gly Lys Lys Gln Gln
        995                 1000                1005
Gly Thr Lys Ile Ala Glu Thr Glu Glu Asp Thr Lys Gly Lys Val Gly
    1010                1015                1020
Thr Val Ser Ser Arg Asp Asn Pro His Leu Ser Thr Phe Asn Leu Glu
1025                1030                1035                1040
Arg Thr Ala Phe Arg Lys Asp Ser Gln Arg Trp Tyr Val Ala Ser Asp
                1045                1050                1055
Gly Gly Val Val Glu Lys Ser Gly Val Glu Ala Pro Ala Met Lys Ala
            1060                1065                1070
Phe Pro Arg Gly Pro Gly Leu Gly Asn Arg Glu Ala Glu Gly Lys Glu
        1075                1080                1085
Asp Gly Thr Met Glu Gly Glu Ala Asp Asp Ala Ser Gly Leu Gly Gln
    1090                1095                1100
Gly Glu Arg Phe Leu Ser Asp Met Ala Cys Val Ala Ser Ala Lys Asp
1105                1110                1115                1120
Leu Asp Asn Pro Glu Asp Thr Asp Ser Pro Thr Cys Asp His Ala Thr
                1125                1130                1135
Lys Leu Pro Glu Ala Glu Asp Asn Val Ala Arg Leu Cys Asp Tyr His
            1140                1145                1150
Leu Ala Lys Arg Met Ser Ser Leu Gln Ser Glu Gly His Phe Ser Leu
        1155                1160                1165
Gln Ser Ser Gln Gly Ser Ser Val Asp Thr Gly Cys Gly Pro Gly Ser
    1170                1175                1180
Ser Ser Ser Ala Cys Ala Thr Pro Val Glu Ser Pro Leu Cys Pro Ser
1185                1190                1195                1200
Met Gly Lys His Leu Ile Pro Asp Ala Ser Gly Lys Gly Gly Ser Tyr
                1205                1210                1215
Ile Ser Pro Glu Glu Arg Val Ala Gly His Pro Asn His Gly Ala Thr
            1220                1225                1230
Phe Lys Glu Leu His Pro Gln Thr Glu Gly Met Cys Pro Arg Met Thr
        1235                1240                1245
Val Pro Ala Leu His Thr Ala Ile Asn Ala Asp Pro Leu Phe Gly Thr
    1250                1255                1260
Leu Arg Asp Gly Cys His Arg Leu Pro Lys Ile Lys Glu Thr Thr Val
1265                1270                1275                1280

<210> 17
<211> 1583
<212> DNA
<213> Homo sapiens

<220>
<221> CDS
<222> (160)...(1258)

<223> I30

<400> 17
ggcacgagtg agcatgcctg ccctttgcaa gcaggtttgg gtctcacgca gaggaaacca      60
aaagcaataa gagggaggga aggcagagca accaatcaag ggcagggtga gactcaaaac     120
gagcgggctc cctggggagc cagacagagg ctgggggtg atg gcg gag cta cag        174
                                           Met Ala Glu Leu Gln
                                           1               5

cag ctg cag gag ttt gag atc ccc act ggc cgg gag gct ctg agg ggc       222
Gln Leu Gln Glu Phe Glu Ile Pro Thr Gly Arg Glu Ala Leu Arg Gly
                10                  15                  20

aac cac agt gcc ctg ctg cgg gtc gct gac tac tgc gag gac aac tat       270
Asn His Ser Ala Leu Leu Arg Val Ala Asp Tyr Cys Glu Asp Asn Tyr
            25                  30                  35

gtg cag gcc aca gac aag cgg aag gcg ctg gag gag acc atg gcc ttc       318
Val Gln Ala Thr Asp Lys Arg Lys Ala Leu Glu Glu Thr Met Ala Phe
        40                  45                  50

act acc cag gca ctg gcc agc gtg gcc tac cag gtg ggc aac ctg gcc       366
Thr Thr Gln Ala Leu Ala Ser Val Ala Tyr Gln Val Gly Asn Leu Ala
    55                  60                  65

ggg cac act ctg cgc atg ttg gac ctg cag ggg gcc gcc ctg cgg cag       414
Gly His Thr Leu Arg Met Leu Asp Leu Gln Gly Ala Ala Leu Arg Gln
70                  75                  80                  85

gtg gaa gcc cgt gta agc acg ctg ggc cag atg gtg aac atg cat atg       462
Val Glu Ala Arg Val Ser Thr Leu Gly Gln Met Val Asn Met His Met
                90                  95                  100

gag aag gtg gcc cga agg gag atc ggc acc tta gcc act gtc caa cgg       510
Glu Lys Val Ala Arg Arg Glu Ile Gly Thr Leu Ala Thr Val Gln Arg
            105                 110                 115

ctg ccc ccc ggc cag aag gtc atc gcc cca gag aac cta ccc cct ctc       558
Leu Pro Pro Gly Gln Lys Val Ile Ala Pro Glu Asn Leu Pro Pro Leu
        120                 125                 130

acg ccc tac tgc agg aga acc ctc aac ttt ggc tgc ctg gac gac att       606
Thr Pro Tyr Cys Arg Arg Thr Leu Asn Phe Gly Cys Leu Asp Asp Ile
    135                 140                 145

ggc cat ggg atc aag gac ctc agc acg cag ctg tca aga aca ggc acc       654
Gly His Gly Ile Lys Asp Leu Ser Thr Gln Leu Ser Arg Thr Gly Thr
150                 155                 160                 165

ctg tct cga aag agc atc aag gcc cct gcc aca ccc gcc tcc gcc acc       702
Leu Ser Arg Lys Ser Ile Lys Ala Pro Ala Thr Pro Ala Ser Ala Thr
                170                 175                 180

ttg ggg aga cca ccc cgg att ccc gag cca gtg cac ctg ccg gtg gtg       750
Leu Gly Arg Pro Pro Arg Ile Pro Glu Pro Val His Leu Pro Val Val
            185                 190                 195

ccc gac ggc aga ctc tcc gcc gcc tcc tct gcg tct tcc ctg gcc tcg       798
Pro Asp Gly Arg Leu Ser Ala Ala Ser Ser Ala Ser Ser Leu Ala Ser
        200                 205                 210

gcc ggc agc gcc gaa ggt gtc ggt ggg gcc ccc acg ccc aag ggg cag       846
Ala Gly Ser Ala Glu Gly Val Gly Gly Ala Pro Thr Pro Lys Gly Gln

215                 220                 225

gca gca cct cca gcc cca cct ctc ccc agc tcc ttg gac cca cct cct      894
Ala Ala Pro Pro Ala Pro Pro Leu Pro Ser Ser Leu Asp Pro Pro Pro
230                 235                 240                 245

cca cca gca gcc gtc gag gtg ttc cag cgg cct ccc acg ctg gag gag      942
Pro Pro Ala Ala Val Glu Val Phe Gln Arg Pro Pro Thr Leu Glu Glu
                250                 255                 260

ttg tcc cca ccc cca ccg gac gaa gag ctg ccc ctg cca ctg gac ctg      990
Leu Ser Pro Pro Pro Pro Asp Glu Glu Leu Pro Leu Pro Leu Asp Leu
            265                 270                 275

cct cct cct cca ccc ctg gat gga gat gaa ttg ggg ctg cct cca ccc     1038
Pro Pro Pro Pro Pro Leu Asp Gly Asp Glu Leu Gly Leu Pro Pro Pro
        280                 285                 290

cca cca gga ttt ggg cct gat gag ccc agc tgg gtg cct gcc tca tac     1086
Pro Pro Gly Phe Gly Pro Asp Glu Pro Ser Trp Val Pro Ala Ser Tyr
    295                 300                 305

ttg gag aaa gtg gtg aca ctg tac cca tac acc agc cag aag gac aat     1134
Leu Glu Lys Val Val Thr Leu Tyr Pro Tyr Thr Ser Gln Lys Asp Asn
310                 315                 320                 325

gag ctc tcc ttc tct gag ggc act gtc atc tgt gtc act cgc cgc tac     1182
Glu Leu Ser Phe Ser Glu Gly Thr Val Ile Cys Val Thr Arg Arg Tyr
                330                 335                 340

tcc gat ggc tgg tgc gag ggc gtc agc tca gag ggg act gga ttc ttc     1230
Ser Asp Gly Trp Cys Glu Gly Val Ser Ser Glu Gly Thr Gly Phe Phe
            345                 350                 355

cct ggg aac tat gtg gag ccc agc tgc t gacagcccag ggctctctgg         1278
Pro Gly Asn Tyr Val Glu Pro Ser Cys
        360                 365

gcagctgatg tctgcactga gtgggtttca tgagccccaa gccaaaacca gctccagtca   1338
cagctggact gggtctgccc acctcttggg ctgtgagctg tgttctgtcc ttcctcccat   1398
cggagggaga aggggtcctg gggagagaga atttatccag aggcctgctg cagatgggga   1458
agagctggaa accaagaagt ttgtcaacag aggaccccta ctccatgcag gacagggtct   1518
cctgctgcaa gtcccaactt tgaataaaac agatgatgtc ctgtgaaaaa aaaaaaaaaa   1578
aaaaa                                                               1583

<210> 18
<211> 366
<212> PRT
<213> Homo sapiens

<400> 18
Met Ala Glu Leu Gln Gln Leu Gln Glu Phe Glu Ile Pro Thr Gly Arg
1               5                   10                  15
Glu Ala Leu Arg Gly Asn His Ser Ala Leu Leu Arg Val Ala Asp Tyr
            20                  25                  30
Cys Glu Asp Asn Tyr Val Gln Ala Thr Asp Lys Arg Lys Ala Leu Glu
        35                  40                  45
Glu Thr Met Ala Phe Thr Thr Gln Ala Leu Ala Ser Val Ala Tyr Gln
    50                  55                  60
Val Gly Asn Leu Ala Gly His Thr Leu Arg Met Leu Asp Leu Gln Gly
65                  70                  75                  80
Ala Ala Leu Arg Gln Val Glu Ala Arg Val Ser Thr Leu Gly Gln Met
                85                  90                  95

Val Asn Met His Met Glu Lys Val Ala Arg Arg Glu Ile Gly Thr Leu
            100                 105                 110
Ala Thr Val Gln Arg Leu Pro Pro Gly Gln Lys Val Ile Ala Pro Glu
        115                 120                 125
Asn Leu Pro Pro Leu Thr Pro Tyr Cys Arg Arg Thr Leu Asn Phe Gly
    130                 135                 140
Cys Leu Asp Asp Ile Gly His Gly Ile Lys Asp Leu Ser Thr Gln Leu
145                 150                 155                 160
Ser Arg Thr Gly Thr Leu Ser Arg Lys Ser Ile Lys Ala Pro Ala Thr
                165                 170                 175
Pro Ala Ser Ala Thr Leu Gly Arg Pro Pro Arg Ile Pro Glu Pro Val
            180                 185                 190
His Leu Pro Val Val Pro Asp Gly Arg Leu Ser Ala Ala Ser Ser Ala
        195                 200                 205
Ser Ser Leu Ala Ser Ala Gly Ser Ala Glu Gly Val Gly Gly Ala Pro
    210                 215                 220
Thr Pro Lys Gly Gln Ala Ala Pro Pro Ala Pro Pro Leu Pro Ser Ser
225                 230                 235                 240
Leu Asp Pro Pro Pro Pro Pro Ala Ala Val Glu Val Phe Gln Arg Pro
                245                 250                 255
Pro Thr Leu Glu Glu Leu Ser Pro Pro Pro Pro Asp Glu Glu Leu Pro
            260                 265                 270
Leu Pro Leu Asp Leu Pro Pro Pro Pro Pro Leu Asp Gly Asp Glu Leu
        275                 280                 285
Gly Leu Pro Pro Pro Pro Pro Gly Phe Gly Pro Asp Glu Pro Ser Trp
    290                 295                 300
Val Pro Ala Ser Tyr Leu Glu Lys Val Val Thr Leu Tyr Pro Tyr Thr
305                 310                 315                 320
Ser Gln Lys Asp Asn Glu Leu Ser Phe Ser Glu Gly Thr Val Ile Cys
                325                 330                 335
Val Thr Arg Arg Tyr Ser Asp Gly Trp Cys Glu Gly Val Ser Ser Glu
            340                 345                 350
Gly Thr Gly Phe Phe Pro Gly Asn Tyr Val Glu Pro Ser Cys
        355                 360                 365

<210> 19
<211> 6935
<212> DNA
<213> Homo sapiens

<220>
<221> CDS
<222> (498)...(3780)
<223> I42

<400> 19
tttccagcca acgccaaaca gtgactgttg acaatttcat attgtcatca ggggaaccaa      60
ggcttattca gatgcctatt tcagaaccta ggacagttcc attgaaaagg cgcaggcgtt     120
cgggctggct gactagatgg atcaggcctg gctgcctgat ggctatattc ctccttcctc     180
cctctccact tccatctcaa cccttgaggc tgcatattga atagttggag aattcagtga     240
actaagagat gcaaatgcac agtacaaaat tcaaatgtcc aattcggggc agggctgcat     300
ctaactttaa tggcaaccac tgcatgtgat gtctggggac tctatagata catggcctca     360
gaccctgaag acatctggat tctgtcactg gattgttcac aaagtgaggc tgaactttcc     420
acaggacgaa gtcttcaggc tggccgcctc cctcgggaac ctggggcttg agccaggtgc     480
cgccctatgg atgggag atg acg gca aac cga gat ggg cga gac tac ttc        530
                   Met Thr Ala Asn Arg Asp Gly Arg Asp Tyr Phe
                    1               5                   10

atc aat cac atg aca cag gca atc cct ttt gac gac cct cgg tta gag       578
Ile Asn His Met Thr Gln Ala Ile Pro Phe Asp Asp Pro Arg Leu Glu
            15                  20                  25

agc tgc caa atc atc cct ccg gct cct cgg aag gtg gag atg aga agg       626

Ser Cys Gln Ile Ile Pro Pro Ala Pro Arg Lys Val Glu Met Arg Arg
        30                  35                  40

gac ccc gtg ctg gga ttt ggt ttt gtg gca ggc agt gaa aag cca gtg      674
Asp Pro Val Leu Gly Phe Gly Phe Val Ala Gly Ser Glu Lys Pro Val
    45                  50                  55

gtc gtt cgc tca gta aca cca ggt ggc ccc tct gaa ggc aag ctg atc      722
Val Val Arg Ser Val Thr Pro Gly Gly Pro Ser Glu Gly Lys Leu Ile
60                  65                  70                  75

ccg gga gat cag att gta atg att aat gat gaa ccg gtc agc gct gca      770
Pro Gly Asp Gln Ile Val Met Ile Asn Asp Glu Pro Val Ser Ala Ala
                80                  85                  90

ccc aga gag cgg gtc atc gat ctg gtc aga agc tgc aaa gaa tcg ata      818
Pro Arg Glu Arg Val Ile Asp Leu Val Arg Ser Cys Lys Glu Ser Ile
            95                  100                 105

ctc ctc act gtc att cag cct tac cct tct ccc aaa tca gca ttt att      866
Leu Leu Thr Val Ile Gln Pro Tyr Pro Ser Pro Lys Ser Ala Phe Ile
        110                 115                 120

agt gct gca aaa aag gca aga tta aag tcc aat cct gtc aaa gta cgc      914
Ser Ala Ala Lys Lys Ala Arg Leu Lys Ser Asn Pro Val Lys Val Arg
    125                 130                 135

ttc tct gag gag gtc atc atc aac ggc caa gtg tcg gaa act gtt aag      962
Phe Ser Glu Glu Val Ile Ile Asn Gly Gln Val Ser Glu Thr Val Lys
140                 145                 150                 155

gac aac tca ctt ctt ttt atg cca aat gtt ttg aaa gtc tat ctg gaa      1010
Asp Asn Ser Leu Leu Phe Met Pro Asn Val Leu Lys Val Tyr Leu Glu
                160                 165                 170

aat ggg cag acc aaa tca ttt cgt ttt gac tgc agc act tcc att aag      1058
Asn Gly Gln Thr Lys Ser Phe Arg Phe Asp Cys Ser Thr Ser Ile Lys
            175                 180                 185

gat gtc atc tta acc ctt caa gag aag ctc tcc atc aaa ggc att gaa      1106
Asp Val Ile Leu Thr Leu Gln Glu Lys Leu Ser Ile Lys Gly Ile Glu
        190                 195                 200

cac ttc tct ctc atg ctg gag cag agg aca gaa ggg gct gga acg aag      1154
His Phe Ser Leu Met Leu Glu Gln Arg Thr Glu Gly Ala Gly Thr Lys
    205                 210                 215

ctg ctc ttg ctt cat gaa cag gag act cta act cag gtg aca cag agg      1202
Leu Leu Leu Leu His Glu Gln Glu Thr Leu Thr Gln Val Thr Gln Arg
220                 225                 230                 235

ccc agc tcc cat aag atg aga tgt ctt ttc cga att agc ttc gtc cca      1250
Pro Ser Ser His Lys Met Arg Cys Leu Phe Arg Ile Ser Phe Val Pro
                240                 245                 250

aaa gat cca att gac ctt tta agg aga gat cca gtt gct ttc gag tat      1298
Lys Asp Pro Ile Asp Leu Leu Arg Arg Asp Pro Val Ala Phe Glu Tyr
            255                 260                 265

ctc tat gtt cag agt tgt aac gat gtg gtt cag gag cga ttt ggg ccg      1346
Leu Tyr Val Gln Ser Cys Asn Asp Val Val Gln Glu Arg Phe Gly Pro
        270                 275                 280

gag ctg aaa tat gac ata gcc ctg cgg ctg gcc gca tta caa atg tac 1394
Glu Leu Lys Tyr Asp Ile Ala Leu Arg Leu Ala Ala Leu Gln Met Tyr
    285                 290                 295

att gca acc gtt acc acc aag caa acg cag aaa atc tcc ctc aaa tac 1442
Ile Ala Thr Val Thr Thr Lys Gln Thr Gln Lys Ile Ser Leu Lys Tyr
300                 305                 310                 315

atc gaa aaa gaa tgg gga tta gag act ttt ctt ccc tct gct gtg ctg 1490
Ile Glu Lys Glu Trp Gly Leu Glu Thr Phe Leu Pro Ser Ala Val Leu
                320                 325                 330

caa agc atg aaa gag aag aac ata aag aaa gca ctt tca cac ctt gtc 1538
Gln Ser Met Lys Glu Lys Asn Ile Lys Lys Ala Leu Ser His Leu Val
            335                 340                 345

aaa gca aat caa aac ttg gta cca ccg ggt aaa aag ctc tct gca cta 1586
Lys Ala Asn Gln Asn Leu Val Pro Pro Gly Lys Lys Leu Ser Ala Leu
        350                 355                 360

caa gcc aag gtc cat tat ctc aag ttc ctc agt gac cta cga ttg tat 1634
Gln Ala Lys Val His Tyr Leu Lys Phe Leu Ser Asp Leu Arg Leu Tyr
    365                 370                 375

ggg ggc cgt gtg ttc aag gca aca tta gtg cag gca gaa aag cgc tcg 1682
Gly Gly Arg Val Phe Lys Ala Thr Leu Val Gln Ala Glu Lys Arg Ser
380                 385                 390                 395

gaa gtg act ctc ctg gtt ggg ccc cgg tat ggc ata agc cat gtc atc 1730
Glu Val Thr Leu Leu Val Gly Pro Arg Tyr Gly Ile Ser His Val Ile
                400                 405                 410

aac acc aaa acc aat ctg gtg gct ctt tta gcc gac ttt agc cac gtc 1778
Asn Thr Lys Thr Asn Leu Val Ala Leu Leu Ala Asp Phe Ser His Val
            415                 420                 425

aac agg atc gaa atg ttt tcc gag gag gag agc ttg gtg cgg gta gaa 1826
Asn Arg Ile Glu Met Phe Ser Glu Glu Glu Ser Leu Val Arg Val Glu
        430                 435                 440

ctc cac gtg cta gat gtg aag cct atc acg ctt ctg atg gaa tcc tca 1874
Leu His Val Leu Asp Val Lys Pro Ile Thr Leu Leu Met Glu Ser Ser
    445                 450                 455

gat gcc atg aac ctg gcc tgc ttg acg gct gga tac tac cgg ctg ctt 1922
Asp Ala Met Asn Leu Ala Cys Leu Thr Ala Gly Tyr Tyr Arg Leu Leu
460                 465                 470                 475

gtt gat tcc agg agg tcg ata ttt aac atg gcc aac aag aaa aac aca 1970
Val Asp Ser Arg Arg Ser Ile Phe Asn Met Ala Asn Lys Lys Asn Thr
                480                 485                 490

gcg acc cag gaa aca gga cct gaa aac aag ggg aag cat aac ctc ctt 2018
Ala Thr Gln Glu Thr Gly Pro Glu Asn Lys Gly Lys His Asn Leu Leu
            495                 500                 505

ggc cca gat tgg aac tgt ata ccc caa atg acc acc ttt att ggc gaa 2066
Gly Pro Asp Trp Asn Cys Ile Pro Gln Met Thr Thr Phe Ile Gly Glu
        510                 515                 520

ggg gaa caa gaa gcc cag ata aca tac ata gat tca aag cag aag acg 2114
Gly Glu Gln Glu Ala Gln Ile Thr Tyr Ile Asp Ser Lys Gln Lys Thr
    525                 530                 535

gtg gag atc aca gac agc acc atg tgt cca aaa gag cac cgg cac ttg 2162
Val Glu Ile Thr Asp Ser Thr Met Cys Pro Lys Glu His Arg His Leu
540                 545                 550                 555

tac ata gac aat gcc tat agt tca gat gga ctt aac cag cag ctg agc 2210
Tyr Ile Asp Asn Ala Tyr Ser Ser Asp Gly Leu Asn Gln Gln Leu Ser
                560                 565                 570

cag ccc ggg gag gcc ccc tgt gag gca gac tac aga agt cta gct cag 2258
Gln Pro Gly Glu Ala Pro Cys Glu Ala Asp Tyr Arg Ser Leu Ala Gln
            575                 580                 585

cgg tcc cta ttg acc ctc tca gga cca gaa act ctg aag aaa gca cag 2306
Arg Ser Leu Leu Thr Leu Ser Gly Pro Glu Thr Leu Lys Lys Ala Gln
        590                 595                 600

gaa tct ccg aga gga gct aaa gtg tcc ttt att ttt gga gac ttc gcc 2354
Glu Ser Pro Arg Gly Ala Lys Val Ser Phe Ile Phe Gly Asp Phe Ala
    605                 610                 615

ttg gat gat ggt att agt ccc cca acc ctt ggc tat gaa acg cta cta 2402
Leu Asp Asp Gly Ile Ser Pro Pro Thr Leu Gly Tyr Glu Thr Leu Leu
620                 625                 630                 635

gat gag ggt cct gaa atg ctg gag aag cag aga aat ctc tac att ggc 2450
Asp Glu Gly Pro Glu Met Leu Glu Lys Gln Arg Asn Leu Tyr Ile Gly
                640                 645                 650

agt gcc aat gac atg aag ggc ctg gat ctc act cca gag gca gag ggc 2498
Ser Ala Asn Asp Met Lys Gly Leu Asp Leu Thr Pro Glu Ala Glu Gly
            655                 660                 665

atc cag ttt gtg gaa aat tct gtt tat gca aac ata ggc gat gtg aag 2546
Ile Gln Phe Val Glu Asn Ser Val Tyr Ala Asn Ile Gly Asp Val Lys
        670                 675                 680

agc ttc cag gcc gcg gag ggg atc gag gaa ccc ctc ttg cat gac atc 2594
Ser Phe Gln Ala Ala Glu Gly Ile Glu Glu Pro Leu Leu His Asp Ile
    685                 690                 695

tgt tat gca gaa aac act gat gac gcg gag gac gag gac gag gtg agc 2642
Cys Tyr Ala Glu Asn Thr Asp Asp Ala Glu Asp Glu Asp Glu Val Ser
700                 705                 710                 715

tgc gag gag gac ctc gtg gtg ggg gag atg aac cag ccg gcc atc ctc 2690
Cys Glu Glu Asp Leu Val Val Gly Glu Met Asn Gln Pro Ala Ile Leu
                720                 725                 730

aac ctg tct ggg tca agc gat gac atc att gac ctc aca tcc ctg ccc 2738
Asn Leu Ser Gly Ser Ser Asp Asp Ile Ile Asp Leu Thr Ser Leu Pro
            735                 740                 745

cct cca gaa ggt gat gac aat gag gat gac ttc ctg ttg cgt tcc ttg 2786
Pro Pro Glu Gly Asp Asp Asn Glu Asp Asp Phe Leu Leu Arg Ser Leu
        750                 755                 760

aac atg gcc att gcc gca ccc cca cct ggc ttt aga gac agt tca gat 2834
Asn Met Ala Ile Ala Ala Pro Pro Pro Gly Phe Arg Asp Ser Ser Asp
    765                 770                 775

gaa gag gac tct cag agc cag gca gct tcc ttc ccc gag gac aag gag 2882
Glu Glu Asp Ser Gln Ser Gln Ala Ala Ser Phe Pro Glu Asp Lys Glu

780                 785                 790                 795

aaa ggc agc agc ctg caa aat gat gag atc ccc gtg tcc ctc att gac      2930
Lys Gly Ser Ser Leu Gln Asn Asp Glu Ile Pro Val Ser Leu Ile Asp
                800                 805                 810

gct gtg ccc acc agc gcc gaa ggc aag tgt gag aag gga ctg gat aat      2978
Ala Val Pro Thr Ser Ala Glu Gly Lys Cys Glu Lys Gly Leu Asp Asn
            815                 820                 825

gcc gtc gtc tcc acg ctg gga gct cta gag gct cta tcc gtg tca gaa      3026
Ala Val Val Ser Thr Leu Gly Ala Leu Glu Ala Leu Ser Val Ser Glu
        830                 835                 840

gaa cag cag acc agt gac aat tca ggt gta gcc atc ttg cgg gct tat      3074
Glu Gln Gln Thr Ser Asp Asn Ser Gly Val Ala Ile Leu Arg Ala Tyr
    845                 850                 855

agt cct gag tct tcg tca gac tcg ggc aat gaa act aac tct tct gaa      3122
Ser Pro Glu Ser Ser Ser Asp Ser Gly Asn Glu Thr Asn Ser Ser Glu
860                 865                 870                 875

atg act gag agt tct gaa ctg gcc aca gca caa aaa cag tca gaa aac      3170
Met Thr Glu Ser Ser Glu Leu Ala Thr Ala Gln Lys Gln Ser Glu Asn
                880                 885                 890

ctc tcc cgc atg ttc ttg gcc act cac gaa ggc tac cac ccc ctt gca      3218
Leu Ser Arg Met Phe Leu Ala Thr His Glu Gly Tyr His Pro Leu Ala
            895                 900                 905

gaa gag cag acc gag ttc ccg gcc tcc aag acc ccc gct ggg ggc ttg      3266
Glu Glu Gln Thr Glu Phe Pro Ala Ser Lys Thr Pro Ala Gly Gly Leu
        910                 915                 920

cct cca aag tcc tcg cac gcc ctg gct gct agg cca gca acc gac ctc      3314
Pro Pro Lys Ser Ser His Ala Leu Ala Ala Arg Pro Ala Thr Asp Leu
    925                 930                 935

ccg ccc aaa gtt gtg cct tcc aag cag tta ctt cac tca gac cac atg      3362
Pro Pro Lys Val Val Pro Ser Lys Gln Leu Leu His Ser Asp His Met
940                 945                 950                 955

gag atg gag cct gaa act atg gag act aag tcg gtc act gac tat ttt      3410
Glu Met Glu Pro Glu Thr Met Glu Thr Lys Ser Val Thr Asp Tyr Phe
                960                 965                 970

agc aaa ctg cac atg ggg tcg gtg gca tac tcc tgc act agc aaa agg      3458
Ser Lys Leu His Met Gly Ser Val Ala Tyr Ser Cys Thr Ser Lys Arg
            975                 980                 985

aaa agc aag ctg gcc gat ggt gag ggg aag gca ccc cct aat ggg aac      3506
Lys Ser Lys Leu Ala Asp Gly Glu Gly Lys Ala Pro Pro Asn Gly Asn
        990                 995                 1000

aca aca gga aaa aaa cag cag ggg acc aaa acg gca gag atg gag gag      3554
Thr Thr Gly Lys Lys Gln Gln Gly Thr Lys Thr Ala Glu Met Glu Glu
    1005                1010                1015

gag gcc agt ggt aaa ttt ggt act gtg tct tca cga gac agt caa cac      3602
Glu Ala Ser Gly Lys Phe Gly Thr Val Ser Ser Arg Asp Ser Gln His
1020                1025                1030                1035

ctg agc act ttt aat ctg gag aga act gcc ttt cgc aag gac agt caa      3650

Leu Ser Thr Phe Asn Leu Glu Arg Thr Ala Phe Arg Lys Asp Ser Gln
                1040                1045                1050

aga tgg tat gtg gcc act gaa ggt ggg atg gct gaa aaa aag tgg att 3698
Arg Trp Tyr Val Ala Thr Glu Gly Gly Met Ala Glu Lys Lys Trp Ile
            1055                1060                1065

aga agc agc aac agg gaa aac ctt tcc aag agc ttc tgg tct tgg ggc 3746
Arg Ser Ser Asn Arg Glu Asn Leu Ser Lys Ser Phe Trp Ser Trp Gly
        1070                1075                1080

aag gga ggc cga agg gaa gga aga agg agc tcc t gatggagaaa 3790
Lys Gly Gly Arg Arg Glu Gly Arg Arg Ser Ser
    1085                1090

ccagtgatgg ctcaggactt ggtcaagggg accgcttctt aactgacgtg acctgtgcat 3850
cttcagccaa agacttagat aacccagagg acgctgactc gtccacctgc gaccatcctt 3910
ccaagcttcc tgaggctgat gagagtgtgg cccgcctttg tgactaccac ttggccaagc 3970
ggatgtcatc actgcaaagc gagggccatt tttctctgca gagctcccaa ggctcttcag 4030
tggatgcagg ctgtggcaca ggcagcagtg gcagtgcctg tgccacaccc gtggagtcgc 4090
cgctctgccc ctccctgggg aagcacttga ttcctgacgc ttctgggaaa ggcgtgaatt 4150
acattccttc agaggagaga gcccctgggc ttcccaacca cggagccacc tttaaggaac 4210
tgcacccaca gacagaaggg atgtgtccac ggatgacagt gcctgctctg cacacagcca 4270
ttaacaccga acccctgttt ggcacattga gagatggatg ccatcggctc cccaagatta 4330
aggaaaccac agtgtagctt tgacagagcc tgggaaggag agacgaggag gcatgccttc 4390
agcttggtct caacatcctg aagctgatcc catcctgcta ccatcaaaca ttcactcgga 4450
atcaaaggtg ccaattccaa atcaagaccc taatgatttc tcccaagcaa atcaggcata 4510
cggagaggct gtgagctggc ggccaccgga tctgagaggg gggagcctca ggacacctcc 4570
cagccagaag gctctgagac atagcagcag tatcctctcc ggatctgtcg atttggagac 4630
cttccgagag agaaccaagg gtgcagtcag cttaaagtgt ccaggcatca cagaagcaca 4690
ggaggccagt tctgaaaggc gagcagaact ccccctgggg aggaagctca ccaaaagttt 4750
ttcccaaagc tcaatgcact tgagctctga ggggaggttt cacaaaaggt ccccagtggc 4810
tcataaagac tcaaagctgt ataggacatt acccttgcgg aagctggagg gcagcaattg 4870
gagatgccgg ggacccttca gctattgctt cctgaaccga gggcaggatg aagatggtga 4930
ggaagaagag gagaggggag aggccaccgt ccaggtctct tgcctctata gaccacaggt 4990
gactcaagcc atgccagaac caagcagccc atgcctggct gtggcgattc agaagcaacg 5050
aggggagcta tccagagggt cagtgctgaa ggtctgggca gaagacctgc gagacccaga 5110
tgacttggac ttcagcaacc tggcttttga tgcccggatt gcaagaataa atgccctaaa 5170
ggagagcaca tatgcaatgc ctgatgggtt ccttgcagcc caaaatgatg ccaatgagct 5230
gctctgtctc gtcagggcaa ccaaggagaa gagggaggag tcacgccctg aagcgtacga 5290
ccttacactt tctcagtaca agcaactgtt atccattgag tccagacagt tgggaagtgc 5350
ctgtaggaaa atggcgatgg ctgagaaaag cccggaggag atgctcctag ctatgacttc 5410
cagctttcaa gtgctctgtt gcctaacaga agcttgcatg cgattagtta aagtcgtgaa 5470
ctcagaaaca cagcggcagg aaattgtagg gaagatcgat gaagtggtca taaattacat 5530
ttgtctactg aaagctgccg aagcagccac tggaaagaac cctggggacc ctaatgttgg 5590
actctcggcg cgacactcaa ccaccatggc cgctctcgta agcacactga cacgttctct 5650
caagaggctt ttaaacaaat aaatatggaa gtcacgtcat aatctacctt tgcaaagcca 5710
tacatgaact tttatttact ttgtgtgtat gatgaacaga tgtctccttt cttctctctg 5770
tatattttgt tattttatat aaaataggag ataaaagtca cactgatgaa atgttgaaat 5830
gtactaatca gatgtattct gtttatatta tacatatata tacacgtaaa agaaatatcc 5890
aagaaagtga tgacatttgg ctatttttca tatagttaaa actccaggta tatgatgtga 5950
aattttaaat tctaccatgt tagagcaaaa caatgaatcc tatccccttt ctttccaagt 6010
agctacttgg aaaccatatc attcatattt agaagtaaaa cacaaaacaa aaaagagaga 6070
gaaaagaaaa gaaatcacaa tgtatataaa acagtactta tgttttaaaa ttatgatttt 6130
taagcattgg aaatagcaaa aagacattta aaattcaaga agctattatg aattactaga 6190
gaatatatct gtaataaatt aattttttgc tcatagtatt tggttactgg atgctttctt 6250
ccaagaatcc cacatattta atttgggttt ttgctactgg ggctacaaat tggtggggat 6310
ggattctact gtgtcagcac aaatgctctt cacagtggtt ctagcattta aaaaacttcc 6370
cggggagaag aacagagggg atgatgggca gtttcctagg taacacctag agttatagaa 6430
tatctcatta cataaaatgt atggaattaa taataccaaa attaattatt tgatggaaag 6490
atctgctttg actaaatgtc aaaaatctgc aaaccaaaga cattatcttc ccctcatccc 6550
aactcaacta cgaaacttaa aattcccttt agagtgatag gacatttagt aaagtatttg 6610
caaacttaaa aaaaggaaca tttaatgatc atcaaaatta agtacagatt cagtaatgta 6670

gaccagacca cacaccagca cctgtgagtc tcatctcaga tcacagctct cagcataggg 6730
cttcatgcat caccgcctct acagaggcta aggctgccag tcaaatttgg aattatagcg 6790
tagtactggg acaaaatctc aaatcttgga tgttccagaa aatcagggag tgatggctac 6850
tgtaatcatg ggagccatga gtaaatagtt aagtatttat taaataaata cttaatctgg 6910
attggctgat aaaaatatga aatct 6935

<210> 20
<211> 1094
<212> PRT
<213> Homo sapiens

<400> 20
Met Thr Ala Asn Arg Asp Gly Arg Asp Tyr Phe Ile Asn His Met Thr
1               5                   10                  15
Gln Ala Ile Pro Phe Asp Asp Pro Arg Leu Glu Ser Cys Gln Ile Ile
            20                  25                  30
Pro Pro Ala Pro Arg Lys Val Glu Met Arg Arg Asp Pro Val Leu Gly
        35                  40                  45
Phe Gly Phe Val Ala Gly Ser Glu Lys Pro Val Val Val Arg Ser Val
    50                  55                  60
Thr Pro Gly Gly Pro Ser Glu Gly Lys Leu Ile Pro Gly Asp Gln Ile
65                  70                  75                  80
Val Met Ile Asn Asp Glu Pro Val Ser Ala Ala Pro Arg Glu Arg Val
                85                  90                  95
Ile Asp Leu Val Arg Ser Cys Lys Glu Ser Ile Leu Leu Thr Val Ile
            100                 105                 110
Gln Pro Tyr Pro Ser Pro Lys Ser Ala Phe Ile Ser Ala Ala Lys Lys
        115                 120                 125
Ala Arg Leu Lys Ser Asn Pro Val Lys Val Arg Phe Ser Glu Glu Val
    130                 135                 140
Ile Ile Asn Gly Gln Val Ser Glu Thr Val Lys Asp Asn Ser Leu Leu
145                 150                 155                 160
Phe Met Pro Asn Val Leu Lys Val Tyr Leu Glu Asn Gly Gln Thr Lys
                165                 170                 175
Ser Phe Arg Phe Asp Cys Ser Thr Ser Ile Lys Asp Val Ile Leu Thr
            180                 185                 190
Leu Gln Glu Lys Leu Ser Ile Lys Gly Ile Glu His Phe Ser Leu Met
        195                 200                 205
Leu Glu Gln Arg Thr Glu Gly Ala Gly Thr Lys Leu Leu Leu Leu His
    210                 215                 220
Glu Gln Glu Thr Leu Thr Gln Val Thr Gln Arg Pro Ser Ser His Lys
225                 230                 235                 240
Met Arg Cys Leu Phe Arg Ile Ser Phe Val Pro Lys Asp Pro Ile Asp
                245                 250                 255
Leu Leu Arg Arg Asp Pro Val Ala Phe Glu Tyr Leu Tyr Val Gln Ser
            260                 265                 270
Cys Asn Asp Val Val Gln Glu Arg Phe Gly Pro Glu Leu Lys Tyr Asp
        275                 280                 285
Ile Ala Leu Arg Leu Ala Ala Leu Gln Met Tyr Ile Ala Thr Val Thr
    290                 295                 300
Thr Lys Gln Thr Gln Lys Ile Ser Leu Lys Tyr Ile Glu Lys Glu Trp
305                 310                 315                 320
Gly Leu Glu Thr Phe Leu Pro Ser Ala Val Leu Gln Ser Met Lys Glu
                325                 330                 335
Lys Asn Ile Lys Lys Ala Leu Ser His Leu Val Lys Ala Asn Gln Asn
            340                 345                 350
Leu Val Pro Pro Gly Lys Lys Leu Ser Ala Leu Gln Ala Lys Val His
        355                 360                 365
Tyr Leu Lys Phe Leu Ser Asp Leu Arg Leu Tyr Gly Gly Arg Val Phe
    370                 375                 380
Lys Ala Thr Leu Val Gln Ala Glu Lys Arg Ser Glu Val Thr Leu Leu
385                 390                 395                 400
Val Gly Pro Arg Tyr Gly Ile Ser His Val Ile Asn Thr Lys Thr Asn

                405                 410                 415
Leu Val Ala Leu Leu Ala Asp Phe Ser His Val Asn Arg Ile Glu Met
            420                 425                 430
Phe Ser Glu Glu Glu Ser Leu Val Arg Val Glu Leu His Val Leu Asp
        435                 440                 445
Val Lys Pro Ile Thr Leu Leu Met Glu Ser Ser Asp Ala Met Asn Leu
    450                 455                 460
Ala Cys Leu Thr Ala Gly Tyr Tyr Arg Leu Leu Val Asp Ser Arg Arg
465                 470                 475                 480
Ser Ile Phe Asn Met Ala Asn Lys Lys Asn Thr Ala Thr Gln Glu Thr
                485                 490                 495
Gly Pro Glu Asn Lys Gly Lys His Asn Leu Leu Gly Pro Asp Trp Asn
            500                 505                 510
Cys Ile Pro Gln Met Thr Thr Phe Ile Gly Glu Gly Glu Gln Glu Ala
        515                 520                 525
Gln Ile Thr Tyr Ile Asp Ser Lys Gln Lys Thr Val Glu Ile Thr Asp
    530                 535                 540
Ser Thr Met Cys Pro Lys Glu His Arg His Leu Tyr Ile Asp Asn Ala
545                 550                 555                 560
Tyr Ser Ser Asp Gly Leu Asn Gln Gln Leu Ser Gln Pro Gly Glu Ala
                565                 570                 575
Pro Cys Glu Ala Asp Tyr Arg Ser Leu Ala Gln Arg Ser Leu Leu Thr
            580                 585                 590
Leu Ser Gly Pro Glu Thr Leu Lys Lys Ala Gln Glu Ser Pro Arg Gly
        595                 600                 605
Ala Lys Val Ser Phe Ile Phe Gly Asp Phe Ala Leu Asp Asp Gly Ile
    610                 615                 620
Ser Pro Pro Thr Leu Gly Tyr Glu Thr Leu Leu Asp Glu Gly Pro Glu
625                 630                 635                 640
Met Leu Glu Lys Gln Arg Asn Leu Tyr Ile Gly Ser Ala Asn Asp Met
                645                 650                 655
Lys Gly Leu Asp Leu Thr Pro Glu Ala Glu Gly Ile Gln Phe Val Glu
            660                 665                 670
Asn Ser Val Tyr Ala Asn Ile Gly Asp Val Lys Ser Phe Gln Ala Ala
        675                 680                 685
Glu Gly Ile Glu Glu Pro Leu Leu His Asp Ile Cys Tyr Ala Glu Asn
    690                 695                 700
Thr Asp Asp Ala Glu Asp Glu Asp Glu Val Ser Cys Glu Glu Asp Leu
705                 710                 715                 720
Val Val Gly Glu Met Asn Gln Pro Ala Ile Leu Asn Leu Ser Gly Ser
                725                 730                 735
Ser Asp Asp Ile Ile Asp Leu Thr Ser Leu Pro Pro Pro Glu Gly Asp
            740                 745                 750
Asp Asn Glu Asp Asp Phe Leu Leu Arg Ser Leu Asn Met Ala Ile Ala
        755                 760                 765
Ala Pro Pro Pro Gly Phe Arg Asp Ser Ser Asp Glu Glu Asp Ser Gln
    770                 775                 780
Ser Gln Ala Ala Ser Phe Pro Glu Asp Lys Glu Lys Gly Ser Ser Leu
785                 790                 795                 800
Gln Asn Asp Glu Ile Pro Val Ser Leu Ile Asp Ala Val Pro Thr Ser
                805                 810                 815
Ala Glu Gly Lys Cys Glu Lys Gly Leu Asp Asn Ala Val Val Ser Thr
            820                 825                 830
Leu Gly Ala Leu Glu Ala Leu Ser Val Ser Glu Glu Gln Gln Thr Ser
        835                 840                 845
Asp Asn Ser Gly Val Ala Ile Leu Arg Ala Tyr Ser Pro Glu Ser Ser
    850                 855                 860
Ser Asp Ser Gly Asn Glu Thr Asn Ser Ser Glu Met Thr Glu Ser Ser
865                 870                 875                 880
Glu Leu Ala Thr Ala Gln Lys Gln Ser Glu Asn Leu Ser Arg Met Phe
                885                 890                 895
Leu Ala Thr His Glu Gly Tyr His Pro Leu Ala Glu Glu Gln Thr Glu
            900                 905                 910

Phe Pro Ala Ser Lys Thr Pro Ala Gly Gly Leu Pro Pro Lys Ser Ser
        915                 920                 925
His Ala Leu Ala Ala Arg Pro Ala Thr Asp Leu Pro Pro Lys Val Val
    930                 935                 940
Pro Ser Lys Gln Leu Leu His Ser Asp His Met Glu Met Glu Pro Glu
945                 950                 955                 960
Thr Met Glu Thr Lys Ser Val Thr Asp Tyr Phe Ser Lys Leu His Met
                965                 970                 975
Gly Ser Val Ala Tyr Ser Cys Thr Ser Lys Arg Lys Ser Lys Leu Ala
            980                 985                 990
Asp Gly Glu Gly Lys Ala Pro Pro Asn Gly Asn Thr Thr Gly Lys Lys
        995                 1000                1005
Gln Gln Gly Thr Lys Thr Ala Glu Met Glu Glu Glu Ala Ser Gly Lys
    1010                1015                1020
Phe Gly Thr Val Ser Ser Arg Asp Ser Gln His Leu Ser Thr Phe Asn
1025                1030                1035                1040
Leu Glu Arg Thr Ala Phe Arg Lys Asp Ser Gln Arg Trp Tyr Val Ala
                1045                1050                1055
Thr Glu Gly Gly Met Ala Glu Lys Lys Trp Ile Arg Ser Ser Asn Arg
            1060                1065                1070
Glu Asn Leu Ser Lys Ser Phe Trp Ser Trp Gly Lys Gly Gly Arg Arg
        1075                1080                1085
Glu Gly Arg Arg Ser Ser
    1090

<210> 21
<211> 2139
<212> DNA
<213> Mus musculus

<220>
<221> CDS
<222> (806)...(1364)
<223> Homer 1a

<221> misc_feature
<222> (1)...(2139)
<223> n = A,T,C or G

<400> 21
agcggggctc cattgtgctc ggcgggggcc gggaagccaa aggaggtggg ctcgggcccc      60
tgcgctgctc cccggcggct gcgcccccag ctagctgcca gcctggaaat ggctccgctg     120
ctgctcctcg ggaaaacgaa tcgatccttc ccagccttct ctgcctgctc tccacctcct     180
ctctgctccg agtcttagga ggacgaacat tcaaaggaca gattccaatg tggtgtgccg     240
tgcacatcgg gagcggctgg ggtttgcact tcgagatttc ttctatataa tttttttttt     300
ttaaacgtaa gggaggcagt agcattgctg cctgtaggat tttttattca agtgcacgtc     360
gcgttgggtt gcacgntcca cccccaggga cctggtgtgg tgaaatttga acccaccgcc     420
ttagcccaaa aaggccgagt aacctggctg cctgagtgtc gtggaagacg tgagcgaaat     480
gaccagcgaa ctcatttttt atcagacttg ctgaagctgg cttttgcgtt ttttctacac     540
gtacgcttaa ttttgtggaa tagttaagtg ctatattctc cgcgcaacct tttcaaattc     600
caaatgtttg aacatttggg tgtcagcgcg agtgaaatca ttttaccgac aagaactaac     660
tgaattgtct gccttgttga gttgcctccg gaaaagatct cgggggtgga aaagcaactg     720
caaaataaca gacggagaaa attccttgga agttatttct gtagcataag agcagaaact     780
tcagagcaag ttttcattgg gcaaa atg ggg gag caa cct atc ttc agc act      832
                            Met Gly Glu Gln Pro Ile Phe Ser Thr
                             1               5

cga gct cat gtc ttc cag att gac ccg aac aca aag aag aac tgg gta      880
Arg Ala His Val Phe Gln Ile Asp Pro Asn Thr Lys Lys Asn Trp Val
 10                  15                  20                  25

ccc acc agc aag cat gca gtt act gta tct tat ttt tat gac agc aca      928
Pro Thr Ser Lys His Ala Val Thr Val Ser Tyr Phe Tyr Asp Ser Thr

                    30                  35                  40

aga aat gtg tat agg ata atc agt tta gat ggc tca aag gca ata ata      976
Arg Asn Val Tyr Arg Ile Ile Ser Leu Asp Gly Ser Lys Ala Ile Ile
            45                  50                  55

aat agc acc atc aca cca aac atg aca ttt act aaa aca tct caa aag      1024
Asn Ser Thr Ile Thr Pro Asn Met Thr Phe Thr Lys Thr Ser Gln Lys
        60                  65                  70

ttt ggc caa tgg gct gat agc cgg gca aac act gtt tat gga ctg gga      1072
Phe Gly Gln Trp Ala Asp Ser Arg Ala Asn Thr Val Tyr Gly Leu Gly
    75                  80                  85

ttc tcc tct gag cat cat ctt tca aaa ttc gca gaa aag ttt cag gaa      1120
Phe Ser Ser Glu His His Leu Ser Lys Phe Ala Glu Lys Phe Gln Glu
 90                  95                 100                 105

ttt aag gaa gct gct cgg ctt gca aag gag aag tcg cag gag aag atg      1168
Phe Lys Glu Ala Ala Arg Leu Ala Lys Glu Lys Ser Gln Glu Lys Met
                110                 115                 120

gag ctg acc agt acc cct tca cag gaa tca gca gga gga gat ctt cag      1216
Glu Leu Thr Ser Thr Pro Ser Gln Glu Ser Ala Gly Gly Asp Leu Gln
            125                 130                 135

tct cct ttg aca cca gaa agt atc aat ggg aca gac gat gag aga aca      1264
Ser Pro Leu Thr Pro Glu Ser Ile Asn Gly Thr Asp Asp Glu Arg Thr
        140                 145                 150

ccc gat gtg aca cag aac tca gag cca agg gct gag cca act cag aat      1312
Pro Asp Val Thr Gln Asn Ser Glu Pro Arg Ala Glu Pro Thr Gln Asn
    155                 160                 165

gca ttg cca ttt cca cat agg tac aca ttc aat tca gca atc atg att      1360
Ala Leu Pro Phe Pro His Arg Tyr Thr Phe Asn Ser Ala Ile Met Ile
170                 175                 180                 185

aag t aaggtggata aatatggaag ttcatttggt ttcagaaact cttgaagtta      1414
Lys

caacctttga gtgaaaaatc tcaggtcaga ctcctttaat ttattgttct tggttgctca   1474
agttgactga attactatat ttccattatc tatgtggaaa aaggagcatt gagctaatta   1534
taggagaaat tttttaaatg gagaaaatat aattcctttc tatctatatt ttaaagatcc   1594
cttttgttaa cccgttttct gtntttatat atgttatgta agatttataa tgtgtaatta   1654
gaaacataga atttctactc tgaaggaaag ctttaccaca ggcctacaga gttttcacag   1714
aagacagggt accaagcacg agcctgttag cattgatggc agatgccagc agaaggaagg   1774
cttgacttcc taattctgta ttctaaaaga tacatcatgt tctaaatgca tttcaaacat   1834
tagttattgg ccgtaccgtg gcattactgg actgtaaaca tgaatgtgaa atggcactat   1894
tgaaaatatt tttttaaagc ccatctacct taacactaat ttttaccctt atttaaatgc   1954
tttttactaa atagttttag gtaaaattaa gaaaataggg gttttttgac tgcacatttt   2014
tttgaagaac caagttttag aaaattatat tctttgacag attaaaaatt gcaaagtgag   2074
atatttcaaa ctctcctagg tgagttttta ttgtgtttga acttgcatta ataggggcat   2134
aggat                                                               2139

<210> 22
<211> 186
<212> PRT
<213> Mus musculus

<400> 22
Met Gly Glu Gln Pro Ile Phe Ser Thr Arg Ala His Val Phe Gln Ile

1                5                   10                  15
Asp Pro Asn Thr Lys Lys Asn Trp Val Pro Thr Ser Lys His Ala Val
            20                  25                  30
Thr Val Ser Tyr Phe Tyr Asp Ser Thr Arg Asn Val Tyr Arg Ile Ile
        35                  40                  45
Ser Leu Asp Gly Ser Lys Ala Ile Ile Asn Ser Thr Ile Thr Pro Asn
    50                  55                  60
Met Thr Phe Thr Lys Thr Ser Gln Lys Phe Gly Gln Trp Ala Asp Ser
65                  70                  75                  80
Arg Ala Asn Thr Val Tyr Gly Leu Gly Phe Ser Ser Glu His His Leu
                85                  90                  95
Ser Lys Phe Ala Glu Lys Phe Gln Glu Phe Lys Glu Ala Ala Arg Leu
            100                 105                 110
Ala Lys Glu Lys Ser Gln Glu Lys Met Glu Leu Thr Ser Thr Pro Ser
        115                 120                 125
Gln Glu Ser Ala Gly Gly Asp Leu Gln Ser Pro Leu Thr Pro Glu Ser
    130                 135                 140
Ile Asn Gly Thr Asp Asp Glu Arg Thr Pro Asp Val Thr Gln Asn Ser
145                 150                 155                 160
Glu Pro Arg Ala Glu Pro Thr Gln Asn Ala Leu Pro Phe Pro His Arg
                165                 170                 175
Tyr Thr Phe Asn Ser Ala Ile Met Ile Lys
            180                 185

<210> 23
<211> 1418
<212> DNA
<213> Mus musculus

<220>
<221> CDS
<222> (153)...(1215)
<223> Homer 1b

<400> 23
gaattcggca cgagtctgcc ttgttgagtt gcctccggaa aagatctcgg gggtggaaaa      60
gcaactgcaa aataacagac ggagaaaatt ccttggaagt tatttctgta gcataagagc     120
agaaacttca gagcaagttt tcattgggca aa atg ggg gag caa cct atc ttc       173
                                    Met Gly Glu Gln Pro Ile Phe
                                     1               5

agc act cga gct cat gtc ttc cag att gac ccg aac aca aag aag aac       221
Ser Thr Arg Ala His Val Phe Gln Ile Asp Pro Asn Thr Lys Lys Asn
        10                  15                  20

tgg gta ccc acc agc aag cat gca gtt act gta tct tat ttt tat gac       269
Trp Val Pro Thr Ser Lys His Ala Val Thr Val Ser Tyr Phe Tyr Asp
    25                  30                  35

agc aca aga aat gtg tat agg ata atc agt tta gat ggc tca aag gca       317
Ser Thr Arg Asn Val Tyr Arg Ile Ile Ser Leu Asp Gly Ser Lys Ala
40                  45                  50                  55

ata ata aat agc acc atc aca cca aac atg aca ttt act aaa aca tct       365
Ile Ile Asn Ser Thr Ile Thr Pro Asn Met Thr Phe Thr Lys Thr Ser
                60                  65                  70

caa aag ttt ggc caa tgg gct gat agc cgg gca aac act gtt tat gga       413
Gln Lys Phe Gly Gln Trp Ala Asp Ser Arg Ala Asn Thr Val Tyr Gly
            75                  80                  85

ctg gga ttc tcc tct gag cat cat ctt tca aaa ttc gca gaa aag ttt       461
Leu Gly Phe Ser Ser Glu His His Leu Ser Lys Phe Ala Glu Lys Phe

90                  95                  100

cag gaa ttt aag gaa gct gct cgg ctt gca aag gag aag tcg cag gag     509
Gln Glu Phe Lys Glu Ala Ala Arg Leu Ala Lys Glu Lys Ser Gln Glu
    105                 110                 115

aag atg gag ctg acc agt acc cct tca cag gaa tca gca gga gga gat     557
Lys Met Glu Leu Thr Ser Thr Pro Ser Gln Glu Ser Ala Gly Gly Asp
120                 125                 130                 135

ctt cag tct cct ttg aca cca gaa agt atc aat ggg aca gac gat gag     605
Leu Gln Ser Pro Leu Thr Pro Glu Ser Ile Asn Gly Thr Asp Asp Glu
                140                 145                 150

aga aca ccc gat gtg aca cag aac tca gag cca agg gct gag cca act     653
Arg Thr Pro Asp Val Thr Gln Asn Ser Glu Pro Arg Ala Glu Pro Thr
            155                 160                 165

cag aat gca ttg cca ttt cca cat agt tca gca atc agc aaa cac tgg     701
Gln Asn Ala Leu Pro Phe Pro His Ser Ser Ala Ile Ser Lys His Trp
        170                 175                 180

gag gct gag cta gct acc ctc aaa ggc aac aat gcc aaa ctc act gca     749
Glu Ala Glu Leu Ala Thr Leu Lys Gly Asn Asn Ala Lys Leu Thr Ala
    185                 190                 195

gcc ctg ctg gag tcc act gcc aat gtg aag cag tgg aag caa cag ctt     797
Ala Leu Leu Glu Ser Thr Ala Asn Val Lys Gln Trp Lys Gln Gln Leu
200                 205                 210                 215

gct gcg tac cag gag gaa gca gag cgg ctg cac aag cgg gtc act gag     845
Ala Ala Tyr Gln Glu Glu Ala Glu Arg Leu His Lys Arg Val Thr Glu
                220                 225                 230

ctg gag tgt gtt agt agt caa gca aac gct gtg cac agc cac aag aca     893
Leu Glu Cys Val Ser Ser Gln Ala Asn Ala Val His Ser His Lys Thr
            235                 240                 245

gag ctg aac cag aca gtg cag gaa ctg gaa gag acc ctg aaa gta aag     941
Glu Leu Asn Gln Thr Val Gln Glu Leu Glu Glu Thr Leu Lys Val Lys
        250                 255                 260

gaa gag gaa ata gaa aga tta aaa caa gaa atc gat aat gcc aga gaa     989
Glu Glu Glu Ile Glu Arg Leu Lys Gln Glu Ile Asp Asn Ala Arg Glu
    265                 270                 275

ctc caa gaa cag agg gac tct ttg act cag aaa cta cag gaa gtt gaa     1037
Leu Gln Glu Gln Arg Asp Ser Leu Thr Gln Lys Leu Gln Glu Val Glu
280                 285                 290                 295

att cga aat aaa gac ctg gag ggg cag ctg tct gac cta gaa cag cgc     1085
Ile Arg Asn Lys Asp Leu Glu Gly Gln Leu Ser Asp Leu Glu Gln Arg
                300                 305                 310

ctg gag aag agc cag aac gaa caa gag gct ttc cgc agt aac ctg aag     1133
Leu Glu Lys Ser Gln Asn Glu Gln Glu Ala Phe Arg Ser Asn Leu Lys
            315                 320                 325

aca ctc cta gaa att ctg gat gga aaa ata ttt gaa cta aca gaa tta     1181
Thr Leu Leu Glu Ile Leu Asp Gly Lys Ile Phe Glu Leu Thr Glu Leu
        330                 335                 340

cga gat aat ttg gcc aag cta ctg gaa tgc agc t aaagagagtg            1225

Arg Asp Asn Leu Ala Lys Leu Leu Glu Cys Ser
    345                 350

aaatttcagt gccaatagat ggagagatgc tgtctgtctt cctaggactg tttgggctcc    1285
gtaccaagat tgcacaaaat tttttgaata tcattcctcc aggaggaggg tgttttgaaa    1345
attggaattg tatatttcag tataaatttt tgaatttagc ttatagctaa ttgggaaaaa    1405
aaaaaaaaaa aaa                                                       1418

<210> 24
<211> 354
<212> PRT
<213> Mus musculus

<400> 24
Met Gly Glu Gln Pro Ile Phe Ser Thr Arg Ala His Val Phe Gln Ile
 1               5                  10                  15
Asp Pro Asn Thr Lys Lys Asn Trp Val Pro Thr Ser Lys His Ala Val
            20                  25                  30
Thr Val Ser Tyr Phe Tyr Asp Ser Thr Arg Asn Val Tyr Arg Ile Ile
        35                  40                  45
Ser Leu Asp Gly Ser Lys Ala Ile Ile Asn Ser Thr Ile Thr Pro Asn
    50                  55                  60
Met Thr Phe Thr Lys Thr Ser Gln Lys Phe Gly Gln Trp Ala Asp Ser
65                  70                  75                  80
Arg Ala Asn Thr Val Tyr Gly Leu Gly Phe Ser Ser Glu His His Leu
                85                  90                  95
Ser Lys Phe Ala Glu Lys Phe Gln Glu Phe Lys Glu Ala Ala Arg Leu
            100                 105                 110
Ala Lys Glu Lys Ser Gln Glu Lys Met Glu Leu Thr Ser Thr Pro Ser
        115                 120                 125
Gln Glu Ser Ala Gly Gly Asp Leu Gln Ser Pro Leu Thr Pro Glu Ser
    130                 135                 140
Ile Asn Gly Thr Asp Asp Glu Arg Thr Pro Asp Val Thr Gln Asn Ser
145                 150                 155                 160
Glu Pro Arg Ala Glu Pro Thr Gln Asn Ala Leu Pro Phe Pro His Ser
                165                 170                 175
Ser Ala Ile Ser Lys His Trp Glu Ala Glu Leu Ala Thr Leu Lys Gly
            180                 185                 190
Asn Asn Ala Lys Leu Thr Ala Ala Leu Leu Glu Ser Thr Ala Asn Val
        195                 200                 205
Lys Gln Trp Lys Gln Gln Leu Ala Ala Tyr Gln Glu Glu Ala Glu Arg
    210                 215                 220
Leu His Lys Arg Val Thr Glu Leu Glu Cys Val Ser Ser Gln Ala Asn
225                 230                 235                 240
Ala Val His Ser His Lys Thr Glu Leu Asn Gln Thr Val Gln Glu Leu
                245                 250                 255
Glu Glu Thr Leu Lys Val Lys Glu Glu Glu Ile Glu Arg Leu Lys Gln
            260                 265                 270
Glu Ile Asp Asn Ala Arg Glu Leu Gln Glu Gln Arg Asp Ser Leu Thr
        275                 280                 285
Gln Lys Leu Gln Glu Val Glu Ile Arg Asn Lys Asp Leu Glu Gly Gln
    290                 295                 300
Leu Ser Asp Leu Glu Gln Arg Leu Glu Lys Ser Gln Asn Glu Gln Glu
305                 310                 315                 320
Ala Phe Arg Ser Asn Leu Lys Thr Leu Leu Glu Ile Leu Asp Gly Lys
                325                 330                 335
Ile Phe Glu Leu Thr Glu Leu Arg Asp Asn Leu Ala Lys Leu Leu Glu
            340                 345                 350
Cys Ser

<210> 25
<211> 1640

<212> DNA
<213> Mus musculus

<220>
<221> CDS
<222> (68)...(1106)
<223> Homer 2a

<400> 25
ggcttggcca cgcgtcgact agtacggggg ggggggcgtc ggagcggccg cacgagcagc 60
gccggag atg gga gaa cag ccc atc ttc acc acg cga gcg cac gtc ttc 109
Met Gly Glu Gln Pro Ile Phe Thr Thr Arg Ala His Val Phe
1               5                   10

cag att gac ccc agc acc aag aag aac tgg gtg ccg gca agc aag cag 157
Gln Ile Asp Pro Ser Thr Lys Lys Asn Trp Val Pro Ala Ser Lys Gln
15                  20                  25                  30

gcc gtc acg gtt tcc tac ttc tat gat gtc acc agg aac agc tat cgg 205
Ala Val Thr Val Ser Tyr Phe Tyr Asp Val Thr Arg Asn Ser Tyr Arg
                35                  40                  45

atc atc agt gtg gat gga gcc aag gtg atc ata aac agc act atc acc 253
Ile Ile Ser Val Asp Gly Ala Lys Val Ile Ile Asn Ser Thr Ile Thr
            50                  55                  60

ccg aac atg act ttc acc aaa acg tca cag aag ttc ggg cag tgg gct 301
Pro Asn Met Thr Phe Thr Lys Thr Ser Gln Lys Phe Gly Gln Trp Ala
        65                  70                  75

gac agc aga gcc aac acc gtg ttc ggt ttg gga ttc tcc tcc gag ctg 349
Asp Ser Arg Ala Asn Thr Val Phe Gly Leu Gly Phe Ser Ser Glu Leu
    80                  85                  90

cag ctc acg aag ttt gca gag aag ttc cag gag gta aga gaa gct gcc 397
Gln Leu Thr Lys Phe Ala Glu Lys Phe Gln Glu Val Arg Glu Ala Ala
95                  100                 105                 110

agg cta gcc aga gac aag tcc cag gag aaa acc gag acc tcc agc aat 445
Arg Leu Ala Arg Asp Lys Ser Gln Glu Lys Thr Glu Thr Ser Ser Asn
                115                 120                 125

cat tcc caa gca tcc agc gtc aat ggc aca gac gac gaa aag gcc tct 493
His Ser Gln Ala Ser Ser Val Asn Gly Thr Asp Asp Glu Lys Ala Ser
            130                 135                 140

cac gcg agc cca gcc gac act cac ctc aag tct gag aat gac aag ctg 541
His Ala Ser Pro Ala Asp Thr His Leu Lys Ser Glu Asn Asp Lys Leu
        145                 150                 155

aag atc gcg ctg aca cag agt gct gcc aat gtg aag aag tgg gag atg 589
Lys Ile Ala Leu Thr Gln Ser Ala Ala Asn Val Lys Lys Trp Glu Met
    160                 165                 170

gag ctg cag acc ctg cgg gag agc aac gcc cgg ctg acc acg gca ctg 637
Glu Leu Gln Thr Leu Arg Glu Ser Asn Ala Arg Leu Thr Thr Ala Leu
175                 180                 185                 190

cag gag tcg gcg gcc agc gtg gag cag tgg aag cgg cag ttc tcc atc 685
Gln Glu Ser Ala Ala Ser Val Glu Gln Trp Lys Arg Gln Phe Ser Ile
                195                 200                 205

tgc agg gac gag aat gac agg ctc cgc agc aag atc gag gag ctg gaa 733

Cys Arg Asp Glu Asn Asp Arg Leu Arg Ser Lys Ile Glu Glu Leu Glu
            210                 215                 220

gaa cag tgc agc gag ata aac agg gag aag gag aag aac aca cag ctg 781
Glu Gln Cys Ser Glu Ile Asn Arg Glu Lys Glu Lys Asn Thr Gln Leu
        225                 230                 235

aag agg agg atc gag gag ctg gag tca gag gtc cga gac aag gag atg 829
Lys Arg Arg Ile Glu Glu Leu Glu Ser Glu Val Arg Asp Lys Glu Met
    240                 245                 250

gag ttg aaa gat ctc cga aaa cag agt gaa atc ata cct cag ctc atg 877
Glu Leu Lys Asp Leu Arg Lys Gln Ser Glu Ile Ile Pro Gln Leu Met
255                 260                 265                 270

tcc gag tgt gaa tat gtc tct gag aag tta gag gcg gcc gaa aga gac 925
Ser Glu Cys Glu Tyr Val Ser Glu Lys Leu Glu Ala Ala Glu Arg Asp
                275                 280                 285

aat caa aac ttg gaa gac aaa gtg cgg tct cta aag aca gac atc gag 973
Asn Gln Asn Leu Glu Asp Lys Val Arg Ser Leu Lys Thr Asp Ile Glu
            290                 295                 300

gag agt aaa tac cga cag cgc cac ctg aag ggg gag ctg aag agc ttc 1021
Glu Ser Lys Tyr Arg Gln Arg His Leu Lys Gly Glu Leu Lys Ser Phe
        305                 310                 315

ctt gag gtg ctg gat gga aag atc gac gac ctc cat gac ttc cgt aga 1069
Leu Glu Val Leu Asp Gly Lys Ile Asp Asp Leu His Asp Phe Arg Arg
    320                 325                 330

gga ctc tcc aag tta ggc aca gat aac tag ggc ggg g cggagcaagt 1116
Gly Leu Ser Lys Leu Gly Thr Asp Asn * Gly Gly
335                 340               345

gtgtgtgaga ggtgtggtag acgtaggaca ttctccattt gcttctgtaa atgcaggtgc 1176
gatctgtctg tctccagacc aattgtgccg tccgctcact cctccagaat aggaaatctc 1236
tcgcttctct ggctttgtga ggtcatggac agctggaagc ttctgactca ggaatccaga 1296
acttggtcta ccttagccgt ttacgcagtc agggcaggga tgtttagatc ttcccttaag 1356
ggctgttgta accctatgaa ccggggatgg gggagtattt tctaatccaa gtaccattat 1416
cctttacagc aggccctcgg gtgccttctg ctgcgtggca ttcagtgtat gtgactctcc 1476
agcaggttct agaccacggg catgtggagg gagcatcttt tcccagtatg cattttgttg 1536
ctttagcaga tgtgacatga cattgtcaac cacaaagttc acactcaaaa actgcacaac 1596
tgacttactc aaaaagaaat aattgtaaaa aaaaaaaaaa aaaa 1640

<210> 26
<211> 345
<212> PRT
<213> Mus musculus

<400> 26
Met Gly Glu Gln Pro Ile Phe Thr Thr Arg Ala His Val Phe Gln Ile
1               5                   10                  15
Asp Pro Ser Thr Lys Lys Asn Trp Val Pro Ala Ser Lys Gln Ala Val
            20                  25                  30
Thr Val Ser Tyr Phe Tyr Asp Val Thr Arg Asn Ser Tyr Arg Ile Ile
        35                  40                  45
Ser Val Asp Gly Ala Lys Val Ile Ile Asn Ser Thr Ile Thr Pro Asn
    50                  55                  60
Met Thr Phe Thr Lys Thr Ser Gln Lys Phe Gly Gln Trp Ala Asp Ser
65                  70                  75                  80
Arg Ala Asn Thr Val Phe Gly Leu Gly Phe Ser Ser Glu Leu Gln Leu
                85                  90                  95

Thr Lys Phe Ala Glu Lys Phe Gln Glu Val Arg Glu Ala Ala Arg Leu
            100                 105                 110
Ala Arg Asp Lys Ser Gln Glu Lys Thr Glu Thr Ser Ser Asn His Ser
        115                 120                 125
Gln Ala Ser Ser Val Asn Gly Thr Asp Asp Glu Lys Ala Ser His Ala
    130                 135                 140
Ser Pro Ala Asp Thr His Leu Lys Ser Glu Asn Asp Lys Leu Lys Ile
145                 150                 155                 160
Ala Leu Thr Gln Ser Ala Ala Asn Val Lys Lys Trp Glu Met Glu Leu
                165                 170                 175
Gln Thr Leu Arg Glu Ser Asn Ala Arg Leu Thr Thr Ala Leu Gln Glu
            180                 185                 190
Ser Ala Ala Ser Val Glu Gln Trp Lys Arg Gln Phe Ser Ile Cys Arg
        195                 200                 205
Asp Glu Asn Asp Arg Leu Arg Ser Lys Ile Glu Glu Leu Glu Glu Gln
    210                 215                 220
Cys Ser Glu Ile Asn Arg Glu Lys Glu Lys Asn Thr Gln Leu Lys Arg
225                 230                 235                 240
Arg Ile Glu Glu Leu Glu Ser Glu Val Arg Asp Lys Glu Met Glu Leu
                245                 250                 255
Lys Asp Leu Arg Lys Gln Ser Glu Ile Ile Pro Gln Leu Met Ser Glu
            260                 265                 270
Cys Glu Tyr Val Ser Glu Lys Leu Glu Ala Ala Glu Arg Asp Asn Gln
        275                 280                 285
Asn Leu Glu Asp Lys Val Arg Ser Leu Lys Thr Asp Ile Glu Glu Ser
    290                 295                 300
Lys Tyr Arg Gln Arg His Leu Lys Gly Glu Leu Lys Ser Phe Leu Glu
305                 310                 315                 320
Val Leu Asp Gly Lys Ile Asp Asp Leu His Asp Phe Arg Arg Gly Leu
                325                 330                 335
Ser Lys Leu Gly Thr Asp Asn Gly Gly
            340                 345

<210> 27
<211> 1673
<212> DNA
<213> Mus musculus

<220>
<221> CDS
<222> (68)...(1130)
<223> Homer 2b

<400> 27
ggcttggcca cgcgtcgact agtacggggg ggggggcgtc ggagcggccg cacgagcagc      60
gccggag atg gga gaa cag ccc atc ttc acc acg cga gcg cac gtc ttc      109
        Met Gly Glu Gln Pro Ile Phe Thr Thr Arg Ala His Val Phe
         1               5                  10

cag att gac ccc agc acc aag aag aac tgg gtg ccg gca agc aag cag      157
Gln Ile Asp Pro Ser Thr Lys Lys Asn Trp Val Pro Ala Ser Lys Gln
 15                  20                  25                  30

gcc gtc acg gtt tcc tac ttc tat gat gtc acc agg aac agc tat cgg      205
Ala Val Thr Val Ser Tyr Phe Tyr Asp Val Thr Arg Asn Ser Tyr Arg
                 35                  40                  45

atc atc agt gtg gat gga gcc aag gtg atc ata aac agc act atc acc      253
Ile Ile Ser Val Asp Gly Ala Lys Val Ile Ile Asn Ser Thr Ile Thr
             50                  55                  60

ccg aac atg act ttc acc aaa acg tca cag aag ttc ggg cag tgg gct      301
Pro Asn Met Thr Phe Thr Lys Thr Ser Gln Lys Phe Gly Gln Trp Ala

        65                  70                  75

gac agc aga gcc aac acc gtg ttc ggt ttg gga ttc tcc tcc gag ctg 349
Asp Ser Arg Ala Asn Thr Val Phe Gly Leu Gly Phe Ser Ser Glu Leu
    80                  85                  90

cag ctc acg aag ttt gca gag aag ttc cag gag gta aga gaa gct gcc 397
Gln Leu Thr Lys Phe Ala Glu Lys Phe Gln Glu Val Arg Glu Ala Ala
95                  100                 105                 110

agg cta gcc aga gac aag tcc cag gag aaa acc gag acc tcc agc aat 445
Arg Leu Ala Arg Asp Lys Ser Gln Glu Lys Thr Glu Thr Ser Ser Asn
                115                 120                 125

cat tcc caa gaa tct ggg tgt gaa acc ccg tct tcc act cag gca tcc 493
His Ser Gln Glu Ser Gly Cys Glu Thr Pro Ser Ser Thr Gln Ala Ser
            130                 135                 140

agc gtc aat ggc aca gac gac gaa aag gcc tct cac gcg agc cca gcc 541
Ser Val Asn Gly Thr Asp Asp Glu Lys Ala Ser His Ala Ser Pro Ala
        145                 150                 155

gac act cac ctc aag tct gag aat gac aag ctg aag atc gcg ctg aca 589
Asp Thr His Leu Lys Ser Glu Asn Asp Lys Leu Lys Ile Ala Leu Thr
    160                 165                 170

cag agt gct gcc aat gtg aag aag tgg gag atg gag ctg cag acc ctg 637
Gln Ser Ala Ala Asn Val Lys Lys Trp Glu Met Glu Leu Gln Thr Leu
175                 180                 185                 190

cgg gag agc aac gcc cgg ctg acc acg gca ctg cag gag tcg gcg gcc 685
Arg Glu Ser Asn Ala Arg Leu Thr Thr Ala Leu Gln Glu Ser Ala Ala
                195                 200                 205

agc gtg gag cag tgg aag cgg cag ttc tcc atc tgc agg gac gag aat 733
Ser Val Glu Gln Trp Lys Arg Gln Phe Ser Ile Cys Arg Asp Glu Asn
            210                 215                 220

gac agg ctc cgc agc aag atc gag gag ctg gaa gaa cag tgc agc gag 781
Asp Arg Leu Arg Ser Lys Ile Glu Glu Leu Glu Glu Gln Cys Ser Glu
        225                 230                 235

ata aac agg gag aag gag aag aac aca cag ctg aag agg agg atc gag 829
Ile Asn Arg Glu Lys Glu Lys Asn Thr Gln Leu Lys Arg Arg Ile Glu
    240                 245                 250

gag ctg gag tca gag gtc cga gac aag gag atg gag ttg aaa gat ctc 877
Glu Leu Glu Ser Glu Val Arg Asp Lys Glu Met Glu Leu Lys Asp Leu
255                 260                 265                 270

cga aaa cag agt gaa atc ata cct cag ctc atg tcc gag tgt gaa tat 925
Arg Lys Gln Ser Glu Ile Ile Pro Gln Leu Met Ser Glu Cys Glu Tyr
                275                 280                 285

gtc tct gag aag tta gag gcg gcc gaa aga gac aat caa aac ttg gaa 973
Val Ser Glu Lys Leu Glu Ala Ala Glu Arg Asp Asn Gln Asn Leu Glu
            290                 295                 300

gac aaa gtg cgg tct cta aag aca gac atc gag gag agt aaa tac cga 1021
Asp Lys Val Arg Ser Leu Lys Thr Asp Ile Glu Glu Ser Lys Tyr Arg
        305                 310                 315

cag cgc cac ctg aag ggg gag ctg aag agc ttc ctt gag gtg ctg gat 1069

Gln Arg His Leu Lys Gly Glu Leu Lys Ser Phe Leu Glu Val Leu Asp
    320                 325                 330

gga aag atc gac gac ctc cat gac ttc cgt aga gga ctc tcc aag tta      1117
Gly Lys Ile Asp Asp Leu His Asp Phe Arg Arg Gly Leu Ser Lys Leu
335                 340                 345                 350

ggc aca gat aac t agggcggggc ggagcaagtg tgtgtgagag gtgtggtaga      1170
Gly Thr Asp Asn

cgtaggacat tctccatttg cttctgtaaa tgcaggtgcg atctgtctgt ctccagacca   1230
attgtgccgt ccgctcactc ctccagaata ggaaatctct cgcttctctg gctttgtgag   1290
gtcatggaca gctggaagct tctgactcag gaatccagaa cttggtctac cttagccgtt   1350
tacgcagtca gggcagggat gtttagatct tcccttaagg gctgttgtaa ccctatgaac   1410
cggggatggg ggagtatttt ctaatccaag taccattatc ctttacagca ggccctcggg   1470
tgccttctgc tgcgtggcat tcagtgtatg tgactctcca gcaggttcta gaccacgggc   1530
atgtggaggg agcatctttt cccagtatgc attttgttgc tttagcagat gtgacatgac   1590
attgtcaacc acaaagttca cactcaaaaa ctgcacaact gacttactca aaaagaaata   1650
attgtaaaaa aaaaaaaaaa aaa                                           1673

<210> 28
<211> 354
<212> PRT
<213> Mus musculus

<400> 28
Met Gly Glu Gln Pro Ile Phe Thr Thr Arg Ala His Val Phe Gln Ile
1               5                   10                  15
Asp Pro Ser Thr Lys Lys Asn Trp Val Pro Ala Ser Lys Gln Ala Val
            20                  25                  30
Thr Val Ser Tyr Phe Tyr Asp Val Thr Arg Asn Ser Tyr Arg Ile Ile
        35                  40                  45
Ser Val Asp Gly Ala Lys Val Ile Ile Asn Ser Thr Ile Thr Pro Asn
    50                  55                  60
Met Thr Phe Thr Lys Thr Ser Gln Lys Phe Gly Gln Trp Ala Asp Ser
65                  70                  75                  80
Arg Ala Asn Thr Val Phe Gly Leu Gly Phe Ser Ser Glu Leu Gln Leu
                85                  90                  95
Thr Lys Phe Ala Glu Lys Phe Gln Glu Val Arg Glu Ala Ala Arg Leu
            100                 105                 110
Ala Arg Asp Lys Ser Gln Glu Lys Thr Glu Thr Ser Ser Asn His Ser
        115                 120                 125
Gln Glu Ser Gly Cys Glu Thr Pro Ser Ser Thr Gln Ala Ser Ser Val
    130                 135                 140
Asn Gly Thr Asp Asp Glu Lys Ala Ser His Ala Ser Pro Ala Asp Thr
145                 150                 155                 160
His Leu Lys Ser Glu Asn Asp Lys Leu Lys Ile Ala Leu Thr Gln Ser
                165                 170                 175
Ala Ala Asn Val Lys Lys Trp Glu Met Glu Leu Gln Thr Leu Arg Glu
            180                 185                 190
Ser Asn Ala Arg Leu Thr Thr Ala Leu Gln Glu Ser Ala Ala Ser Val
        195                 200                 205
Glu Gln Trp Lys Arg Gln Phe Ser Ile Cys Arg Asp Glu Asn Asp Arg
    210                 215                 220
Leu Arg Ser Lys Ile Glu Glu Leu Glu Glu Gln Cys Ser Glu Ile Asn
225                 230                 235                 240
Arg Glu Lys Glu Lys Asn Thr Gln Leu Lys Arg Arg Ile Glu Glu Leu
                245                 250                 255
Glu Ser Glu Val Arg Asp Lys Glu Met Glu Leu Lys Asp Leu Arg Lys
            260                 265                 270
Gln Ser Glu Ile Ile Pro Gln Leu Met Ser Glu Cys Glu Tyr Val Ser
        275                 280                 285

Glu Lys Leu Glu Ala Ala Glu Arg Asp Asn Gln Asn Leu Glu Asp Lys
    290                 295                 300
Val Arg Ser Leu Lys Thr Asp Ile Glu Glu Ser Lys Tyr Arg Gln Arg
305                 310                 315                 320
His Leu Lys Gly Glu Leu Lys Ser Phe Leu Glu Val Leu Asp Gly Lys
                325                 330                 335
Ile Asp Asp Leu His Asp Phe Arg Arg Gly Leu Ser Lys Leu Gly Thr
            340                 345                 350
Asp Asn

<210> 29
<211> 2297
<212> DNA
<213> Mus musculus

<400> 29
tccacagcca gggaacagcc aatcttcagc acccgggcgc acgtattcca gatcgacccc 60
actacaaagc ggaactggat ccccgccggc aagcacgcac ttaccgtgtc ctatttctat 120
gatgcaaccc gaaatgtgta ccgcatcatc agcatcgggg gtgccaaggc catcatcaac 180
agcactgtca ctcccaacat gaccttcacc aaaacctctc agaagttcgg gcaatgggca 240
gacagtcgag ccaacactgt ctacggccta ggctttgcct ctgaacagca gctgacccag 300
tttgctgaga agtttcagga ggtgaaagaa gctgccaggc tggctcgaga gaaatctcaa 360
gatggtggag aattcactag tactggcctg gcccttgcct cccatcaggt tcctccaagc 420
cccttggtca gcaccaatgg tccaggcgag gaaaagctgt tccgtagcca gagtgcggac 480
acccctggcc ccaccgagcg ggaacggttg aagaagatgc tgtcagaagg ctctgtaggg 540
gaagtccagt gggaagcaga gttcttcgcg cttcaggaca gcaaccagag gttggcggga 600
gcccttcggg aagcgaacgc agcggccact cagtggaggc aacaactgga ggtccaacgt 660
gcagaggctg aactcttgag gcagcgggta gcagagctgg aggcccaggt ggctgtagag 720
ccagtccggg caggagagaa agaagcaacc agccagtcgg tggagcagct ggaggctcgg 780
gtgcagacca aggaccagga gatccagact ttgaagaatc agagcactgg cacccgagag 840
gctccagaca ctgccgagcg cgaagagaca cagcagcaag ttcaggacct ggagacccgg 900
aatgcagagc tggagcagca gctgcgggcg atggagtgca acctggagga ggcgcgggcc 960
gagcgggagc gcgcacgggc ggaggtgggc cgggctgcgc agctgctgga tgttcggctg 1020
tttgagctca gcgagctgcg tgaaggcctg gcacgcctgg cagaggcagc accctagtct 1080
gccatggagt gtctgcggcc tcaaggcgcc ctggcagggg ccaggggacc ccagctgtct 1140
ctgagctttg cactgtgtag agttttctag aatccttggg caatgcttct acccaggtta 1200
catttctacg tgtggcgttg ctgtccctgg ctgctgctgc cctgcgcccc agggacactg 1260
cgagggaagg ctgcactagt catccccatg gggcaacaga ggctttggga tcctgagacc 1320
tgaaggccct gtactcatcc caccccattc tcaagtcaga ctgacaactt caaagagtgt 1380
ttactgaagt caggggccac cagcaccagg tttacagctc agtcctgagc ctcagcctgg 1440
gctggctctt ggggccgaga tctgggagga cgcgaccgtc ggacagtgct ccctgctttc 1500
tgccgccgaa gtgtctgccc cactttctcc ttgaagcgtc ggttttgttg cttgatcttg 1560
gccagctcag ctttgcgttt ggcctccagg tctgggtcct gcggaaggga gctgagaatg 1620
taactgggca gcttcccagg gactggctcc cccaccccta cccgtcccca ggtcccaccc 1680
acccttactg gccacactct tatgcctgtc cctgcatacc catgcctccc tatactacct 1740
tcccctccag gatcatctgt ttccgcttgt tgatctcttt cttttcatca aaatgcgaag 1800
cctccagttt ctaggggtgg ggaggggaac aggtcagtca ggcctggggc aggaagcccc 1860
gcccacctca ccccactcca ccctaccctg acaggctggc cacacttact atttcgcact 1920
cccttcgcac tacgttgacc tgcgtgagga tttgtagaac ctcagcctcc tccaccacca 1980
gctctgccag ctgctgctct gcagggacag gaaacactga gttgggctgg gagtgcaacc 2040
agccctctgc acccccagct ctggatgtct ggatccaacc aaatgtggac tgatgatatt 2100
tagaaaaagc aaaatgctgc caagcttggc agcacatgct tgtcatcaca gcactgggag 2160
gtggaggcag gggcatcact cgtttcagct gagttccagg ccagctctgt agagcaagaa 2220
tctgtctcaa attaatgact gaataaacaa atgaacaagt aaaaaaaaaa aaaaaaaaaa 2280
aaaaaaaaaa aaaaaaa 2297

<210> 30
<211> 359
<212> PRT
<213> Mus musculus

<400> 30

Met Ser Thr Ala Arg Glu Gln Pro Ile Phe Ser Thr Arg Ala His Val
1               5                   10                  15
Phe Gln Ile Asp Pro Thr Thr Lys Arg Asn Trp Ile Pro Ala Gly Lys
            20                  25                  30
His Ala Leu Thr Val Ser Tyr Phe Tyr Asp Ala Thr Arg Asn Val Tyr
        35                  40                  45
Arg Ile Ile Ser Ile Gly Gly Ala Lys Ala Ile Ile Asn Ser Thr Val
    50                  55                  60
Thr Pro Asn Met Thr Phe Thr Lys Thr Ser Gln Lys Phe Gly Gln Trp
65                  70                  75                  80
Ala Asp Ser Arg Ala Asn Thr Val Tyr Gly Leu Gly Phe Ala Ser Glu
                85                  90                  95
Gln Gln Leu Thr Gln Phe Ala Glu Lys Phe Gln Glu Val Lys Glu Ala
            100                 105                 110
Ala Arg Leu Ala Arg Glu Lys Ser Gln Asp Gly Gly Glu Phe Thr Ser
        115                 120                 125
Thr Gly Leu Ala Leu Ala Ser His Gln Val Pro Pro Ser Pro Leu Val
    130                 135                 140
Ser Thr Asn Gly Pro Gly Glu Glu Lys Leu Phe Arg Ser Gln Ser Ala
145                 150                 155                 160
Asp Thr Pro Gly Pro Thr Glu Arg Glu Arg Leu Lys Lys Met Leu Ser
                165                 170                 175
Glu Gly Ser Val Gly Glu Val Gln Trp Glu Ala Glu Phe Phe Ala Leu
            180                 185                 190
Gln Asp Ser Asn Gln Arg Leu Ala Gly Ala Leu Arg Glu Ala Asn Ala
        195                 200                 205
Ala Ala Thr Gln Trp Arg Gln Gln Leu Glu Val Gln Arg Ala Glu Ala
    210                 215                 220
Glu Leu Leu Arg Gln Arg Val Ala Glu Leu Glu Ala Gln Val Ala Val
225                 230                 235                 240
Glu Pro Val Arg Ala Gly Glu Lys Glu Ala Thr Ser Gln Ser Val Glu
                245                 250                 255
Gln Leu Glu Ala Arg Val Gln Thr Lys Asp Gln Glu Ile Gln Thr Leu
            260                 265                 270
Lys Asn Gln Ser Thr Gly Thr Arg Glu Ala Pro Asp Thr Ala Glu Arg
        275                 280                 285
Glu Glu Thr Gln Gln Gln Val Gln Asp Leu Glu Thr Arg Asn Ala Glu
    290                 295                 300
Leu Glu Gln Gln Leu Arg Ala Met Glu Cys Asn Leu Glu Glu Ala Arg
305                 310                 315                 320
Ala Glu Arg Glu Arg Ala Arg Ala Glu Val Gly Arg Ala Ala Gln Leu
                325                 330                 335
Leu Asp Val Arg Leu Phe Glu Leu Ser Glu Leu Arg Glu Gly Leu Ala
            340                 345                 350
Arg Leu Ala Glu Ala Ala Pro
        355

<210> 31
<211> 5798
<212> DNA
<213> Rattus norvegicus

<220>
<221> CDS
<222> (567)...(1125)
<223> Homer 1a

<400> 31
   gtgctgtgca catcgcgagc ggctggggtt tgcacttcga gatttcttct ttataatttt      60
   ttttttttaa tgtaagggag acagtggaat tgctacccgt agaattttta ttcaagtgca     120
   cgtcgcgttg ggttgcacgc tccaccccca gggacctggt gtggtgaaat ttgaacccac     180
   cgccttagcc caaaggccga gtaacctggc tgcttgagtg tcgtggaaga cgtgagcgaa     240
   atgatcagcg aactcatttt ttatcagact cgctgaagct ggcttttgcg tttttctaca 300
   cgtacactaa ttttatggaa tagttaaagt gctatattct ccgcgcaacc ttttcaaatt 360
   ccaaatgttt gaacgttttg gtgtcagcgc gagtgaaatc attttaccga caagaactaa 420
   ctgaattgtc tgcctcgttg agttgcctcc ggaaaagatc tcgggggtgg aaaagcaact 480
   gcaaaataac agacggagaa aattccttgg aagttatttc tgtagcataa gagcagaaac 540
   ttcagagcaa gttttcattg ggcaaa atg ggg gaa caa cct atc ttc agc act 593
   Met Gly Glu Gln Pro Ile Phe Ser Thr
   1 5
   cga gct cat gtc ttc cag atc gac cca aac aca aag aag aac tgg gta 641
   Arg Ala His Val Phe Gln Ile Asp Pro Asn Thr Lys Lys Asn Trp Val
   10 15 20 25
   ccc acc agc aag cat gca gtt act gtg tct tat ttc tat gac agc aca 689
   Pro Thr Ser Lys His Ala Val Thr Val Ser Tyr Phe Tyr Asp Ser Thr
   30 35 40
   agg aat gtg tat agg ata atc agt cta gac ggc tca aag gca ata ata 737
   Arg Asn Val Tyr Arg Ile Ile Ser Leu Asp Gly Ser Lys Ala Ile Ile
   45 50 55
   aat agc acc atc act cca aac atg aca ttt act aaa aca tct caa aag 785
   Asn Ser Thr Ile Thr Pro Asn Met Thr Phe Thr Lys Thr Ser Gln Lys
   60 65 70
   ttt ggc caa tgg gct gat agc cgg gca aac act gtt tat gga ctg gga 833
   Phe Gly Gln Trp Ala Asp Ser Arg Ala Asn Thr Val Tyr Gly Leu Gly
   75 80 85
   ttc tcc tct gag cat cat ctc tca aaa ttt gca gaa aag ttt cag gaa 881
   Phe Ser Ser Glu His His Leu Ser Lys Phe Ala Glu Lys Phe Gln Glu
   90 95 100 105
   ttt aaa gaa gct gct cgg ctg gca aag gag aag tcg cag gag aag atg 929
   Phe Lys Glu Ala Ala Arg Leu Ala Lys Glu Lys Ser Gln Glu Lys Met
   110 115 120
   gaa ctg acc agt acc cct tca cag gaa tca gca gga gga gat ctt cag 977
   Glu Leu Thr Ser Thr Pro Ser Gln Glu Ser Ala Gly Gly Asp Leu Gln
   125 130 135
   tct cct tta aca cca gaa agt atc aat ggg aca gat gat gag aga aca 1025
   Ser Pro Leu Thr Pro Glu Ser Ile Asn Gly Thr Asp Asp Glu Arg Thr
   140 145 150
   ccc gat gtg aca cag aac tca gag cca agg gct gag cca gct cag aat 1073
   Pro Asp Val Thr Gln Asn Ser Glu Pro Arg Ala Glu Pro Ala Gln Asn
   155 160 165
   gca ttg cca ttt tca cat agg tac aca ttc aat tca gca atc atg att 1121
   Ala Leu Pro Phe Ser His Arg Tyr Thr Phe Asn Ser Ala Ile Met Ile
   170 175 180 185
   aaa t gagatggata aatatgaagt tcatttggtt tcagaaactc ttgagtgaaa 1175
   Lys
   aatcccaggt cagacttctt taattaatta attgtttgct gttgctcaga ttgactgaat 1235
   atttccatta tctgtgtaga aaaaggaacg ttaattatag gagaaacttt ttcaatggac 1295
   aaaacattcc attctatcta tattttaaag atcccttttg ctaaccagtt ttctgatttt 1355
   ctacatgtta cgtaagacta ataacttgtg attaggatca atggactcct gctccaaagg 1415
   aaagccttgc cacaggccca cagaggtgcc acagaggacg gggccaggca ggaacccgtc 1475
   agcattgaag gttgtttttg tatgccaaca ggaggaaagc ttgagttgct gctgattctt 1535
   aaaagaattc tgtattctaa aagatacaca tcatgttcta aatgcatttt aaactagtga 1595
   cattagttat tgggcatact gtggtattac tagactacaa agaggaatat gaagtggcac 1655
   cattgaaagt atttttttaa aaagcctgtc taccttaaca ctaattttta cccttattta 1715
   aatgcttttt actaaacagt tttaggtaaa attaagaaaa cagttttgtt gactgcacat 1775
   cttttagaag gaccaacttt tagagaatta cattctttga cagattaaaa attgcaaagt 1835
   gagatatttc aaactcttaa gtgagtttta ttgccgttgg actgcattaa tacggacata 1895
   cgattaaact tagtagacca acactgaggg atctccttac caggctgcag aacaaggaaa 1955
   ttaagcaata aatgggactt gtgaatggaa ggacactcta ctgctagtgc tagtaattct 2015
   gcataagatg gtatacattt tgaagaaagc tgcttttaat tacttttaat aatgatttta 2075
   attactctag tgcaagtgct tcctcgagct ataaaggtag ctgagcacag cagaccttta 2135
   ctccctcagt ctgacttctg tactcatatt catttagtga acatagtctt ttaacagaag 2195
   accacagttc tttgatagcg ttacaaaact tacgttattt aaacgttata aagaacgtta 2255
   ttgtaggata aaatgttaaa aactgtgtca aggacaggaa gaattcctat ctattaagta 2315
   gtggtttcca cccccactta agactgaact gcactgaacg gtaactgtat acttggtttg 2375
   acacctcgac tgagccatgc gcactgaata ctgtgacatt gaggagtaag aacttttaaa 2435
   tttaacattt aaagaagcta cttgcagttt atgcaccgaa atttgtctaa atgttctcca 2495
   ttttgctgac cccgttgtat tcatactgct ccccagagcc tagagttgtc ctcatcctga 2555
   cttcctgtgc ctgagtgtct gagaggagtc actttcactg tgaagacact gcttctgcgc 2615
   ctcgtaggga ggacttgaca gtgctcccgt agaaatccta cattatttca acctcagagt 2675
   tacagtaaag gcaggttata accagtcttt cttattattt taagaatttc cagccctagt 2735
   gttttatgaa agtattcctg tgaatttgac accttatgat cctatattca tctaattcct 2795
   taatgaaata aaaatgtcca tgtgaggtag gttatttaca gcgattgcag gagacatggt 2855
   gttcttcaga gttcccaaac caggatagtt tcaaataggt ttttcatggc ttctgacgaa 2915
   gaagaccata aagttccctg cagtgtgtca gtgatgtgca agctgaatta gtgcgaagtg 2975
   tcacactgtg aaagcacgtg cttttggctt attatgagaa aacgaaatct ttaaattcag 3035
   tttatgtgtc ttaggtccag tttactttga tttgactact cagttcttct gaccccacct 3095
   agtatgtatg tatatgtgtg tgtatgtgtg tgtatgtctg tatgtatata catacatata 3155
   cacacacatt gtatacatat gctatatata cagtatgtgt atatatatac tatatatgaa 3215
   tatatgaata tatatattca attagttaat agtacattta agccaaatat ccaacataag 3275
   cacactatgt aagtatctat ctggaaagac ctatatagaa ttgagatcaa catttcatga 3335
   gttagaaaca aaggatttta taattaatat tacttaagtc taaagtaccc atatatttaa 3395
   attagatatg caatttttcc ctcttggcaa agaaagacaa aaatcttgtg tttagagatg 3455
   atgtagattg tcatttttgc ctttccttcc tgagtacttg ttttaacaac aacaaaaaaa 3515
   gactagttta agaaaaggga ttgtccagta tttttctgct ttgttaagtc taattttact 3575
   gttaaacaga gagcagaatc actggagtac tgggggggtt ttttgttgtt tttttttttt 3635
   tcttttctgt ttttttcgga gctggggagc gaacccaggg ccttgcgctc actaggcaag 3695
   cactctaccg ctgagctaaa tccccaaccc ctggagtatc tgttttaaaa gaaagccagg 3755
   accgttatga tggccatacc cagggtacat agtgaaaaca acagagacca agcaatgaga 3815
   gtgtgagagt accaatccac cagtactgct gccggacatg gcagctgcct gtgcttttct 3875
   gaagagtcat agtgtatgct aagtctagaa ccattactta gtaaagaggc tatgactttt 3935
   atttgggcct gacaatttta gtggtgtggt catagtctat tctgtatttg taagctttat 3995
   ttttaaatta ctgtgttgat ttaggaacac aagaaatgtt tttattttta attatgagtg 4055
   tatataaggt tttcagatat gcacagacta caataataga ctcccatgga gataccactt 4115
   cagccttaac agtcagggag aaggagcctc actttatcac cgcactcacc ctgctctcca 4175
   ctgatctgtt gttactgcgg tgtggaggtt cacacgcatg caggtcttca cacatgatgg 4235
   gtaggcccgc accaagtgag cctctcccag ccttgctgtt tcgttttttt attttaatct 4295
   tacatgtatg ggtgttttgc atccaggcat gtcatgcctg tgtccacaga agccagaaag 4355
   ggtatcagat tccctaaaac tggagttctc gatgatcgtg agcgagccat tgtgggtgct 4415
   gggaactgaa gctgggtcct ctacaagagc agccagcgct cttaaccatt gagccactat 4475
   ctgccctgtg tttgttttat ttatttattt atttatttat ttatttattt atttatttat 4535
   ttatttattt atttatttat tggttctttt tttttggact ggggaccgaa gccagggcct 4595
   tgcacttcct aggcaagcgc tctaccactg agctaaatcc ccaacccctt gttttatttt 4655
   taaagcaaac gagatacata atttcaacca tgataattta agattatctt gaactcttaa 4715
   ggaaatgtat atactaagct attatagttt ttattttccc taattcagtg gcataatacc 4775
   ttaccttgag tcgtttacta ctttctttgg tttctaaaaa ctctactgct aaattacaat 4835
   gtaaaaacat agggctcgta tatactgtag agtgctgtag atgtcctcgt catcaactat 4895
   gcaataacag tctgatcgac acatttcagg agcgatcact ctttggtgtg cttctttaaa 4955
   tactttcaga agcttaggat gtgcaaagca ggaagaccgt gggtgtaaat gtttacttat 5015
   ttctttgaga gtgttagtaa gtcttttcta aattgctttt ctcttcaaaa ttatcgttaa 5075
   cttaaatgat aattatcttt gaggttaaac agaagctcat tgacaaacta aagtgacttt 5135
   ttagggcatt ctttgagatc atagtcttat atctggggac taaaatgtca ttagacccta 5195
   atagactaac ttgtatgttt gtgtggggaa acgttttcct ctctcattca aggtaactgt 5255
   ttgctgcctg ttgttacttg tgtagcattc tagaaaatgg ctaggttttt tataagattt 5315
   aagacaatag aagtagtttt atattattat agttctgttg gaatgtgatc ctgaaattat 5375
   tactgaaaat tagaattttt atttcgctaa tgacaacctt gactctcaga gatgcagtgt 5435
   aaattgatac ctcatctttc cgagagttca gagcacaggg cggcagtatg tgaagctgct 5495
   tttgcactga cgcattttga taagtttggc tactgtaatg gtaaaaggct cctcaggcac 5555
   tgactgcatt tgggttcttc cgatggggga tgatccgttc tcgtggtgct gctggactta 5615
   tgcattttgg aggtactgca tgtatcttcc acactgcttg acattttctc tgatctgtgt 5675
   gtttgcacca actcattaaa agaaatatgc agaaatatct tctaattcgt tgatcttcgc 5735
   tgtatgacag ttataatatt aaacacttgg gttgatcaaa aaaaaaaaaa aaaaaaaaaa 5795
   aaa 5798

<210> 32
<211> 186
<212> PRT
<213> Rattus norvegicus

<400> 32
Met Gly Glu Gln Pro Ile Phe Ser Thr Arg Ala His Val Phe Gln Ile
1               5                   10                  15
Asp Pro Asn Thr Lys Lys Asn Trp Val Pro Thr Ser Lys His Ala Val
            20                  25                  30
Thr Val Ser Tyr Phe Tyr Asp Ser Thr Arg Asn Val Tyr Arg Ile Ile
        35                  40                  45
Ser Leu Asp Gly Ser Lys Ala Ile Ile Asn Ser Thr Ile Thr Pro Asn
    50                  55                  60
Met Thr Phe Thr Lys Thr Ser Gln Lys Phe Gly Gln Trp Ala Asp Ser
65                  70                  75                  80
Arg Ala Asn Thr Val Tyr Gly Leu Gly Phe Ser Ser Glu His His Leu
                85                  90                  95
Ser Lys Phe Ala Glu Lys Phe Gln Glu Phe Lys Glu Ala Ala Arg Leu
            100                 105                 110
Ala Lys Glu Lys Ser Gln Glu Lys Met Glu Leu Thr Ser Thr Pro Ser
        115                 120                 125
Gln Glu Ser Ala Gly Gly Asp Leu Gln Ser Pro Leu Thr Pro Glu Ser
    130                 135                 140
Ile Asn Gly Thr Asp Asp Glu Arg Thr Pro Asp Val Thr Gln Asn Ser
145                 150                 155                 160
Glu Pro Arg Ala Glu Pro Ala Gln Asn Ala Leu Pro Phe Ser His Arg
                165                 170                 175
Tyr Thr Phe Asn Ser Ala Ile Met Ile Lys
            180                 185

<210> 33
<211> 3339
<212> DNA
<213> Rattus norvegicus

<220>
<221> CDS
<222> (740)...(1802)
<223> Homer 1b

<400> 33
ctagtggatc ccccgggctg caggaattct gcggccgcaa caccgcactg tggtggacag 60
tgagggccgg agagagacca cagtgaccca tcaagaagcc catgacagtt ccagaagtga 120
tccagatcct ccaagatctt cagctttgga tgatcccttt tccatcctgg acctgcttct 180
aggacgttgg tttcggtccc gatagctttc ttgaacttca gaggccttca ggtccttccc 240
accccctccc tccctgttgc ccattgccaa taagcatagc ttttgctgtc atcctggggt 300
cttaaatgtg tggaaccccc ccagggacct ggtgtggtga aatttgaacc caccgcctta 360
gcccaaaggc cgagtaacct ggctgcttga gtgtcgtgga agacgtgagc gaaatgatca 420
gcgaactcat tttttatcag actcgctgaa gctggctttt gcgtttttct acacgtacac 480
taattttatg gaatagttaa agtgctatat tctccgcgca accttttcaa attccaaatg 540
tttgaacgtt ttggtgtcag cgcgagtgaa atcattttac cgacaagaac taactgaatt 600
gtctgcctcg ttgagttgcc tccggaaaag atctcggggg tggaaaagca actgcaaaat 660

aacagacgga gaaaattcct tggaagttat ttctgtagca taagagcaga aacttaagag 720
caagttttca ttgggcaaa atg ggg gaa caa cct atc ttc agc act cga gct 772
Met Gly Glu Gln Pro Ile Phe Ser Thr Arg Ala
1               5                   10

cat gtc ttc cag atc gac cca aac aca aag aag aac tgg gta ccc acc 820
His Val Phe Gln Ile Asp Pro Asn Thr Lys Lys Asn Trp Val Pro Thr
            15                  20                  25

agc aag cat gca gtt act gtg tct tat ttc tat gac agc aca agg aat 868
Ser Lys His Ala Val Thr Val Ser Tyr Phe Tyr Asp Ser Thr Arg Asn
        30                  35                  40

gtg tat agg ata atc agt cta gac ggc tca aag gca ata ata aat agc 916
Val Tyr Arg Ile Ile Ser Leu Asp Gly Ser Lys Ala Ile Ile Asn Ser
    45                  50                  55

acc atc act cca aac atg aca ttt act aaa aca tct caa aag ttt ggc 964
Thr Ile Thr Pro Asn Met Thr Phe Thr Lys Thr Ser Gln Lys Phe Gly
60                  65                  70                  75

caa tgg gct gat agc cgg gca aac act gtt tat gga ctg gga ttc tcc 1012
Gln Trp Ala Asp Ser Arg Ala Asn Thr Val Tyr Gly Leu Gly Phe Ser
                80                  85                  90

tct gag cat cat ctc tca aaa ttt gca gaa aag ttt cag gaa ttt aaa 1060
Ser Glu His His Leu Ser Lys Phe Ala Glu Lys Phe Gln Glu Phe Lys
            95                  100                 105

gaa gct gct cgg ctg gca aag gag aag tcg cag gag aag atg gaa ctg 1108
Glu Ala Ala Arg Leu Ala Lys Glu Lys Ser Gln Glu Lys Met Glu Leu
        110                 115                 120

acc agt acc cct tca cag gaa tca gca gga gga gat ctt cag tct cct 1156
Thr Ser Thr Pro Ser Gln Glu Ser Ala Gly Gly Asp Leu Gln Ser Pro
    125                 130                 135

tta aca cca gaa agt atc aat ggg aca gat gat gag aga aca ccc gat 1204
Leu Thr Pro Glu Ser Ile Asn Gly Thr Asp Asp Glu Arg Thr Pro Asp
140                 145                 150                 155

gtg aca cag aac tca gag cca agg gct gag cca gct cag aat gca ttg 1252
Val Thr Gln Asn Ser Glu Pro Arg Ala Glu Pro Ala Gln Asn Ala Leu
                160                 165                 170

cca ttt tca cat agt tca gcc atc agc aaa cac tgg gag gct gaa cta 1300
Pro Phe Ser His Ser Ser Ala Ile Ser Lys His Trp Glu Ala Glu Leu
            175                 180                 185

gcc acg ctc aag ggg aac aat gcc aag ctc acc gca gcg ctg ctg gag 1348
Ala Thr Leu Lys Gly Asn Asn Ala Lys Leu Thr Ala Ala Leu Leu Glu
        190                 195                 200

tcc act gcc aac gtg aag cag tgg aag caa cag ctg gct gcc tac cag 1396
Ser Thr Ala Asn Val Lys Gln Trp Lys Gln Gln Leu Ala Ala Tyr Gln
    205                 210                 215

gag gag gca gag cgg ctg cac aag cgg gtc acg gag ctg gaa tgt gtt 1444
Glu Glu Ala Glu Arg Leu His Lys Arg Val Thr Glu Leu Glu Cys Val
220                 225                 230                 235

agt agt caa gca aac gcg gtg cac agc cac aag aca gag ctg agt cag 1492
Ser Ser Gln Ala Asn Ala Val His Ser His Lys Thr Glu Leu Ser Gln

240                 245                 250

aca gtg cag gag ctg gaa gag acc cta aaa gta aag gaa gag gaa ata      1540
Thr Val Gln Glu Leu Glu Glu Thr Leu Lys Val Lys Glu Glu Glu Ile
            255                 260                 265

gaa aga tta aaa caa gaa att gat aac gcc aga gaa ctt caa gaa cag      1588
Glu Arg Leu Lys Gln Glu Ile Asp Asn Ala Arg Glu Leu Gln Glu Gln
        270                 275                 280

agg gac tct ttg act cag aaa cta cag gaa gtt gag att cga aat aaa      1636
Arg Asp Ser Leu Thr Gln Lys Leu Gln Glu Val Glu Ile Arg Asn Lys
    285                 290                 295

gac ctg gag ggg cag ctg tcg gag ctg gag cag cgc ctg gag aag agc      1684
Asp Leu Glu Gly Gln Leu Ser Glu Leu Glu Gln Arg Leu Glu Lys Ser
300                 305                 310                 315

cag agc gag cag gac gct ttc cgc agt aac ctg aag act ctc cta gag      1732
Gln Ser Glu Gln Asp Ala Phe Arg Ser Asn Leu Lys Thr Leu Leu Glu
                320                 325                 330

att ctg gac ggg aaa ata ttt gaa cta aca gaa ttg cgg gat aat ttg      1780
Ile Leu Asp Gly Lys Ile Phe Glu Leu Thr Glu Leu Arg Asp Asn Leu
            335                 340                 345

gcc aag cta cta gaa tgc agc t aaagaaagtg aaatttcagt gccaatagat      1832
Ala Lys Leu Leu Glu Cys Ser
        350

gaagagatac tgtctgtctt cgtaggactg tttgggctct gtaccaagat tgcacaaaat      1892
tttttgaata tcattcctcc agaaggaggg tgttttgaaa attggaattg tatatttcag      1952
tataaatttt agaatttagc ttatagctag ttgggggaaa aaaagacatg aaaaacttga      2012
accacaaatt acctccatgt acattggcca tagttacaat gggagaatta acaatgtctg      2072
ggtcccttct cctttttctg ttcaacacag tgaagattat ctgcttttta aatttattta      2132
cgatatctac agctgtgttt tgtgtaaaaa cttagtaatg gaagccctgt ctttgttgtt      2192
atctgaataa tttctcagga tatttttttg ctgctgagaa agggccatta ccaattaatc      2252
cttgccagga gttggggagc tatgtctcta attggaatca ctataactgg gtgtctggag      2312
ttcttccctt ttcgtactga gagtgttctc actctagtga ctcctctggt acactccgtg      2372
ttctccaatc ttgtctgttg tactttactt ttccatattg actccatgta tttatgagaa      2432
gatattatct cccattttat tatacatttt gaagccaact aaacaaaggc agctgagtcc      2492
ttcagatatt tttcttttta aatttatagt aaatttgaca cagaactgaa attcagcagt      2552
ccgtctttga cggtttagtc tagcaatgtt aaggatattt agagaaaata tgcagttacg      2612
tttatttata tatttggcaa gaaatttttt ctggatgatc aatgcttttc aatttatgat      2672
aaataatggt tagggggcgc tgtttattat agataatttt aaggtatata gctgttttca      2732
aggaggtcca cttccgtcta gcagccaagc agaggactgt atctaaatcg tgatcgtggc      2792
agatgggtct tcatagaaac catgtcttta ttcaaacttc atagggcaat attttgaact      2852
gttacctagg catttcaaaa caggaaatac cgtcaacaga ctcttctcca agagcaggtt      2912
ttactgttgt tttgatgtaa ttttaagaca tttagcaaac atgcatttct ttatatgata      2972
catttctttc acaaaacaat ttaaaagtaa gccacgtgct gtctgctctg cccgggtagg      3032
aattgcatca gaatacatat atcttgctgt acaatgcctg tgatattgaa gagggttctt      3092
ttcatgtatg cttgagtatc taactctgga gtcaatgaat gcactgactt tttttttgtt      3152
cgtaccccaa atgattgaat tgttaagtac aaattaagca gattaactca ttttttcact      3212
cataaacaga ttcttagtac tagttttgtt ttatatttat gtgtatgtat gtaaatacat      3272
acatattaat ttatattaga gtgaaaaata aattgtttgt ttctaacatt aaaaaaaaaa      3332
aaaaaaa                                                              3339

<210> 34
<211> 354
<212> PRT
<213> Rattus norvegicus

<400> 34

Met Gly Glu Gln Pro Ile Phe Ser Thr Arg Ala His Val Phe Gln Ile
1               5                   10                  15
Asp Pro Asn Thr Lys Lys Asn Trp Val Pro Thr Ser Lys His Ala Val
            20                  25                  30
Thr Val Ser Tyr Phe Tyr Asp Ser Thr Arg Asn Val Tyr Arg Ile Ile
        35                  40                  45
Ser Leu Asp Gly Ser Lys Ala Ile Ile Asn Ser Thr Ile Thr Pro Asn
    50                  55                  60
Met Thr Phe Thr Lys Thr Ser Gln Lys Phe Gly Gln Trp Ala Asp Ser
65                  70                  75                  80
Arg Ala Asn Thr Val Tyr Gly Leu Gly Phe Ser Ser Glu His His Leu
                85                  90                  95
Ser Lys Phe Ala Glu Lys Phe Gln Glu Phe Lys Glu Ala Ala Arg Leu
            100                 105                 110
Ala Lys Glu Lys Ser Gln Glu Lys Met Glu Leu Thr Ser Thr Pro Ser
        115                 120                 125
Gln Glu Ser Ala Gly Gly Asp Leu Gln Ser Pro Leu Thr Pro Glu Ser
    130                 135                 140
Ile Asn Gly Thr Asp Asp Glu Arg Thr Pro Asp Val Thr Gln Asn Ser
145                 150                 155                 160
Glu Pro Arg Ala Glu Pro Ala Gln Asn Ala Leu Pro Phe Ser His Ser
                165                 170                 175
Ser Ala Ile Ser Lys His Trp Glu Ala Glu Leu Ala Thr Leu Lys Gly
            180                 185                 190
Asn Asn Ala Lys Leu Thr Ala Ala Leu Leu Glu Ser Thr Ala Asn Val
        195                 200                 205
Lys Gln Trp Lys Gln Gln Leu Ala Ala Tyr Gln Glu Glu Ala Glu Arg
    210                 215                 220
Leu His Lys Arg Val Thr Glu Leu Glu Cys Val Ser Ser Gln Ala Asn
225                 230                 235                 240
Ala Val His Ser His Lys Thr Glu Leu Ser Gln Thr Val Gln Glu Leu
                245                 250                 255
Glu Glu Thr Leu Lys Val Lys Glu Glu Glu Ile Glu Arg Leu Lys Gln
            260                 265                 270
Glu Ile Asp Asn Ala Arg Glu Leu Gln Glu Gln Arg Asp Ser Leu Thr
        275                 280                 285
Gln Lys Leu Gln Glu Val Glu Ile Arg Asn Lys Asp Leu Glu Gly Gln
    290                 295                 300
Leu Ser Glu Leu Glu Gln Arg Leu Glu Lys Ser Gln Ser Glu Gln Asp
305                 310                 315                 320
Ala Phe Arg Ser Asn Leu Lys Thr Leu Leu Glu Ile Leu Asp Gly Lys
                325                 330                 335
Ile Phe Glu Leu Thr Glu Leu Arg Asp Asn Leu Ala Lys Leu Leu Glu
            340                 345                 350
Cys Ser

<210> 35
<211> 3706
<212> DNA
<213> Rattus norvegicus

<220>
<221> CDS
<222> (732)...(1836)
<223> Homer 1c

<400> 35
gcggccgcgt cgactacggc tgcgagaaga cgacagaagg gggctccgct gatgctcctc      60
gtgagaacga atcgatcctt cccagccttc tctgcctgct ctccacctcc tctctgctcc     120
gagtcttagg agaacgaaca ttcaaaggac agattccaat gtggtgtgct gtgcacatcg     180
cgagcggctg gggtttgcac ttcgagattt cttctttata attttttttt tttaatgtaa     240
gggagacagt ggaattgcta cccgtagaat ttttattcaa gtgcacgtcg cgttgggttg     300

cacgctccac ccccagggac ctggtgtggt gaaatttgaa cccaccgcct tagcccaaag 360
gccgagtaac ctggctgctt gagtgtcgtg gaagacgtga gcgaaatgat cagcgaactc 420
attttttatc agactcactg aagctggctt ttgcgttttt ctacacgtac actaatttta 480
tggaatagtt aaagtgctat attctccgcg caaccttttc aaattccaaa tgtttgaacg 540
ttttggtgtc agcgcgagtg aaatcatttt accgacaaga actaactgaa ttgtctgcct 600
cgttgagttg cctccggaaa agatctcggg ggtggaaaag caactgcaaa ataacagacg 660
gagaaaattc cttggaagtt atttctgtag cataagagca gaaacttcag agcaagtttt 720
cattgggcaa a atg ggg gaa caa cct atc ttc agc act cga gct cat gtc 770
Met Gly Glu Gln Pro Ile Phe Ser Thr Arg Ala His Val
1               5                   10

ttc cag atc gac cca aac aca aag aag aac tgg gta ccc acc agc aag 818
Phe Gln Ile Asp Pro Asn Thr Lys Lys Asn Trp Val Pro Thr Ser Lys
    15                  20                  25

cat gca gtt act gtg tct tat ttc tat gac agc aca agg aat gtg tat 866
His Ala Val Thr Val Ser Tyr Phe Tyr Asp Ser Thr Arg Asn Val Tyr
30                  35                  40                  45

agg ata atc agt cta gac ggc tca aag gca ata ata aat agc acc atc 914
Arg Ile Ile Ser Leu Asp Gly Ser Lys Ala Ile Ile Asn Ser Thr Ile
                50                  55                  60

act cca aac atg aca ttt act aaa aca tct caa aag ttt ggc caa tgg 962
Thr Pro Asn Met Thr Phe Thr Lys Thr Ser Gln Lys Phe Gly Gln Trp
            65                  70                  75

gct gat agc cgg gca aac act gtt tat gga ctg gga ttc tcc tct gag 1010
Ala Asp Ser Arg Ala Asn Thr Val Tyr Gly Leu Gly Phe Ser Ser Glu
        80                  85                  90

cat cat ctc tca aaa ttt gca gaa aag ttt cag gaa ttt aaa gaa gct 1058
His His Leu Ser Lys Phe Ala Glu Lys Phe Gln Glu Phe Lys Glu Ala
    95                  100                 105

gct cgg ctg gca aag gag aag tcg cag gag aag atg gaa ctg acc agt 1106
Ala Arg Leu Ala Lys Glu Lys Ser Gln Glu Lys Met Glu Leu Thr Ser
110                 115                 120                 125

acc cct tca cag gaa tca gca gga gga gat ctt cag tct cct tta aca 1154
Thr Pro Ser Gln Glu Ser Ala Gly Gly Asp Leu Gln Ser Pro Leu Thr
                130                 135                 140

cca gaa agt atc aat ggg aca gat gat gag aga aca ccc gat gtg aca 1202
Pro Glu Ser Ile Asn Gly Thr Asp Asp Glu Arg Thr Pro Asp Val Thr
            145                 150                 155

cag aac tca gag cca agg gct gag cca gct cag aat gca ttg cca ttt 1250
Gln Asn Ser Glu Pro Arg Ala Glu Pro Ala Gln Asn Ala Leu Pro Phe
        160                 165                 170

tca cat agt gcc ggg gat cga acc cag ggc ctc tct cat gct agt tca 1298
Ser His Ser Ala Gly Asp Arg Thr Gln Gly Leu Ser His Ala Ser Ser
    175                 180                 185

gcc atc agc aaa cac tgg gag gct gaa cta gcc acg ctc aag ggg aac 1346
Ala Ile Ser Lys His Trp Glu Ala Glu Leu Ala Thr Leu Lys Gly Asn
190                 195                 200                 205

aat gcc aag ctc acc gca gcg ctg ctg gag tcc act gcc aac gtg aag 1394
Asn Ala Lys Leu Thr Ala Ala Leu Leu Glu Ser Thr Ala Asn Val Lys
                210                 215                 220

cag tgg aag caa cag ctg gct gcc tac cag gag gag gca gag cgg ctg     1442
Gln Trp Lys Gln Gln Leu Ala Ala Tyr Gln Glu Glu Ala Glu Arg Leu
            225                 230                 235

cac aag cgg gtc acg gag ctg gaa tgt gtt agt agt caa gca aac gcg     1490
His Lys Arg Val Thr Glu Leu Glu Cys Val Ser Ser Gln Ala Asn Ala
        240                 245                 250

gtg cac agc cac aag aca gag ctg agt cag aca gtg cag gag ctg gaa     1538
Val His Ser His Lys Thr Glu Leu Ser Gln Thr Val Gln Glu Leu Glu
    255                 260                 265

gag acc cta aaa gta aag gaa gag gaa ata gaa aga tta aaa caa gaa     1586
Glu Thr Leu Lys Val Lys Glu Glu Glu Ile Glu Arg Leu Lys Gln Glu
270                 275                 280                 285

att gat aac gcc aga gaa ctt caa gaa cag agg gac tct ttg act cag     1634
Ile Asp Asn Ala Arg Glu Leu Gln Glu Gln Arg Asp Ser Leu Thr Gln
                290                 295                 300

aaa cta cag gaa gtt gag att cga aat aaa gac ctg gag ggg cag ctg     1682
Lys Leu Gln Glu Val Glu Ile Arg Asn Lys Asp Leu Glu Gly Gln Leu
            305                 310                 315

tcg gag ctg gag cag cgc ctg gag aag agc cag agc gag cag gac gct     1730
Ser Glu Leu Glu Gln Arg Leu Glu Lys Ser Gln Ser Glu Gln Asp Ala
        320                 325                 330

ttc cgc agt aac ctg aag act ctc cta gag att ctg gac ggg aaa ata     1778
Phe Arg Ser Asn Leu Lys Thr Leu Leu Glu Ile Leu Asp Gly Lys Ile
    335                 340                 345

ttt gaa cta aca gaa ttg cgg gat aat ttg gcc aag cta cta gaa tgc     1826
Phe Glu Leu Thr Glu Leu Arg Asp Asn Leu Ala Lys Leu Leu Glu Cys
350                 355                 360                 365

agc taa aga a agtgaaattt cagtgccaat agatgaagag atactgtctg     1876
Ser  *  Arg

tcttcgtagg actgtttggg ctctgtacca agattgcaaa aaattttttg aatatcattc     1936
ctccagaagg agggtgtttt gaaaattgga attgtatatt tcagtataaa ttttagaatt     1996
tagcttatag ctagttgggg gaaaaaaaga catgaaaaac ttgaaccaca aataatgcaa     2056
tcttttcccc tgatagtagc caatgggaga attaacaatg tctgggtccc ttctcctttt     2116
tctgttcaac acagtgaaga ttatctgctt tttaaattta tttacgatat ctacagctgt     2176
gttttgtgta aaaacttagt aatggaagcc ctgtctttgt tgttatctga ataatttctc     2236
aggatatttt tttgctgctg agaaagggcc attaccaatt aatccttgcc aggagttggg     2296
gagctatgtc tctaattgga atcactataa ctgggtgtct ggagttcttc ccttttcgta     2356
ctgagagtgt tctcactcta gtgactactc tggtacactc cgtgttctcc aatcttgtct     2416
gttgtacttt acttttccat attgactcca tgtatttatg agaagatatt atctcccatt     2476
ttattataca ttttgaagcc aactaaacaa aggcagctga gtccttcaga tatttttctt     2536
tttaaattta tagtaaattt gacacagaac tgaaattcag cagtccgtct ttgacggttt     2596
agtctagcaa tgttaaggat atttagagaa aatatgcagt tacgtttatt tatatatttg     2656
gcaagaaatt ttttctggat gatcaatgct tttcaattta tgataaataa tggttagggg     2716
cgctgtttat tatagataat tttaaggtgt atagctgttt tcaaggaggt ccactcccgt     2776
ctagcagcca agcagaggac tgtatctaaa tcgtgatcgt ggcagatggg tcttcataga     2836
aaccatgtct ttattcaaac ttcatagggc aatattttga actgttacct aggcatttca     2896
aaacaggaaa taccgtcaac agactcttct ccaagagcag gttttactgt tgttttgatg     2956
taattttaag acatttagca aacatgcatt tctttatatg atacatttct ttcacaaaac     3016
aatttaaaag taagccacgt gctgtctgct ctgcccgggt aggaattgca tcagaataca     3076
tatatcttgc tgtacaatgc ctgtgatatt gaagagggtt cttttcatgt atgcttgagt     3136
atctaactct ggagtcaatg aatgcactga cttttttttt tgttcgtacc ccaaatgatt     3196
gaattgttaa gtacaaatta agcagattaa ctcatttttt cactcataaa cagattctta     3256

gtactagttt tgttttatat ttatgtgtat gtatgtaaat acatacatat taatttatat 3316
tagagtgaaa aataaattgt ttgtttctaa cattagtttc tacagtaagg tgtctctgaa 3376
acatgtgtgt cagacactta gccaccatgc attctatgtg ctaccccatc atgccagtca 3436
cctccatcga cgttagggta ttttccttac ctgtctatta taaagagaat aacttaggta 3496
cacatgctca gagccgagat atttctctga taaatcaggt aataaaatct atttgatggg 3556
tagaattttg aaaacagaca tgattttatc tatgagtttc tgaatatcaa agaacaccag 3616
gttttcattt aaatagaggt ctaacactag ggatcaggga atttagttat gaagagttga 3676
aaaaaaaaaa aaaaaaaaaa aaaaaaaaaa 3706

<210> 36
<211> 367
<212> PRT
<213> Rattus norvegicus

<400> 36
Met Gly Glu Gln Pro Ile Phe Ser Thr Arg Ala His Val Phe Gln Ile
1               5                   10                  15
Asp Pro Asn Thr Lys Lys Asn Trp Val Pro Thr Ser Lys His Ala Val
            20                  25                  30
Thr Val Ser Tyr Phe Tyr Asp Ser Thr Arg Asn Val Tyr Arg Ile Ile
        35                  40                  45
Ser Leu Asp Gly Ser Lys Ala Ile Ile Asn Ser Thr Ile Thr Pro Asn
    50                  55                  60
Met Thr Phe Thr Lys Thr Ser Gln Lys Phe Gly Gln Trp Ala Asp Ser
65                  70                  75                  80
Arg Ala Asn Thr Val Tyr Gly Leu Gly Phe Ser Ser Glu His His Leu
                85                  90                  95
Ser Lys Phe Ala Glu Lys Phe Gln Glu Phe Lys Glu Ala Ala Arg Leu
            100                 105                 110
Ala Lys Glu Lys Ser Gln Glu Lys Met Glu Leu Thr Ser Thr Pro Ser
        115                 120                 125
Gln Glu Ser Ala Gly Gly Asp Leu Gln Ser Pro Leu Thr Pro Glu Ser
    130                 135                 140
Ile Asn Gly Thr Asp Asp Glu Arg Thr Pro Asp Val Thr Gln Asn Ser
145                 150                 155                 160
Glu Pro Arg Ala Glu Pro Ala Gln Asn Ala Leu Pro Phe Ser His Ser
                165                 170                 175
Ala Gly Asp Arg Thr Gln Gly Leu Ser His Ala Ser Ser Ala Ile Ser
            180                 185                 190
Lys His Trp Glu Ala Glu Leu Ala Thr Leu Lys Gly Asn Asn Ala Lys
        195                 200                 205
Leu Thr Ala Ala Leu Leu Glu Ser Thr Ala Asn Val Lys Gln Trp Lys
    210                 215                 220
Gln Gln Leu Ala Ala Tyr Gln Glu Glu Ala Glu Arg Leu His Lys Arg
225                 230                 235                 240
Val Thr Glu Leu Glu Cys Val Ser Ser Gln Ala Asn Ala Val His Ser
                245                 250                 255
His Lys Thr Glu Leu Ser Gln Thr Val Gln Glu Leu Glu Glu Thr Leu
            260                 265                 270
Lys Val Lys Glu Glu Glu Ile Glu Arg Leu Lys Gln Glu Ile Asp Asn
        275                 280                 285
Ala Arg Glu Leu Gln Glu Gln Arg Asp Ser Leu Thr Gln Lys Leu Gln
    290                 295                 300
Glu Val Glu Ile Arg Asn Lys Asp Leu Glu Gly Gln Leu Ser Glu Leu
305                 310                 315                 320
Glu Gln Arg Leu Glu Lys Ser Gln Ser Glu Gln Asp Ala Phe Arg Ser
                325                 330                 335
Asn Leu Lys Thr Leu Leu Glu Ile Leu Asp Gly Lys Ile Phe Glu Leu
            340                 345                 350
Thr Glu Leu Arg Asp Asn Leu Ala Lys Leu Leu Glu Cys Ser Arg
        355                 360                 365

<210> 37

<211> 7424
<212> DNA
<213> Rattus norvegicus

<220>
<221> CDS
<222> (267)...(5486)
<223> Shank 3a

<400> 37
ctctagaact agtggatccc ccgggctgca ggattctgcg gccgcgctaa accgtgccgc 60
cgtcgccgcc gccgctgcgc ctgcggagcc cccggagccg ctgtcccccg cgctggcccc 120
ggccccggcc ccatacggcc ccctcccgca gtagcgcggt cggcgggact ctggcggggg 180
gtcagggggg gccagggcgc cgcgcggagt ccccgtgcgc tcctctctcc gccgggaaca 240
gtccgggccc cggcgctagc accggg atg gac ggc ccc ggg gcc agc gcc gtg 293
Met Asp Gly Pro Gly Ala Ser Ala Val
1               5

gtc gtg cgc gtc ggc atc ccg gac ctg caa caa acg aag tgc ctg cgt 341
Val Val Arg Val Gly Ile Pro Asp Leu Gln Gln Thr Lys Cys Leu Arg
10                  15                  20                  25

ctg gat cca acc gcg ccc gtg tgg gcc gcc aag cag cgt gtg ctc tgc 389
Leu Asp Pro Thr Ala Pro Val Trp Ala Ala Lys Gln Arg Val Leu Cys
                30                  35                  40

gcc ctc aac cac agc ctt cag gac gcg ctc aac tac ggg cta ttc cag 437
Ala Leu Asn His Ser Leu Gln Asp Ala Leu Asn Tyr Gly Leu Phe Gln
            45                  50                  55

cct ccc tcc cgg ggt cgc gcc ggc aag ttc ctg gat gaa gag cgg ctc 485
Pro Pro Ser Arg Gly Arg Ala Gly Lys Phe Leu Asp Glu Glu Arg Leu
        60                  65                  70

tta cag gac tac ccg cct aac ctg gac acg ccc ctg ccc tat ctg gag 533
Leu Gln Asp Tyr Pro Pro Asn Leu Asp Thr Pro Leu Pro Tyr Leu Glu
    75                  80                  85

ttt cga tac aag cgg aga gtt tat gcc cag aac ctc ata gat gac aag 581
Phe Arg Tyr Lys Arg Arg Val Tyr Ala Gln Asn Leu Ile Asp Asp Lys
90                  95                  100                 105

cag ttt gca aag ctg cac aca aag gca aac ctg aag aag ttc atg gac 629
Gln Phe Ala Lys Leu His Thr Lys Ala Asn Leu Lys Lys Phe Met Asp
                110                 115                 120

tat gtc cag cta cac agc aca gac aag gtg gcc cgc ctg ctg gac aag 677
Tyr Val Gln Leu His Ser Thr Asp Lys Val Ala Arg Leu Leu Asp Lys
            125                 130                 135

ggg ctg gac ccc aat ttc cat gac cct gac tca gga gag tgc cct ctg 725
Gly Leu Asp Pro Asn Phe His Asp Pro Asp Ser Gly Glu Cys Pro Leu
        140                 145                 150

agc ctt gca gca cag ttg gac aac gcc act gac ctc ctg aag gtt ctt 773
Ser Leu Ala Ala Gln Leu Asp Asn Ala Thr Asp Leu Leu Lys Val Leu
    155                 160                 165

cgc aat ggc ggt gct cat ctg gac ttc cga acc cga gat ggg cta acc 821
Arg Asn Gly Gly Ala His Leu Asp Phe Arg Thr Arg Asp Gly Leu Thr
170                 175                 180                 185

gct gtc cac tgc gcc acc cga cag cgg aat gcg gga gca ttg acg acc 869

Ala Val His Cys Ala Thr Arg Gln Arg Asn Ala Gly Ala Leu Thr Thr
                190                 195                 200

ctg ctg gac ctg ggg gct tca cct gac tac aag gac agc cgc ggc ctg      917
Leu Leu Asp Leu Gly Ala Ser Pro Asp Tyr Lys Asp Ser Arg Gly Leu
            205                 210                 215

acg ccc ctg tac cat agt gcc cta ggg ggc ggg gat gcc ctc tgc tgt      965
Thr Pro Leu Tyr His Ser Ala Leu Gly Gly Gly Asp Ala Leu Cys Cys
        220                 225                 230

gag ctg ctt ctc cat gat cac gca cag ttg ggg acc act gac gag aat     1013
Glu Leu Leu Leu His Asp His Ala Gln Leu Gly Thr Thr Asp Glu Asn
    235                 240                 245

ggc tgg cag gag atc cat cag gcc tgt cgc ttt ggg cat gta cag cac     1061
Gly Trp Gln Glu Ile His Gln Ala Cys Arg Phe Gly His Val Gln His
250                 255                 260                 265

ttg gag cac ctg ctg ttc tat ggg gcc aac atg ggt gcc cag aac gcc     1109
Leu Glu His Leu Leu Phe Tyr Gly Ala Asn Met Gly Ala Gln Asn Ala
                270                 275                 280

tcg gga aac aca gcc ttg cac atc tgt gcc ctc tat aac cag gag agc     1157
Ser Gly Asn Thr Ala Leu His Ile Cys Ala Leu Tyr Asn Gln Glu Ser
            285                 290                 295

tgt gcc cgc gtc ctg ctt ttc cgt ggt gcc aac aag gac gtc cgc aat     1205
Cys Ala Arg Val Leu Leu Phe Arg Gly Ala Asn Lys Asp Val Arg Asn
        300                 305                 310

tac aac agc cag aca gcc ttc cag gtg gcc att att gca ggg aac ttt     1253
Tyr Asn Ser Gln Thr Ala Phe Gln Val Ala Ile Ile Ala Gly Asn Phe
    315                 320                 325

gag ctt gcc gag gta atc aag acc cac aaa gac tcg gat gtc gta cca     1301
Glu Leu Ala Glu Val Ile Lys Thr His Lys Asp Ser Asp Val Val Pro
330                 335                 340                 345

ttc agg gaa acc ccc agc tat gca aag cga cga cgt ctg gct ggc ccg     1349
Phe Arg Glu Thr Pro Ser Tyr Ala Lys Arg Arg Arg Leu Ala Gly Pro
                350                 355                 360

agt ggc ttg gca tcc cct cgg ccc tta cag cgc tca gcc agt gat atc     1397
Ser Gly Leu Ala Ser Pro Arg Pro Leu Gln Arg Ser Ala Ser Asp Ile
            365                 370                 375

aac ctg aag ggt gac cag ccc gca gct tct ccc ggg ccc act ctc cga     1445
Asn Leu Lys Gly Asp Gln Pro Ala Ala Ser Pro Gly Pro Thr Leu Arg
        380                 385                 390

agc ctc cct cac caa ctg ctg ctc cag agg ctt cag gag gag aaa gac     1493
Ser Leu Pro His Gln Leu Leu Leu Gln Arg Leu Gln Glu Glu Lys Asp
    395                 400                 405

cgg gac agg gat ggt gag cag gag aac gac atc agc ggt ccc tca gca     1541
Arg Asp Arg Asp Gly Glu Gln Glu Asn Asp Ile Ser Gly Pro Ser Ala
410                 415                 420                 425

ggc agg ggc ggc cac agc aag atc agc ccc agc ggg ccc ggc gga tcc     1589
Gly Arg Gly Gly His Ser Lys Ile Ser Pro Ser Gly Pro Gly Gly Ser
                430                 435                 440

ggc ccc gcg ccc ggc ccc ggc ccg gcg tct ccc gcg ccc ccc gcg ccg 1637
Gly Pro Ala Pro Gly Pro Gly Pro Ala Ser Pro Ala Pro Pro Ala Pro
            445                 450                 455

ccg ccc cgg ggc ccg aag cgg aaa ctt tac agt gcc gtc ccc ggc cgc 1685
Pro Pro Arg Gly Pro Lys Arg Lys Leu Tyr Ser Ala Val Pro Gly Arg
        460                 465                 470

aag ttc atc gct gtg aag gcg cac agc ccg cag ggc gag ggc gag atc 1733
Lys Phe Ile Ala Val Lys Ala His Ser Pro Gln Gly Glu Gly Glu Ile
    475                 480                 485

ccg ctg cac cgc ggc gag gcc gtg aag gtg ctc agc att ggg gag ggc 1781
Pro Leu His Arg Gly Glu Ala Val Lys Val Leu Ser Ile Gly Glu Gly
490                 495                 500                 505

ggt ttc tgg gag gga acc gtg aag ggc cgt aca ggc tgg ttc cca gct 1829
Gly Phe Trp Glu Gly Thr Val Lys Gly Arg Thr Gly Trp Phe Pro Ala
                510                 515                 520

gac tgt gtg gag gaa gtg cag atg cga cag tat gac aca cgg cat gaa 1877
Asp Cys Val Glu Glu Val Gln Met Arg Gln Tyr Asp Thr Arg His Glu
            525                 530                 535

act cga gag gac cgg acg aag cgt ctt ttc cgc cac tac act gtg ggt 1925
Thr Arg Glu Asp Arg Thr Lys Arg Leu Phe Arg His Tyr Thr Val Gly
        540                 545                 550

tcc tat gac agc ctc act tca cac agt gat tat gtc att gat gat aag 1973
Ser Tyr Asp Ser Leu Thr Ser His Ser Asp Tyr Val Ile Asp Asp Lys
    555                 560                 565

gtg gct atc ctg caa aaa cgg gac cat gag ggt ttt ggc ttt gtt ctc 2021
Val Ala Ile Leu Gln Lys Arg Asp His Glu Gly Phe Gly Phe Val Leu
570                 575                 580                 585

cgg gga gcc aaa gca gag acc ccc att gag gag ttt aca ccc aca cct 2069
Arg Gly Ala Lys Ala Glu Thr Pro Ile Glu Glu Phe Thr Pro Thr Pro
                590                 595                 600

gcc ttc cct gcg ctc cag tac ctt gag tct gta gat gtg gaa ggt gtg 2117
Ala Phe Pro Ala Leu Gln Tyr Leu Glu Ser Val Asp Val Glu Gly Val
            605                 610                 615

gcc tgg aag gct ggg ctt cgc act ggg gac ttc ctc att gag gta aac 2165
Ala Trp Lys Ala Gly Leu Arg Thr Gly Asp Phe Leu Ile Glu Val Asn
        620                 625                 630

gga gtg aac gtc gtg aag gtt gga cac aag caa gtg gtg ggt ctc atc 2213
Gly Val Asn Val Val Lys Val Gly His Lys Gln Val Val Gly Leu Ile
    635                 640                 645

cgt cag ggt ggc aac cgt ctg gtc atg aag gtt gtg tct gtt acc agg 2261
Arg Gln Gly Gly Asn Arg Leu Val Met Lys Val Val Ser Val Thr Arg
650                 655                 660                 665

aag cca gag gag gat agt gct cgg cgc aga gcc cca cca cct ccc aag 2309
Lys Pro Glu Glu Asp Ser Ala Arg Arg Arg Ala Pro Pro Pro Pro Lys
                670                 675                 680

agg gcc ccc agc acc acg ctg acc ctg cgg tcc aag tcc atg acg gct 2357
Arg Ala Pro Ser Thr Thr Leu Thr Leu Arg Ser Lys Ser Met Thr Ala
            685                 690                 695

gag ctc gag gaa ctc gct tcc att cgg aga agg aaa ggg gag aag ttg     2405
Glu Leu Glu Glu Leu Ala Ser Ile Arg Arg Arg Lys Gly Glu Lys Leu
        700                 705                 710

gat gag atc ctg gcg gtt gct gcg gaa cca acg ctg agg cca gac att     2453
Asp Glu Ile Leu Ala Val Ala Ala Glu Pro Thr Leu Arg Pro Asp Ile
    715                 720                 725

gca gac gct gat tcc agg gca gcc act gtc aag cag cgg ccc acc agc     2501
Ala Asp Ala Asp Ser Arg Ala Ala Thr Val Lys Gln Arg Pro Thr Ser
730                 735                 740                 745

cgg agg att acc cct gcc gag atc agc tca ttg ttt gag cga cag ggc     2549
Arg Arg Ile Thr Pro Ala Glu Ile Ser Ser Leu Phe Glu Arg Gln Gly
                750                 755                 760

ctc ccg ggc cca gag aag ctg ccg ggc tct ctg cgg aag ggg att cca     2597
Leu Pro Gly Pro Glu Lys Leu Pro Gly Ser Leu Arg Lys Gly Ile Pro
            765                 770                 775

cgg acc aaa tct gta ggg gag gat gag aag ctg gca tcc cta ctg gaa     2645
Arg Thr Lys Ser Val Gly Glu Asp Glu Lys Leu Ala Ser Leu Leu Glu
        780                 785                 790

ggg cgt ttc cca cgc agc aca tca atg caa gac aca gtg cgt gaa ggc     2693
Gly Arg Phe Pro Arg Ser Thr Ser Met Gln Asp Thr Val Arg Glu Gly
    795                 800                 805

cga ggc att ccg ccc cca ccg cag acc gcc ccg cca ccc cca ccc gcg     2741
Arg Gly Ile Pro Pro Pro Pro Gln Thr Ala Pro Pro Pro Pro Pro Ala
810                 815                 820                 825

ccc tac tac ttc gac tcc ggg cca ccc ccc acc ttc tca cca ccg cca     2789
Pro Tyr Tyr Phe Asp Ser Gly Pro Pro Pro Thr Phe Ser Pro Pro Pro
                830                 835                 840

cca cca ccg ggc cgg gcc tat gac act gtg cgc tcc agc ttc aag cca     2837
Pro Pro Pro Gly Arg Ala Tyr Asp Thr Val Arg Ser Ser Phe Lys Pro
            845                 850                 855

ggc ctg gag gct cgt ctg ggt gca ggg gca gct ggc ctg tat gat tct     2885
Gly Leu Glu Ala Arg Leu Gly Ala Gly Ala Ala Gly Leu Tyr Asp Ser
        860                 865                 870

ggc aca cct ctg ggc ccg ctg ccc tac cct gag cgc cag aag cgt gca     2933
Gly Thr Pro Leu Gly Pro Leu Pro Tyr Pro Glu Arg Gln Lys Arg Ala
    875                 880                 885

cgc tcc atg atc ata ttg cag gac tct gcg cca gaa gtg ggc gat gta     2981
Arg Ser Met Ile Ile Leu Gln Asp Ser Ala Pro Glu Val Gly Asp Val
890                 895                 900                 905

ccc cgg cct gcg cct gca gcc aca ccg cct gag cgc ccc aag cgc cgg     3029
Pro Arg Pro Ala Pro Ala Ala Thr Pro Pro Glu Arg Pro Lys Arg Arg
                910                 915                 920

cct cgg ccg tca ggc cct gat agt ccc tat gcc aac ctg ggc gcc ttc     3077
Pro Arg Pro Ser Gly Pro Asp Ser Pro Tyr Ala Asn Leu Gly Ala Phe
            925                 930                 935

agt gcc agc ctc ttt gct ccg tcg aaa ccg cag cgc cgc aag agt ccg     3125
Ser Ala Ser Leu Phe Ala Pro Ser Lys Pro Gln Arg Arg Lys Ser Pro

940                 945                 950

ctg gtg aag cag ctt cag gtg gag gac gct cag gag cgc gcg gcg ttg     3173
Leu Val Lys Gln Leu Gln Val Glu Asp Ala Gln Glu Arg Ala Ala Leu
    955                 960                 965

gcc gtg ggt agc ccg gga cca gtg ggt gga agc ttt gca cga gaa ccc     3221
Ala Val Gly Ser Pro Gly Pro Val Gly Gly Ser Phe Ala Arg Glu Pro
970                 975                 980                 985

tcc cca acg cac cgc ggg ccc cga ccg ggc ggc ctt gac tac agc tct     3269
Ser Pro Thr His Arg Gly Pro Arg Pro Gly Gly Leu Asp Tyr Ser Ser
                990                 995                 1000

gga gaa ggc ctg ggg ctc acc ttt ggc ggc cct agc cct ggc cca gtc     3317
Gly Glu Gly Leu Gly Leu Thr Phe Gly Gly Pro Ser Pro Gly Pro Val
            1005                1010                1015

aag gag cgg cgc ctg gag gag cga cgc cgt tcc act gtg ttc ctg tct     3365
Lys Glu Arg Arg Leu Glu Glu Arg Arg Arg Ser Thr Val Phe Leu Ser
        1020                1025                1030

gtg ggt gcc atc gag ggc aac cct ccc agc gcg gat ctg cca tcc cta     3413
Val Gly Ala Ile Glu Gly Asn Pro Pro Ser Ala Asp Leu Pro Ser Leu
    1035                1040                1045

caa ccc tcc cgc tcc att gat gag cgc ctc ctg ggg aca ggc gcc acc     3461
Gln Pro Ser Arg Ser Ile Asp Glu Arg Leu Leu Gly Thr Gly Ala Thr
1050                1055                1060                1065

act ggc cga gat ttg ctg ctc ccc tcc cct gtc tct gct ctg aag cca     3509
Thr Gly Arg Asp Leu Leu Leu Pro Ser Pro Val Ser Ala Leu Lys Pro
                1070                1075                1080

ttg gtc ggt ggt ccc aac ctt ggg ccc tca agc tcc acc ttc atc cat     3557
Leu Val Gly Gly Pro Asn Leu Gly Pro Ser Ser Ser Thr Phe Ile His
            1085                1090                1095

cct ctt act ggc aaa ccc ttg gat cct agc tca ccc cta gct ctt gct     3605
Pro Leu Thr Gly Lys Pro Leu Asp Pro Ser Ser Pro Leu Ala Leu Ala
        1100                1105                1110

ctg gct gcc cga gag cgg gct ctg gcc tcg caa aca cct tcc cgg tcc     3653
Leu Ala Ala Arg Glu Arg Ala Leu Ala Ser Gln Thr Pro Ser Arg Ser
    1115                1120                1125

ccc aca ccc gtg cac agt cct gat gct gac cgc cct gga ccc ctc ttt     3701
Pro Thr Pro Val His Ser Pro Asp Ala Asp Arg Pro Gly Pro Leu Phe
1130                1135                1140                1145

gtg gat gtg caa acc cga gac tcc gag aga gga ccc ttg gcc tcc cca     3749
Val Asp Val Gln Thr Arg Asp Ser Glu Arg Gly Pro Leu Ala Ser Pro
                1150                1155                1160

gcc ttc tcc cct cgg agt cca gcc tgg att cca gtg cct gct cgc aga     3797
Ala Phe Ser Pro Arg Ser Pro Ala Trp Ile Pro Val Pro Ala Arg Arg
            1165                1170                1175

gag gca gag aag ccc act cgg gaa gag cgg aag tca cca gag gac aag     3845
Glu Ala Glu Lys Pro Thr Arg Glu Glu Arg Lys Ser Pro Glu Asp Lys
        1180                1185                1190

aaa tcc atg atc ctc agc gtc ttg gac acg tcc ttg caa cgg cca gct     3893

Lys Ser Met Ile Leu Ser Val Leu Asp Thr Ser Leu Gln Arg Pro Ala
    1195                1200                1205

ggc ctc att gtt gtg cat gcc acc agc aat gga cag gag ccc aac agg      3941
Gly Leu Ile Val Val His Ala Thr Ser Asn Gly Gln Glu Pro Asn Arg
1210                1215                1220                1225

ctg ggg gct gaa gag gag cgc ccg ggt act ccg gag ctg gcc cca acc      3989
Leu Gly Ala Glu Glu Glu Arg Pro Gly Thr Pro Glu Leu Ala Pro Thr
                1230                1235                1240

ccc atg cag gca gca gct gtg gca gag ccc atg cca agc cca cga gcc      4037
Pro Met Gln Ala Ala Ala Val Ala Glu Pro Met Pro Ser Pro Arg Ala
            1245                1250                1255

caa ccc cct ggc aac atc cca gca gat ccc ggg cca agc caa ggc aac      4085
Gln Pro Pro Gly Asn Ile Pro Ala Asp Pro Gly Pro Ser Gln Gly Asn
        1260                1265                1270

tca gag gag gag cca aag ctg gta ttc gct gtg aac ctg cca cct gct      4133
Ser Glu Glu Glu Pro Lys Leu Val Phe Ala Val Asn Leu Pro Pro Ala
    1275                1280                1285

caa ctg tcc tcc aac gat gag gag acc aga gag gag ctg gcc cgc att      4181
Gln Leu Ser Ser Asn Asp Glu Glu Thr Arg Glu Glu Leu Ala Arg Ile
1290                1295                1300                1305

ggg cta gtg cca ccc cct gaa gag ttt gcc aat ggg atc ctg ctg gcc      4229
Gly Leu Val Pro Pro Pro Glu Glu Phe Ala Asn Gly Ile Leu Leu Ala
                1310                1315                1320

acc cca ccc cca gga ccg ggc ccc ttg ccc acc acg gta ccc agc ccg      4277
Thr Pro Pro Pro Gly Pro Gly Pro Leu Pro Thr Thr Val Pro Ser Pro
            1325                1330                1335

gcc tca ggg aag ccc agc agc gag ctg ccc cct gcc ccg gag tct gca      4325
Ala Ser Gly Lys Pro Ser Ser Glu Leu Pro Pro Ala Pro Glu Ser Ala
        1340                1345                1350

gct gac tct gga gta gag gag gcc gac act cga agc tcc agt gac ccc      4373
Ala Asp Ser Gly Val Glu Glu Ala Asp Thr Arg Ser Ser Ser Asp Pro
    1355                1360                1365

cac ctg gag acc aca agc acc att tcc aca gtg tcc agc atg tcc acc      4421
His Leu Glu Thr Thr Ser Thr Ile Ser Thr Val Ser Ser Met Ser Thr
1370                1375                1380                1385

ctg agc tcg gag agt gga gaa ctc act gac acc cac acc tcc ttt gcc      4469
Leu Ser Ser Glu Ser Gly Glu Leu Thr Asp Thr His Thr Ser Phe Ala
                1390                1395                1400

gat gga cac act ttt cta ctc gag aag cca cca gtg cct ccc aag ccc      4517
Asp Gly His Thr Phe Leu Leu Glu Lys Pro Pro Val Pro Pro Lys Pro
            1405                1410                1415

aaa ctc aag tcc ccg ctg ggg aag ggg ccg gtg acc ttc agg ggc ccg      4565
Lys Leu Lys Ser Pro Leu Gly Lys Gly Pro Val Thr Phe Arg Gly Pro
        1420                1425                1430

ctg ctg aag caa tcc tcg gac agt gag ctc atg gcc cag cag cac cat      4613
Leu Leu Lys Gln Ser Ser Asp Ser Glu Leu Met Ala Gln Gln His His
    1435                1440                1445

gcc acc tct act ggg ttg act tct gct gct ggg cct gcc cgc cct cgc 4661
Ala Thr Ser Thr Gly Leu Thr Ser Ala Ala Gly Pro Ala Arg Pro Arg
1450                1455                1460                1465

tac ctc ttc cag aga agg tcc aag ctg tgg ggg gac ccc gtg gag agt 4709
Tyr Leu Phe Gln Arg Arg Ser Lys Leu Trp Gly Asp Pro Val Glu Ser
                1470                1475                1480

cgg ggg ctc cct ggg cct gag gat gac aaa cca act gtg atc agt gag 4757
Arg Gly Leu Pro Gly Pro Glu Asp Asp Lys Pro Thr Val Ile Ser Glu
            1485                1490                1495

ctc agc tcc cgt ctg cag cag ctg aat aaa gac act cgc tcc ttg ggg 4805
Leu Ser Ser Arg Leu Gln Gln Leu Asn Lys Asp Thr Arg Ser Leu Gly
        1500                1505                1510

gag gaa cca gtt ggt ggc ctg ggt agc ctg ctg gac cct gct aag aag 4853
Glu Glu Pro Val Gly Gly Leu Gly Ser Leu Leu Asp Pro Ala Lys Lys
    1515                1520                1525

tcg ccc att gca gca gct cgc tgc gcg gtg gtc ccg agt gcc ggc tgg 4901
Ser Pro Ile Ala Ala Ala Arg Cys Ala Val Val Pro Ser Ala Gly Trp
1530                1535                1540                1545

ctc ttc agc agc ctc ggt gag ctg agc acc atc tca gcg cag cgc agc 4949
Leu Phe Ser Ser Leu Gly Glu Leu Ser Thr Ile Ser Ala Gln Arg Ser
                1550                1555                1560

ccc ggg ggc ccg ggc gga ggg gcc tcc tac tcg gtg cgg ccc agc ggc 4997
Pro Gly Gly Pro Gly Gly Gly Ala Ser Tyr Ser Val Arg Pro Ser Gly
            1565                1570                1575

cgg tac ccc gtg gcg aga cga gcc ccg agc cca gtg aaa ccc gca tcg 5045
Arg Tyr Pro Val Ala Arg Arg Ala Pro Ser Pro Val Lys Pro Ala Ser
        1580                1585                1590

ctg gag cgg gtg gag ggg ctg ggg gcg ggc gtg gga ggc gcg ggg cgg 5093
Leu Glu Arg Val Glu Gly Leu Gly Ala Gly Val Gly Gly Ala Gly Arg
    1595                1600                1605

ccc ttc ggc ctc acg cct ccc acc atc ctc aag tcg tcc agc ctc tcc 5141
Pro Phe Gly Leu Thr Pro Pro Thr Ile Leu Lys Ser Ser Ser Leu Ser
1610                1615                1620                1625

atc ccg cac gaa ccc aag gaa gtg cgc ttc gtg gtg cga agt gcg agt 5189
Ile Pro His Glu Pro Lys Glu Val Arg Phe Val Val Arg Ser Ala Ser
                1630                1635                1640

gcg cgc agc cgc tcc ccc tca cca tct ccg ctg ccc tcg cct tct cct 5237
Ala Arg Ser Arg Ser Pro Ser Pro Ser Pro Leu Pro Ser Pro Ser Pro
            1645                1650                1655

ggc tct ggc ccc agt gcc ggc ccg cgt cgg cca ttt caa cag aag ccc 5285
Gly Ser Gly Pro Ser Ala Gly Pro Arg Arg Pro Phe Gln Gln Lys Pro
        1660                1665                1670

ctg cag ctt tgg agc aag ttc gat gtg ggc gac tgg ctg gag agc atc 5333
Leu Gln Leu Trp Ser Lys Phe Asp Val Gly Asp Trp Leu Glu Ser Ile
    1675                1680                1685

cac tta ggc gag cac cga gac cgc ttc gag gac cat gag atc gaa ggc 5381
His Leu Gly Glu His Arg Asp Arg Phe Glu Asp His Glu Ile Glu Gly
1690                1695                1700                1705

gca cac ctg cct gcg ctc acc aag gaa gac ttc gtg gag ctg gga gtc     5429
Ala His Leu Pro Ala Leu Thr Lys Glu Asp Phe Val Glu Leu Gly Val
            1710                1715                1720

aca cgc gtt ggc cac cgc atg aac atc gag cgt gcg ctc agg cag ctg     5477
Thr Arg Val Gly His Arg Met Asn Ile Glu Arg Ala Leu Arg Gln Leu
        1725                1730                1735

gat ggc agc tgacgcccct ctccctctcc tgttcctgct gcgccctgcc             5526
Asp Gly Ser
    1740

ggcagggccc ccacccctac tccaggccgc aggctcggct cgccccctac cacggcgccc   5586
gggccaggaa tgttgcatga atcgtcctgt ttgctgttgc ttggagactt gccctgtaca   5646
ttgcttagtg ccctcccctg ccgctgaacc ccacccagca cacagtaagg gcgcggacca   5706
ggggggctgg gtggaagggg gttggggcag ggtgctctgg cctgaccacc tcctccacag   5766
ctcctggtgg ccattcttcc agagggggaa cctagtccag catgcgaggt caggacacgc   5826
cttggtgact cggggggagg ggggagacat tggggttctc gataggggcc aaggagcccc   5886
ctgttttaca tattttaatc cactctatat ttggaaagag aaaaggaaca aatatctctg   5946
tccgtaacag ttcccgccct cttcccctca agtcctctcg ctggtcccgc cacagctacc   6006
cagtcttcca tctccggccc ctcactgcca ccccatatag ggcaggggac actccagctg   6066
gcctggggtt agccagggtc ctggcagccc accctgggga ccccggctca gcccccttcc   6126
ctcgctgagc tatagtatgc cccacccacc ctttaggtgc tgctcagggg gacgggtggc   6186
aggcattgcc tgctgggcac tagcagggcc aggtggcctg ggagattatt gccctggggc   6246
tgggccccgg taacccaacc ccagccatca tcttcacagg gtctctccca aaggaggggt   6306
ctaacctttc cccacttctt gggcaactac agcagagaag cctccctgcc tcgcgcccca   6366
aagactcccc aattcctgcc ctgtgtgtgt gcaccacatg tgtgtgtgca cgcctgcgtg   6426
cttgtgaaaa ttgggtgtgg ctgagcgcat gggtgccctg tatgtgcttg attgtggagt   6486
ggtccccagg ggctgttctg gatgggtggg aggttgagga agcttgcaca ggggtgcatg   6546
catgggtgtg tgcctgtgaa agggccctgt cttctccaaa gaaaggctgt cctgctcttg   6606
ggtcctgctg ttttctcagc ctgttctccc tgaacctcac ccagcttaag caggggttct   6666
tggtgaatcc tttcagcttt gggaggcctc aagggctccc gtgcaggcag cacccctttg   6726
ggcttctaag ggaattgtgg ggaccactaa aatcaggcca caacagccct tggagagagg   6786
caaagactcc tgagggtacc ctggcccccc ttactgtgac tcctcacaat tcagcaatga   6846
cctgtggggc ggggggcctt ggggcatttt taacataggg tttggagtct ggactaagct   6906
ccatccacgt cactcacaag tttctgtttc tatttctagc tttttttaat aaaatatata   6966
tatatatata tataaaagac agaaaacagg tgttttcatg gcccaggggc ttggcacgcc   7026
ggtctgtgcc cacccgcccc gccccaccct ggcccaccgg ccccattcct tagacacaga   7086
gtcacgccca ctaaccctct taccaacaga gcaggtcaca cacacagcag cggtcactgt   7146
aacagactgc cacatacaca gtctcacatt tacctgtggg tttttggttc tgttcagttt   7206
gggtttttaa ctttacaggg tcagttccgc ttcatccccc ttttgtatgg agttccatct   7266
cggggctttc aaccccctgc tccagtcctg aggcctcctg accctgacgt tgtgatacac   7326
cccacagaga tctatgtttc ttatattatt attattaata ataattatta taatattatg   7386
taataaattt ataagaaatg aaaaaaaaaa aaaaaaaa                           7424

<210> 38
<211> 1740
<212> PRT
<213> Rattus norvegicus

<400> 38
   Met Asp Gly Pro Gly Ala Ser Ala Val Val Val Arg Val Gly Ile Pro
   1               5                   10                  15
   Asp Leu Gln Gln Thr Lys Cys Leu Arg Leu Asp Pro Thr Ala Pro Val
               20                  25                  30
   Trp Ala Ala Lys Gln Arg Val Leu Cys Ala Leu Asn His Ser Leu Gln
           35                  40                  45
   Asp Ala Leu Asn Tyr Gly Leu Phe Gln Pro Pro Ser Arg Gly Arg Ala
       50                  55                  60
   Gly Lys Phe Leu Asp Glu Glu Arg Leu Leu Gln Asp Tyr Pro Pro Asn
   65                  70                  75                  80
   Leu Asp Thr Pro Leu Pro Tyr Leu Glu Phe Arg Tyr Lys Arg Arg Val
   85                  90                  95
   Tyr Ala Gln Asn Leu Ile Asp Asp Lys Gln Phe Ala Lys Leu His Thr
               100                 105                 110
   Lys Ala Asn Leu Lys Lys Phe Met Asp Tyr Val Gln Leu His Ser Thr
           115                 120                 125
   Asp Lys Val Ala Arg Leu Leu Asp Lys Gly Leu Asp Pro Asn Phe His
       130                 135                 140
   Asp Pro Asp Ser Gly Glu Cys Pro Leu Ser Leu Ala Ala Gln Leu Asp
   145                 150                 155                 160
   Asn Ala Thr Asp Leu Leu Lys Val Leu Arg Asn Gly Gly Ala His Leu
                   165                 170                 175
   Asp Phe Arg Thr Arg Asp Gly Leu Thr Ala Val His Cys Ala Thr Arg
               180                 185                 190
   Gln Arg Asn Ala Gly Ala Leu Thr Thr Leu Leu Asp Leu Gly Ala Ser
           195                 200                 205
   Pro Asp Tyr Lys Asp Ser Arg Gly Leu Thr Pro Leu Tyr His Ser Ala
       210                 215                 220
   Leu Gly Gly Gly Asp Ala Leu Cys Cys Glu Leu Leu Leu His Asp His
   225                 230                 235                 240
   Ala Gln Leu Gly Thr Thr Asp Glu Asn Gly Trp Gln Glu Ile His Gln
                   245                 250                 255
   Ala Cys Arg Phe Gly His Val Gln His Leu Glu His Leu Leu Phe Tyr
               260                 265                 270
   Gly Ala Asn Met Gly Ala Gln Asn Ala Ser Gly Asn Thr Ala Leu His
           275                 280                 285
   Ile Cys Ala Leu Tyr Asn Gln Glu Ser Cys Ala Arg Val Leu Leu Phe
       290                 295                 300
   Arg Gly Ala Asn Lys Asp Val Arg Asn Tyr Asn Ser Gln Thr Ala Phe
   305                 310                 315                 320
   Gln Val Ala Ile Ile Ala Gly Asn Phe Glu Leu Ala Glu Val Ile Lys
                   325                 330                 335
   Thr His Lys Asp Ser Asp Val Val Pro Phe Arg Glu Thr Pro Ser Tyr
               340                 345                 350
   Ala Lys Arg Arg Arg Leu Ala Gly Pro Ser Gly Leu Ala Ser Pro Arg
           355                 360                 365
   Pro Leu Gln Arg Ser Ala Ser Asp Ile Asn Leu Lys Gly Asp Gln Pro
       370                 375                 380
   Ala Ala Ser Pro Gly Pro Thr Leu Arg Ser Leu Pro His Gln Leu Leu
   385                 390                 395                 400
   Leu Gln Arg Leu Gln Glu Glu Lys Asp Arg Asp Arg Asp Gly Glu Gln
                   405                 410                 415
   Glu Asn Asp Ile Ser Gly Pro Ser Ala Gly Arg Gly Gly His Ser Lys
               420                 425                 430
   Ile Ser Pro Ser Gly Pro Gly Gly Ser Gly Pro Ala Pro Gly Pro Gly
           435                 440                 445
   Pro Ala Ser Pro Ala Pro Pro Ala Pro Pro Pro Arg Gly Pro Lys Arg
       450                 455                 460
   Lys Leu Tyr Ser Ala Val Pro Gly Arg Lys Phe Ile Ala Val Lys Ala
   465                 470                 475                 480
   His Ser Pro Gln Gly Glu Gly Glu Ile Pro Leu His Arg Gly Glu Ala
                   485                 490                 495
   Val Lys Val Leu Ser Ile Gly Glu Gly Gly Phe Trp Glu Gly Thr Val
               500                 505                 510
   Lys Gly Arg Thr Gly Trp Phe Pro Ala Asp Cys Val Glu Glu Val Gln
           515                 520                 525
   Met Arg Gln Tyr Asp Thr Arg His Glu Thr Arg Glu Asp Arg Thr Lys
       530                 535                 540
   Arg Leu Phe Arg His Tyr Thr Val Gly Ser Tyr Asp Ser Leu Thr Ser
   545                 550                 555                 560
   His Ser Asp Tyr Val Ile Asp Asp Lys Val Ala Ile Leu Gln Lys Arg
                   565                 570                 575
   Asp His Glu Gly Phe Gly Phe Val Leu Arg Gly Ala Lys Ala Glu Thr
               580                 585                 590
   Pro Ile Glu Glu Phe Thr Pro Thr Pro Ala Phe Pro Ala Leu Gln Tyr
           595                 600                 605
   Leu Glu Ser Val Asp Val Glu Gly Val Ala Trp Lys Ala Gly Leu Arg
       610                 615                 620
   Thr Gly Asp Phe Leu Ile Glu Val Asn Gly Val Asn Val Val Lys Val
   625                 630                 635                 640
   Gly His Lys Gln Val Val Gly Leu Ile Arg Gln Gly Gly Asn Arg Leu
                   645                 650                 655
   Val Met Lys Val Val Ser Val Thr Arg Lys Pro Glu Glu Asp Ser Ala
               660                 665                 670
   Arg Arg Arg Ala Pro Pro Pro Pro Lys Arg Ala Pro Ser Thr Thr Leu
           675                 680                 685
   Thr Leu Arg Ser Lys Ser Met Thr Ala Glu Leu Glu Glu Leu Ala Ser
       690                 695                 700
   Ile Arg Arg Arg Lys Gly Glu Lys Leu Asp Glu Ile Leu Ala Val Ala
   705                 710                 715                 720
   Ala Glu Pro Thr Leu Arg Pro Asp Ile Ala Asp Ala Asp Ser Arg Ala
                   725                 730                 735
   Ala Thr Val Lys Gln Arg Pro Thr Ser Arg Arg Ile Thr Pro Ala Glu
               740                 745                 750
   Ile Ser Ser Leu Phe Glu Arg Gln Gly Leu Pro Gly Pro Glu Lys Leu
           755                 760                 765
   Pro Gly Ser Leu Arg Lys Gly Ile Pro Arg Thr Lys Ser Val Gly Glu
       770                 775                 780
   Asp Glu Lys Leu Ala Ser Leu Leu Glu Gly Arg Phe Pro Arg Ser Thr
   785                 790                 795                 800
   Ser Met Gln Asp Thr Val Arg Glu Gly Arg Gly Ile Pro Pro Pro Pro
                   805                 810                 815
   Gln Thr Ala Pro Pro Pro Pro Pro Ala Pro Tyr Tyr Phe Asp Ser Gly
               820                 825                 830
   Pro Pro Pro Thr Phe Ser Pro Pro Pro Pro Pro Pro Gly Arg Ala Tyr
           835                 840                 845
   Asp Thr Val Arg Ser Ser Phe Lys Pro Gly Leu Glu Ala Arg Leu Gly
       850                 855                 860
   Ala Gly Ala Ala Gly Leu Tyr Asp Ser Gly Thr Pro Leu Gly Pro Leu
   865                 870                 875                 880
   Pro Tyr Pro Glu Arg Gln Lys Arg Ala Arg Ser Met Ile Ile Leu Gln
                   885                 890                 895
   Asp Ser Ala Pro Glu Val Gly Asp Val Pro Arg Pro Ala Pro Ala Ala
               900                 905                 910
   Thr Pro Pro Glu Arg Pro Lys Arg Arg Pro Arg Pro Ser Gly Pro Asp
           915                 920                 925
   Ser Pro Tyr Ala Asn Leu Gly Ala Phe Ser Ala Ser Leu Phe Ala Pro
       930                 935                 940
   Ser Lys Pro Gln Arg Arg Lys Ser Pro Leu Val Lys Gln Leu Gln Val
   945                 950                 955                 960
   Glu Asp Ala Gln Glu Arg Ala Ala Leu Ala Val Gly Ser Pro Gly Pro
                   965                 970                 975
   Val Gly Gly Ser Phe Ala Arg Glu Pro Ser Pro Thr His Arg Gly Pro
               980                 985                 990
   Arg Pro Gly Gly Leu Asp Tyr Ser Ser Gly Glu Gly Leu Gly Leu Thr
           995                 1000                1005
   Phe Gly Gly Pro Ser Pro Gly Pro Val Lys Glu Arg Arg Leu Glu Glu
       1010                1015                1020
   Arg Arg Arg Ser Thr Val Phe Leu Ser Val Gly Ala Ile Glu Gly Asn
   1025                1030                1035                1040
   Pro Pro Ser Ala Asp Leu Pro Ser Leu Gln Pro Ser Arg Ser Ile Asp
                   1045                1050                1055
   Glu Arg Leu Leu Gly Thr Gly Ala Thr Thr Gly Arg Asp Leu Leu Leu
               1060                1065                1070
   Pro Ser Pro Val Ser Ala Leu Lys Pro Leu Val Gly Gly Pro Asn Leu
           1075                1080                1085
   Gly Pro Ser Ser Ser Thr Phe Ile His Pro Leu Thr Gly Lys Pro Leu
   1090 1095 1100
   Asp Pro Ser Ser Pro Leu Ala Leu Ala Leu Ala Ala Arg Glu Arg Ala
   1105 1110 1115 1120
   Leu Ala Ser Gln Thr Pro Ser Arg Ser Pro Thr Pro Val His Ser Pro
   1125 1130 1135
   Asp Ala Asp Arg Pro Gly Pro Leu Phe Val Asp Val Gln Thr Arg Asp
   1140 1145 1150
   Ser Glu Arg Gly Pro Leu Ala Ser Pro Ala Phe Ser Pro Arg Ser Pro
   1155 1160 1165
   Ala Trp Ile Pro Val Pro Ala Arg Arg Glu Ala Glu Lys Pro Thr Arg
   1170 1175 1180
   Glu Glu Arg Lys Ser Pro Glu Asp Lys Lys Ser Met Ile Leu Ser Val
   1185 1190 1195 1200
   Leu Asp Thr Ser Leu Gln Arg Pro Ala Gly Leu Ile Val Val His Ala
   1205 1210 1215
   Thr Ser Asn Gly Gln Glu Pro Asn Arg Leu Gly Ala Glu Glu Glu Arg
   1220 1225 1230
   Pro Gly Thr Pro Glu Leu Ala Pro Thr Pro Met Gln Ala Ala Ala Val
   1235 1240 1245
   Ala Glu Pro Met Pro Ser Pro Arg Ala Gln Pro Pro Gly Asn Ile Pro
   1250 1255 1260
   Ala Asp Pro Gly Pro Ser Gln Gly Asn Ser Glu Glu Glu Pro Lys Leu
   1265 1270 1275 1280
   Val Phe Ala Val Asn Leu Pro Pro Ala Gln Leu Ser Ser Asn Asp Glu
   1285 1290 1295
   Glu Thr Arg Glu Glu Leu Ala Arg Ile Gly Leu Val Pro Pro Pro Glu
   1300 1305 1310
   Glu Phe Ala Asn Gly Ile Leu Leu Ala Thr Pro Pro Pro Gly Pro Gly
   1315 1320 1325
   Pro Leu Pro Thr Thr Val Pro Ser Pro Ala Ser Gly Lys Pro Ser Ser
   1330 1335 1340
   Glu Leu Pro Pro Ala Pro Glu Ser Ala Ala Asp Ser Gly Val Glu Glu
   1345 1350 1355 1360
   Ala Asp Thr Arg Ser Ser Ser Asp Pro His Leu Glu Thr Thr Ser Thr
   1365 1370 1375
   Ile Ser Thr Val Ser Ser Met Ser Thr Leu Ser Ser Glu Ser Gly Glu
   1380 1385 1390
   Leu Thr Asp Thr His Thr Ser Phe Ala Asp Gly His Thr Phe Leu Leu
   1395 1400 1405
   Glu Lys Pro Pro Val Pro Pro Lys Pro Lys Leu Lys Ser Pro Leu Gly
   1410 1415 1420
   Lys Gly Pro Val Thr Phe Arg Gly Pro Leu Leu Lys Gln Ser Ser Asp
   1425 1430 1435 1440
   Ser Glu Leu Met Ala Gln Gln His His Ala Thr Ser Thr Gly Leu Thr
   1445 1450 1455
   Ser Ala Ala Gly Pro Ala Arg Pro Arg Tyr Leu Phe Gln Arg Arg Ser
   1460 1465 1470
   Lys Leu Trp Gly Asp Pro Val Glu Ser Arg Gly Leu Pro Gly Pro Glu
   1475 1480 1485
   Asp Asp Lys Pro Thr Val Ile Ser Glu Leu Ser Ser Arg Leu Gln Gln
   1490 1495 1500
   Leu Asn Lys Asp Thr Arg Ser Leu Gly Glu Glu Pro Val Gly Gly Leu
   1505 1510 1515 1520
   Gly Ser Leu Leu Asp Pro Ala Lys Lys Ser Pro Ile Ala Ala Ala Arg
   1525 1530 1535
   Cys Ala Val Val Pro Ser Ala Gly Trp Leu Phe Ser Ser Leu Gly Glu
   1540 1545 1550
   Leu Ser Thr Ile Ser Ala Gln Arg Ser Pro Gly Gly Pro Gly Gly Gly
   1555 1560 1565
   Ala Ser Tyr Ser Val Arg Pro Ser Gly Arg Tyr Pro Val Ala Arg Arg
   1570 1575 1580
   Ala Pro Ser Pro Val Lys Pro Ala Ser Leu Glu Arg Val Glu Gly Leu
   1585 1590 1595 1600
   Gly Ala Gly Val Gly Gly Ala Gly Arg Pro Phe Gly Leu Thr Pro Pro
                   1605                1610                1615
   Thr Ile Leu Lys Ser Ser Ser Leu Ser Ile Pro His Glu Pro Lys Glu
               1620                1625                1630
   Val Arg Phe Val Val Arg Ser Ala Ser Ala Arg Ser Arg Ser Pro Ser
           1635                1640                1645
   Pro Ser Pro Leu Pro Ser Pro Ser Pro Gly Ser Gly Pro Ser Ala Gly
       1650                1655                1660
   Pro Arg Arg Pro Phe Gln Gln Lys Pro Leu Gln Leu Trp Ser Lys Phe
   1665                1670                1675                1680
   Asp Val Gly Asp Trp Leu Glu Ser Ile His Leu Gly Glu His Arg Asp
                   1685                1690                1695
   Arg Phe Glu Asp His Glu Ile Glu Gly Ala His Leu Pro Ala Leu Thr
               1700                1705                1710
   Lys Glu Asp Phe Val Glu Leu Gly Val Thr Arg Val Gly His Arg Met
           1715                1720                1725
   Asn Ile Glu Arg Ala Leu Arg Gln Leu Asp Gly Ser
       1730                1735                1740

<210> 39
<211> 474
<212> DNA
<213> Homo sapiens

<220>
<221> CDS
<222> (1)...(435)
<223> Homer 3a

<400> 39
atg tcc aca gcc agg gag cag cca atc ttc agc aca cgg gcg cac gtg      48
Met Ser Thr Ala Arg Glu Gln Pro Ile Phe Ser Thr Arg Ala His Val
 1               5                  10                  15

ttc caa att gac cca gcc acc aag cga aac tgg atc cca gcg ggc aag      96
Phe Gln Ile Asp Pro Ala Thr Lys Arg Asn Trp Ile Pro Ala Gly Lys
            20                  25                  30

cac gca ctc act gtc tcc tat ttc tac gat gcc acc cgc aat gtg tac     144
His Ala Leu Thr Val Ser Tyr Phe Tyr Asp Ala Thr Arg Asn Val Tyr
        35                  40                  45

cgc atc atc agc atc gga ggc gcc aag gcc atc atc aac agc act gtc     192
Arg Ile Ile Ser Ile Gly Gly Ala Lys Ala Ile Ile Asn Ser Thr Val
    50                  55                  60

act ccc aac atg acc ttc acc aaa act tcc cag aag ttc ggg cag tgg     240
Thr Pro Asn Met Thr Phe Thr Lys Thr Ser Gln Lys Phe Gly Gln Trp
65                  70                  75                  80

gcc gac agt cgc gcc aac aca gtc tat ggc ctg ggc ttt gcc tct gaa     288
Ala Asp Ser Arg Ala Asn Thr Val Tyr Gly Leu Gly Phe Ala Ser Glu
                85                  90                  95

cag cat ctg aca cag ttt gcc gag aag ttc cag gaa gtg aag gaa gca     336
Gln His Leu Thr Gln Phe Ala Glu Lys Phe Gln Glu Val Lys Glu Ala
            100                 105                 110

gcc agg ctg gcc agg gag aaa tct cag gat ggc tgg ggt ggg ccc cag     384
Ala Arg Leu Ala Arg Glu Lys Ser Gln Asp Gly Trp Gly Gly Pro Gln
        115                 120                 125

tcg gct ctg gtt gtt ggc agc ttt ggg gct gtt ttt gag ctt ctc att     432

Ser Ala Leu Val Val Gly Ser Phe Gly Ala Val Phe Glu Leu Leu Ile
    130                 135                 140

gtg tagaatttct agatcccccg attacatttc taagcgtga                474
Val
145

<210> 40
<211> 145
<212> PRT
<213> Homo sapiens

<400> 40
Met Ser Thr Ala Arg Glu Gln Pro Ile Phe Ser Thr Arg Ala His Val
 1               5                  10                  15
Phe Gln Ile Asp Pro Ala Thr Lys Arg Asn Trp Ile Pro Ala Gly Lys
            20                  25                  30
His Ala Leu Thr Val Ser Tyr Phe Tyr Asp Ala Thr Arg Asn Val Tyr
        35                  40                  45
Arg Ile Ile Ser Ile Gly Gly Ala Lys Ala Ile Ile Asn Ser Thr Val
    50                  55                  60
Thr Pro Asn Met Thr Phe Thr Lys Thr Ser Gln Lys Phe Gly Gln Trp
65                  70                  75                  80
Ala Asp Ser Arg Ala Asn Thr Val Tyr Gly Leu Gly Phe Ala Ser Glu
                85                  90                  95
Gln His Leu Thr Gln Phe Ala Glu Lys Phe Gln Glu Val Lys Glu Ala
            100                 105                 110
Ala Arg Leu Ala Arg Glu Lys Ser Gln Asp Gly Trp Gly Gly Pro Gln
        115                 120                 125
Ser Ala Leu Val Val Gly Ser Phe Gly Ala Val Phe Glu Leu Leu Ile
    130                 135                 140
Val
145

<210> 41
<211> 859
<212> DNA
<213> Rattus norvegicus

<220>
<221> CDS
<222> (1)...(858)
<223> INADL

<400> 41
cac gcg tcc ggt gtg gtg cac ctt ggc atc tgt aag cct ttg gtg gag      48
His Ala Ser Gly Val Val His Leu Gly Ile Cys Lys Pro Leu Val Glu
 1               5                  10                  15

gag gag aag gag gag aag gag gaa cat ttt att ttc cat tca aac aac      96
Glu Glu Lys Glu Glu Lys Glu Glu His Phe Ile Phe His Ser Asn Asn
            20                  25                  30

aat gga gat aac agt gag tct cca gaa acc gtt cac gag atc cac tca     144
Asn Gly Asp Asn Ser Glu Ser Pro Glu Thr Val His Glu Ile His Ser
        35                  40                  45

tct tta atc ctc gag gca ccc cag gga ttt aga gat gag ccg tat ctt     192
Ser Leu Ile Leu Glu Ala Pro Gln Gly Phe Arg Asp Glu Pro Tyr Leu
    50                  55                  60

gaa gaa ctc gtg gat gaa cct ttt cta gat ttg gga aag tct ttg cag     240

Glu Glu Leu Val Asp Glu Pro Phe Leu Asp Leu Gly Lys Ser Leu Gln
65                  70                  75                  80

ttc caa caa aaa gac atg gac agc agc tca gaa gcc tgg gaa atg cat      288
Phe Gln Gln Lys Asp Met Asp Ser Ser Ser Glu Ala Trp Glu Met His
                85                  90                  95

gaa ttc ctg agc cct cgg ctg gag aga agg ggt gag gaa aga gag atg      336
Glu Phe Leu Ser Pro Arg Leu Glu Arg Arg Gly Glu Glu Arg Glu Met
            100                 105                 110

ctt gtt gac gag gag tat gag atc tac caa gac cgc ctc cgg gac atg      384
Leu Val Asp Glu Glu Tyr Glu Ile Tyr Gln Asp Arg Leu Arg Asp Met
        115                 120                 125

gaa gca cac cca cca cct cct cac att cgg gag ccc act tct gca tct      432
Glu Ala His Pro Pro Pro Pro His Ile Arg Glu Pro Thr Ser Ala Ser
    130                 135                 140

ccc agg ctg gat ctc cag gcc ggc ccc cag tgg ctg cat gct gac ctc      480
Pro Arg Leu Asp Leu Gln Ala Gly Pro Gln Trp Leu His Ala Asp Leu
145                 150                 155                 160

tca gga gga gag ata ctc gag tgt cac gac aca gag tcc atg atg act      528
Ser Gly Gly Glu Ile Leu Glu Cys His Asp Thr Glu Ser Met Met Thr
                165                 170                 175

gct tat ccc cag gag atg cag gac tat agc ttc agc acc aca gac atg      576
Ala Tyr Pro Gln Glu Met Gln Asp Tyr Ser Phe Ser Thr Thr Asp Met
            180                 185                 190

atg aaa gaa aca ttt ggc ctt gac tcc cgg ccg ccc atg ccc tcc tct      624
Met Lys Glu Thr Phe Gly Leu Asp Ser Arg Pro Pro Met Pro Ser Ser
        195                 200                 205

gaa gga aat ggt cag cac ggc cga ttt gat gac ttg gaa cat ctt cat      672
Glu Gly Asn Gly Gln His Gly Arg Phe Asp Asp Leu Glu His Leu His
    210                 215                 220

tca cta gca agc cac ggc ctg gat tta ggc atg atg act cca agt gac      720
Ser Leu Ala Ser His Gly Leu Asp Leu Gly Met Met Thr Pro Ser Asp
225                 230                 235                 240

ttg caa ggc cct ggc gtg ctt gta gat ctt cca gct gtc acc cca aga      768
Leu Gln Gly Pro Gly Val Leu Val Asp Leu Pro Ala Val Thr Pro Arg
                245                 250                 255

aga ggc tgc ggc cgc taa gta agt aag acg tcg agc tct aag taa gta      816
Arg Gly Cys Gly Arg  *  Val Ser Lys Thr Ser Ser Ser Lys  *  Val
            260                 265                 270

acg gcc gcc acc gcg gtg gag ctt tgg act tct tcg cca gag              858
Thr Ala Ala Thr Ala Val Glu Leu Trp Thr Ser Ser Pro Glu
                275                 280

g                                                                    859

<210> 42
<211> 284
<212> PRT
<213> Rattus norvegicus

<400> 42

His Ala Ser Gly Val Val His Leu Gly Ile Cys Lys Pro Leu Val Glu
1               5                   10                  15
Glu Glu Lys Glu Glu Lys Glu Glu His Phe Ile Phe His Ser Asn Asn
            20                  25                  30
Asn Gly Asp Asn Ser Glu Ser Pro Glu Thr Val His Glu Ile His Ser
        35                  40                  45
Ser Leu Ile Leu Glu Ala Pro Gln Gly Phe Arg Asp Glu Pro Tyr Leu
    50                  55                  60
Glu Glu Leu Val Asp Glu Pro Phe Leu Asp Leu Gly Lys Ser Leu Gln
65                  70                  75                  80
Phe Gln Gln Lys Asp Met Asp Ser Ser Ser Glu Ala Trp Glu Met His
                85                  90                  95
Glu Phe Leu Ser Pro Arg Leu Glu Arg Arg Gly Glu Glu Arg Glu Met
            100                 105                 110
Leu Val Asp Glu Glu Tyr Glu Ile Tyr Gln Asp Arg Leu Arg Asp Met
        115                 120                 125
Glu Ala His Pro Pro Pro Pro His Ile Arg Glu Pro Thr Ser Ala Ser
    130                 135                 140
Pro Arg Leu Asp Leu Gln Ala Gly Pro Gln Trp Leu His Ala Asp Leu
145                 150                 155                 160
Ser Gly Gly Glu Ile Leu Glu Cys His Asp Thr Glu Ser Met Met Thr
                165                 170                 175
Ala Tyr Pro Gln Glu Met Gln Asp Tyr Ser Phe Ser Thr Thr Asp Met
            180                 185                 190
Met Lys Glu Thr Phe Gly Leu Asp Ser Arg Pro Pro Met Pro Ser Ser
        195                 200                 205
Glu Gly Asn Gly Gln His Gly Arg Phe Asp Asp Leu Glu His Leu His
    210                 215                 220
Ser Leu Ala Ser His Gly Leu Asp Leu Gly Met Met Thr Pro Ser Asp
225                 230                 235                 240
Leu Gln Gly Pro Gly Val Leu Val Asp Leu Pro Ala Val Thr Pro Arg
                245                 250                 255
Arg Gly Cys Gly Arg Val Ser Lys Thr Ser Ser Ser Lys Val Thr Ala
            260                 265                 270
Ala Thr Ala Val Glu Leu Trp Thr Ser Ser Pro Glu
        275                 280

<210> 43
<211> 5
<212> PRT
<213> Artificial Sequence

<220>
<223> optimal ligand

<400> 43
Phe Pro Pro Pro Pro
1               5

<210> 44
<211> 4
<212> PRT
<213> Artificial Sequence

<220>
<223> motif ligand

<400> 44
Lys Ile Ala Ala
1

<210> 45
<211> 21

<212> DNA
<213> Artificial Sequence

<220>
<223> oligonucleotide for PCR

<400> 45
gacagcagag ccaacaccgt g                                                21

<210> 46
<211> 21
<212> DNA
<213> Artificial Sequence

<220>
<223> oligonucleotide for PCR

<400> 46
gtctgcagct ccatctccca c                                                21

<210> 47
<211> 21
<212> DNA
<213> Artificial Sequence

<220>
<223> oligonucleotide for PCR

<400> 47
cacggtgttg gctctgctgt c                                                21

<210> 48
<211> 21
<212> DNA
<213> Artificial Sequence

<220>
<223> degenerate primer

<221> misc_feature
<222> (0)...(0)
<223> r = A or G;  y = C or T;  v = A, G, or C;  b = G,
      C, or T.

<400> 48
atgggvgarc arccbatytt c                                                21

<210> 49
<211> 7
<212> PRT
<213> Artificial Sequence

<220>
<223> conserved amino acid sequence

<400> 49
Met Gly Glu Gln Pro Ile Phe
 1               5

<210> 50
<211> 21
<212> DNA
<213> Artificial Sequence

<220>
<223> oligonucleotide for PCR

<400> 50
gagggtagcc agttcagcct c                                                21

<210> 51
<211> 21
<212> DNA
<213> Artificial Sequence

<220>
<223> oligonucleotide for PCR

<400> 51
gttgatctca ctgcattgtt c                                                21

<210> 52
<211> 18
<212> PRT
<213> Artificial Sequence

<220>
<223> peptide from Homer 1b/c

<400> 52
Ile Phe Glu Leu Thr Glu Leu Arg Asp Asn Leu Ala Lys Leu Leu Glu
1               5                   10                  15
Cys Ser

<210> 53
<211> 20
<212> PRT
<213> Artificial Sequence

<220>
<223> peptide from Homer 2a/b

<400> 53
Gly Lys Ile Asp Asp Leu His Asp Phe Arg Arg Gly Leu Ser Lys Leu
1               5                   10                  15
Gly Thr Asp Asn
            20

<210> 54
<211> 19
<212> PRT
<213> Artificial Sequence

<220>
<223> peptide from Homer 3

<400> 54
Arg Leu Phe Glu Leu Ser Glu Leu Arg Glu Gly Leu Ala Arg Leu Ala
1               5                   10                  15
Glu Ala Ala

<210> 55
<211> 10
<212> PRT

<213> Artificial Sequence

<220>
<223> peptide with Homer ligand peptide consensus

<400> 55
Leu Val Pro Pro Pro Glu Glu Phe Ala Asn
 1               5                   10

<210> 56
<211> 10
<212> PRT
<213> Artificial Sequence

<220>
<223> peptide with Homer ligand peptide consensus

<400> 56
Pro Leu Pro Pro Pro Leu Glu Phe Ser Asn
 1               5                   10

<210> 57
<211> 10
<212> PRT
<213> Artificial Sequence

<220>
<223> peptide with Homer ligand peptide consensus

<400> 57
Pro Leu Pro Pro Pro Leu Glu Phe Ala Asn
 1               5                   10

<210> 58
<211> 10
<212> PRT
<213> Artificial Sequence

<220>
<223> peptide with Homer ligand peptide consensus

<400> 58
Phe Leu Pro Pro Pro Glu Ser Phe Asp Ala
 1               5                   10

## Description

### STATEMENT AS TO FEDERALLY SPONSORED RESEARCH

This invention was made with Government support under Grant No. R01 DA10309, RO1 DA11742 and KO2 MHO1152, awarded by the National Institutes of Health. The government may have certain rights in the invention.

### FIELD OF THE INVENTION

The present invention relates generally to protein-protein interactions and more specifically to molecules involved in mediating receptor-activated or ion channel-mediated intracellular calcium mobilization or concentration.

### BACKGROUND OF THE INVENTION

The mature central nervous system exhibits the capacity to alter cellular interactions as a function of the activity of specific neuronal circuits. This capacity is believed to underlie learning and memory storage, age-related memory loss, tolerance to and dependence on drugs of abuse, recovery from brain injury, epilepsy as well as aspects of postnatal development of the brain (Schatz, *C., Neuron*, 5:745, 1990). Currently, the role of activity-dependent synaptic plasticity is best understood in the context of learning and memory. Cellular mechanisms underlying activity-dependent plasticity are known to be initiated by rapid, transmitter-induced changes in membrane conductance properties and activation of intracellular signaling pathways (Bliss and Collingridge, *Nature*, 361:31, 1993). Several lines of evidence also indicate a role for rapid synthesis of mRNA and protein in long-term neuroplasticity. For example, classical studies of learning and memory demonstrate a requirement for protein synthesis in long-term, but not short-term memory (Flexner, *et al., Science,* 141:57, 1963; Agranoff, B., *Basic Neurochemistry, 3rd Edition,* 1981; Davis and Squire, *Physiol. Bull.,* 96:518, 1984), and long-term enhancement of synaptic connectivity, studied in cultured invertebrate neurons (Montarolo, *et al., Science,* 234:1249, 1986; Bailey, *et al., Neuron,* 9:749, 1992) or in the rodent hippocampus (Frey, *et al., Science,* 260:1661, 1993; Nguyen*, et al., Science,* 265:1104, 1994), is blocked by inhibitors of either RNA or protein synthesis. Importantly, inhibitors of macromolecular synthesis are most effective when administered during a brief time window surrounding the conditioning stimulus indicating a special requirement for molecules that are rapidly induced (Goelet, *et al., Nature,* 322:419, 1986).

Immediate early genes (IEGs) are rapidly induced in neurons by neurotransmitter stimulation and synaptic activity and are hypothesized to be part of the macromolecular response required for long-term plasticity (Goelet, *et al*., supra; Sheng and Greenberg, *Neuron,* 4:477, 1990; Silva and Giese, *Neurobiology,* 4:413, 1994). To identify cellular mechanisms that may contribute to long-term plasticity in the vertebrate brain, differential cloning techniques have been used to identify genes that are rapidly induced by depolarizing stimuli (Nedivi, *et al., Nature*, 363:713, 1993; Qian, *et al., Nature,* 361:453, 1993; Yamagata, *et al., Neuron,* 11:371, 1993; Yamagata, *et al., Learning and Memory* 1:140, 1994; Yamagata, *et al., Journal of Biological Chemistry,* 269:16333, 1994; Andreasson and Worley, *Neuroscience,* 69:781, 1995; Lyford, *et al., Neuron,* 14:433, 1995). In contrast to the earlier focus on transcription factors, many of the newly characterized IEGs represent molecules that can directly modify the function of cells and include growth factors (Nedivi, *et al., supra*; Andreasson and Worley, *supra*), secreted enzymes that can modify the extracellular matrix, such as tissue plasminogen activator (Qian, *et al., supra*), enzymes involved in intracellular signaling, such as prostaglandin synthase (Yamagata, *et al., supra*), and a novel homolog of H-Ras, termed Rheb (Yamagata, *et al., supra*), as well as a novel cytoskeleton-associated protein, termed Arc (Lyford, *et al., supra*). The remarkable functional diversity of this set of rapid response genes is representative of the repertoire of cellular mechanisms that are likely to contribute to activity-dependent neuronal plasticity.

Pharmaceutical agents often act by modulating signaling between cells or within cells. For example, Prozac alters the reuptake of the neurotransmitter serotonin and enhances aspects of its signaling function in brain. Nonsteroidal antiinflammatory drugs (NSAIDs) act by inhibiting the activity of cyclooxygenase enzyme, which is involved in the signaling pathways of inflammation. Viagra modifies the intracellular guanylate cyclase response to autonomic neurotransmitters in erectile tissues. These, and other precedent setting pharmaceuticals, validate the notion that specific signaling pathways may be targeted for therapeutic development.

Cellular mechanisms that modify important intracellular signals can involve changes in intracellular calcium. This type of mechanism is used in brain neurons to adapt to changes in intercellular signaling, and is demonstrated to exert powerful effects on cellular responses induced by glutamate. Similar, though distinct, cellular mechanism may be used to modulate intracellular calcium signals in other tissues including heart, lung, liver and skeletal muscle. Compounds that can modify this mechanism can modulate natural transmitter signals and may exert therapeutic effects.

Classical studies demonstrated that activation of receptors on the cell surface evoke changes in the level of specific, diffusable molecules inside the cell. The regulated production of these molecules serves to signal events happening at the membrane surface to intracellular receptors and are therefore termed second messenger signaling pathways. Major second messenger pathways include the phosphoinositide pathway, which regulates intracellular calcium; the adenylate cyclase pathway, which regulates levels of cyclic AMP; the guanylate cyclase pathway, which regulates levels of cGMP; and the nitric oxide pathway which regulates NO.

The regulated release of intracellular calcium is essential to the function of all tissues. Each tissue possesses a distinct physiology that is dependent on receptor/transmitter-regulated release of intracellular calcium. For example, synaptic function is modulated in brain neurons by glutamate receptor regulated release of intracellular calcium. Contractility of cardiac and smooth muscle is also regulated by intracellular calcium. Recent reviews of the role of calcium signaling in cellular responses include: Berridge, *Nature* 386:759 (1997); Berridge, *J. Physiol. (London)* 499:291 (1997); Bootman *et al., Cell* 91:367 (1997).

Recent studies demonstrate that molecules that function together in signaling networks are frequently clustered together in macromolecular complexes. For example, components of the MAP kinase pathway form a complex of cytosolic kinases with their specific substrates (Davis, *Mol. Reprod. Dev*. 42:459 (1995)). Similarly, proteins such as AKAP function as scaffolds for specific kinases and their substrates (Lester and Scott, *Recent Prog. Horm. Res.* 52:409 (1997)). Recently, a multi-PDZ containing protein was identified in *Drosophila* (termed InaD) that couples the membrane-associated, light-activated ion channel with its effector enzymes (Tsunoda *et al., Nature* 388:243 (1997)). The biochemical consequence of this clustering is that the local concentrations of molecules that convey the signals between proteins are as high as possible. Consequently, signaling takes place efficiently. The clustering activity of these proteins is essential to normal function of the signaling cascade (Lester and Scott, *supra* 1997; Tsunoda *et al., supra* 1997). Accordingly, Accordingly, agents that alter these signaling complexes will modify the response due to transmitter or other form of cellular stimulation in a way that mimics more classical receptor agonists or antagonists. For example, a metabotropic glutamate receptor signaling may be blocked either at the receptor by conventional receptor antagonists or by uncoupling the metabotropic receptor from its intracellular IP3 receptor by agents that block the cross-linking activity of Homer family proteins.

The identification of molecules regulating the aggregation of neurotransmitter receptors at synapses is central to understanding the mechanisms of neural development, synaptic plasticity and learning. The most well characterized model for the synaptic aggregation of ionotropic receptors is the neuromuscular junction. Early work showed that contact between the axon of a motor neuron and the surface of a myotube rapidly triggers the accumulation of preexisting surface acetylcholine receptors (Anderson and Cohen, *J Physiol* 268:757-773, 1977; Frank and Fischbach, *J Cell Biol* 83:143-158, 1979). Subsequent work has shown that agrin, a complex glycoprotein secreted by the presynaptic terminal, activates a postsynaptic signal transduction cascade (reviewed by Colledge and Froehner, *Curr Opin Neurobiol* 8:357-63, 1998), that leads to receptor clustering by the membrane associated protein rapsyn.

### SUMMARY OF THE INVENTION

Homer proteins, the products of neuronal immediate early genes, selectively bind the carboxy-termini of certain cell-surface receptors (*e*.*g*., group 1 metabotropic receptors), certain intracellular receptors and binding proteins (*e.g*., inositol trisphosphate receptors, ryanodine receptor, Shank proteins, I42). Many forms of Homer proteins contain a "coiled-coil" structure in the carboxy-terminal domain which mediates homo- and heteromultimerization between Homer proteins. The present invention is based on the seminal discovery that Homer plays a significant role in mediating receptor-activated calcium mobilization from internal stores and that Homer proteins regulate aspects of receptor clustering

In one embodiment, a method is provided for identifying a compound that modulates a cellular response mediated by a cell-surface receptor. The method includes incubating a test compound and a cell expressing a cell-surface receptor and a Homer protein under conditions sufficient to permit the compound to interact with the cell, and exposing the cell to a cell-surface receptor ligand. A cellular response to the ligand by the cell incubated with the compound is compared with a cellular response of the cell not incubated with the compound wherein a difference in cellular response identify a compound that modulates a Homer-associated cellular response.

In another embodiment, a method is provided for identifying a compound that modulates a cellular response mediated by an intracellular receptor. The method includes incubating the compound, and a cell expressing an intracellular receptor and a Homer protein under conditions sufficient to permit the compound to interact with the cell and exposing the cell to conditions that activate the intracellular receptor. A cellular response by a cell incubated with the compound is compared with a cellular response of a cell not incubated with the compound wherein a difference in a cellular response identifies a compound that modulates a Homer-associated cellular response.

In yet another embodiment, a method is provided for identifying a compound that modulates receptor activated calcium mobilization in a cell. The method includes incubating the compound and a cell expressing a Homer protein under conditions sufficient to permit the compound to interact with the cell and exposing the cell to conditions sufficient to activate calcium mobilization. The receptor-activated calcium mobilization of a cell incubated with said the compound is compared with the receptor-activated calcium mobilization of a cell not incubated with the compound wherein a difference in calcium mobilization is indicative of an effect of the compound on Homer-associated calcium mobilization.

In another embodiment, a method is provided for modulating receptor-mediated calcium mobilization. The method includes exposing a cell expressing Homer protein to a compound in a sufficient amount to modulate the calcium mobilization that typically occurs when a cell is exposed to an amount of ligand sufficient to activate an intercellualr signaling pathway that includes Homer protein.

In another embodiment, a method is provided for identifying a compound that inhibits Homer protein activity. The method includes identifying an inhibitor of Homer binding or crosslinking activity and identifying an inhibitor of Homer protein activity that forms covalent or non-covalent bonds with amino acids in a Homer protein binding site, based upon the crystal structure coordinates of Homer protein binding domain. and synthesizing the inhibitor.

In one embodiment, a method is provided for identifying a compound that affects the formation of cell surface receptors into clusters. The method includes incubating the compound and a cell expressing a Homer protein and a Homer interacting protein, e.g., a Shank protein, under conditions sufficient to allow the compound to interact with the cell and determining the effect of the compound on the formation of cell-surface receptors into clusters. The formation of cell-surface receptors into clusters of a cell contacted with the compound is compared to the formation of cell-surface receptors into clusters of a cell not contacted with the compound, wherein a difference in the formation of clusters is indicative of a compound that affects formation of cell surface receptors into clusters.

In another embodiment, a method is provided for treating a disorder associated with glutamate receptors, including metabotropic and NMDA-type glutamate receptors, in a subject. The method includes administering to a subject in need, a therapeutically effective amount of a compound that modulates Homer protein activity.

In another embodiment, a method is provided for treating a disorder associated with Homer protein activity including administering to a subject in need a therapeutically effective amount of a compound that modulates Homer protein activity. The compound may be identified by a method of the invention described herein.

In another embodiment, there is provided an isolated nucleic acid encoding Homer protein 1b, having the nucleotide sequence as set forth in SEQ ID NO:3 as well as an isolated Homer protein having substantially the same amino acid sequence as set forth in SEQ ID NO:4.

In another embodiment, there is provided an isolated nucleic acid encoding Homer protein 1c, having the nucleotide sequence as set forth in SEQ ID NO:5 as well as an isolated Homer protein having substantially the same amino acid sequence as set forth in SEQ ID NO:6.

In another embodiment, there is provided an isolated nucleic acid encoding Homer protein 2a, having the nucleotide sequence as set forth in SEQ ID NO:7 as well as an isolated Homer protein having substantially the same amino acid sequence as set forth in SEQ ID NO:8.

In another embodiment, there is provided an isolated nucleic acid encoding Homer protein 2b, having the nucleotide sequence as set forth in SEQ ID NO:9 as well as an isolated Homer protein having substantially the same amino acid sequence as set forth in SEQ ID NO:10.

In another embodiment, there is provided an isolated nucleic acid encoding Homer protein 3, having the nucleotide sequence as set forth in SEQ ID NO:11 as well as an isolated Homer protein having substantially the same amino acid sequence as set forth in SEQ ID NO:12.

In another embodiment, there is provided an isolated peptide having the amino acid sequence set forth in SEQ ID NO:13 an isolated peptide having the amino acid sequence set forth in SEQ ID NO:14.

In yet another embodiment, there is provided an isolated nucleic acid encoding Homer Interacting Protein, having the nucleotide sequence as set forth in SEQ ID NO: 15 or 17 with a deduced amino acid sequence as set forth in SEQ ID NO: 16 or 18, respectively.

In another embodiment, there is provided an isolated Homer Interacting Protein having substantially the same amino acid sequence as set forth in SEQ ID NO:19.

In another embodiment, there is provided an isolated Homer Interacting Protein having substantially the same amino acid sequence as set forth in SEQ ID NO:20.

In yet a further embodiment, there is provided a substantially purified polypeptide containing a proline rich region that is specifically capable of specifically binding to polypeptides of the Homer family.

In still another embodiment, there is provided a transgenic non-human animal having a transgene that expresses a Homer protein, e.g., Homer 1a, chromosomally integrated into the germ cells of the animal.

### BRIEF DESCRIPTION OF THE DRAWINGS

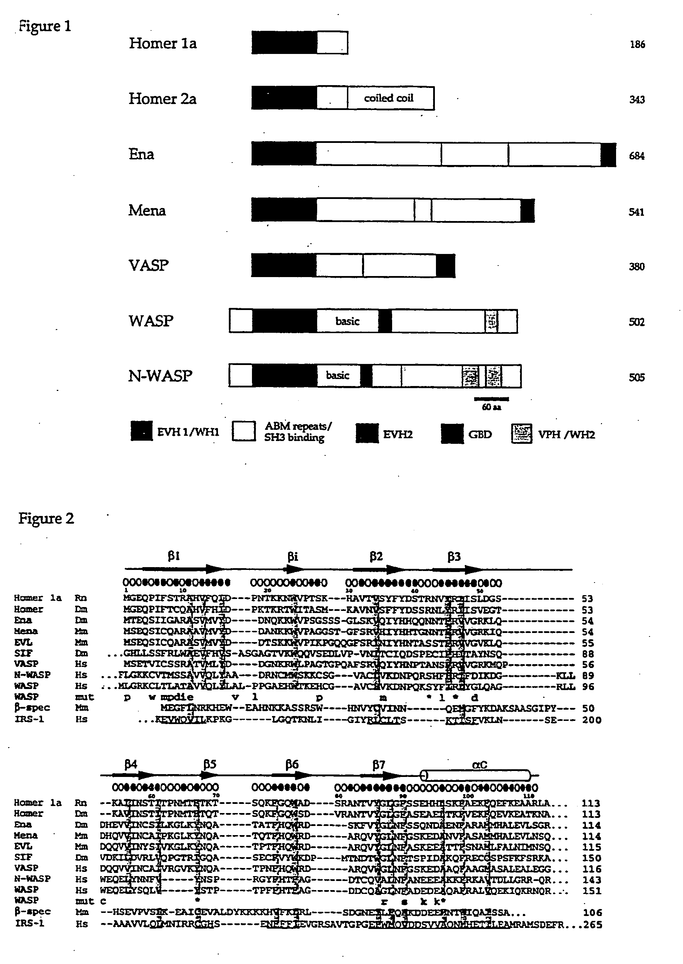

Figure 1. Schematic Representation of EVH1 Domain-containing Proteins. EVH1 domains are found at or near the N-termini of Homer, Ena, Mena, VASP, and WASP proteins. Homer 1b/2/3 encode a CC domain which mediates multimerization between various Homer proteins. In ENA, Mena, VASP, WASP, and N-WASP, the EVH1 domain is followed by a central proline rich region of variable length. The proteins are drawn to the scale shown, and the respective amino acid lengths are shown at the right.
Figure 2. Structure-Based Alignment of EVH1, PH, and PTB Domain Sequences. A structure-based sequence alignment between EVH1 domains and the β-spectrin PH domain and the IRS-1 PTB domain is shown. Species are indicated by Rn (rat), Hs (human), Mm (mouse), and Dm *(Drosophila).* Elements of the Homer EVH1 domain secondary structure are represented by arrows (β-strands), cylinders (α-helices), and lines (coils). Conserved residues (among EVH1 domains) are highlighted. The fractional solvent accessibility (FAS) of each residue in Homer 1a is indicated by ovals. Filled ovals = 0≤FAS≤0.1 (buried); shaded ovals = 0.1 <FAS≤0.4 (partially accessible); open ovals = FAS> 0.4. Mutations in the EVH1 domain of the WASP gene are indicated in lower case letters below the WASP amino acid sequence. Mutations that are associated with the severe WAS phenotype are show in bold letters (Zhu et al, 1997). Sites mutated to more than one residue are indicated by asterisks. Bold asterisks indicate residues that, when mutated, affect the interaction of WASP with WIP (Stewart et al., 1999). Residues of Homer, β-spectrin, and IRS-1 that align well following structural superposition and were used to calculate rms differences in Cα positions between these domains are underlined in the IRS-1 sequence. Gaps are indicated by dashes while continued sequences at amino- and carboxy-termini are indicated by periods. Residue numbering for Homer 1a is shown above its amino acid sequence. The number of the last included residue of each protein is shown at the end of each row.

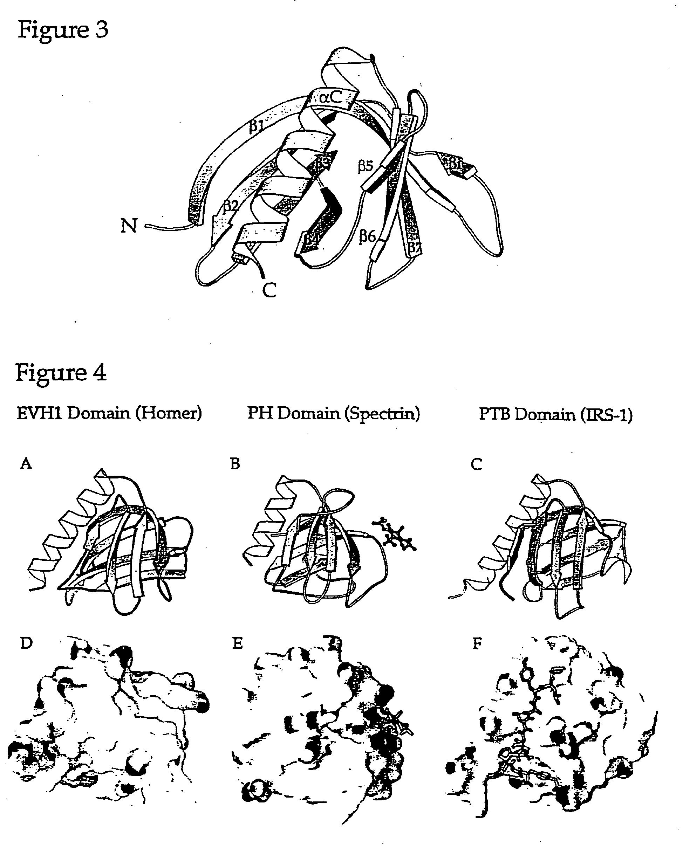

Figure 3. Ribbon Diagram of the Homer 1a EVH1 Domain. The amino and carboxy termini are indicated, and elements of secondary structure are labeled to correspond to homologous structures in PH and PTB domains. An additional short region of β-strand between β1 and β2 has been labeled βi.
Figure 4. Structural Comparison of EVH1, PH, and PTB Domains. Ribbon diagrams (A)-(C) and surface representations (D)-(F) of the Homer 1 EVH1, β-spectrin PH, and IRS-1 PTB domains, respectively, are shown. All molecules are shown in a similar orientation, which is rotated about 45° about the vertical axis from orientations shown in Figure 3. The β-spectrin PH domain is shown with bound inositol trisphosphate (Hyvonen et al, 1995). The IRS-1 domain is shown complexed to a phosphotyrosine-containing peptide derived from the insulin receptor (ECk et al., 1996).

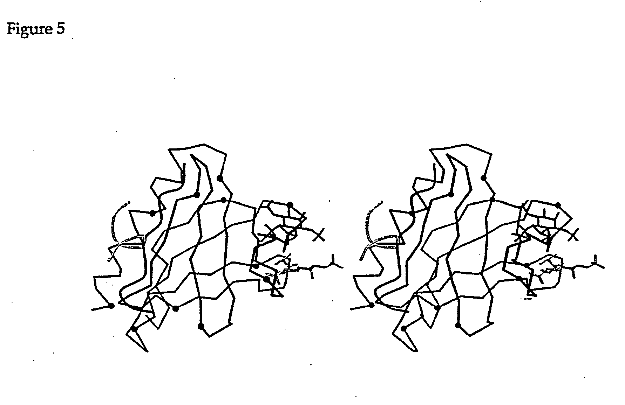

Figure 5. Versatile Ligand Recognition by PH-Like Domain. Sterodiagram of a backbone trace of Homer 1 EVH1 doamin showing the relative positions of IP3 as bound by the β-spectrin and PLC-δ PH domains, as well as the peptide ligands for the IRS-1 and Numb PTB domains is shown. The orientations of the EVH1 domain is similar to that in Figure 4. Ligand positions were determined by superimposing the backbone traces of the EVH1, PH and PTB domains in the program) (Jones et al., 1991).

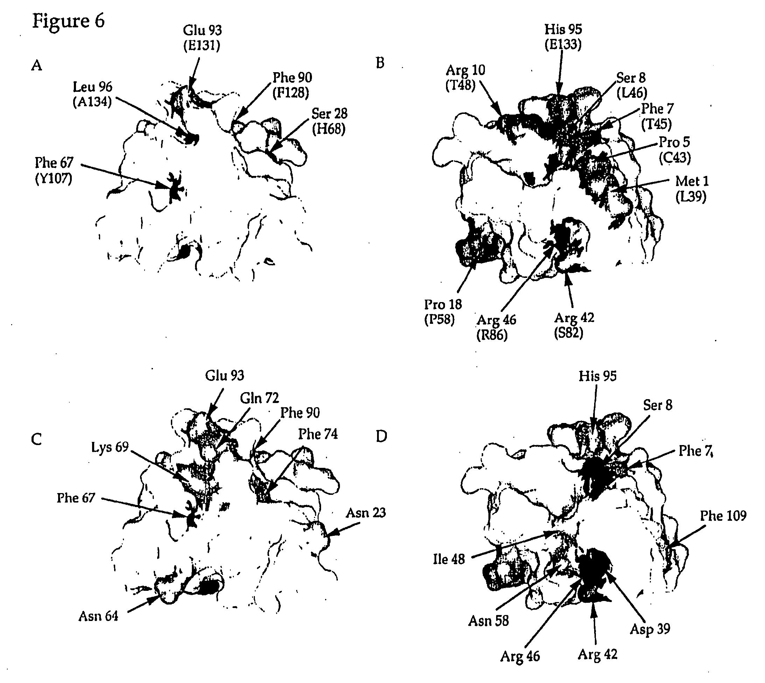

Figure 6. Mapping of WAS-Causing and Homer Binding Mutations on the EVH1 Surface. (A) and (B) Surface representations of the Homer1 EVH1 doamin with sites homologous to positions of WASP mutations (in parentheses) colored according to solvent accessibility. Solvent exposed residues are shown in magenta, and buried or partially buried residues are shown in blue. Residue assignments are based on the sequence shown in Figure 2. WASP EVH1 mutations are listed in Table 2. Surface representations of Homer 1 EVH1 domain showing the location of residues targeted by site-directed mutagenesis. Mutations that disrupt binding of Homer EVH1 to ligands in an in vitro binding assay are shown in red, while those that have no effect on binding are shown in light blue (see Table 3). The orientation of the EVH1 domain in panels A and C is identical to that in Figure 4A and D. IN panes1 B and D,. the moleucle is rotated about 180 degrees about the vertical axis.

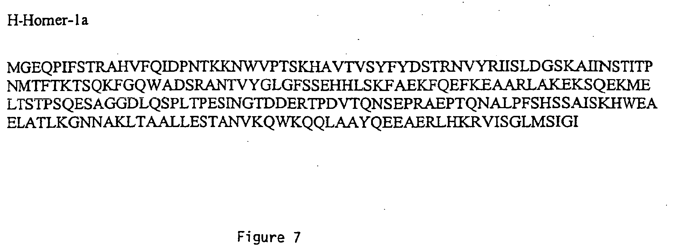

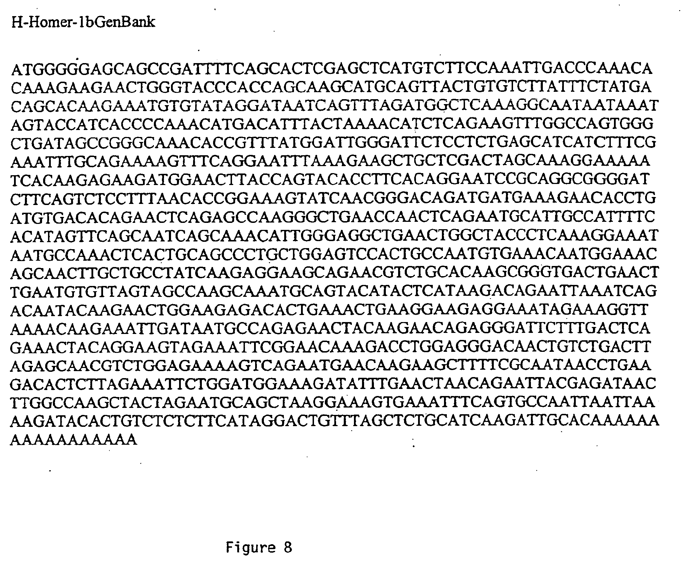

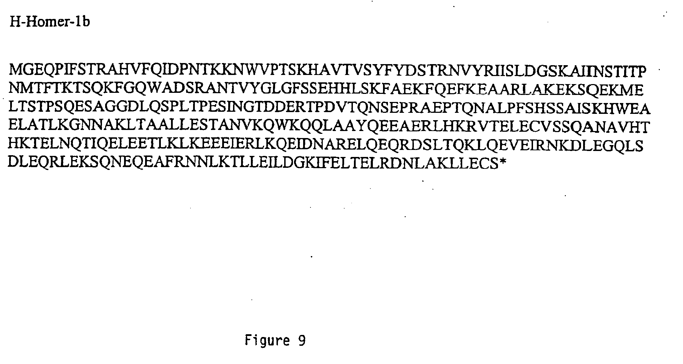

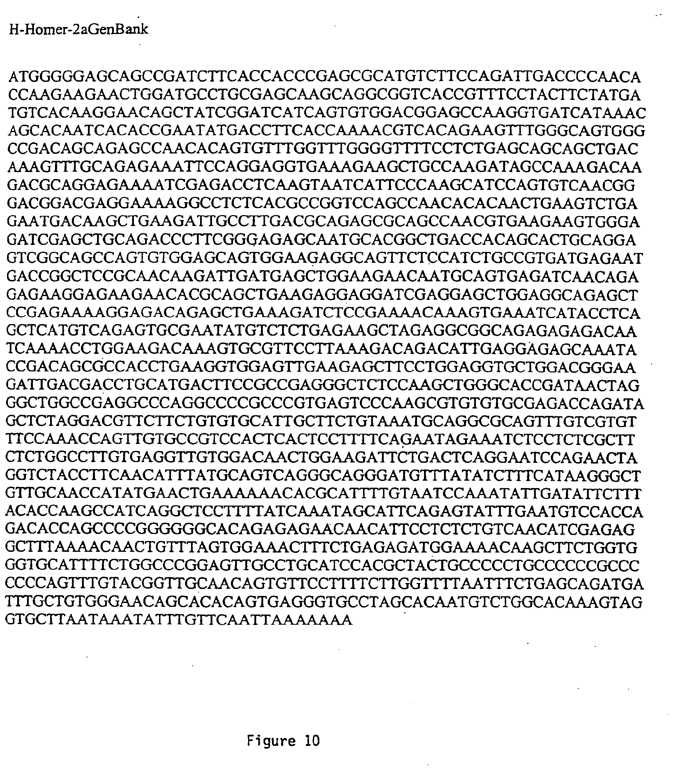

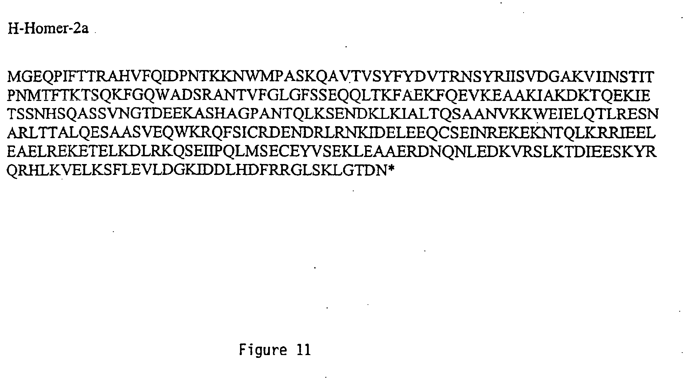

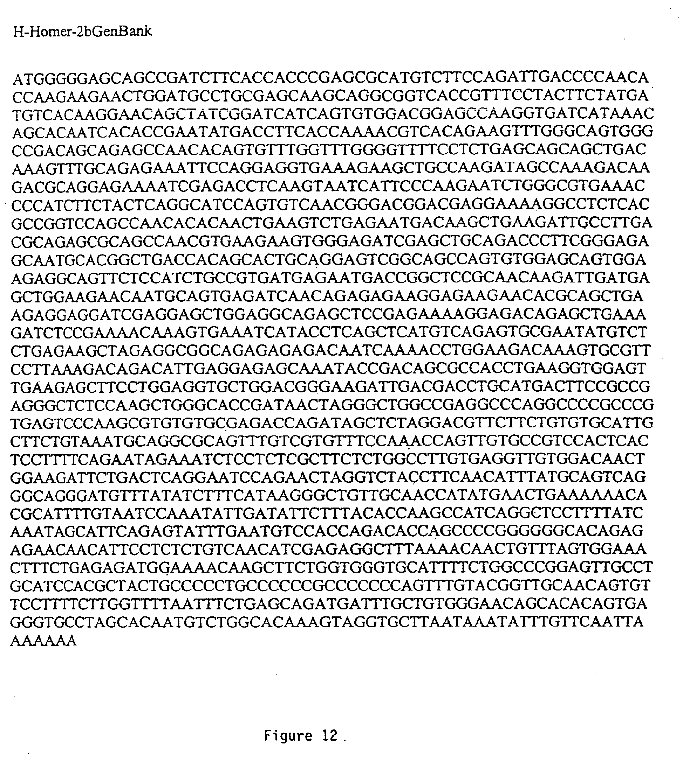

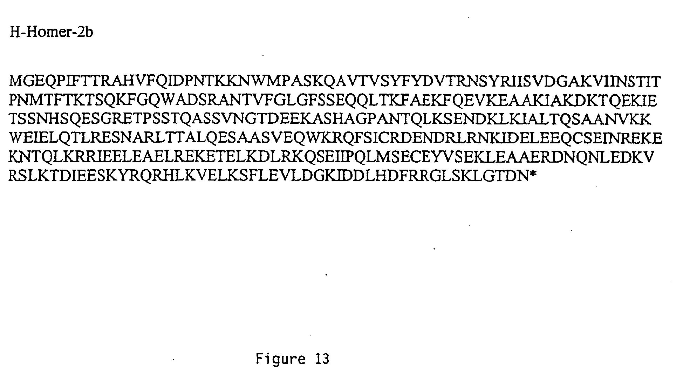

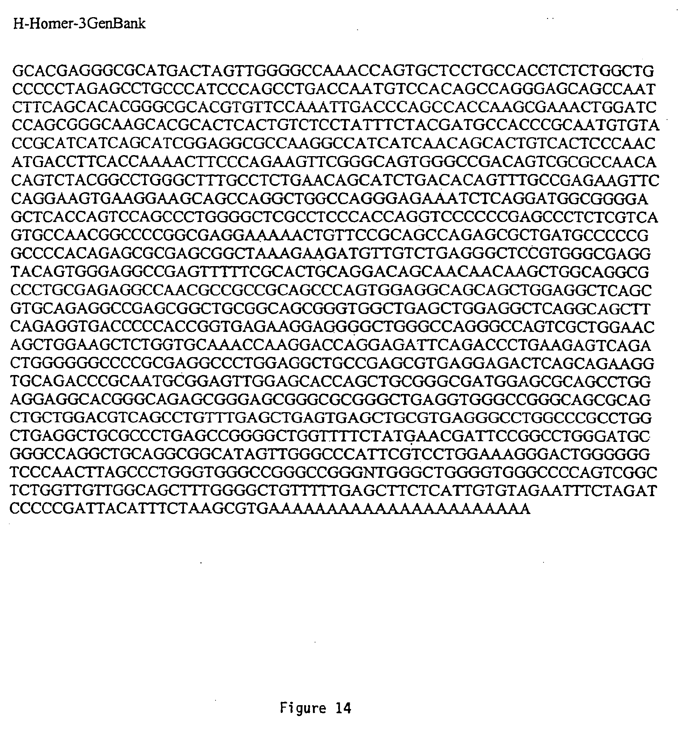

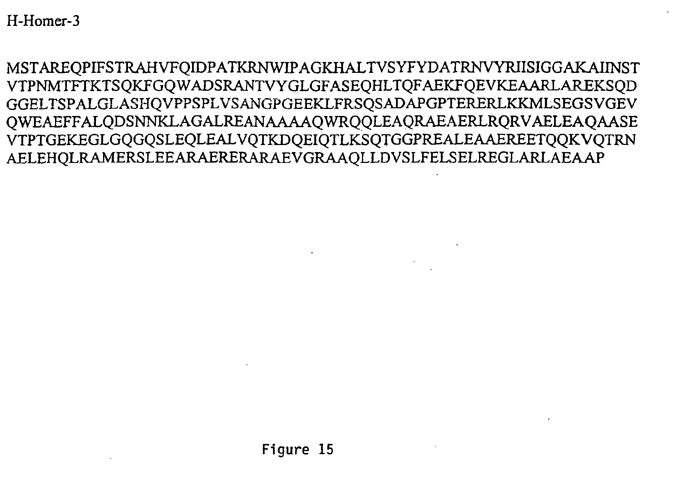

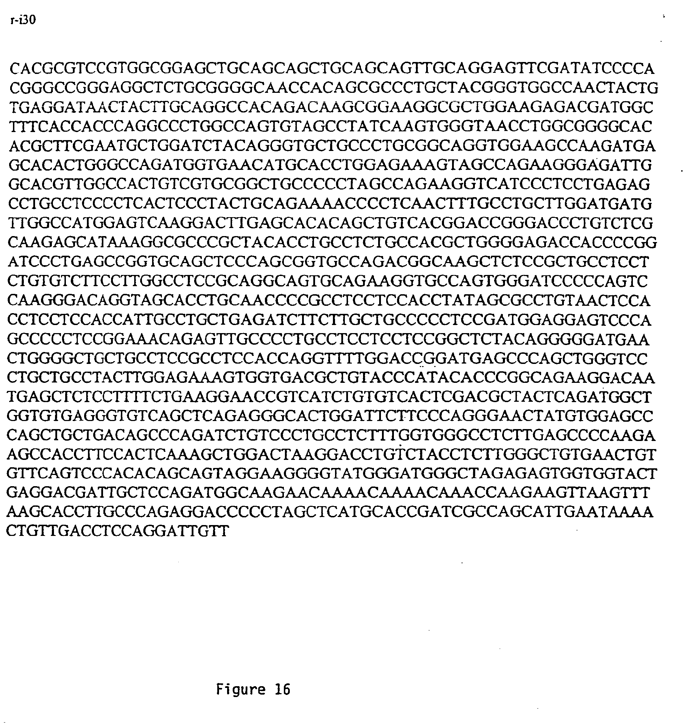

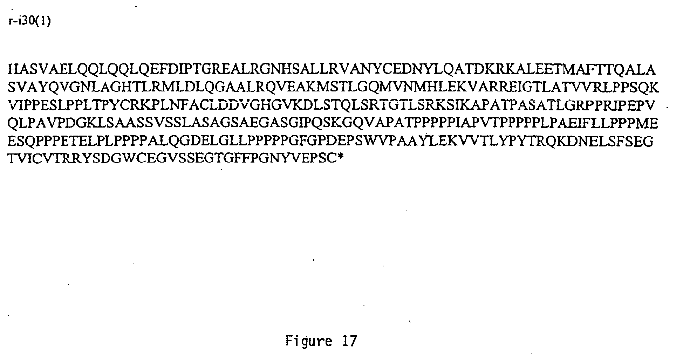

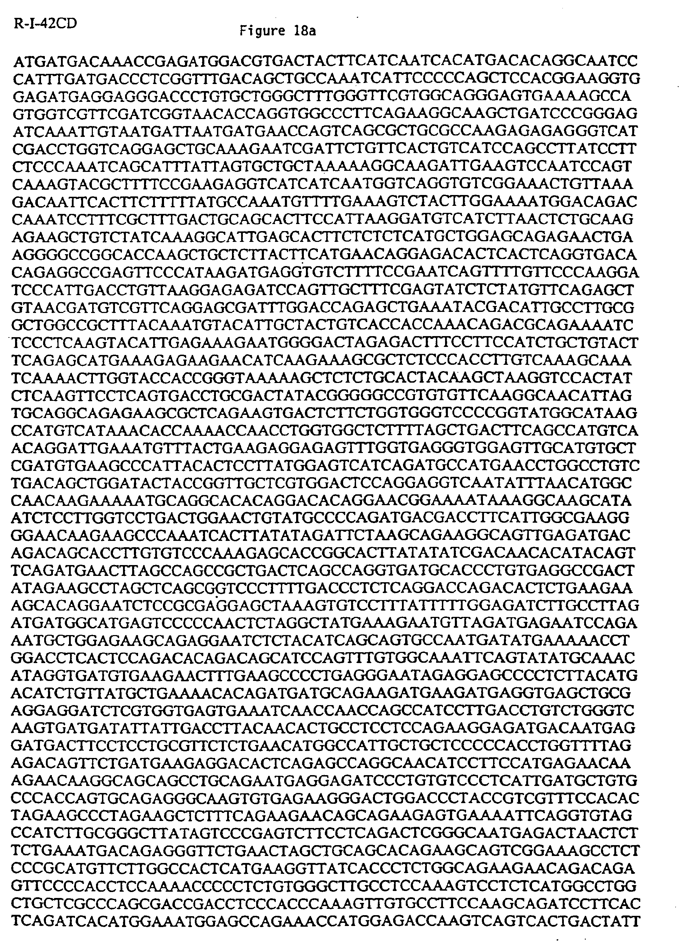

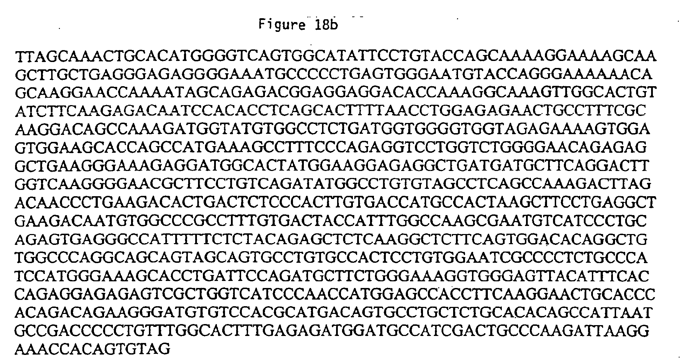

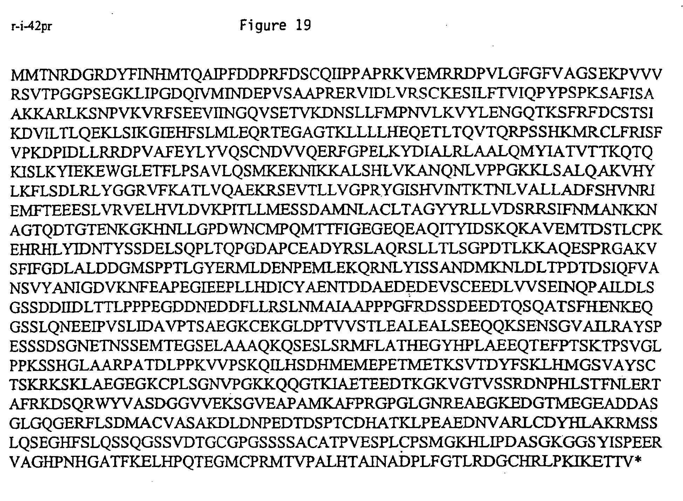

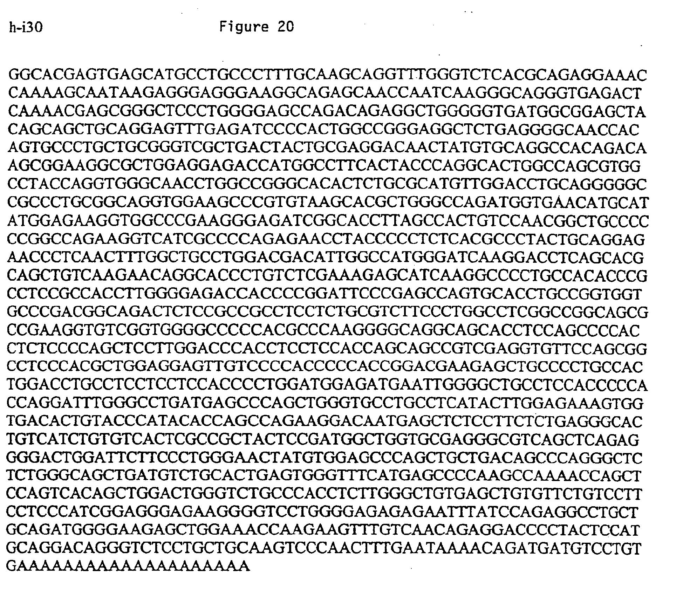

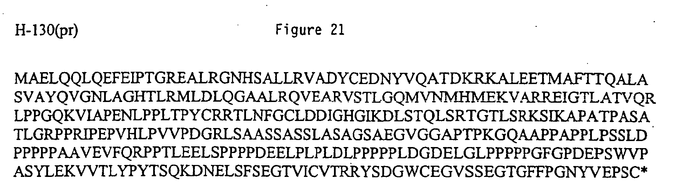

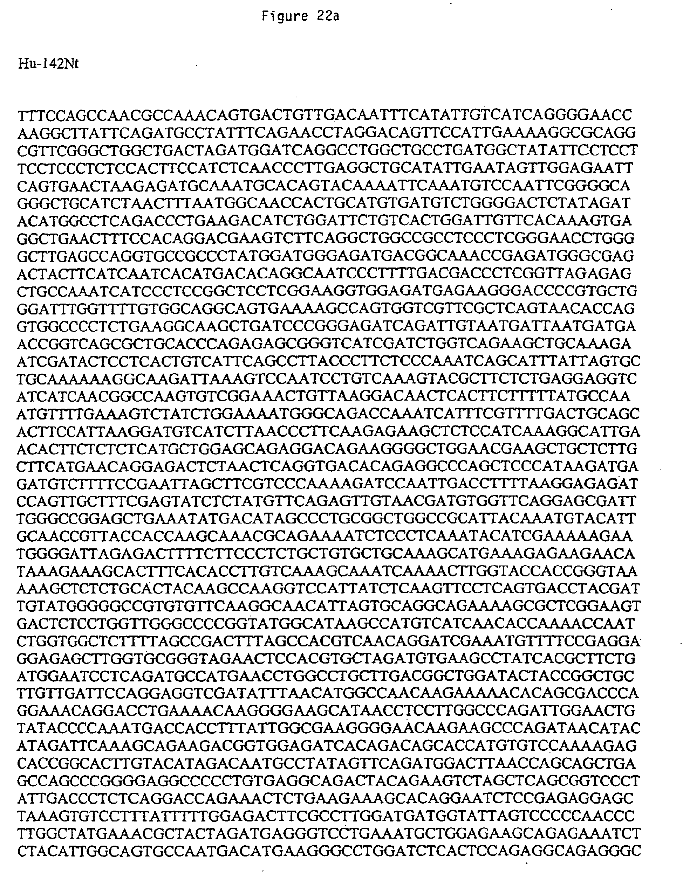

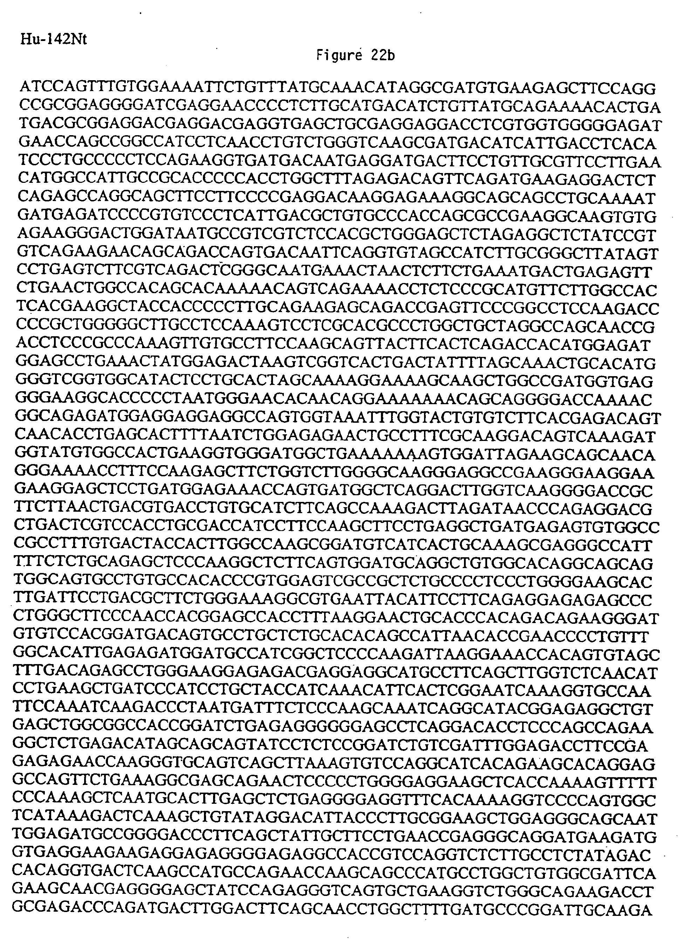

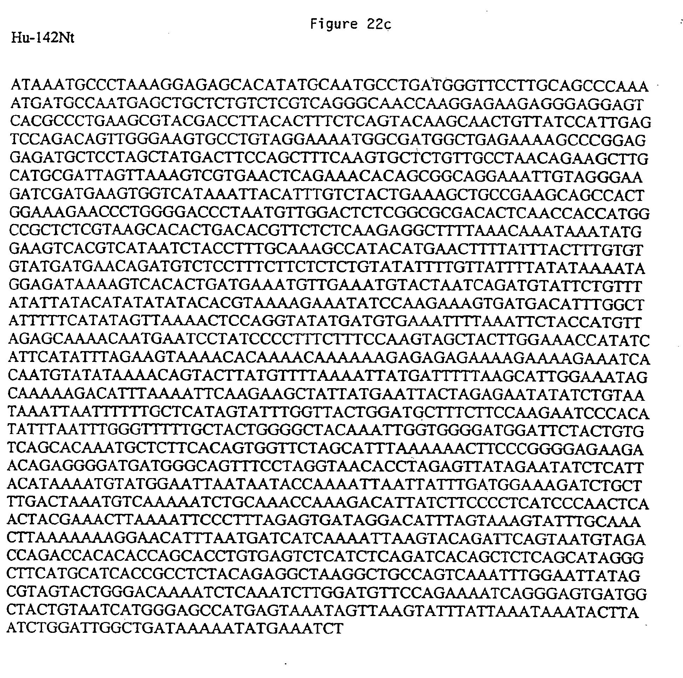

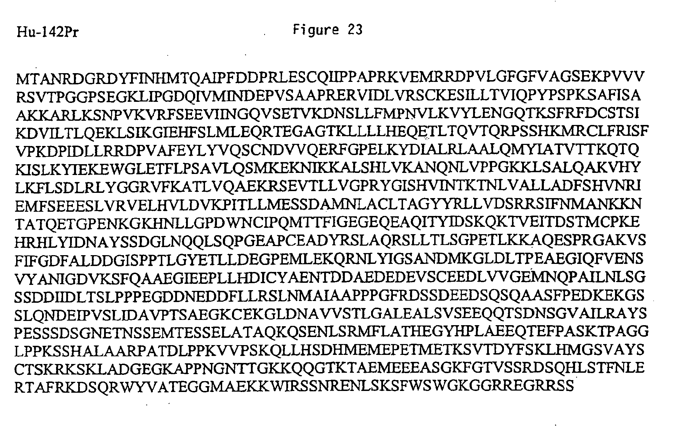

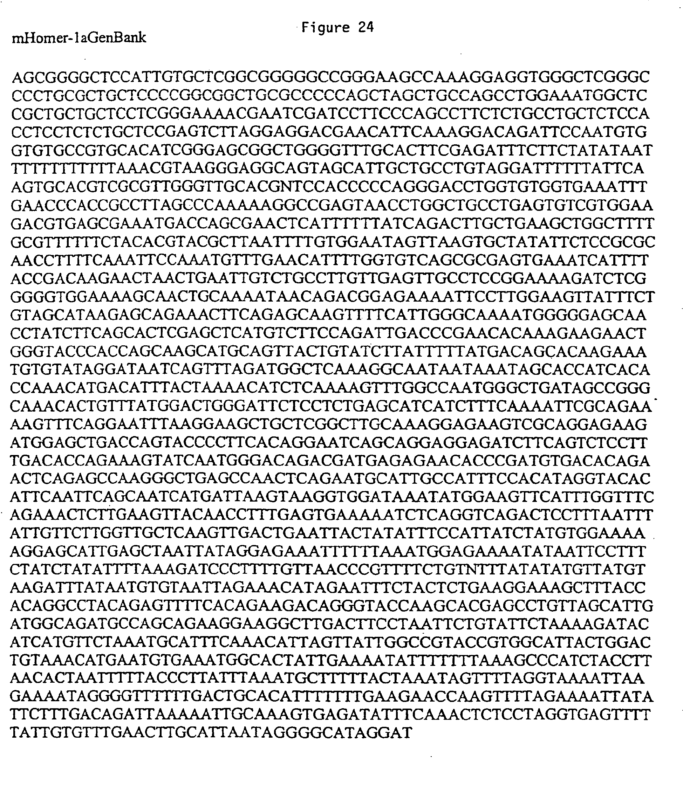

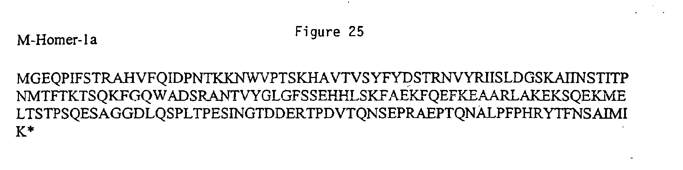

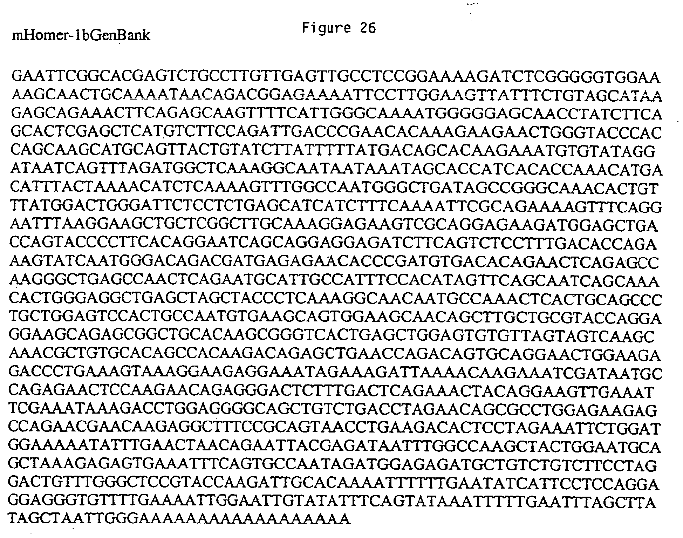

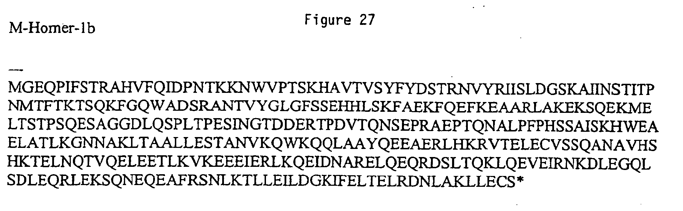

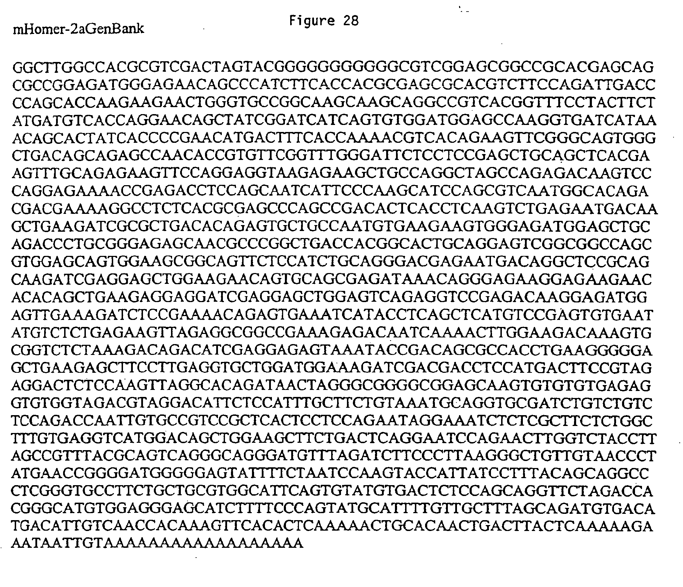

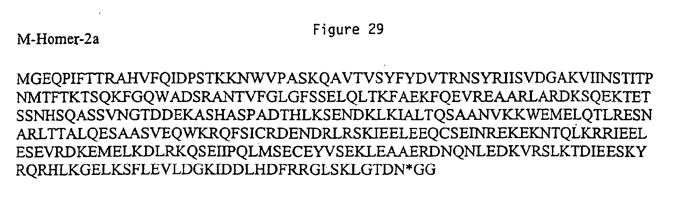

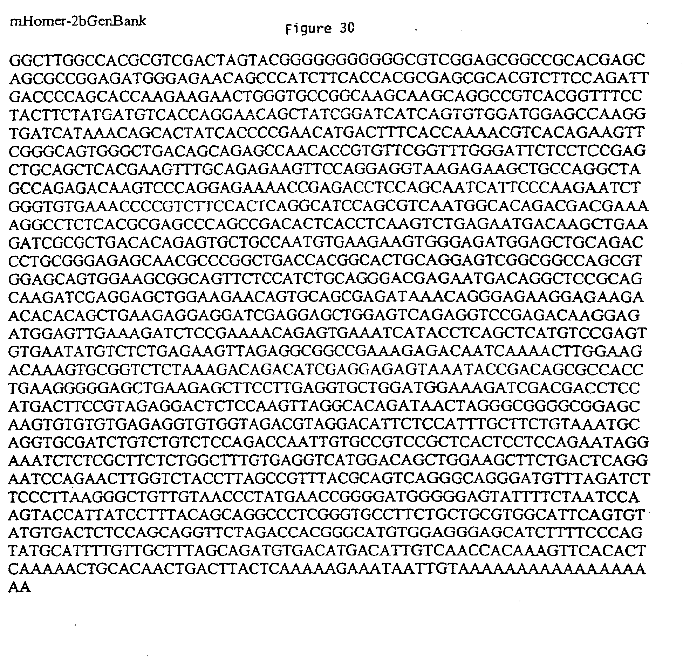

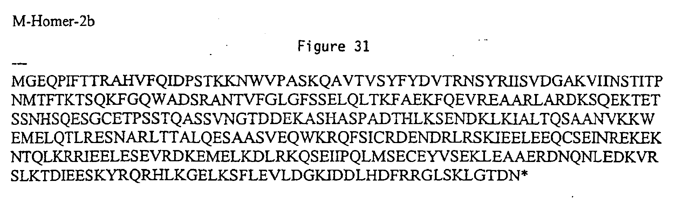

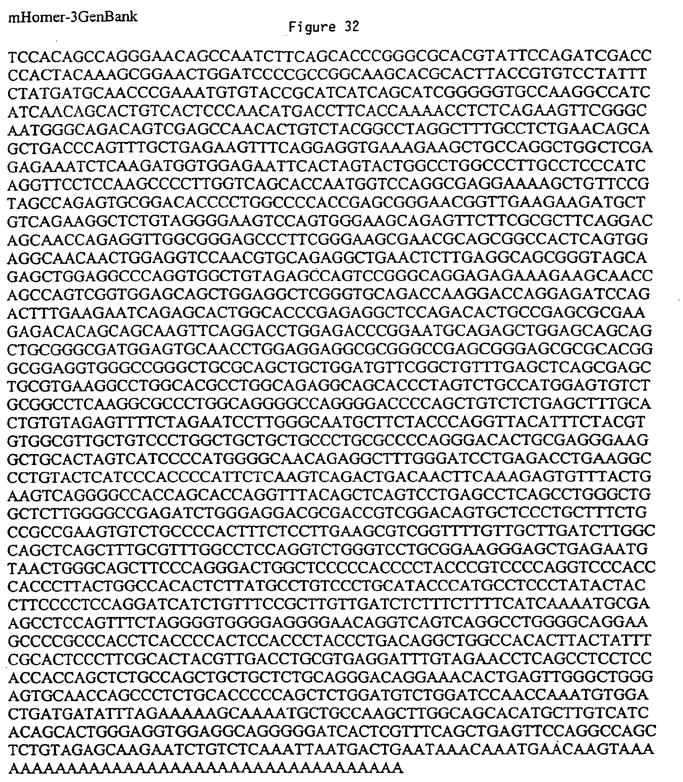

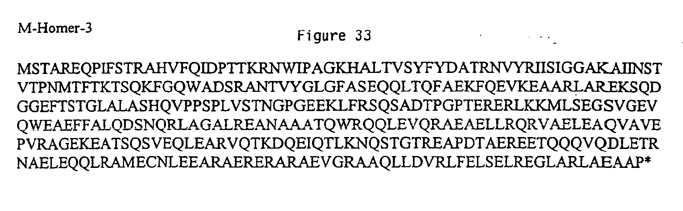

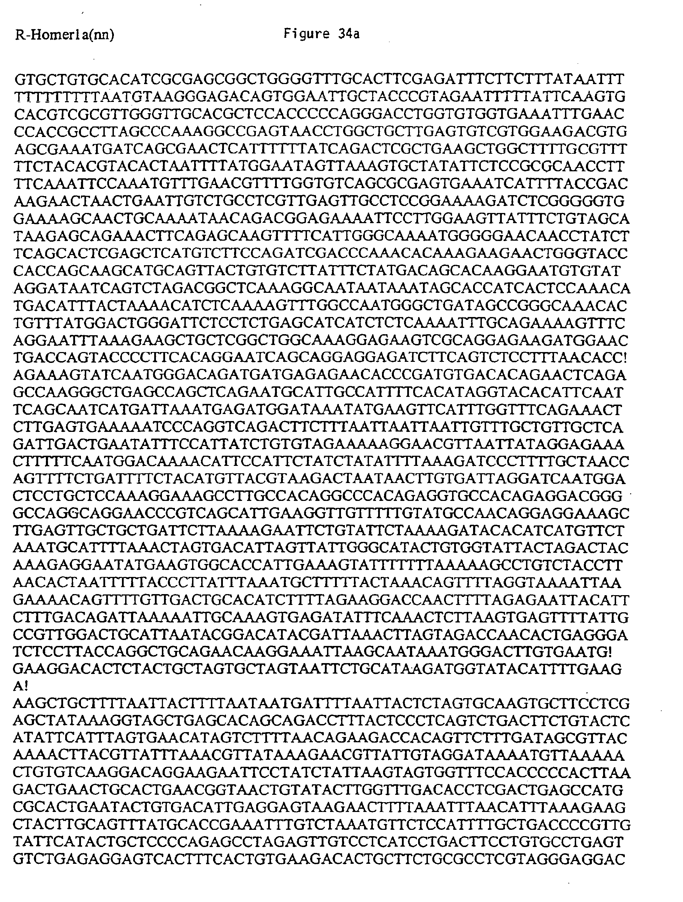

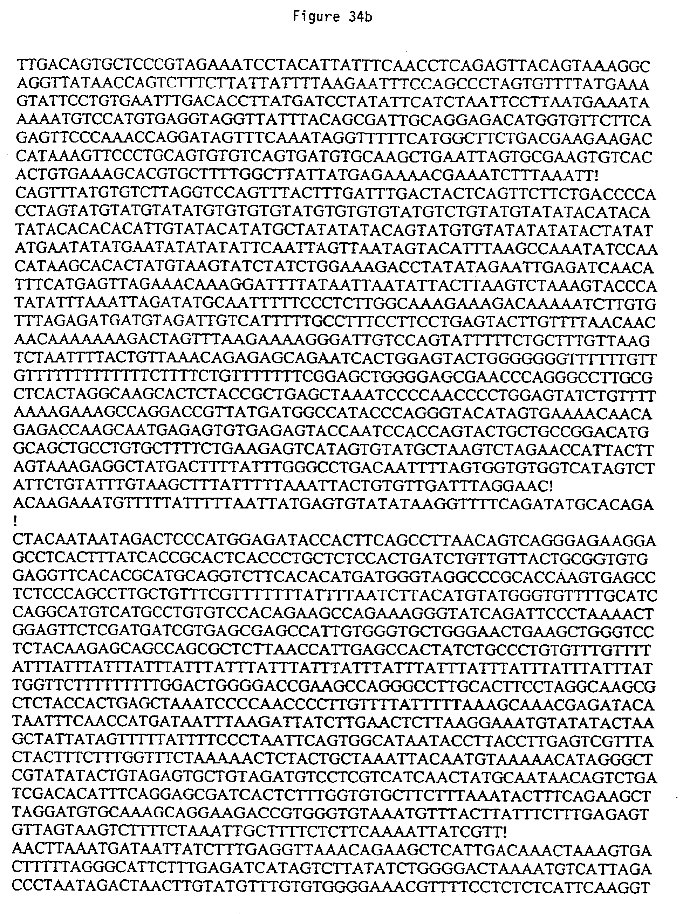

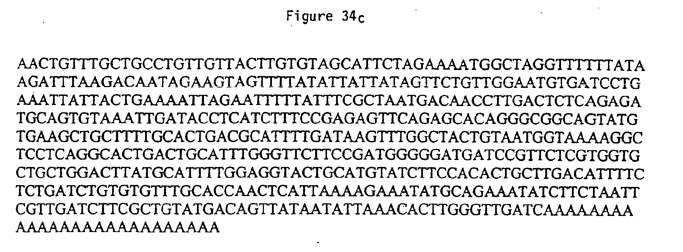

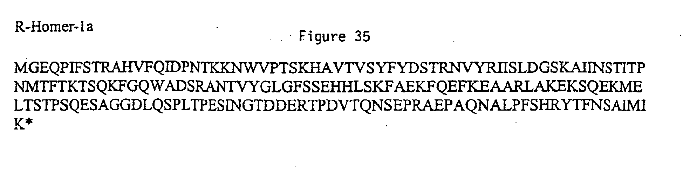

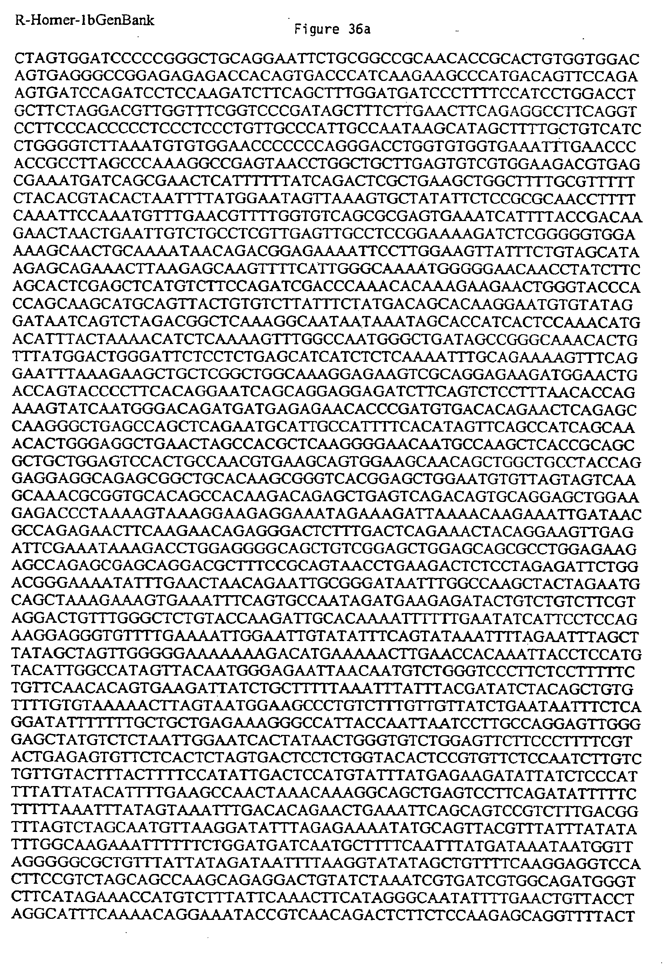

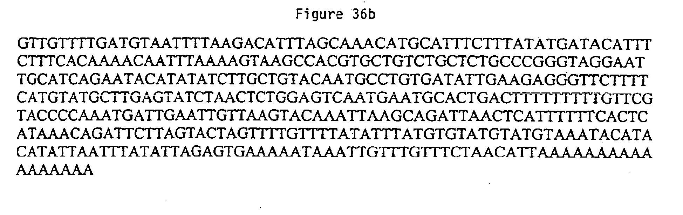

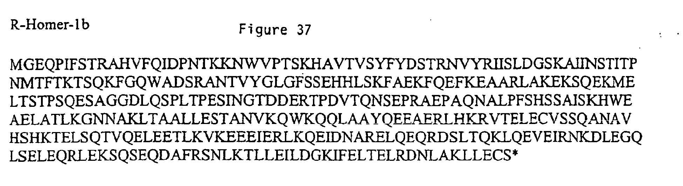

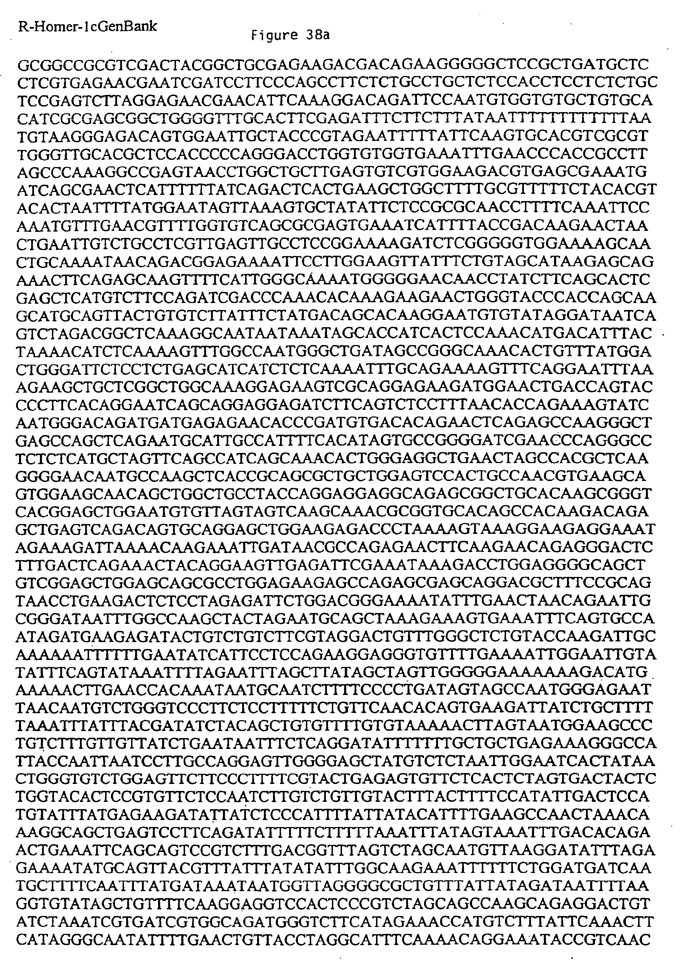

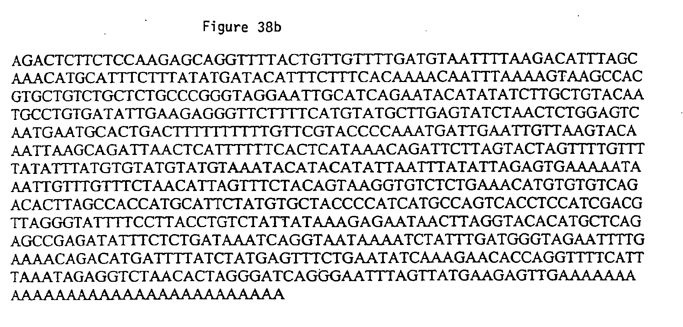

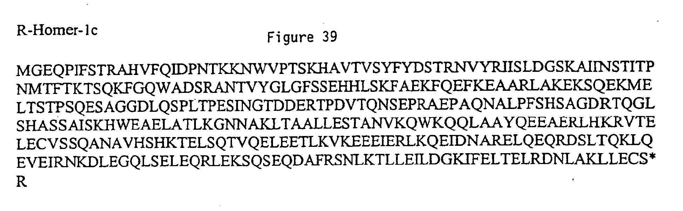

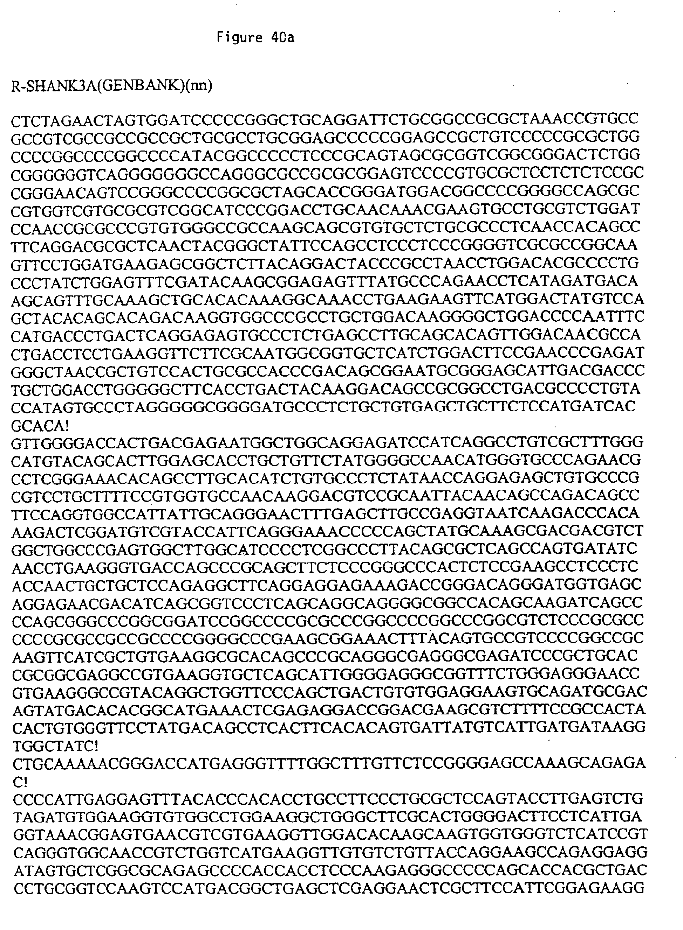

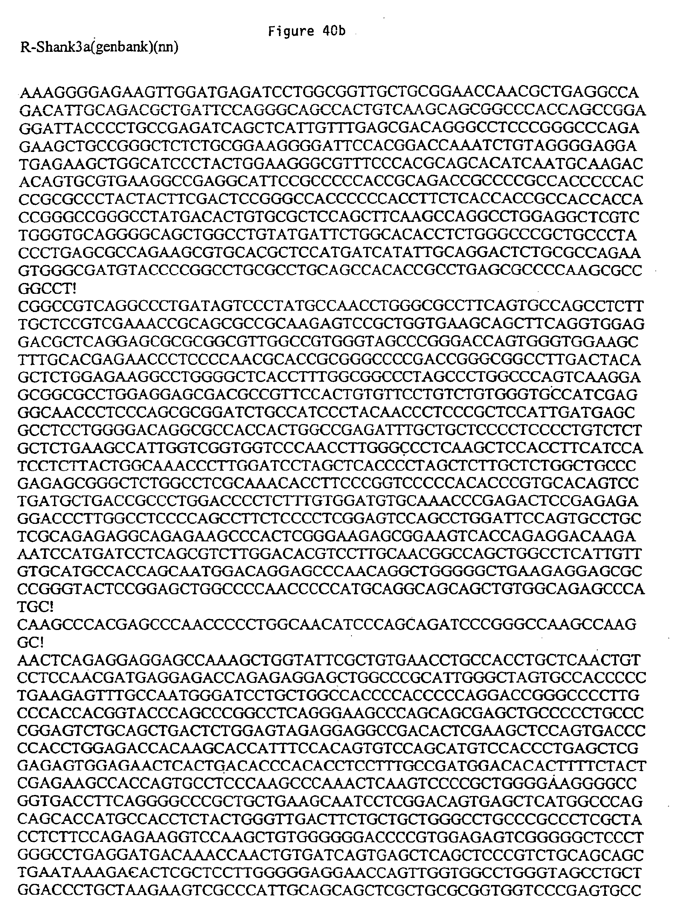

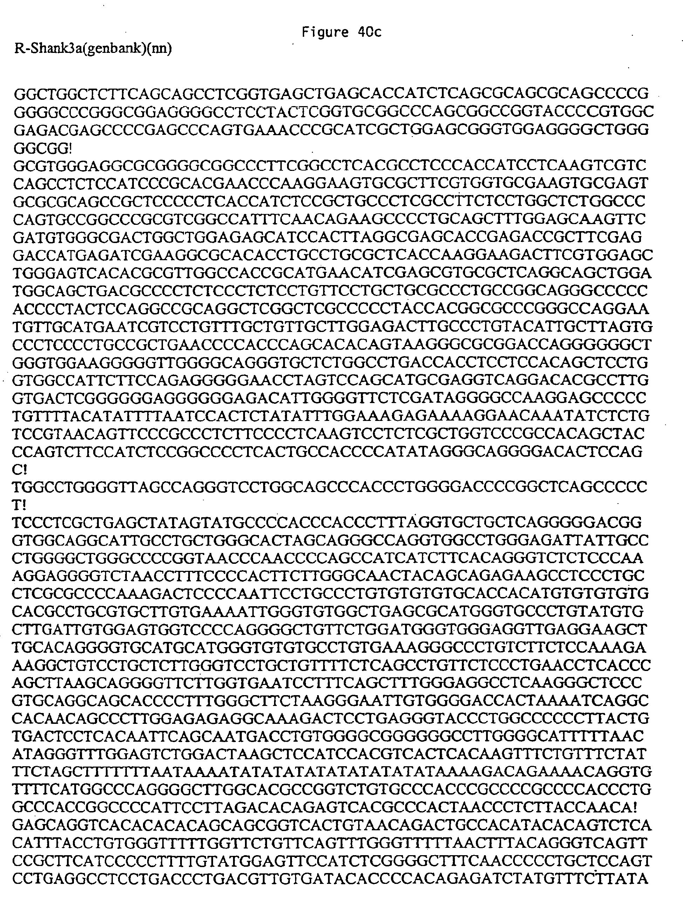

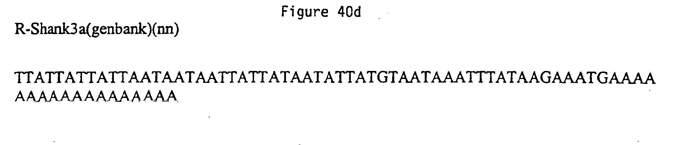

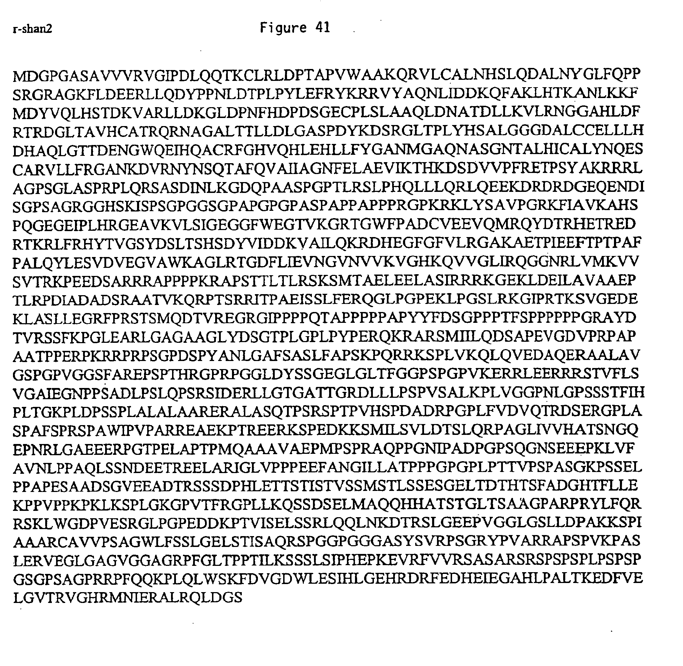

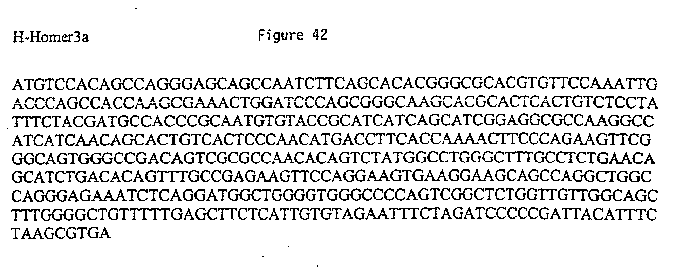

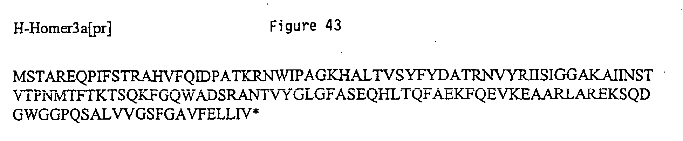

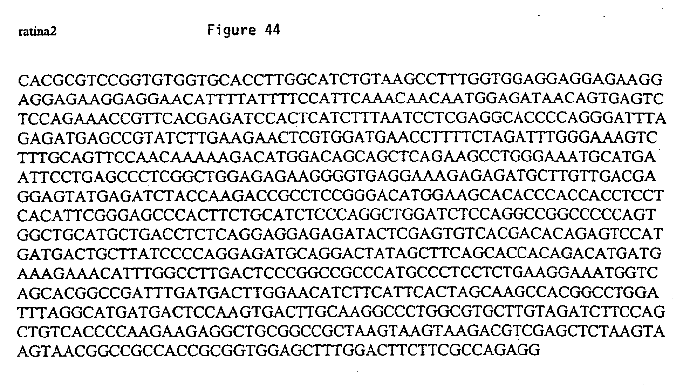

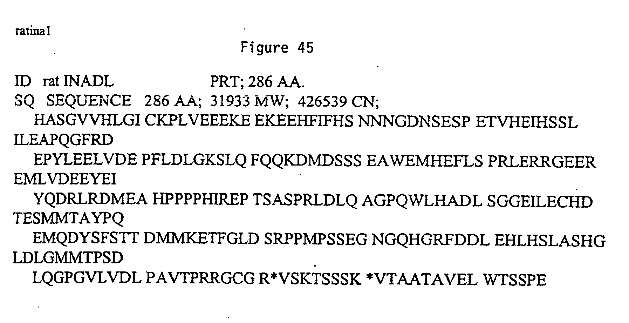

Figures 7 through 47 are described in the following table.

**Figure:s Homer Family Proteins and Homer Interacting Proteins**

| **FIGURE** | **SEQ ID No.** | **Sequence** |
|---|---|---|
| | 1 | Human Homer 1 a (nucleic acid) |
| **7** | 2 | Human Homer 1a (amino acid) |
| **8** | 3 | Human Homer 1b (nucleic acid) |
| **9** | 4 | Human Homer 1b (amino acid) |
| | 5 | Homer 1c (nucleic acid) |
| | 6 | Homer 1c (amino acid) |
| **10** | 7 | Human Homer 2a (nucleic acid) |
| **11** | 8 | Human Homer 2a (amino acid) |
| **12** | 9 | Human Homer 2b (nucleic acid) |
| **13** | 10 | Human Homer 2b (amino acid) |
| **14** | 11 | Human Homer 3 (nucleic acid) |
| **15** | 12 | Human Homer 3 (amino acid) |
| **16** | 13 | peptide binding―core region: PPXXFR |
| **17** | 14 | peptide binding―extended region: ALTPPSPFRD |
| **18** | 15 | Homer interacting protein: rat I30 (nucleic acid) |
| **19** | 16 | Homer interacting protein: rat I30 (amino acid) |
| **20** | 17 | Homer interacting protein: rat I42 (nucleic acid) |
| **21** | 18 | Homer interacting protein: rat I42 (amino acid) |
| **22** | 19 | Homer interacting protein: human I30 (nucleic acid) |
| **23** | 20 | Homer interacting protein: human I30 (amino acid) |
| **24** | 21 | Homer interacting protein: human I42 (nucleic acid) |
| **25** | 22 | Homer interactin protein: human I42 (amino acid) |
| **26** | 23 | Mouse Homer 1a (nucleic acid) |
| **27** | 24 | Mouse Homer 1a (amino acid) |
| **28** | 25 | Mouse Homer 1b (nucleic acid) |
| **29** | 26 | Mouse Homer 1b (amino acid) |
| **30** | 27 | Mouse Homer 2a (nucleic acid) |
| **31** | 28 | Mouse Homer 2a (amino acid) |
| **32** | 29 | Mouse Homer 2b (nucleic acid) |
| **33** | 30 | Mouse Homer 2b (amino acid) |
| **34** | 31 | Mouse Homer 3 (nucleic acid) |
| **35** | 32 | Mouse Homer 3 (amino acid) |
| **36** | 33 | Rat Homer 1a (nucleic acid) |
| **37** | 34 | Rat Homer 1a (amino acid) |
| **38** | 35 | Rat Homer 1b (nucleic acid) |
| **39** | 36 | Rat Homer 1b (amino acid) |
| **40** | 37 | Rat Homer 1c (nucleic acid) |
| **41** | 38 | Rat Homer 1c (amino acid) |
| **42** | 39 | Rat Shank 3a (nucleic acid) |
| **43** | 40 | Rat Shank 3a (amino acid) |
| **44** | 41 | Human Homer 3a (nucleic acid) |
| **45** | 42 | Human Homer 3a (amino acid) |
| **46** | 43 | Rat INADL partial nucleic acid sequence |
| **47** | 44 | Rat INADL partial amino acid sequence |

### DETAILED DESCRIPTION OF THE INVENTION

Homer represents a family of proteins that selectively binds the carboxy-terminus of group 1 metabotropic receptors and is enriched at excitatory synapses (Brakeman *et al*., 1977). In the adult brain, Homer is rapidly and transiently induced by physiological synaptic stimuli that evoke ion-term potentiation in the hippocampus (Brakeman *et al., 1997;* Kato *et al*., 1997), and is also induced in the striatum by dopaminetic drugs of addiction (Brakeman *et al*., 1997). The first Homer gene identified, now termed Homer 1a (Brakeman *et al., Nature* 386:2284-288 (1997); GenBank Accession No. U92079), is a member of a family of closely related Homer proteins that are constitutively expressed in brain (Kato *et al*., 1998; Sun *et al*., 1998; Xiao *et al*., 1998). There are now three mammalian genes identified and at least six distinct transcripts expressed in brain (Xiao *et al*., 1998). All Homer family members, including Homer 1a, contain an amino-terminal region of about 110 amino acids that binds metabotropic glutamate receptors 1a and 5 (mGluR1a and mGluR5) (Xiao *et al*., 1998). The region of Homer that interacts with mGluR1a or 5 is termed "EVH1 domain", based on homology to similar domains in a family of proteins that include *Drosophila* Enabled (Gertler *et al*., 1996), mammalian VASP (Haffner *et al*., 1995) and the Wescott-Aldrige protein (WASP) (Ponting and Phillips, 1997; Symons *et al*., 1996). The EVH1 domain of Homer is conserved at a level of about 80% between *Drosophila*, rodent and human (Xiao *et al*., 1998) The Homer family EVH1 domain also can bind to intracellular receptors such as the inositol trisphosphate receptor and dyamin III. Binding of Homer proteins in the EVH1 region is mediated by an amino acid sequence motif that is rich in proline residues.

To explore the proline-rich motif and its role in Homer interactions, a deletion mutation strategy was used. A 50-amino acid deletion at the carboxy-terminal end of mGluR5 destroyed binding to Homer. By contrast, a 41 amino acid deletion of mGluR5 retained full binding activity. The intervening sequence is proline rich and shares sequence similarity with the previously described SH3 ligand sequence (Yu, 1994). A series of point mutants based on the known structure-function relationship for SH3 ligands was prepared and binding assays confirmed general characteristics of SH3 ligand binding, but also demonstrated that that the Homer binding site is distinct in the positioning of critical amino acids (Tu *et al*., 1998). A consensus for binding was determined to be PPXXFR, consistent with the observation that mutation of either of the proline residues or the phenylalanine, or a change in their relative position, interrupted binding. The arginine in the last position was preferred over other tested amino acids, but is not essential. Mutations were identically effective in interrupting binding to each of the Homer family members including Homer 1a, 1b/c, 2a/b, 3 and an EVH1 fragment (110 amino acids) of Homer 1. Thus, it was concluded that the interaction with mGluR5 was mediated by the Homer EVH1 domain.

To further explore Homer binding, mutations of mGluR5 were tested using a 250 amino acid carboxy-terminal fragment of the receptor, which had an identical effect on binding when placed in the full length mGluR5 protein (Tu *et al*., 1998). This exquisite sensitivity of Homer binding to changes in single amino acids within the Homer-ligand site was confirmed in other Homer-interacting proteins including mGluR1a (Tu *et al*., 1998), Shank (Tu *et al*., 1999), and I42 (see below). To further confirm that the interaction was mediated by a direct interaction at the Homer-ligand site (as opposed to a secondary allosteric effect on a remote binding site), synthetic 10-mer peptides with either the wild type, or F-to-R mutation were prepared. The wild type peptide blocked binding of mGluR1a or mGluR5 to each of the Homer family members (Tu *et al*., 1998). Approximately half of the binding was blocked at a peptide concentration of 3.4 micromolar. By contrast, the F-to-R mutant peptide did not alter binding at concentrations as high as 340 micromolar.

Most forms of Homer protein encode a carboxy-terminal domain with a "coiled-coil" structure. This coiled-coil domain mediates homo- and heteromultermization between Homer proteins (Kato *et al*., 1998; Xiao *et al*., 1998) and such multimers can be identified in normal brain tissue (Xiao *et al*., 1998). Homer proteins are enriched in brain tissue fractions from postsynaptic densities and are localized at the ultrastructural level to postsynaptic densities. Homer 1a differs from the other members of the Homer family in that Homer 1a is not constitutively expressed and it does not contain a carboxy terminal coiled-coil domain. Experimental data showing that Homer proteins interact with cell-surface receptors and with intracellular receptors, and form multimeric complexes with other Homer proteins indicates an important role for Homer proteins in intracellular signaling.

An exemplary polynucleotide encoding a Homer protein is set forth as SEQ ID NO: 1. The term "polynucleotide", "nucleic acid", "nucleic acid sequence", or "nucleic acid molecule" refers to a polymeric form of nucleotides at least 10 bases in length. By "isolated polynucleotide" is meant a polynucleotide that is not immediately contiguous with both of the coding sequences with which it is immediately contiguous (one on the 5' end and one on the 3' end) in the naturally occurring genome of the organism from which it is derived. The term therefore includes, for example, a recombinant DNA which is incorporated into a vector; into an autonomously replicating plasmid or virus; or into the genomic DNA of a prokaryote or eukaryote, or which exists as a separate molecule (*e*.*g*., a cDNA) independent of other sequences. The nucleotides of the invention can be ribonucleotides, deoxyribonucleotides, or modified forms of either nucleotide. A polynucleotide encoding Homer includes "degenerate variants", sequences that are degenerate as a result of the genetic code. There are 20 natural amino acids, most of which are specified by more than one codon. Therefore, all degenerate nucleotide sequences are included in the invention as long as the amino acid sequence of a polypeptide encoded by the nucleotide sequence of SEQ ID NO: 1 is functionally unchanged.

A nucleic acid molecule encoding Homer includes sequences encoding functional Homer polypeptides as well as functional fragments thereof. As used herein, the term "functional polypeptide" refers to a polypeptide which possesses biological function or activity which is identified through a defined functional assay (e.g., EXAMPLE 3), and which is associated with a particular biologic, morphologic, or phenotypic alteration in the cell. The term "functional fragments of Homer polypeptide," refers to fragments of a Homer polypeptide that retain a Homer activity, e.g., the ability to interact with cell-surface or intracellular receptors or mediate intracellular calcium mobilization, and the like. Additionally, functional Homer fragments may act as competitive inhibitors of Homer binding, for example, biologically functional fragments, for example, can vary in size from a polypeptide fragment as small as an epitope capable of binding an antibody molecule to a large polypeptide capable of participating in the characteristic induction or programming of phenotypic changes within a cell.

A functional Homer polypeptide includes a polypeptide as set forth in SEQ ID NO:2 and conservative variations thereof. The terms "conservative variation" and "substantially similar" as used herein denotes the replacement of an amino acid residue by another, biologically similar residue. Examples of conservative variations include the substitution of one hydrophobic residue such as isoleucine, valine, leucine or methionine for another, or the substitution of one polar residue for another, such as the substitution of arginine for lysine, glutamic acid for aspartic acid, or glutamine for asparagine, and the like. The terms "conservative variation" and "substantially similar" also include the use of a substituted amino acid in place of an unsubstituted parent amino acid provided that antibodies raised to the substituted polypeptide also immunoreact with the unsubstituted polypeptide.

Also included are other Homer nucleic acid and amino acid sequences, including Homer 1b (SEQ ID NOS:3 and 4); Homer 1c (SEQ ID NOS:5 and 6); Homer 2a (SEQ ID NOS:7 and 8); Homer 2b (SEQ ID NOS:9 and 10); Homer 3 (SEQ ID NOS:11 and 12).

Cell-surface receptors are important intermediaries in intercellular signaling. A "cell-surface receptor" is a protein, usually having at least one binding domain on the outer surface of a cell where specific molecules may bind to, activate, or block the cell surface receptor. Cell surface receptors usually have at least one extracellular domain, a membrane spanning region ("transmembrane") and an intracellular domain. Activation of a cell-surface receptor can lead to changes in the levels of various molecules inside the cell. Several types of cell-surface receptors have been identified in a variety of cell types, including ligand-gated receptors, ligand-gated channels, voltage-activated receptors, voltage-activated channels, ion channels and the like.

One class of cell-surface receptor is excitatory amino acid receptors (EAA receptors) which are the major class of excitatory neurotransmitter receptors in the central nervous system. "EAA receptors" are membrane spanning proteins that mediate the stimulatory actions of glutamate and possibly other endogenous acidic amino acids. EAA are crucial for fast excitatory neurotransmission and they have been implicated in a variety of diseases including Alzheimer's disease, stroke schizophrenia, head trauma and epilepsy. EAA have also been implicated in the process of aging In addition, EAA are integral to the processes of long-term potentiation, one of the synaptic mechanisms underlying learning and memory. There are three main subtypes of EAA receptors: (1) the metabotropic or trans ACPD receptors; (2) the ionotropic NMDA receptors; and (3) the non-NMDA receptors, which include the AMPA receptors and kainate receptors.

Ionotropic glutamate receptors are generally divided into two classes: the NMDA and non-NMDA receptors. Both classes of receptors are linked to integral cation channels and share some amino acid sequence homology. GluR1-4 are termed AMPA (α-amino -3-hydroxy-5-methylisoxazole-4-propionic acid) receptors because AMPA preferentially activates receptors composed of these subunits, while GluR5-7 are termed kainate receptors as these are preferentially sensitive to kainic acid. Thus, an "AMPA receptor" is a non-NMDA receptor that can be activated by AMPA. AMPA receptors include the GluR1-4 family, which form homo-oligomeric and hetero-oligomeric complexes which display different current-voltage relations and Ca²⁺ permeability. Polypeptides encoded by GluR1-4 nucleic acid sequences can form functional ligand-gated ion channels. An AMPA receptor includes a receptor having a GluR1, GluR2, GluR3 or GluR4 subunit. NMDA receptor subtypes include class NR2B and NR2D, for example.

Metabotropic glutamate receptors are divided into three groups based on amino acid sequence homology, transduction mechanism and binding selectivity: Group I, Group II and Group III. Each Group of receptors contains one or more types of receptors. For example, Group I includes metabotropic glutamate receptors 1 and 5 (mGluRl and mGluR5), Group II includes metabotropic glutamate receptors 2 and 3 (mGluR2 and mGluR3) and Group III includes metabotropic glutamate receptors 4, 6, 7 and 8 (mGluR4, mGluR6, mGluR7 and mGluR8). Each mGluR type may be found in several subtypes. For example, subtypes of mGluR1 include mGluR1a, mGluRlb and mGluR1c.

Group I metabotropic glutamate receptors represent a family of seven membrane spanning proteins that couple to G-proteins and activate phospholipase C (Nakanishi, 1994). Members of the family include mGluR1 and mGluR5. Activation of these receptors results in the hydrolysis of memberane phosphatidylinositol bisphosphate to diacylglycerol, which activates protein kinase C. and inositol trisphosphate, which in turn activates the inositol trisphosphate receptor to release intracellular calcium. (Aramori and Nakanishi, 1992; Joly *et al*., 1995 Kawabata *et al*., 1998)

Activation of a glutamate receptor on the cell surface results in a cellular response. A "cellular response" is an event or sequence of events that singly or together are a direct or indirect response by a cell to activation of a cell surface receptor. A "cellular response" is also the blockade or activation of selective and non-selective cation channels and potentiation or inhibition of other cell-surface receptor responses. In addition, a "cellular response" may be the activation of an intracellular signaling pathway, including the activation of all steps or any one step in an intracellular signaling pathway.

An "intracellular signaling pathway" is a sequence of events that transduces information about an extracellular event into a signal to intracellular receptors or effector molecules such as enzymes. One type of intracellular signaling pathway is a second messenger signaling pathway. It may begin with the activation of receptors on the cell surface, which activation evokes changes in the level of specific, diffusible molecules inside the cell. The regulated production of these molecules serves to signal events to the intracellular receptors and is therefore termed a second messenger signaling pathway. Major second messenger pathways include the adenylate cyclase pathway, which regulates levels of cyclic AMP, the phosphoinositide pathway, which regulates intracellular calcium, guanylate cyclase, which regulates levels of cGMP, and the nitric oxide pathway, which regulates nitric oxide.

A cellular response mediated by cell surface receptors can also include calcium mobilization. A compound can modulate cellular responses mediated by cell surface receptors by inhibiting or potentiating the release of calcium from intracellular stores. A compound increases calcium mobilization by increasing the release of calcium from intracellular stores. A compound decreases calcium mobilization by inhibiting of the release of calcium from intracellular stores.

Cell-surface receptors are known to mediate cellular responses. Methods for demonstrating cellular responses are well known in the art (e.g. electrophysiological and biochemical methods). (See Examples section for additional methodology). A method is provided for identifying a compound that modulates a cellular response mediated by a cell-surface receptor. The method includes incubating the compound and a cell expressing a cell-surface receptor and a Homer protein under conditions sufficient to permit the compound to interact with the cell. The cell may be any cell of interest, including but not limited to neuronal cells, glial cells, cardiac cells, bronchial cells, uterine cells, testicular cells, liver cells, renal cells, intestinal cells, cells from the thymus and spleen, placental cells, endothelial cells, endocrine cells including thyroid, parathyroid, pituitary and the like, smooth muscle cells and skeletal muscle cells. The cell is exposed to a cell-surface receptor ligand. A "cell surface receptor ligand" is a compound that binds to the binding site of the cell-surface receptor thereby initiating a sequence of events that singly or together embrace a "cellular response". The effect of the compound on the cellular response is determined, either directly or indirectly, and a cellular response is then compared with a cellular response of a control cell. A suitable control includes, but is not limited to, a cellular response of a cell not contacted with the compound. The term "incubating" includes conditions which allow contact between the test compound and the cell of interest. "Contacting" may include in solution or in solid phase.

Compounds which modulate a cellular response can include peptides, peptidomimetics, polypeptides, pharmaceuticals, chemical compounds and biological agents, for example. Antibodies, neurotropic agents, anti-epileptic compounds and combinatorial compound libraries can also be tested using the method of the invention. One class of organic molecules, preferably small organic compounds having a molecular weight of more than 50 and less than about 2,500 Daltons. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more of the above functional groups.

The test agent may also be a combinatorial library for screening a plurality of compounds. Compounds such as peptides identified in the method of the invention can be further cloned, sequenced, and the like, either in solution of after binding to a solid support, by any method usually applied to the isolation of a specific DNA sequence Molecular techniques for DNA analysis (Landegren *et al., Science* 242:229-237, 1988) and cloning have been reviewed (Sambrook *et al*., Molecular Cloning: a Laboratory Manual, 2nd Ed.; Cold Spring Harbor Laboratory Press, Plainview, NY, 1998, herein incorporated by reference).

Candidate compounds are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc., to produce structural analogs. Candidate agents are also found among biomolecules including, but not limited to: peptides, saccharides, fatty acids, steroids, purines, pyrimidines, derivatives, structural analogs or combinations thereof.

A variety of other agents may be included in the screening assay. These include agents like salts, neutral proteins, *e*.*g*., albumin, detergents, etc. that are used to facilitate optimal protein-protein binding and/or reduce nonspecific or background interactions. Reagents that improve the efficiency of the assay, such as protease inhibitors, nuclease inhibitors, antimicrobial agents and the like may be used. The mixture of components are added in any order that provides for the requisite binding. Incubations are performed at any suitable temperature, typically between 4 and 40°C. Incubation periods are selected for optimum activity, but may also be optimized to facilitate rapid high-throughput screening. Typically between 0.1 and 10 h will be sufficient.

In another embodiment, a method is provided for identifying a compound that modulates a cellular response mediated by an intracellular receptor. An "intracellular receptor" is a protein that binds particular intracellular molecules. Intracelluar receptors include ryanodine receptors and inositol trisphosphate receptors, for example, an "inositol trisphosphate receptor" is a receptor that binds the compound inositol 1,4,5 trisphosphate, which is an important intracellular second messenger. Inositol 1,4,5 trisphosphate is released from phosphatidyl inositol bisphosphate by the action of a specific phospholipase C enzyme (PLC) and binds to and activates a calcium channel in the endoplasmic reticulum (ER).

A compound can modulate a cellular response mediated by an intracellular receptor by inhibiting or potentiating the release of calcium from intracellular stores, for example, a compound increases calcium mobilization by increasing the release of calcium from intracellular stores. A compound decreases calcium mobilization by inhibiting of the release of calcium from intracellular stores.

The method of the invention includes incubating the compound and a cell expressing an intracellular receptor and a Homer protein under conditions sufficient to permit the compound to interact with the cell, exposing the cell to conditions that activate said intracellular receptor, and comparing a cellular response in a cell incubated with said compound with the response of a cell not incubated with said compound. Methods for determining cellular responses mediated by intracellular signals are well known to one of skill in the art (*e.g.,* biochemical assays) and provided in the Examples as well.

A method is also provided for identifying a compound that modulates receptor-activated calcium mobilization. The term "calcium mobilization" means a change in the amount or concentration of free calcium (Ca⁺²) sequestered in the endoplasmic reticulum, sarcoplasmic reticulum or mitochondria of a cell. The method includes incubating the compound and a cell expressing a Homer protein under conditions sufficient to permit the compound to interact with the cell and exposing the cell to conditions sufficient to activate calcium mobilization. Then, the cellular response of the cell exposed to the compound is compared to the cellular response of a cell not exposed to the compound. A difference in a cellular response is indicative of a compound that modulates receptor-activated calcium mobilization in a cell.

In another embodiment of the invention, a method is provided for modulating receptor-mediated calcium mobilization in a cell including exposing a cell to a compound in a sufficient amount to modulate the calcium mobilization that normally occurs when a cell is exposed to an amount of ligand sufficient to activate an intracellular signaling pathway. Those of skill in the art will understand that "the calcium mobilization that normally occurs" depends on the cell type and on the ligand activating the intracellular pathway (Berridge, 1997 *supra*; Berridge, 1998 *supra;* Bootman, 1997 *supra*). Methods of measuring free calcium flux are well known in the art (e.g., imaging methodology using calcium-sensitive dyes such as fura-2 and the like).

A ligand which activates the intracellular signaling pathway may be an agonist or antagonist of metabotropic glutamate receptors. The terms "agonist" and "antagonist" are meant to include compounds that bind to the receptor and, respectively, activate or block activation of the receptor. Known agonists of metabotropic glutamate receptors include glutamate, quisqualate, Ibotenate, homocysteine sulfinate and the neurotoxin β-N-methylamino-L-alanine. Antagonists of metabotropic glutamate receptors include MCPG. Known agonists of the NMDA type glutamate receptor include glutamate and NMDA and known antagonists include MK-801 and APV.

Another embodiment of the invention includes a method of identifying a compound that inhibits Homer protein activity. The method relies on functional properties of the Homer EVH1 and coiled-coil binding domains that can be used to establish high-throughput screens for molecules that influence these and other functional properties of Homer family members. Homer protein activity may be blocked, partially or completely, by interfering with a protein or other molecule in the intracellular signaling pathway though which Homer proteins act. For example, Homer activity can be modulated, for example, by modulating Homer protein expression, by modifying the activity of the Homer EVH1 domain, by modification of the activity of the Homer CC domain, by modification of Homer crosslinking activity, and the like. Homer activity can also be modulated with by interfering with the expression or activity of Homer Interacting Protein 142, Homer Interacting Protein 130, NR2D, ACK-2, Shank proteins, ryanodine, inositol trisphosphate, and hInaD, and the like

Homer proteins function as a regulated adapter network that cross-links interacting proteins. Cross-linking is determined by the binding properties of the Homer EVH1 domain, which recognize a unique proline-rich ligand with a core sequence consensus of PXNF. This Homer ligand is present in all identified proteins that naturally associate with Homer, and the ability of Homer proteins to bind can be disrupted by single amino acid changes in this motif. Cross-linking activity of Homer proteins has demonstrated effects on glutamate receptor signaling and this action is due to the formation of signaling complexes that link cell-surface receptors with intracellular receptors. Cross-linking by Homer proteins may also have consequences on receptor trafficking or other cellular functions of the interacting proteins.

Development of agents that modulate activity of the Homer EVH1 domain is furthered by knowledge of the crystal structure of Homer protein. The method includes designing inhibitors of Homer protein that form non-covalent bonds with amino acids in the Homer binding sites based upon the crystal structure co-ordinates of Homer protein binding domain; synthesizing the inhibitor; and determining whether the inhibitor inhibits the activity of Homer protein.

The "Homer protein binding domain" is a conserved sequence of amino acids in the amino-terminal region of the that interacts with other proteins. All Homer proteins possess a conserved region of about 175 amino acids at their amino-termini. The 110 terminal amino acids in this region interact with the carboxy-termini of other proteins, for example metabotropic glutamate receptors, inositol trisphosphate receptors, Shank, and the like. The carboxy-termini region of the proteins to which the Homer protein binding domain may bind usually contains an amino acid sequence that contains a high number of proline residues.

One aspect of the invention resides in the obtaining of crystals of Homer protein of sufficient quality to determine the three dimensional (tertiary) structure of the protein by X-ray diffraction methods. The knowledge obtained concerning Homer proteins may be used in the determination of the three dimensional structure of the binding domain of Homer proteins. The binding domain can also be predicted by various computer models. Upon discovering the three-dimensional protein structure of the binding domain, small molecules which mimic the functional binding of Homer protein to its ligands can be designed and synthesized This is the method of "rational" drug design. Another approach to "rational" drug design is based on a lead compound that is discovered using high thoughput screens; the lead compound is further modified based on a crystal stucture of the binding regions of the molecule in question. Accordingly, another aspect of the invention is to provide material which is a starting material in the rational design of drugs which mimic or prevents the action of Homer proteins.

The term "crystal structure coordinates" refers to mathematical coordinates derived from mathematical equations related to the patterns obtained on diffraction of a monochromatic beam of X-rays by the atoms (scattering centers) of a Homer protein molecule in crystal form. The diffraction data are used to calculate an electron density map of the repeating unit of the crystal. The electron density maps are used to establish the positions of the individual atoms within the unit cell of the crystal. The crystal structure coordinates of the Homer protein binding domain are obtained from a Homer protein crystal having orthorhombic space group symmetry P2₁2₁2₁ with a = 33.79, b = 51.40, and c = 66.30 Angstroms. The coordinates of the Homer protein binding domain can also be obtained by means of computational analysis.

The term "selenomethione substitution refers to the method of producing a chemically modified form of the crystal of Homer. The Homer protein is expressed by bacterial in meida that is depleted in methionine and supplement in selenomethionine. Selenium is thereby incorporated into the crystal in place of methionine sulfurs.. The location(s) of selenium are determined by X-ray diffraction analysis of the crystal. This information is used to generate the phase information used to construct three-dimensional structure of the protein.

The term "heavy atom derivatization" refers to the method of producing a chemically modified form of the crystal of Homer. A crystal is soaked in a solution containing heavy metal atom salts or organometallic compounds, which can diffuse through the crystal and bind to the surface of the protein. The location(s) of the bound heavy metal atom(s) are determined by X-ray diffraction analysis of the soaked crystal. This information is used to generate the phase information used to construct three-dimensional structure of the protein.

Those of skill in the art understand that a set of structure coordinates determined by X-ray crystallography is not without standard error.

The term "unit cell" refers to the basic parallelipiped shaped block. The entire volume of a crystal may be constructed by regular assembly of such blocks.

The term "space group" refers to the arrangement of symmetry elements of a crystal.

The term "molecular replacement" refers to a method that involves generating a preliminary model of an Homer crystal whose structure coordinates are not known, by orienting and positioning a molecule whose structure coordinates are known. Phases are then calculated from this model and combined with observed amplitudes to give an approximate Fourier synthesis of the structure whose coordinates are known.

The crystal structure coordinates of Homer protein may be used to design compounds that bind to the protein and alter its physical or physiological properties in a variety of ways. The structure coordinates of the protein may also be used to computationally screen small molecule data bases for compounds that bind to the protein. The structure coordinates of Homer mutants (*e.g*., missense mutations, deletion mutations, and the like, obtained by site-directed mutagenesis, by exposure to mutagenic agents, through selection of naturally occurring mutants, *etc*.) may also facilitate the identification of related proteins, thereby further leading to novel therapeutic modes for treating or preventing Homer-mediated conditions. A potential inhibitor is designed to form hydrogen bonds with tryptophan²⁴, phenylalanine⁷⁴, threonine⁶⁶, threonine⁶⁸, glutamine⁷⁶, alanine⁷⁸, threonine⁷⁰, and valine⁸⁵ of the Homer binding domain.

A method is also provided for identifying a compound that affects the formation of cell surface receptors into clusters. The method includes incubating the compound and a cell expressing a Homer protein and a Homer Interacting protein, such as a Shank protein, a Homer Interacting Protein, and the like, under conditions sufficient to allow the compound to interact with the cell, determining the effect of the compound on the formation of cell-surface receptors into clusters, and comparing the formation of cell-surface receptors into clusters in cells contacted with the compound with the formation of cell surface receptors into clusters in cells not contacted with the compound.

Shank proteins are a novel family of proteins found at the postsynaptic density (PSD) and which are capable of binding to other proteins. Shank proteins contain multiple protein interaction domains, including ankyrin repeats, SH3 domain, PDZ domain, at least one proline rich domain and at least one SAM domain. The PDZ domain of Shank mediates binding to the carboxy-terminus of guanylate kinase associated protein (GKAP), and this interaction is important in neuronal cells for the synaptic localization of Shank proteins. Shank proteins also interact with Homer proteins and therefore Shank and Homer may serve as a protein bridge that links specific proteins that bind to Homer and specific proteins that bind to Shank. Exemplary Shank proteins include Shank 1a, Shank 1b and Shank 3, and cortactin binding protein, and the like.

A compound can affect the formation of cell-surface receptors into clusters by either stimulating the formation of cell-surface receptors into clusters or by inhibiting the recruitment of cell-surface receptors into clusters. When the effect is "inhibition", cell-surface clustering is decreased as compared with the level in the absence of the test compound. When the effect is "stimulation", cell-surface clustering is increased as compared to a control in the absence of the test compound.

A method is further provided for treating a subject with a disorder associated with metabotropic receptors or ion channel receptors comprising administering to the subject a therapeutically effective amount of a compound that modulates Homer protein activity. In yet another embodiment, a method is provided for treating a subject with a disorder associated with Homer protein activity, comprising administering to the subject a therapeutically effective amount of a compound that modulates Homer protein activity.

Essentially, any disorder that is etiologically linked to a glutamate receptor, an inositol trisphosphate receptor, a ryanodine receptor, a Shank protein , 142 (or other Homer interacting proteins) or to a Homer protein could be considered susceptible to treatment with an agent that modulates Homer protein activity. The disorder may be a neuronal cell disorder. Examples of neuronal cell disorders include but are not limited to Alzheimer's disease, Parkinson's disease, stroke, epilepsy, neurodegenerative disease, Huntington's disease, and brain or spinal cord injury/damage, including ischemic injury. The disorder may also be a disorder of a cardiac disorder, a disorder of musculature, a renal disorder, a uterine disorder or a disorder of bronchial tissue. The disorder may be epilepsy, glutamate toxicity, a disorder of memory, a disorder of learning or a disorder of brain development.

Detection of altered (decreased or increased) levels of "Homer protein activity" can be accomplished by hybridization of nucleic acids isolated from a cell of interest with a Homer polynucleotide of the invention. Analysis, such as Northern Blot analysis, are utilized to quantitate expression of Homer, such as to measure Homer transcripts. Other standard nucleic acid detection techniques will be known to those of skill in the art. Detection of altered levels of Homer can also accomplished using assays designed to detect Homer polypeptide. For example, antibodies or petides that specifically bind a Homer polypeptide can be utilized. Analyses, such as radioimmune assay or immunohistochemistry, are then used to measure Homer, such as to measure protein concentration qualitatively or quantitatively.

Treatment can include modulation of Homer activity by administration of a therapeutically effective amount of a compound that modulates Homer or Homer protein activity. The term "modulate" envisions the suppression of Homer activity or expression when Homer is overexpressed or has an increased activity as compared to a control. The term "modulate" also includes the augmentation of the expression of Homer when it is underexpressed or has a decreased activity as compared to a control. The term "compound" as used herein describes any molecule, *e.g.,* protein, nucleic acid, or pharmaceutical, with the capability of altering the expression of Homer polynucleotide or activity of Homer polypeptide. Treatment may inhibit the interaction of the EVH1 domain of Homer with its target protein , may increase the avidity of this interaction by means of allosteric effects, may block the binding activity of the coiled-coil doamin of Homer or influence other functional properties of Homer proteins.

Candidate agents include nucleic acids encoding a Homer, or that interfere with expression of Homer, such as an antisense nucleic acid, ribozymes, and the like. Candidate agents also encompass numerous chemical classes wherein the agent modulates Homer expression or activity.

Where a disorder is associated with the increased expression of Homer, nucleic acid sequences that interfere with the expression of Homer can be used. In this manner, the coupling of cell-surface and intracellular receptors can be inhibited. This approach also utilizes, for example, antisense nucleic acid, ribozymes, or triplex agents to block transcription or translation of Homer mRNA, either by masking that mRNA with an antisense nucleic acid or triplex agent, or by cleaving it with a ribozyme in disorders associated with increased Homer. Alternatively, a dominant negative form of Homer polypeptide could be administered.

When Homer is overexpressed, candidate agents include antisense nucleic acid sequences. Antisense nucleic acids are DNA or RNA molecules that are complementary to at least a portion of a specific mRNA molecule (Weintraub, 1990, *Scientific American,* **262**:40). In the cell, the antisense nucleic acids hybridize to the corresponding mRNA, forming a double-stranded molecule. The antisense nucleic acids interfere with the translation of the mRNA, since the cell will not translate a mRNA that is double-stranded. Antisense oligomers of about 15 nucleotides are preferred, since they are easily synthesized and are less likely to cause problems than larger molecules when introduced into the target cell. The use of antisense methods to inhibit the *in vitro* translation of genes is well known in the art (Marcus-Sakura, 1988, *Anal.Biochem*., **172**:289).

Use of an oligonucleotide to stall transcription is known as the triplex strategy since the oligomer winds around double-helical DNA, forming a three-strand helix. Therefore, these triplex compounds can be designed to recognize a unique site on a chosen gene (Maher, *et al.,* 1991, *Antisense Res. and Dev.,* **1**(3):227; Helene, C., 1991, *Anticancer Drug Design,* 6(6):569).

Ribozymes are RNA molecules possessing the ability to specifically cleave other single-stranded RNA in a manner analogous to DNA restriction endonucleases. Through the modification of nucleotide sequences which encode these RNAs, it is possible to engineer molecules that recognize specific nucleotide sequences in an RNA molecule and cleave it (Cech, 1988, *J.Amer.Med. Assn.,* **260**:3030). A major advantage of this approach is that, because they are sequence-specific, only mRNAs with particular sequences are inactivated.

There are two basic types of ribozymes namely, *tetrahymena*-type (Hasselhoff, 1988, *Nature,* **334:**585) and "hammerhead"-type. *Tetrahymena*-type ribozymes recognize sequences which are four bases in length, while "hammerhead"-type ribozymes recognize base sequences 11-18 bases in length. The longer the recognition sequence, the greater the likelihood that the sequence will occur exclusively in the target mRNA species. Consequently, hammerhead-type ribozymes are preferable to *tetrahymena*-type ribozymes for inactivating a specific mRNA species and 18-based recognition sequences are preferable to shorter recognition sequences.

When a disorder is associated with the decreased expression of Homer, nucleic acid sequences that encode Homer can be used. An agent which modulates Homer expression includes a polynucleotide encoding a polypeptide of SEQ ID NO:2, 4, 6, 8, 10 or 12, or a conservative variant thereof. Alternatively, an agent of use with the subject invention includes agents that increase the expression of a polynucleotide encoding Homer or an agent that increases the activity of Homer polypeptide.

In another embodiment of the invention, there is provided a transgenic non-human animal having a transgene that expresses Homer 1a chromosomally integrated into the germ cells of the animal. Animals are referred to as "transgenic" when such animal has had a heterologous DNA sequence, or one or more additional DNA sequences normally endogenous to the animal (collectively referred to herein as "transgenes") chromosomally integrated into the germ cells of the animal. The transgenic animal (including its progeny) will also have the transgene fortuitously integrated into the chromosomes of somatic cells.

Various methods to make the transgenic animals of the subject invention can be employed. Generally speaking, three such methods may be employed. In one such method, an embryo at the pronuclear stage (a "one cell embryo") is harvested from a female and the transgene is microinjected into the embryo, in which case the transgene will be chromosomally integrated into both the germ cells and somatic cells of the resulting mature animal. In another such method, embryonic stem cells are isolated and the transgene incorporated therein by electroporation, plasmid transfection or microinjection, followed by reintroduction of the stem cells into the embryo where they colonize and contribute to the germ line. Methods for microinjection of mammalian species is described in United States Patent No. 4,873,191. In yet another such method, embryonic cells are infected with a retrovirus containing the transgene whereby the germ cells of the embryo have the transgene chromosomally integrated therein. When the animals to be made transgenic are avian, because avian fertilized ova generally go through cell division for the first twenty h in the oviduct, microinjection into the pronucleus of the fertilized egg is problematic due to the inaccessibility of the pronucleus. Therefore, of the methods to make transgenic animals described generally above, retrovirus infection is preferred for avian species, for example as described in U.S. Patent No. 5,162,215. If microinjection is to be used with avian species, however, a recently published procedure by Love *et al*., (Biotechnology, 12, Jan 1994) can be utilized whereby the embryo is obtained from a sacrificed hen approximately two and one-half h after the laying of the previous laid egg, the transgene is microinjected into the cytoplasm of the germinal disc and the embryo is cultured in a host shell until maturity. When the animals to be made transgenic are bovine or porcine, microinjection can be hampered by the opacity of the ova thereby making the nuclei difficult to identify by traditional differential interference-contrast microscopy. To overcome this problem, the ova can first be centrifuged to segregate the pronuclei for better visualization.

The "non-human animals" of the invention are murine typically (e.g., mouse). The "transgenic non-human animals" of the invention are produced by introducing "transgenes" into the germline of the non-human animal. Embryonal target cells at various developmental stages can be used to introduce transgenes. Different methods are used depending on the stage of development of the embryonal target cell. The zygote is the best target for microinjection. The use of zygotes as a target for gene transfer has a major advantage in that in most cases the injected DNA will be incorporated into the host gene before the first cleavage (Brinster *et al., Proc. Natl*. *Acad. Sci. USA* 82:4438-4442, 1985). As a consequence, all cells of the transgenic non-human animal will carry the incorporated transgene. This will in general also be reflected in the efficient transmission of the transgene to offspring of the founder since 50% of the germ cells will harbor the transgene.

The term "transgenic" is used to describe an animal which includes exogenous genetic material within all of its cells. A "transgenic" animal can be produced by cross-breeding two chimeric animals which include exogenous genetic material within cells used in reproduction. Twenty-five percent of the resulting offspring will be transgenic i.e., animals which include the exogenous genetic material within all of their cells in both alleles. 50% of the resulting animals will include the exogenous genetic material within one allele and 25% will include no exogenous genetic material.

In the microinjection method useful in the practice of the subject invention, the transgene is digested and purified free from any vector DNA *e.g.* by gel electrophoresis. It is preferred that the transgene include an operatively associated promoter which interacts with cellular proteins involved in transcription, ultimately resulting in constitutive expression. Promoters useful in this regard include those from cytomegalovirus (CMV), Moloney leukemia virus (MLV), and herpes virus, as well as those from the genes encoding metallothionin, skeletal actin, P-enolpyruvate carboxylase (PEPCK), phosphoglycerate (PGK), DHFR, and thymidine kinase.

Promoters for viral long terminal repeats (LTRs) such as Rous Sarcoma Virus can also be employed. Constructs useful in plasmid transfection of embryonic stem cells will employ additional regulatory elements well known in the art such as enhancer elements to stimulate transcription, splice acceptors, termination and polyadenylation signals, and ribosome binding sites to permit translation.

Retroviral infection can also be used to introduce transgene into a non-human animal, as described above. The developing non-human embryo can be cultured in vitro to the blastocyst stage. During this time, the blastomeres can be targets for retro viral infection (Jaenich, R., Proc. Natl. Acad. Sci USA 73:1260-1264, 1976). Efficient infection of the blastomeres is obtained by enzymatic treatment to remove the zona pellucida (Hogan, *et al*. (1986) in Manipulating the Mouse Embryo, Cold Spring Harbor Laboratory Press, Cold Spring Harbor, N.Y). The viral vector system used to introduce the transgene is typically a replication-defective retro virus carrying the transgene (Jahner*, et al., Proc. Natl. Acad. Sci. USA* 82:6927-6931, 1985; Van der Putten, *et al., Proc. Natl. Acad. Sci USA* 82:6148-6152, 1985). Transfection is easily and efficiently obtained by culturing the blastomeres on a monolayer of virus-producing cells (Van der Putten, *supra*; Stewart, *et al*., *EMBO J*. 6:383-388, 1987). Alternatively, infection can be performed at a later stage. Virus or virus-producing cells can be injected into the blastocoele (D. Jahner *et al., Nature* 2-98:623-628, 1982). Most of the founders will be mosaic for the transgene since incorporation occurs only in a subset of the cells which formed the transgenic nonhuman animal. Further, the founder may contain various retro viral insertions of the transgene at different positions in the genome which generally will segregate in the offspring. In addition, it is also possible to introduce transgenes into the germ line, albeit with low efficiency, by intrauterine retroviral infection of the midgestation embryo (D. Jahner *et al., supra*).

A third type of target cell for transgene introduction is the embryonal stem cell (ES). ES cells are obtained from pre-implantation embryos cultured in vitro and fused with embryos (M. J. Evans *et al. Nature* 292:154-156, 1981; M.O. Bradley *et al., Nature* 309: 255-258, 1984; Gossler, *et al., Proc. Natl. Acad. Sci USA* 83: 9065-9069, 1986; and Robertson *et al., Nature* 322:445-448, 1986). Transgenes can be efficiently introduced into the ES cells by DNA transfection or by retro virus-mediated transduction. Such transformed ES cells can thereafter be combined with blastocysts from a nonhuman animal. The ES cells thereafter colonize the embryo and contribute to the germ line of the resulting chimeric animal. (For review see Jaenisch, R., *Science* 240: 1468-1474, 1988).

"Transformed" means a cell into which (or into an ancestor of which) has been introduced, by means of recombinant nucleic acid techniques, a heterologous nucleic acid molecule. "Heterologous" refers to a nucleic acid sequence that either originates from another species or is modified from either its original form or the form primarily expressed in the cell.

"Transgene" means any piece of DNA which is inserted by artifice into a cell, and becomes part of the genome of the organism (i.e., either stably integrated or as a stable extrachromosomal element) which develops from that cell. Such a transgene may include a gene which is partly or entirely heterologous (i.e., foreign) to the transgenic organism, or may represent a gene homologous to an endogenous gene of the organism. Included within this definition is a transgene created by the providing of an RNA sequence which is transcribed into DNA and then incorporated into the genome. The transgenes of the invention include DNA sequences which encode Homer protein-sense and antisense polynucleotides, which may be expressed in a transgenic non-human animal. The term "transgenic" as used herein additionally includes any organism whose genome has been altered by in vitro manipulation of the early embryo or fertilized egg or by any transgenic technology to induce a specific gene knockout. As used herein, the term "transgenic" includes any transgenic technology familiar to those in the art which can produce an organism carrying an introduced transgene or one in which an endogenous gene has been rendered non-functional or "knocked out".

Antibodies of the invention may bind to Homer proteins or Homer interacting proteins provided by the invention to prevent normal interactions of the Homer proteins and Homer Interacting proteins. Binding of antibodies to Homer proteins or Homer Interacting Proteins can interfere with cell-signaling by interfering with an intracellular signaling pathway. Binding of antibodies can interfere with Homer protein binding to extracellular receptors, *e.g*., to NMDA receptors, to metabotropic receptors, and the like. Binding of antibodies can interfere with Homer protein binding to intracellular receptors, *e.g*., inositol trisphosphate receptors, and the like. Furthermore, binding to Homer proteins or to Homer Interacting Proteins can interfere with cell-surface receptor clustering mediated by Homer family proteins.

The antibodies of the invention can be used in any subject in which it is desirable to administer in vitro or in vivo immunodiagnosis or immunotherapy. The antibodies of the invention are suited for use, for example, in immunoassays in which they can be utilized in liquid phase or bound to a solid phase carrier. In addition, the antibodies in these immunoassays can be detectably labeled in various ways. Examples of types of immunoassays which can utilize antibodies of the invention are competitive and non-competitive immunoassays in either a direct or indirect format. Examples of such immunoassays are the radioimmunoassay (RIA) and the sandwich (immunometric) assay. Detection of the antigens using the antibodies of the invention can be done utilizing immunoassays which are run in either the forward, reverse, or simultaneous modes, including immunohistochemical assays on physiological samples. Those of skill in the art will know, or can readily discern, other immunoassay formats without undue experimentation.

The term "antibody" as used in this invention includes intact molecules as well as fragments thereof, such as Fab, F(ab')2, and Fv which are capable of binding to an epitopic determinant present in an invention polypeptide. Such antibody fragments retain some ability to selectively bind with its antigen or receptor.

Methods of making these fragments are known in the art. (See for example, Harlow and Lane, Antibodies: A Laboratory Manual, Cold Spring Harbor Laboratory, New York (1988), incorporated herein by reference).Monoclonal antibodies are made from antigen containing fragments of the protein by methods well known to those skilled in the art (Kohler, *et al., Nature*, 256:495, 1975).

Antibodies which bind to an invention polypeptide of the invention can be prepared using an intact polypeptide or fragments containing small peptides of interest as the immunizing antigen. For example, it may be desirable to produce antibodies that specifically bind to the N- or C-terminal domains of an invention polypeptide. The polypeptide or peptide used to immunize an animal is derived from translated cDNA or chemically synthesized and can be conjugated to a carrier protein, if desired. Commonly used carrier proteins which may be chemically coupled to the immunizing peptide include keyhole limpet hemocyanin (KLH), thyroglobulin, bovine serum albumin (BSA), tetanus toxoid, and the like.

Invention polyclonal or monoclonal antibodies can be further purified, for example, by binding to and elution from a matrix to which the polypeptide or a peptide to which the antibodies were raised is bound. Those of skill in the art will know of various techniques common in the immunology arts for purification and/or concentration of polyclonal antibodies, as well as monoclonal antibodies (See, for example, Coligan, *et al*., Unit 9, Current Protocols in Immunology, Wiley Interscience, 1994, incorporated by reference).

The antibodies of the invention can be bound to many different carriers and used to detect the presence of an antigen comprising the polypeptides of the invention. Examples of well-known carriers include glass, polystyrene, polypropylene, polyethylene, dextran, nylon, amylases, natural and modified celluloses, polyacrylamides, agaroses and magnetite. The nature of the carrier can be either soluble or insoluble for purposes of the invention . Those skilled in the art will know of other suitable carriers for binding antibodies, or will be able to ascertain such, using routine experimentation.

There are many different labels and methods of labeling known to those of ordinary skill in the art. Examples of the types of labels which can be used in the present invention include enzymes, radioisotopes, fluorescent compounds, colloidal metals, chemiluminescent compounds, phosphorescent compounds, and bioluminescent compounds. Those of ordinary skill in the art will know of other suitable labels for binding to the antibody, or will be able to ascertain such, using routine experimentation.

Another technique which may also result in greater sensitivity consists of coupling the antibodies to low molecular weight haptens. These haptens can then be specifically detected by means of a second reaction. For example, it is common to use such haptens as biotin, which reacts with avidin, or dinitrophenyi, puridoxal, and fluorescein, which can react with specific antihapten antibodies. In using the monoclonal and polyclonal antibodies of the invention for the in vivo detection of antigen, e.g., Homer, the detectably labeled antibody is given a dose which is diagnostically effective. The term "diagnostically effective" means that the amount of detectably labeled antibody is administered in sufficient quantity to enable detection of the site having the antigen comprising a polypeptide of the invention for which the antibodies are specific. The concentration of detectably labeled antibody which is administered should be sufficient such that the binding to those cells having the polypeptide is detectable compared to the background. Further, it is desirable that the detectably labeled antibody be rapidly cleared from the circulatory system in order to give the best target-to-background signal ratio. As a rule, the dosage of detectably labeled antibody for in vivo treatment or diagnosis will vary depending on such factors as age, sex, and extent of disease of the individual. Such dosages may vary, for example, depending on whether multiple injections are given, antigenic burden, and other factors known to those of skill in the art.

The following examples are intended to illustrate but not to limit the invention in any manner, shape, or form, either explicitly or implicitly. While they are typical of those that might be used, other procedures, methodologies, or techniques known to those skilled in the art may alternatively be used.

### EXAMPLES

Homer 1a is an IEG and is the original member of a family of proteins that function together as a regulated adapter system that is hypothesized to control the coupling of membrane receptors to intracellular pools of releasable calcium. Homer proteins function at excitatory synapses to couple membrane group 1 metabotropic glutamate receptors (mGluR) to endoplasmic reticulum-associated inositol trisphosphate receptors (IP3R) (Brakeman *et al*., 1997; Tu *et al*., 1998; Xiao *et al*., 1998). Current studies suggest a broader role for Homer proteins in calcium signaling and receptor trafficking. The Shank family of proteins was identified based on their association with Homer (Naisbitt *et al.,* 1999; Tu *et al.,* 1999). Shank, together with Homer, appears to be part of both the NMDA and group 1 mGluR signaling complexes. By virtue of its interaction with Shank, Homer provides a mechanism to couple NMDA Ca2+ influx to intracellular Ca2+-induced Ca2+ release pools. The inventors have identified additional Homer-interacting proteins that provide insight into the role of Homer in trafficking of group 1 mGluR (e.g., SEQ ID NOS:16, 18, 20). Because these Homer-dependent cellular processes are regulated by the IEG form of Homer (Homer 1a), mechanisms by which Homer proteins can modulate Ca2+ dynamics of mGluR and NMDA receptors, as well as regulate receptor trafficking are defined.

Homer family proteins possess an N-terminal EVH1 domain that mediates interactions with mGluRs, IP3R, Shank and other novel proteins. The EVH1 domain has been determined to bind the proline rich motif PPXXFR *(Tu et al*., 1998). The present invention provides the crystal structure of the Homer EVH1 domain. In complementary studies, genetic approaches were used to identify critical residues in both the EVH1 domain and the ligand that modulate the affinity of the Homer-mGluR (and other Homer-interacting proteins) interaction. This information is essential to an understanding of the integrative cellular actions of Homer proteins,. Together, these studies define the molecular basis of specificity of EVH1 interaction with its ligands, and provide insight into how the EVH1 interaction is regulated.

This patent application includes a description of several Homer-interacting proteins that are part of the signaling network that is controlled by Homer (e.g., SEQ ID NOS: 16, 18, 20). Yeast two-hybrid screens and searches of NCBI protein data bases identified a set of known and novel candidate interacting proteins for Homer include the ryanodine receptor, NMDA receptor subunit NR2D, human InaD and novel interacting proteins termed I42 and I30. As described below, current data indicate that agents can be developed that specifically modulate the crosslinking activity of Homer for these various receptors and thereby provide novel theraputics that regulate the output of these receptors on cellular function.

Homer acts in several ways to regulate cellular function. Homer and Homer-related proteins function as an adapter system to couple membrane receptors to intracellular pools of releasable Ca. This "signaling" function of Homer is documented in Xiao et (1998), Tu *et al* (1998 and 1999) Naisbett *et al*. (1999), as well as by studies of the novel Homer-interacting protein termed I42 (see below). By virtue of its crosslinking activity, Homer proteins play a role in synaptogenesis and spatial targeting/trafficking of GluRs to other postsynaptic structural proteins. This function of Homer is supported by observations in Tu *et al* (1999) and Naisbett *et al* (1999).

### Initial cloning of Homer; a novel brain Immediate Early Gene (IEG)

Homer was cloned in a differential screen of seizure-stimulated hippocampus. Prior work, in which IEG induction was examined in brain provided a detailed understanding of time course and tissue distribution of the IEG response (Cole *et al*., 1989; Saffen *et al*., 1988; Worley *et al*., 1990), and suggested a paradigm to maximally induce novel IEG mRNAs (Lanahan and Worley, 1998; Worley *et al*., 1990). Once cloned, *in situ* hybridization was used to screen for IEGs that were regulated in other paradigms that activate neurons including LTP stimulation in the hippocampus (Brakeman *et al.,* 1997) and acute administration of cocaine. In these models, Homer was one of the most highly induced of all of the IEGs (Brakeman *et al.,* 1997). Initial characterization of Homer was challenging in that the mRNA was nearly 7 kb, while the best deduced open reading frame was only 186 aa, and was located near the 5' end of the cDNA (Brakeman *et al*., 1997). The 3' UTR was over 5 kb. The ORF was confirmed by in vitro transcription and translation of the cDNA, and rabbit polyclonal antisera were generated against bacterially expressed fusion proteins. With these antibodies, we were able to demonstrate that the protein was rapidly and transiently induced in the hippocampus following a seizure (Brakeman *et al*., 1997). This confirmed the deduced ORF and assured us that the cDNA was indeed translated in brain.

### Homer selectively binds group 1 metabotropic receptors and is enriched at synapses

In an effort to discover the function of Homer, a yeast 2-hybrid technique (Chevray and Nathans, 1992; Fields and Song, 1989) was used to screen a cDNA library prepared from rat hippocampus and cortex. The full length Homer IEG was used as bait. Among ~30 confirmed interacting cDNAs, one encoded the C-terminal 250 aa of mGluR5. We initially confirmed that the proteins bind using a GSTHomer in a pulldown assay with either fragments of mGluR5, or full length mGluR5 expressed in heterologous cells (HEK293 cells) (Brakeman *et al*., 1997). Homer protein also bound to mGluR1a, but not mGluR2, 3, 4, or 7. This was an interesting clue to the function of Homer since mGluR1 and mGluR5 (termed group 1 metabotropic receptors) couple to phospholipase C and active hydrolysis of phosphoinositides to generate inositol trisphosphate and diacylglycerol (Nakanishi *et al.,* 1994). mGluR1a and 5 also share sequence similarity in their long, cytosolically disposed C-terminus. Other metabotropic glutamate receptors (termed group 2 and 3) inhibit adenylate cyclase activity, and have short C-termini that lack homology to group 1 receptors. We proceeded to test whether Homer and mGluR5 naturally associate in brain and confirmed that these proteins co-immunoprecipitate from detergent extracts of hippocampus (Brakeman *et al*., 1997). The next major clue was provided by the observation that Homer immunoreactivity was enriched at excitatory synapses (Brakeman *et al.,* 1997). In brain, Homer protein was associated with dendrites and showed a punctate pattern consistent with a localization in spines. The binding properties and cellular distribution of Homer suggested a role at the excitatory synapse.

### Homer is a member of a family of closely related proteins that are enriched at the excitatory synapse

A search of the NCBI sequence data base identified several ESTs that showed strong homology to Homer, but were clearly distinct in that they encoded additional C-terminal sequence (Brakeman *et al*., 1997). Using a combination of screening strategies, a family of 12 cDNAs was identified from rat, mouse, *Drosophila,* and human (Xiao *et al*., 1998). All of these cDNAs encoded proteins with a similar protein structure and were deduced to be the products of 3 independent mammalian genes (termed Homer 1, 2, 3) and 1 *Drosophilia* gene. Like Homer IEG (now termed Homer 1a), all new family members contain an N-terminal, ~110 amino acid domain that binds mGluR1a/5 ((Xiao *et al*., 1998). The region of Homer that interacts with mGluR1a/5 is termed an EVH1 domain based on its modest homology (20-25% identity) to domains in a family of proteins that include *Drosophilia* Enabled [Gertler, 1996], mammalian VASP (Haffner *et al*., 1995) and the Wiscott-Aldridge protein (WASP) (Ponting and Phillips, 1997; Symons *et al*., 1996). The EVH1 domains of Homer proteins from *Drosophilia,* rodent and human are conserved at a level of 80% identity (Xiao *et al*., 1998). Other than the IEG Homer 1a, all new forms of Homer encode an additional C-terminal domain with predicted coiled-coil (CC) structure.

As the nomenclature suggests, *Homer 1* gene encodes both the IEG form (Homer 1a) and splice forms that encode CC domains termed Homer 1b and 1c. The 1b and 1c splice forms differ in their inclusion of an approximately 10 amino acid sequence located between the EVH1 and CC domains. (Homer family members that encode CC domains are also referred to as CC-Homers to distinguish them from Homer 1a, which lacks a CC domain.) Similarly, *Homer 2* encodes two CC-Homer splice forms termed Homer 2a and 2b, which also differ by a short internal sequence between EVH1 and CC domains. Homer 3 encodes a single form. The CC domains are less conserved than the EVH1 domain (~40% identity between rat Homer 1, 2 and 3) but they are able to specifically bind to themselves and to CC-domains of other Homer family members (Xiao *et al*., 1998). Homer CC domains do not interact with other representative CC-domain proteins in GST pulldown assays, and a yeast 2-hybrid screen of brain cDNA with the CC-domain of Homer 1 identified multiple copies of Homer 1, Homer 2 and Homer 3, but not other CC domains (Xiao *et al*., 1998). As evidence that Homer proteins can naturally self-multimerize, we demonstrated that Homer 1b/Homer 3 heteromultimers co-immunoprecipitate from brain (Xiao *et al*., 1998). These observations indicate that the Homer CC domains mediate specific self-association.

In contrast to Homer 1a, all CC-containing Homer family members are constitutively expressed in brain (Xiao *et al*., 1998)]. This was confirmed using both Northern blot and in situ hybridization assays which compared expression with Homer 1a in the same material. mRNA and protein expression of Homer 1b/c, Homer 2 and Homer 3 are unchanged in hippocampus following a seizure while Homer 1a mRNA and protein are induced at least 10 fold.

Antibodies were generated that specifically recognize each of the CC-Homers. Antibodies were raised against synthetic C-terminal peptide sequences. Because Homer 1b and 1c possess identical C-termini, the C-terminal antibodies recognize both splice forms. Similarly, C-terminal Homer 2 antibodies recognize both Homer 2a and 2b. Accordingly, when using these antibodies to detect Homer proteins, we refer to the immunoreactivity as Homer 1b/c or Homer 2a/b. We used these antibodies to determine that Homer 1b/c and 3 are enriched in a detergent resistant fraction of the postsynaptic density (PSD) (Xiao *et al*., 1998). Homer 2a/b is also enriched in synaptic fractions, but is relatively more soluble than Homer 1b/c and Homer 3. Like Homer 1a, each of the CC-Homers co-immunoprecipitates with group 1 mGluRs from brain (Xiao *et al*., 1998). Immunogold electron microscopy (EM) demonstrated that Homer 1b/c and Homer 3 are ultrastructurally localized at the PSD (Xiao *et al*., 1998). These observations suggest that CC-Homer proteins function as multivalent adapter complexes that bind mGluRs at postsynaptic sites.

### Homer 1a functions as a natural dominant negative protein.

The fact that Homer 1a lacks a CC domain suggested that it may function as a natural dominant negative to disrupt cross-linking of CC-Homers. In this model, the EVH1 domain of Homer 1a can bind and compete for the same target proteins as CC-Homers (such as mGluR5), but because Homer 1a lacks the CC-domain, it cannot self-associate and cannot cross-link. To test the dominant negative hypothesis, we generated a transgenic mouse that constitutively expressed Homer 1a in brain neurons under the control of a modified Thy-1 promoter [Aigner, 1995 #200]. We confirmed transgene expression in hippocampus, cerebellum and cortex in two independent lines (Xiao *et al*., 1998). The level of transgene expression in the hippocampus was similar to natural Homer 1a expression induced by a seizure. In contract to the natural Homer 1 a, however, the transgene was constitutively expressed in the unstimulated mouse. A prediction of the dominant negative hypothesis is that the ability to co-immunoprecipitate mGluR with Homer 1b/c or Homer 3 antibodies should be diminished in the transgenic mouse. As one of the controls for this experiment, we demonstrated by western blot that levels of expression of mGluRla, mGluR5 and Homer 1b/c, 2a/b, 3 were unchanged in the transgenic mouse brain. We then performed IP experiments and observed the anticipated result; the co-immunoprecipitation of mGluR5 with CC-Homers from hippocampus was reduced in the transgenic mouse (Xiao *et al.,* 1998). Similar co-immunoprecipitations of mGluRla with Homer 3 from cerebellum was also reduced. As an additional control, we demonstrated that the ability to co-immunoprecipitate Homer 1b/c with Homer 3 was not altered in the transgenic mouse. This was the predicted result since the association between these proteins is mediated by their CC domains, and this interaction is not altered by the Homer 1a EVH1 domain. These observations support the hypothesis that Homer 1a functions as a natural dominant negative to regulate CC-Homer-dependent cross-linking.

### Homer binds a proline rich sequence that is ~50 aa from the C-terminus of group 1 mGluRs.

When we initially characterized the interaction between Homer and mGluR5, we anticipated that Homer might bind the free C-terminus. This surmise was based on the precedent of PDZ proteins such as PSD95 and GRIP, which bind the free C-terminus of NMDAR2 (Kornau, 1995) and AMPA receptors (Dong *et al*., 1997). Homer was noted to encode a GLGF sequence like the PDZ domain. Additionally, in GST pulldown assays that used brief washes, we noted a modest reduction of binding when the C-terminal 4 or 10 aa were deleted from mGluR5 [Brakeman, 1997 #99]. (In retrospect, this modest reduction of binding may be due to Homer pulldown of Shank which does bind the free C-terminus of mGluR5, but appears to be lower affinity than Homer-mGluR5 binding; see below.) However, with more standard wash conditions, it became clear that the 4 and 10 aa C-terminal deletion mutants of mGluR5 continued to bind avidly to Homer. We continued the deletion strategy until we found that a 50 aa C-terminal deletion of mGluR5 destroyed binding to Homer. By contrast, a 41 aa deletion of mGluR5 retained full binding activity. We noted that the intervening sequence was proline rich and shared sequence similarity with the previously described SH3 ligand sequence [Yu, 1994 #166] We prepared a series of point mutants based on the known structure-function relationship for SH3 ligands. Binding assays confirmed general characteristics of SH3 ligand binding, but also demonstrated that that the Homer binding site is distinct in the positioning of critical amino acids *(Tu et al*., 1998). A consensus for binding was determined to be PPXXFR, consistent with the observation that mutation of either of the prolines or the phenylalanine, or a change in their relative position, interrupted binding. The arginine in the last position is preferred over other amino acids, but is not essential. Mutations were identically effective in interrupting binding to each of the Homer family members including Homer 1a, 1b/c, 2a/b, 3 and an EVH1 only fragment (110aa) of Homer 1. Thus, we conclude that the interaction with mGluR5 is mediated by the Homer EVH1 domain.

Mutations of mGluR5 were initially tested in the context of a 250aa C-terminal fragment, but were also determined to have an identical effect on binding when placed in the full length mGluR5 protein (Tu *et al*., 1998). This exquisite sensitivity of Homer binding to changes in single amino acid within the Homer-ligand site has been confirmed in other Homer-interacting proteins including mGluR1a (Tu *et al*., 1998), Shank (Tu *et al*., 1999) and I42 (see below). To further confirm that the interaction was mediated by a direct interaction at the Homer-ligand site (as opposed to a secondary allosteric effect on a remote binding site), we prepared synthetic 10 mer peptides with either the wild type, or F-to-R mutation, and demonstrated that the wild type peptide blocked binding of mGluR1a or mGluR5 to each of the Homer family members [(Tu *et al*., 1998)]. Approximately half of the binding was blocked at a peptide concentration of 3.4 micromolar. By contrast, the F-to-R mutant peptide did not alter binding at concentrations as high as 340 micromolar.

### Homer binds the IP3 receptor.

Armed with a consensus sequence that predicted binding to Homer, we searched the NCBI data base for other proteins that might bind Homer. A Homer-ligand site was identified in the IP3R, dynamin III, a human alpha adrenergic receptor and the ryanodine receptor [(Tu *et al*., 1998)]. Each of these interactions were determined to be consistent with the known topology of the candidate interacting protein, assuming that Homer proteins are cytosolic. We were able to confirm a biochemical interaction of Homer with the IP3R and dynamin III using GST pull down assays. More importantly, we demonstrated that the IP3R co-immunoprecipitates with each of the Homer 1b/c, 2a/b and 3 from detergent extracts of cerebellum [(Tu *et al*., 1998)]. Homer appears to be associated with a substantial portion of IP3R in the cerebellum, since a cocktail of the three Homer antibodies is able to specifically (compared to a cocktail of preimmune serums) co-immunoprecipitate ~50% of the total IP3R in detergent extracts (CHAPS).

### CC-Homers function to link mGluR5 and IP3R in a signaling complex.

Based on the prior observations, we examined the hypothesis that CC-Homers might cross-link mGluR and IP3R. This notion was appealing in that the IP3R is part of the signaling network that is activated upon glutamate stimulation of mGluR1/5. Signaling complexes had previously been described including; AKAP proteins which function as scaffolds for specific kinases and their substrates [Lester, 1997 #149], and the *Drosophila* protein InaD which couples the membrane light activated channel with its down stream effector enzyme, phospholipase C [Tsunoda, 1997 #147]. Unlike these other examples of signaling complexes, however, Homer would need to form a bridge between receptors in two different membranes. Functional mGluRs are in the plasma membrane while the IP3R is localized primarily to intracellular endoplasmic reticulum (ER). In support of the notion that ER and plasma membranes can come in close apposition in neurons, we noted that Dr. Kristin Harris (Harvard) described the presence of smooth ER (SER, or spine apparatus) in the spines of hippocampal and cerebellar neurons (Tu *et al*., 1998). Remarkably, the SER forms close appositions with the plasma membrane that were uniquely localized to the lateral margin of the PSD. These sites are precisely where the group 1 mGluRs are localized (Baude *et al*., 1993; Lujan *et al*., 1997; Nusser *et al*., 1994). The IP3R is present in spines of cerebellar Purkinje neurons where it is associated with the spine apparatus (Satoh *et al*., 1990). (Interestingly, in hippocampal neurons, the RYR is present in the spine apparatus while the IP3R appears to be restricted to the dendritic shaft [reviewed in (Narasimhan *et al.,* 1998)]. Homer 1b/c and 3 are also enriched in the cytosol at the lateral margin of the PSD [(Xiao *et al.,* 1998)]. Thus, available anatomic evidence supported the notion that synaptic mGluRs come in close apposition with SER-associated IP3Rs at sites that are enriched for CC-Homers.

As a first test of the hypothesis that CC-Homers cross-link mGluR and IP3R, we asked whether we could detect a trimolecular complex of mGluR, Homer and IP3R in brain. Indeed, IP3R antibody specifically co-immunoprecipitateed Homer and mGluR1a from cerebellum (Narasimhan *et al*., 1998). Since IP3Rs are not known to directly interact with mGluR1a, this result supported the hypothesis that Homer bridges these proteins to form a trimolecular signaling complex. A further prediction of the "Homer hypothesis" is that Homer 1a should uncouple the putative mGluR-CC-Homer-IP3R complex. To test this, we monitored the effect of Homer 1a expression on glutamate-induced intracellular calcium release. Plasmids expressing Homer 1a or Homer 1b were transfected along with green fluorescent protein (gene gun) and identified Purkinje neurons were stimulated with quisqualate. A patch electrode containing the Ca2+ detector Fura-2 was attached to the soma and a holding potential of -60 mV was applied. Tetrodotoxin and picrotoxin were included in the bath to block synaptic input and EDTA/MgCl₂ was included to assure that measured Ca2+ increases in the cell were generated from intracellular stores. Under these conditions, quisqualate-induced Ca2+ increases are due to mGluRl-evoked release from IP3R pools (Roche *et al*., accepted). Expression of Homer 1b did not alter the induced Ca2+ transient compared to cells transfected with an empty vector. By contrast, neurons transfected with Homer 1a showed a Ca2+ transient that was reduced in amplitude and delayed in time to peak (Tu *et al*., 1998). This result is consistent with the notion that the IP3 generated by mGluR1a activation of phospholipase C is less effective in releasing Ca2+ from the IP3R pools in neurons expressing Homer 1a, and is anticipated if Homer 1a disrupts the physical linkage between mGluR1a and IP3R. Released IP3 must diffuse further, thereby resulting in a lower effective concentration of IP3 at the receptor.

### CC-Homers alter trafficking of mGluR1a/5 in heterologous cells.

We initiated studies to examine the effect of Homer on mGluR5 expression. When wild type mGluR5 was expressed in heterologous cells (HEK293, COS or HeLa) the receptor reached the plasma membrane surface where it was diffusely localized. This was also true when mGluR5 was co-expressed with Homer 1a. However, we noted that co-expression of mGluR5 with Homer 1b resulted in intracellular inclusions of mGluR5 (Roche *et al*., accepted). This effect of Homer 1b was dependent on the amount of transfected plasmid and was most obvious when equal amounts of Homer 1b and mGluR5 plasmids were co-transfected. There was a trend for higher level expression of mGluR5 when co-transfected with Homer 1b. When ratios of transfected plasmids were titrated so that total mGluR5 expression was the same (comparing expression with or without Homer 1b), a substantial portion of the total mGluR5 was associated with the intracellular pool when co-expressed with Homer 1b. In these cells, relatively less reached the plasma membrane compared to mGluR expressed alone, or co-expressed with Homer 1a. We further noted that at earlier times after transfection of Homer 1b and mGluR5, mGluR5 showed an enrichment in perinuclear organelles with a reticular pattern throughout the cell that resembled the ER. To assess the nature of the CC-Homer-dependent cellular accumulation, we compared the distribution of mGluR5 with the ER specific maker BIP [B (Roche *et al*., accepted)]. Staining with BIP antibodies revealed extensive ER present in both transfected and untransfected cells and co-localization with mGluR5. We also noted that the perinuclear organelles were not present within non-transfected cells and therefore appeared to be ER-derived structures unique to cells overexpressing mGluR5 and Homer 1b. These observations suggest that Homer 1b, but not Homer 1a, causes mGluR5 to be retained in the ER.

As an additional assay for ER retention, we examined the status of the carbohydrates present on mGluR5 in cells co-expressing Homer 1a or Homer 1b. If Homer 1b caused mGluR5 to be retained within the ER, then mGluR5 should contain immature, high mannose carbohydrates which are sensitive to digestion with the enzyme Endoglycosidase H (Endo H). Alternatively, if mGluR5 had successfully traveled through the ER and cis Golgi, it would possess mature, complex carbohydrates which would be Endo H resistant. Mature carbohydrates would be anticipated if mGluR5 was on the cell surface or if it was sequestered in a post-Golgi intracellular compartment such as endosomes. We determined that mGluR5 is Endo H resistant when expressed alone or with Homer 1a (Xiao *et al*., 1998). However, when expressed with H1b, mGluR5 is Endo H sensitive, consistent with the hypothesis that expression of H1b leads to the retention of group I mGluR in the ER.

The subcellular localization of the group II metabotropic glutamate receptor mGluR2 was the same whether expressed alone or with H1b. In addition, we used a series of mGluR5 constructs containing point mutations within the Homer binding site and found that mutations that disrupt mGluR5/Homer interactions *in vitro* also prevented ER retention of mGluR5 co-expressed with H1b [(Takei *et al*., 1994)]. mGluR5 P1125L, which does not bind to Homer *in vitro* (Tu *et al*., 1998), was not retained in the ER when co-expressed with H1b. In contrast, mGluR5 S1126F, which does bind Homer in vitro, was ER retained when co-expressed with H1b. Other point mutations in adjacent residues were analyzed and the results were consistent with *in vitro* binding studies [summarized in [B (Ikeda *et al*., 1995)], demonstrating that mGluR5 is retained within the ER by H1b only when its Homer binding site is intact.

While these experiments were performed in heterologous cells, we also noted enrichment of the group I metabotropic receptor mGluR1a in the ER of Purkinje cells [[(Kammermeier *et al*., submitted). Since Purkinje neurons express particularly high levels of CC-Homers [(Xiao *et al*., 1998)], this suggests Homer proteins may naturally regulate receptor trafficking through the ER. In this model, Homer 1a would be permissive for transfer through the ER Golgi system to insertion into the postsynaptic membrane. The ability of CC-Homers to alter the spatial distribution and metabolism of ER associated proteins may also impact the IP3R. IP3Rs in Purkinje neurons are associated with dense stacks of ER (Satoh *et al*., 1990) and this stacking morphology has been shown to be regulated by neural activity (Takei *et al*., 1994). Since a substantial portion of IP3R in cerebellum is associated with CC-Homers, it is possible that the ability of CC-Homer to crosslink interacting proteins on two adjacent membranes plays a regulatory role in ER morphology and function. Experiments in Aims 2 and 3 will examine this hypothesis.

### Homer Modulates mGluR coupling to ion channels.

Group 1 mGluRs modulate ionic currents by activating pertussis toxin-sensitive and -insensitive G proteins (Naisbitt *et al*., 1999). Modulation of Ca2+ currents by heterologously expressed group 1 mGluRs in superior cervical ganglion (SCG) neurons proceeds through multiple pathways involving both the a and βg - subunits of G proteins. We examined the effect of Homer on mGluR coupling to Ca2+ and M-type potassium channels in SCG neurons. CC-Homers, including 1b, 2b and 3 produced a similar reduction of the effect of group 1 mGluRs (Kim *et al.,* 1997; Naisbitt *et al.,* 1999; Naisbitt *et al.,* 1997; Takeuchi *et al.,* 1997). By contrast, Homer 1a or an engineered short form of Homer 2 did not block group 1 mGluR effects, but were able to partially reverse the effect of the CC-Homers.

### Homer interacts with Shank suggesting a role synaptogenesis and NMDAR function.

To gain further insight into the physiological function of Homer, we characterized a novel family of proteins that were identified based on their interaction with Homer 1a in a yeast 2-hybrid screen of a brain cDNA library. These Homer-interacting proteins were determined to be identical to the Shank family of PSD proteins that interact with GKAP and the PSD-95 complex (Tu *et al*., 1999). Shank proteins are specifically enriched at excitatory synapses and co-localize with NMDA receptors in primary neuronal cultures (Naisbitt *et al*., 1999). Shank proteins appear to be recruited to excitatory synapses by virtue of their interaction with GKAP, a synaptic protein that binds to the guanylate kinase domain of PSD-95 (Kim *et al.,* 1997; Naisbitt *et al.,* 1999; Naisbitt *et al.,* 1997; Takeuchi *et al.,* 1997). In addition to the PDZ domain which binds GKAP, Shank contains domains that mediate self-multimerization and interaction with cortactin (Golshani *et al.,* 1998). Shank also directly interacts with Homer [(Lujan *et al.,* 1997)]. Homer and Shank proteins co-localize at the PSD of CA1 pyramidal neurons [(Tu *et al.,* 1999)], and native Homer-Shank complexes were identified in brain using GST pull down assays of Shank with GKAP [(Otani and Connor, 1998)]. Additionally, Homer and Shank co-immunoprecipitate from brain [(Aniksztejn *et al*., 1991; Ben-Ari *et al*., 1992)]. These observations indicate that Shank and Homer naturally associate in brain. Biochemical studies indicate that the Shank-Homer interaction is mediated by the EVH1 domain of Homer which binds to a single Homer-ligand site present in the proline-rich domain of Shank proteins [(Tu *et al*., 1999)]. A quaternary complex of Homer/Shank/GKAP/PSD-95 is assembled in heterologous cells, with Homer and PSD-95 co-localizing in large clusters [(Berridge, 1998)]. Thus, Shank provides a molecular bridge that links the NMDA receptor complex with Homer and its associated proteins.

The Homer-Shank interaction also produces clustering of group 1 mGluRs [ (Satoh *et al*., 1990; Villa *et al*., 1992)]. Clustering molecules have previously been identified for a variety of receptors and ion channels (Selig *et al*., 1995), but Shank-Homer are the first clustering proteins for group 1 mGluR. It is notable that the mechanism of clustering involves a linkage of mGluRs with the previously defined NMDA receptor scaffold. Thus the Shank-Homer interaction could be relevant to synaptogenesis, by docking mGluRs to a preestablished "core" of NMDA receptors. In support of such a mechanism, functional NMDA receptors appear to precede the emergence of metabotropic receptors in the hippocampus and cerebellum (Xiao *et al*., 1998). Homer proteins, in association with Shank, could function to localize and cluster the mGluRs in proximity to NMDARs, and may contribute to the perisynaptic localization of group 1 metabotropic receptors (Lujan *et al*., 1997).

By linking NMDA and mGluR signaling pathways, the Shank-Homer interaction might also contribute to examples of glutamate receptor crosstalk for which physical proximity of molecules may be important, such as activation of phospholipase C (Beneken *et al*., submitted) or protein kinase C (Aniksztejn *et al*., 1991; Ben-Ari *et al*., 1992). Additionally, the Homer/Shank/GKAP/PSD-95 assembly may mediate physical association (and perhaps functional coupling) of the NMDAR with IP3R/RYR and intracellular Ca2+ stores. Consistent with such a functional interaction, recent studies indicate that NMDA receptor-dependent increases in spine Ca2+ may derive from intracellular stores by a mechanism of Ca2+ -induced Ca2+ release (CICR) [(Emptage *et al*., 1999) and reviewed by (Svoboda and Mainen, 1999)]. Both IP3R and ryanodine receptor channels possess CICR properties (Berridge, 1998), and are similarly localized in dendrites and spines of specific neuronal types (Satoh *et al.,* 1990; Villa *et al.,* 1992). The physical proximity of glutamate receptors with calcium pools may underlie synergistic effects of mGluRs on NMDA-dependent responses as reported in studies of LTP [(Bashir *et al*., 1993; Bortolotto *et al*., 1994) but see also ((Selig *et al*., 1995)], and is consistent with the reduction of LTP in group 1 mGluR mutant mice [(Prehoda *et al*., 1999) but see also (Conquet *et al*., 1994)].
The proposed model for Shank and Homer-dependent clustering requires that Homer be multivalent in order to cross-link Shank/GKAP/PSD95 to IP3R/RYRs and to mGluRs. This is achieved by multimerization of constitutively expressed CC-Homers (Xiao *et al.,* 1998). In this context, the monovalent Homer 1a IEG product appears to function to uncouple proteins that are linked via the constitutively expressed CC-Homer multimers, and thereby dynamically regulate the assembly of this postsynaptic network. Cocaine-induced increases in Homer 1a may thus modulate both mGluR and NMDA Ca2+ responses in spines.

### Homer EVH1 domain crystal structure.

To investigate the structural basis of interactions between EVH1 domains and ligands, we determined the high-resolution crystal structure of the EVH1 domain from rat Homer 1 (). Methods of protein purification and crystallization are described in our manuscript (Niebuhr *et al*., 1997; Tu *et al*., 1998). This structure revealed that the EVH1 module is homologous to both the plextrin homology (PH) domain and the phosphotyrosine binding (PTB) domain. (legend next page)

At the same time we were working to solve the structure of Homer 1 EVH1, Dr. Wendel Lim's group (at UCSF) solved the structure of the related EVH1 protein termed Mena (20% identical to Homer EVH1 domain) (Prehoda *et al*., 1999). Comparison of the Mena and Homer coordinates confirmed that these are related proteins despite the low degree of amino acid identity. The Mena crystal was solved with a 6mer peptide and identified a putative ligand binding surface. Both of our groups determined that co-crystals were not formed with longer synthetic peptides. One issue that concerned us regarding the putative ligand-binding site on Mena was that the affinity of the 6mer used for Mena was 100 fold less than that of a 10 mer (Prehoda *et al.,* 1999). The measured affinity of the 6mer was ~600 micromolar. Additionally, within the EVH1 family, Homer is one of the most divergent members (Prehoda *et al.,* 1999). One important difference between Mena and Homer EVH1 binding, is the orientation of the phenylalanine relative to the polyprolines. The optimal ligand for Mena is FPPPP while the consensus ligand for Homer is PPXXF. This may be important since the F is the single most critical side chain for the interaction when tested with larger peptides for both EVH1 domains (Niebuhr *et al*., 1997; Tu *et al*., 1998). In the Mena structure, the F side chain is not placed in a clear hydrophobic pocket (the ring appears to coordinate an arginine) and superposition of the ligand coordinates in Homer EVH1 is even less obviously stabilized.

To examine the predictive power of the Mena co-crystal for the ligand binding activity of Homer EVH1, we tested a series of missense mutations that targeted sites anticipated to contact the prolines of the ligand (PPXXF) sequence. Based on the homology of the EVH1 domain with the PTB domain, we also tested sites on Homer that would be critical if Homer mimicked the peptide binding surface of the PTB domain. This PTB ligand site is remote from the putative Mena EVH1 ligand site. Our mutation analysis also tested a series of mutants selected based on the homology between Homer and WASP. Genetic data from patients with Wiscott Aldrich syndrome defined a series of mutations in the EVH1 domain that map to sites that are distinct from both the PTB and the putative Mena ligand sites. Our selection of the mutational substitutions was based on the Homer EVH1 structure. Substituted amino acids were selected to be sufficiently conservative as not to disrupt the primary structure.

A total of 30 missense mutants of the Homer EVH1 domain were expressed in HEK293 cells and assayed for binding to either mGluR1a or Shank3 using GST pulldown assays. Surface-exposed mutations within the region homologous to the peptide binding site of PTB domains had no affect on peptide binding. Similarly, mutations based on the WASP data were also ineffective in disrupting binding. By contrast, certain of the mutants based the Mena ligand site did disrupt Homer EVH1 binding. Despite ambiguities involved with interpreting the effects of any single mutation, the nature and distribution of the effects of site-directed mutations in the Homer EVH1 domain on Homer-ligand interactions strongly implicate the Mena ligand region as mediating natural ligand binding by the Homer EVH1 domain.

One interesting finding from our analysis of mutant Homer EVH1 binding is that certain mutations disrupt binding specifically to mGluR1a, but not to Shank3 (and visa versa). One interpretation of this finding is that there are determinants of binding in addition to the core PPXXF motif. An important implication of this observation is that differences in critical determinants of Homer binding to its various targets may be exploited to develop pharmaceuticals that can selectively disrupt interactions with a particular target.

### I42 Interacts with Homer.

I42 (SEQ ID NOS:17 and 18) encodes a novel protein that was first identified in a Y2H screen of brain cDNA with the Homer EVH1 domain. Current information indicates that 142 functions with Homer at the excitatory synapse. We have generated 142 specific antisera and can demonstrate robust co-immunoprecipitation of I42 with Homer from brain. ImmunoEM analysis demonstrates that I42 is localized to the postsynaptic density. The predicted domain structure of I42 indicates that it shares certain properties with Shank including a N-terminal structural domain (a band 4.1 domain in I42), a single PDZ domain, and a central proline rich domain with a single Homer-ligand site. Additionally, there is a C-terminal type 1 PDZ ligand motif. We have identified a related sequence in the data base (KIAA sequence has several errors with frame shifts) suggesting that I42 may represent a gene family.

Current studies indicate a functional interaction of I42, Homer and mGluRs. We have performed a yeast 2-hybrid screen of the I42 PDZ domain and find it binds β-Pix (also termed Cool-1) (Allen *et al*., 1998). β-Pix is a guanine nucleotide exchange factor (GEF) for Rac1/CDC42. This interaction appears robust using GST pulldown assays and we have recently confirmed the interaction using co-immunoprecipitation assays from brain. Biochemical assays indicate that the PDZ domain of I42 binds its own C-terminus (may be intra or inter molecular). Based on these observations, I42 functions as a scaffold/cytoskeletal regulatory protein that responds to specific signals and may link between mGluR activation and Rac-dependent cytoskeletal remodeling. This biochemical association may play a role in mGluR trafficking or synaptic remodeling. An additional functional consequence of the Homer I42 interaction is indicated by the demonstrated association of β-Pix with p21 activated kinase (Pak) *(Tu et al*., 1999). Paks are a family of kinase that can signal both locally and more distally to the nucleus. A mutation of Pak3 has recently been linked to mental retardation (Tu *et al.,* 1998), confirming the importance of this regulated kinase to cognitive function. Accordingly, I42 appears to be part of a novel signaling pathway for the mGluRs that may be regulated by Homer proteins.

In preliminary studies, we observe that 142 co-immunoprecipitates with Homer from brain. Antibodies for I42 also co-immunoprecipitates mGluR1 from brain. In parallel studies, we observed the interaction between 142 and β-Pix (). These observations indicate the involvement of Homer in the function of I42/β-Pix and identify another signaling pathway that can be manipulated by agents that modulate Homer binding function.

*ii) Ultrastructural localization of I42*/*β-Pix*/*Pak at synapses:* We have performed preliminary immunoEM with I42 Ab and observes that it is associated with the PSD region. The methods and approach are identical to our studies of Shank (Naisbitt *et al*., 1999). This observation indicates that I42 is enriched at the excitatory synapse together with Homer, Shank and glutamate receptors.

### Ryanodine receptor (RYR) and Homer.

The RYR encodes a potential Homer binding site near the N-terminus (Bhat *et al*., 1999) and using GST pulldown assays we observe that GSTHomer binds to the relevant fragment of RYR1. Importantly, we have demonstrated that the RYR co-immunoprecipitates with Homer from detergent extracts of skeletal muscle. The interaction between RYR and Homer is understood to be consistent with the function of Homer proteins to regulate the coupling of membrane receptors with intracellular calcium pools. Glutamate mediates an inhibitory postsynaptic potential in dopamine neurons of the midbrain and this is mediated by mGluR1 release of intracellular Ca2+ from RYR sensitive CICR pools (Bhat *et al*., 1999). RYR have recently been implicated as an important source of NMDAR-induced calcium rise in the post synaptic spine (Emptage *et al*., 1999). Since Shank is part of the NMDA receptor signaling complex (Naisbitt *et al*., 1999) and binds Homer, it is compelling to evaluate the possible interaction between RYR and Homer.

### NMDA Receptor Type 2D (NR2D) and Homer.

Independent Y2H screens of adult cortex and cerebellum identified several clones of the NMDA receptor type 2D (NR2D). NR2D has not been as extensively studied as NR2B but is expressed in developing cerebellum and interneurons in the forebrain (Dunah *et al.,* 1998; Goebel and Poosch, 1999). NMDAR that include the NR2DR have slower channel properties (Cull-Candy *et al.,* 1998; Okabe *et al.,* 1998; Vicini *et al.,* 1998). The C-terminus of NR2D is highly proline rich consistent with our observation that Homer binds a specific proline rich sequence. Thus, in the case of NR2D, Homer proteins form a direct coupling to CICR pools. This direct coupling would contrast with NMDAR that include NR2B which appear to couple to Homer indirectly via PSD95-GKAP-Shank (Naisbitt *et al*., 1999). In both cases, modification of Homer crosslinking activity will alter the intracellular release of calcium due to glutamate receptor activation. Because of the differences in the binding properties of the EVH1 domain of Homer to its different targets, it is anticipated that agents that specifically disrupt the linkage of NR2B or NR2D can be developed.

### Mammalian InaD like molecule interaction with Homer.

We have identified two distinct novel members of a family of proteins with similarity to the recently reported human InaD (Philipp and Flockerzi, 1997) and *Drosophila* Discs Lost [DLT (Bhat *et al.,* 1999)]. These proteins encode 5 and 4 PDZ domains, respectively, and a proline rich region that is shared in all clones that is presumed to mediate interaction with Homer. DLT has been demonstrated to be essential for establishment of epithelial cell polarity and binds to the C-terminus of Neurexin IV [DLT (Bhat *et al*., 1999). We currently refer to our clones as rat InaD. In current studies, we observe that full length myc-tagged rInaD co-immunoprecipitates with Homer 2 from co-expressing HEK293 cells.

### I30 interaction with Homer.

I30 is a novel member of the family of abl binding proteins. Related proteins function as adaptor proteins that regulate cell growth [Ziemnicka-Kotula, 1998 #392; Biesova, 1997 #393] and are hypothesized. I30 encodes a SH3 domain and a Homer binding site. Accordingly, Homer is anticipated to link this protein to other Homer-interacting proteins including metabotropic glutamate receptors and IP3R. (See SEQ ID NOS: 15,16,19 and 20).

### Cdc42-associated tyrosine Kinase-2 (ACK-2) interaction with Homer

ACK-2 is a non-receptor tyrosine kinase that is regulated by the Rho-related GTP-binding protein Cdc42 [Yang, 1999 #391]. ACK-2 is activated by signals that result from cell adhesion, by for example activation of the integrin receptor. One cellular consequence of ACK-2 activation is down stream activation of c-Jun kinase. Our observation that ACK-2 interacts with Homer indicates that this signaling pathway can be linked to other membrane receptors by Homer, and identifies another signaling cascade that can be manipulated by agents that alter Homer crosslinking function.

### EXAMPLE 1

### Identification and Sequencing of Homer Family Members

Low stringency screens of phage cDNA libraries and EST Database searches were performed to identify Homer family members. cDNA libraries were screened using the rat Homer 1a coding region as a probe. Screens of mouse and rat brain cDNA libraries identified two isoforms of Homer-1 (Homer-lb and Homer-1c).

Searches of EST Databases identified a mouse EST sequence (ID#442801) which is about 73% homologous to a portion of 5' coding region of Homer-I cDNA sequence. Based on the EST used RT-PCR (Forward: 5'-GAC AGC AGA GCC AAC ACC GTG-3'; Reverse: 5'-GTC TGC AGC TCC ATC TCC CAC-3') to amplify the corresponding region from various mouse tissues. The PCR products (~330 bp) consisted of two different sequences, one of which contains an additional insertion of 33 bp. A mixture of these two cDNA fragments were used as probes to screen an adult mouse brain cDNA library. Out of 10⁶ clones screened, five clones hybridized well to the probe. Sequence analysis of these clones indicated that they are five partial cDNA clones representing two isoforms of a Homer-2 gene. These clones are identical to the isoforms amplified by RT-PCR. The 5' region of Homer-2 was cloned using 5'-RACE technique. Total RNA from E14.5 mouse brain was reverse-transcribed using the reverse primer described above. Another gene-specific primer (5'-CAC GGT GTT GGC TCT GCT GTC-3') was used in the amplification of the 5' region of Homer-2. The sequence authenticity of the 5' RACE clones was further confirmed by sequencing a partial mouse EST clone #441857.

A search of the EST Database allowed the identification of several human EST's corresponding to mouse and rat Homer-1b, Homer-2a and 2b cDNA sequences. RT-PCR was used to clone the human Homer-1b and Homer 2a and 2b coding regions. A 5' degenerate primer (5'-ATG GG(A/G/C) GA(A/G) CA(A/G) CC(T/C/G) AT(T/C) TTC-3') was designed based on an amino-terminal seven residue amino acid sequence (MGEQPIF) that is conserved among human EST clone #HCE003, mouse, rat, and *Drosophila* Homer homologue sequences. The 3' primers (5'-GAG GGT AGC CAG TTC AGC CTC-3') for human Homer-1 and human Homer-2 (5'-GTT GAT CTC ACT GCA TTG TTC-3') were made from the sequences of human EST clones #562862 and #HIBAB15 respectively. Human Homer-1b and Homer-2a and 2b were amplified from new born human frontal cortex. The sequences of human Homer 1b, Homer 2a and Homer 2b were derived from sequencing several PCR clones and EST clones and are shown in SEQ ID NO's:3, 7 and 9.

Human and mouse Homer-3 were identified by searching EST Database, using Homer-1 and Homer-2 sequences. Two full-length human Homer-3 clones were identified (Clone ID #284002 and #38753) and sequenced. Numerous mouse Homer-3 clones were found and one of them (Clone ID #1162828) contains an almost full-length coding region. Also identified were several *Drosophila* EST sequences exhibiting significant homology at the amino acid level to the N-terminal region of Homer family members. The sequence presented in SEQ ID NO:11 is derived from Clone #LD3829.

**Expression Constructs** Mammalian expression constructs were made by cloning cDNA into SalI and NotI sites of pRK5 (Genentech), so that the cDNA was fused in-frame to an N terminal c-Myc tag. GST-fusion constructs were made by cloning Homer cDNA into the SalI and NotI sites of pGEX4T-2 (Pharmacia). The full-length coding regions of mouse Homer-1b, rat Homer-1c, mouse Homer-2b and human Homer-3 were engineered with SalI and NotI sites at the 5' and 3' ends by PCR using high fidelity DNA polymerase *Pfu* (Stratagene). Various truncations of Homer-1b/c and Homer-2b coding regions were made by PCR with specific Primers containing SalI and NotI sites. All the PCR-based constructs were sequenced to confirm the sequences and in-frame fusion.

The sequence of Homer 1a was used to screen cDNA libraries prepared from rat and mouse brain for related gene products. Homer 1a sequence was also used to search GenBank data bases. Several related rodent and human sequences were identified.

cDNAs that are most closely related to Homer 1a appear to represent alternative splice forms. This inference is based on nucleotide sequence identity of their 5'UTRs and the first 175 amino acids of the open reading frames (ORF). The presumptive novel splice variants, termed Homer 1b and 1c, are completely divergent from Homer 1a after residue 175 of the ORF and they possess entirely distinct 3'UTRs. comparison at the point of sequence divergence indicates that Homer 1a encodes a unique eleven amino acid carboxy terminus of the ORF and about 5 kb 3' UTR region. The unique eleven amino acid carboxy-terminal sequence of Homer 1a does not possess a recognizable motif. In Homer 1b and 1c, an additional 168 and 180 amino acids are present that are predicted to possess coiled-coil (CC) secondary structure (Lupas , *Trends Biochem. Sci* 21:375 (1969)). While the 3'UTR sequence of Homer 1a includes multiple AUUUA repeats which are implicated in destabilizing mRNAs of intermediate early genes (IEG) (Shaw and Kamen, *Cell* 46:659 (1986)), the 3'UTR sequence of Homer 1b and 1c does not include this motif. The only difference between Homer 1b and 1c is the inclusion in Homer 1c of a twelve amino acid sequence insertion at residue 177, between the conserved amino-terminus and the CC domain. Thus, Homer 1b and 1c appear to be formed by a splicing event that substitutes a relatively long and unique carboxy-terminus of the ORF and shorter 3'UTR sequence that lacks the characteristic IEG motif. Multiple independent isolates of rat and mouse Homer 1b and 1c were identified and sequenced to confirm their natural expression in brain.

Further searches identified cDNA sequences that appear to represent two additional Homer genes, termed Homer 2 and Homer 3. The sequences of two splice forms of Homer 2 and one Homer 3 sequence is presented (See Figures section). The predicted size of the protein products and general domain structure are similar to Homer 1b and 1c. Like Homer 1b and 1c, each of the Homer 2 and Homer 3 proteins contain about 120 amino acids at the amino-terminal that is highly similar to the amino-terminal domain of Homer 1a. The degree of amino acid identity in these regions is about 88% between Homer 1 and Homer 2 and about 86% between Homer 1 and Homer 3. Many of the amino acid differences are conservative.

In contrast to the high degree of conservation in amino-terminal region, the carboxy-terminal regions of Homer 2 and 3 are only about 22% identical to Homer 1b, but like Homer 1b and 1c are predicted to possess a CC secondary structure. The CC domains of all Homer family members exhibit significant homology (about 40-45% amino acid similarity) to the CC regions of myosin heavy chain (Strehler *et al., J Mol Biol* 190:291 (1986)), kinesin heavy chain (Yang *et al., Cell* 56:879 (1989)) and dynactin (Gill *et al., J Cell Biol* 115:1639 (1991)). The distinct splice forms of Homer 2, termed Homer 2a and Homer 2b, are differentiated by an eleven amino acid insertion at residue 131 in Homer 2b. Human Homer 1, 2 and 3 are mapped to chromosomes 5, 15 and 19, respectively by the Human Genome Project.

*Drosophila* Homer possess the basic domain structure of mammalian Homers. The amino-terminus is highly homologous to that of mammalian Homer and the carboxy terminus is predicted to form a CC secondary structure.

### EXAMPLE 2

### Generation and Characterization of Homer Antisera

Rabbit polyclonal antibodies were generated against synthetic peptides derived from the unique carboxy termini of Homer 1b/c, Homer 2a/b and Homer 3. Synthetic carboxy-terminal peptides of Homer 1, 2 or 3 were conjugated to thyroglobulin with glutaraldehyde and used to immunize rabbits according to a previously published protocol (Martin *et al., Neuron,* 9:259 1992). Peptide sequences used are contained in Homer-1b and Ic: IFELTELRDNLAKLLECS; Homer-2a and 2b: GKIDDLHDFRRGLSKLGTDN; and Homer-3: RLFELSELREGLARLAEAA. Detergent (2% SDS) extracts from rat cortex, hippocampus, and cerebellum were separated on 8% SDS-PAGE gels and transferred to nitrocellulose membranes. Blots was probed with polyclonal anti-Homer sera. Specificity was tested by incubating the antiserum with 10 µg/ml of relevant peptide at room temperature for 10 m prior to use. Rabbit polyclonal antiserum was also generated against the full length GST-Homer 1a fusion protein, as described previously (Brakeman, *et al., Cell* 87:227 1997). This antiserum recognizes all Homer 1 isoforms.

Unpurified antibodies were tested for their sensitivity and specificity in detecting heterologously expressed, full length Homer proteins with amino-terminal c-myc tags. Each Homer protein was selectively detected on Western blot by the appropriate Homer antibody in soluble extracts of transfected HEK293 cells. The myc-tagged Homer proteins migrated with an apparent molecular mass of 50 kDa. There was no cross reactivity between antibodies for one Homer form and other family members.

### EXAMPLE 3

### In Vitro Interaction of Homer Proteins with Cell-Surface mGlu Receptors

To examine the interaction of Homer proteins with mGluR1 and mGluR5, HEK293 cells were transiently transfected (using calcium phosphate) with full length mGluR1α and mGluR5 constructs in pRK5 (Brakeman *et al.,* 1997). Cell lysates were made 24-48 h post-transfection. GST fusion proteins bound to glutathione agarose were prepared of Homer 1a, Homer 1c, Homer 2b, Homer 3 and two amino terminal fragments of Homer 2 according to the following procedure. GST fusion constructs were prepared by polymerase chain reaction with specific primers that included SalI and NotI sequences and subcloned into pGEX4T-2 vector (Pharmacia Biotech, Uppsala, Sweden). Constructs were confirmed by sequencing. GST-fusion proteins were expressed in BL21 bacterial strains. Bacteria were harvested and lysed in PBS, 1% Triton X100, 2mM phenylmethylsulfonyl fluoride (PMSF) and pelleted at 13,000 rpm (Sorvall SS-34) at 4°C for 5 m Proteins were purified by incubating 1 ml bed volume glutathione-sepharose (GST) beads (Sigma USA) with bacterial supernatant at 4°C for 10 m, washing twice with PBS and PBS plus 1% Triton X-100. Protein was eluted with 10 mM glutathione and dialyzed against PBS at 4°C. Protein concentrations were measured by BCA (Pierce, Illinois). Cell lysates of the transfected cells were incubated with equivalent amounts of various Homer-GST fusion proteins at 4 °C for 2 h, washed with PBS and 1% Triton X-100. Proteins were eluted in 2% SDS sample buffer and separated on 8% or 2.5% SDS-PAGE gels and probed with appropriate antibody.

It has been previously demonstrated that the amino-terminal 131 amino acids of Homer 1a is sufficient to bind group I metabotropic glutamate receptors (Brakeman *et al., Nature* 386: 284 (1997)). In view of the high degree of sequence conservation in this region of Homer family members, the possibility that they would also bind group I receptors was examined. GST fusion proteins were prepared of Homer 1a, Homer 1c, Homer 2b Homer 3 and two amino-terminal fragments of Homer 2. The fusion proteins were bound to glutathione agarose and assayed for binding to full length mGluR5 or full length mGluR1a expressed in HEK293 cells. These studies show that mGluR5 bound GST Homer 1a. mGluR5 also bound to all full length Homer constructs and to a Homer 2 amino-terminal fragment of about 141 residues but not to GST alone. The relative binding in the three assays were comparable for each of the three Homer types. A Homer 2 deletion mutant that includes only the amino-terminal 92 residues did not bind mGluR5. Similar binding of Homer proteins to mGluR1 was also observed.

### EXAMPLE 4

### In Vivo Interaction of Homer Proteins with Cell-Surface mGlu Receptors

To examine if Homer proteins are naturally associated with group I metabotropic receptors in the brain, immunoprecipitation studies were performed. Rat or mouse brain tissues were sonicated (3 x 10 s) in PBS (~200 mg/ml wet weight) containing 1% Triton-X100 with protease inhibitors and centrifuged for 10 m at 15,000g. Three µl of antiserum directed against Homer 1b, Homer 1c, Homer 2a, Homer 2b or Homer 3 was added to 60 µl of tissue extract and incubated for 1-1/2 h at 4°C and then washed three times with PBS/Triton. Preimmune and peptide-blocked antisera were used as negative controls. Binding in tissue samples was analyzed by gel electrophoresis and western blot analysis. Proteins were eluted in 2% SDS loading buffer. mGluR1α monoclonal antibody was obtained from PharMingen (San Diego CA). Rabbit polyclonal mGluR5 antibody was a gift from Dr. Richard Huganir, Johns Hopkins School of Medicine.

Homer family members are naturally associated with group I metabotropic receptors in brain. This analysis was performed using cerebellum since all three Homer family members are expressed in this tissue. Detergent extracts of whole adult rat cerebellum were incubated with antibodies to Homer 1b/c. Homer 2a/b or Homer 3 and immunopreciptates were blotted with a mouse monoclonal antibody to mGluR1α. mGluRl α co-immunoprecipitates with each of the antisera directed against Homer proteins. The predominate band after electrophoreses corresponded to the monomer form of mGluR1α (about 150 kDA) and other bands corresponding to multimers of mGluR1α are also observed.

### EXAMPLE 5

### In Vitro Interaction of Homer Proteins with Intracellular Inositol Trisphosphate Receptors

To demonstrate that Homer proteins interact in vivo with inositol trisphosphate receptors immunoprecipitation studies were performed using brain tissue. Rats or mice were sacrificed by decapitation and the cerebella were dissected immediately. Cerebella were sonicated in TE buffer (50 mM Tris, 1 mM EDTA, pH 7.4) containing 1% CHAPS and protease inhibitor cocktail (~100 mg wet weight/ml). The homogenate was centrifuged at 90,000 rpm, 20 m, 4°C in a TLA 100.3 rotor. 100 µl of the cerebellar extract was used for each immunoprecipitation assay with the following antibodies: 3 µl of crude Homer 1, Homer 2 or Homer 3 antibodies (Xiao *et al*., in press); 20 µg of affinity purified inositol trisphosphate antibody (gift from Alan Sharp). Antibodies and extract were incubated for 30m at 4°C, then 60 µl of 1:1 protein A or protein G (for goat antibody) sepharose slurry was added. The antibody/extract/beads were incubated for an additional 90 m at 4°C. After washing 3x10 m in TE-CHAPS buffer, the proteins were eluted from the beads with 30 µl of 4% SDS loading buffer and analyzed by SDS-PAGE and immunoblot.

Results from these studies showed that the inositol trisphosphate receptor specifically co-precipitates with antisera directed against Homer 1, Homer 2 and Homer 3.

### EXAMPLE 6

### Calcium Mobilization is Decreased by Transient Expression of Homer Protein Without a Coiled-Coil Domain

To demonstrate that Homer cross-links metabotropic glutamate receptors and inositol trisphosphate receptors to provide or enhance a functional signaling complex, calcium mobilization was examined in cells transient expressing truncated forms of Homer protein. The truncated Homer protein used lacks the coiled-coil domain and is unable to form a bridge linking the mGluR at the cell surface with intracellular inositol trisphosphate receptors. The truncated form of Homer protein resembled Homer 1a with the exception of 11 residues at the carboxy-terminal. This form of Homer results in enhanced expression of Homer protein as compared with transfection of Homer 1a in heterologous cells. The Homer protein was introduced into Purkinje cells in primary cerebellar cultures and glutamate induced effects on calcium mobilization was measured.

Embryonic mouse cerebellar cultures were prepared and maintained according to the method of Schilling *et al*. (Schilling *et al., Neuron* 7:891 1991). At 4-5 DIV, cultures were transfected with plasmids coding for E-GFP (Clontech) and either full-length Homer 1b or an IEG form of Homer 1. The IEG form of Homer 1 was a 186 amino acid amino-terminal fragment of Homer 1b. Plasmids were purified by cesium banding. Three combinations of the plasmids were transfected. Group I (control), 20µg of E-GFP and 40µg of pRK5 vector; group II, 20µg of E-GFP and 40µg of pRK5 Homer 1 IEG; group III: 20µg of E-GFP and 40µg of pRK5 Homer 1b. Plasmid DNA was mixed with gold particles (0.6 micron), and coated onto plastic tubing. DNA was then ballistically transfected into cells according to the manufacturer's protocol (Helios Gene Gun System, BIO-RAD). After transfection, cultures were returned to the incubator and maintained for an additional 2 days for a total of 7-8 DIV at the time of use for imaging experiments.

Patch electrodes were attached to the somata of GFP-expressing Purkinje cells and a holding potential of -60 mV was applied. Micropressure electrodes (1µm tip diameter) were filled with quisqualate (100 µm in external saline) and were positioned ~20 µm away from large-caliber dendrites. Test pulses were delivered using positive pressure (6 psi, 1 sec). Cells were bathed in a solution that contained (in mM) NaCl (140), KCl (5), EGTA (0.2), MgCl₂(0.8), HEPES (10), glucose (10), tetrodotoxin (0.005), and picrotoxin (0.1), adjusted to pH 7.35 with NaOH, which flowed at a rate of 0.5 ml/m The recording electrode contained CsCl (135), HEPES (10), fura-2 K₅ salt (0.2), and Na₂-ATP (4), adjusted to pH 7.35 with C_{S}OH. Patch electrodes yielded a resistance of 3-5 MΩ when measured with the internal and external salines described above.

Fura-2 ratio imaging of intracellular free Ca²+, was accomplished by measuring the background corrected fluorescence ratio at 340 and 380 nm excitation using a cooled CCD camera system, as previously described (Linden *et al., JNeurosci* 15:5098 1995). Exposure times were 200 msec per single wavelength image. Experiments were conducted at room temperature. Enhanced GFP is weakly excited by illumination in the 380-400 nm spectrum. Based upon the bandpass characteristics of our 340HT15 and 380HT10 excitation filters and the absorption spectrum of enhanced GFP (Clontech), we estimate that <1% of the signal at 340 nm excitation and <5% of the signal at 380 nm excitation is contributed by GFP, even in those cells where the fura/GFP loading ratio is smallest. This could lead to a small (<5%) systemic underestimation of free calcium concentration that should distribute randomly across experimental groups.

Calcium mobilization in the absence of influx was measure by ratio imaging fura-2 in Purkinje cells bathed in Ca⁺²-free external saline and stimulated with a micropressure pulse of quisqualate, a metabotropic glutamate receptor agonist (Linden, *Neuron* 17:483 1996). The resultant Ca⁺² transient is triggered by an mGluR and inositol trisphosphate pathway since it is completely blocked by either an mGluR antagonist ((+)-MCPG, 500µM in the bath) or a novel specific inositol trisphosphate receptor-associated ion channel blocker, xestospongin C (1 µM in the internal saline). Purkinje cells transfected with a truncated form of Homer showed mGluR-evoked Ca⁺² responses with a decreased amplitude (170 ± 9 nM, mean ± SEM, n=30 cells) and an increased latency (10.5 ± 1.8 sec) as compared with cells transfected with Homer 1b (244 ± 17 nM, 4.2 ± 0.9 sec, n=23) or an empty vector control (239 ± 19 nM, 4.5 ± 1.1 sec, n=15). The decay phase of the Ca⁺² response appeared somewhat slower in neurons transfected with the truncated form. While the total Ca⁺² flux appeared similar in cells transfected with truncated and complete Homer proteins and in empty vector controls, the measurement could not be made because the tail of the Ca⁺² response was abbreviated due to the constraints of the image buffer capacity.

### EXAMPLE 7

### Determination of the Crystal Structure of Homer Protein

The crystal structure of Homer protein and a Homer protein binding site were determined. Results of these experiments are presented in Table

### (a) Protein Expression and Purification

Residues 1-120 of rat Homer 1a were expressed in *Escherichia coli* BL21 cells as a C-terminal fusion to glutathione-S-transferase (GST-1aEVH) as previously described (Tu *et al., Neuron* 21:717 1998). Selenomethionine-substituted (SeMet) GST-1aEVH was prepared by expression in the methionine auxotrophic strain B834 (DE3) (Novagen). 5 mL of an overnight culture grown at 37°C in LB media supplemented with 100 µg/mL ampicillin (Sigma) was added to 4L M9 minimal media (Gibco BRL) supplemented with 100 µg/mL ampicillin, 0.05 mg/mL alanine, aspartic acid, glutamic acid, phenylalanine, glycine, histidine, isoleucine, lysine, asparagine, proline, glutamine, arginine, serine, threonine, valine, tryptophan, tyrosine, L-selenomethione , 1 µ/mL thiamine (Sigma), 2mM MgSO₄, 1% glucose, 100 µM CaCl₂. Cells were grown to an A₆₀₀ of 0.5 at which time IPTG (Calbiochem) was added to a final concentration of 0.2 mM. Cells were grown for an additional 3 hours, harvested by centrifugation, and resuspended in 1x PBS/1% Triton. Pepstatin A and leupeptin (Boehringer-Mannheim) were added to a final concentration of 1 µg/mL, and PMSF (Life Technologies) was added to 0.5 mM. Cells were lysed by sonication and centrifuged at 13,000 rpm in an SS-34 rotor to pellet cell debris. The cleared lysate was added to a 5 mL glutathione-agarose (Sigma) column. The column was washed in succession with twenty column volumes of 1x PBS/1% Triton, twenty column volumes of 1x PBS, and ten column volumes of cleavage buffer (50 mM Tris [7.4], 150 mM NaCl, 2.5 mM CaCl₂,. 50 mM β-mercaptoethanol). All buffers were degassed. A 50% slurry of glutathione-agarose beads loaded with fusion protein was incubated with 20 U of biotinylated Thrombin (Novagen) for 16 h at room temperature. The released cleavage product (1a-EVH) was collected, and the biotinylated Thrombin was removed with streptavidin-agarose beads (Novagen). la-EVH was further purified by cation-exchange chromatography using a Resource S column (Amersham-Pharmacia).

### (b) Crystallization and Data Collection

Crystals of native and SeMet protein were grown in hanging drops by the method of vapor diffusion (Wlodawer *et al., Proc Natl Acad Sci USA* 72:777 1975). 1µl of a 9 mg/mL native or SeMet protein solution was mixed with a 1:1 dilution of reservoir buffer (30% PEG 3350, 87 mM MgSO₄, 50 mM HEPES, pH 7.3) with distilled water and equilibrated over I mL of reservoir buffer. All crystallization trials for the SeMet protein were set up under anaerobic conditions to minimize potential problems due to oxidation. Two different crystal forms were observed for both the native and the SeMet protein. Crystals in the orthorhombic space group P2₁2₁2₁, (unit cell dimensions a = 33.79 Å, b = 51.40 Å, c = 66.30 Å) typically grew to a size of 0.5 mm x 0.03 mm x 0.03 mm. Crystals in the trigonal space group P3₂21 (unit cell dimensions a = b = 49.94 Å, c = 80.91 Å) grew to a size of 0.4 mm x 0.1 mm x 0.1 mm. All data used for phasing and refinement were collected from a single trigonal SeMet crystal soaked in mother liquor plus 10% (v/v) ethylene glycol for approximately three minutes prior to flash freezing in a gaseous nitrogen stream at -180°C. X-ray diffraction data suitable for multiwavelength anomalous dispersion (MAD) phasing were collected at four wavelengths at or near the Se absorption edge. These data were collected at beamline X4A of the National Synchrotron Light Source at Brookhaven National Laboratory using an R-AXIS IV image plate detector. Nonoverlapping oscillations (2°) at φ and φ + 180° were measured over a 90° rotation of the crystal, interleaving the four wavelengths. All data were processed and scaled using the DENZO/SCALEPACK programs (Otwinowski and Minor, *Meth Enzymol* 276:307 1997). Data collection statistics are shown in Table 1.

### (c) Structure Solution and Refinement

The expected two selenium sites were determined and refined using the program SOLVE (Terwilliger and Berendzen, *Acta Crystallogr* D53:5711997; Terwilliger and Eisenberg, *Acta Crystallogr* A39:813 1983) and initial Se scattering factors from (Hall *et al., Cell* 91:85 1997). Values for the refined Se scattering factors as determined by SOLVE are shown in Table 1. The electron density maps calculated with the experimental MAD phases as determined by SOLVE were improved by solvent flattening and histogram matching using DM (Collaborative Computational Project, 1994). An initial model of residues 1-105 was built into 1.8 Å experimental electron density maps using the program O (Jones *et al., Acta Crystallogr* A47:110 1991). After one round of simulated annealing with bulk solvent correction and positional and B-factor refmement using CNS (Brünger *et al., Acta Crystallogr* D54:905 1998), residues 106-111 were built into 2F₀-F_{c} maps. The model was refined against the maximum-likelihood target (Pannu and Read, *Acta Crystallogr* A52:659 1996) using data to 1.7 Å Bragg spacing collected at 0.9879 Å. Eight rounds of model building and water addition alternated with B-factor and positional refinement yielded the current model, which includes residues 1-111 and 88 water molecules. No electron density was observed for residues 112-120. This model has a crystallographic R value of 25.3% and a free R value of 28.4%. The solvent content is ca. 40.6%, with one molecule per asymmetric unit. Fractional solvent accessibility for each residue was calculated in X-PLOR (Brünger, X-PLOR, Version 3.1: A system for X-ray crystallography and NMR (New Haven, CT: Yale Univ. 1992).

### (d) Determination of Homer Site by Site Directed Mutagenesis

Point mutants of N-terminally myc-tagged, full-length Homer 1b and 1c and Homer 1 EVH1 were made using the QuikChange TM Site-Directed Mutagenesis Kit (Stratagene). Expression constructs were transiently transfected into HEK293 cells using calcium phosphate methods. About 24-48 h post-transfection, cell lysates were prepared in Ix PBS/1% Triton X-100 (Sigma) and protease inhibitors. GST pull-down assays were performed by mixing 100 µl of cell lysate with GST-mGluR5 or GST-Shank3 (residues 1143-1408) (Tu *et al*., in press) bound to glutathione-agarose, incubating at 4°C for 2 h, and washing with 1x PBS and 1x PBS/1% Triton X-100. Bound products were eluted with 100 µl 2x SDS loading buffer and detected by SDS-PAGE and immunoblot using anti-myc antibody 9EI0 (Invitrogen) and ECL reagents (Amersham).

### EXAMPLE 8

### Homer Expression is Upregulated in Certain Brain Regions in Response to Electrically Induced Seizures

Rat Homer 1a was cloned based on its rapid upregulation in hippocampal granule cell neurons following electrically-induced seizure (MECS; see Brakeman *et al*., *Nature* 386:284 1997) The expression of other members of the Homer family was examined in the brain following seizure. Radio-labeled riboprobes were prepared using unique sequences for Homer 1a, Homer 1b, Homer 1c, Homer 2a, Homer 2b and Homer 3. Probes used did not distinguish between the splice forms of Homer 1b and 1c or Homer 2 and 2b.

### (a) In Situ Hybridization

Anti-sense and sense cRNA probes were generated from each mouse Homer plasmid by in vitro transcription in the presence of [³⁵S]UTP, as previously described (Lyford *et al., Neuron* 14:433 1995). Probe for Homer-1a (Xiao, 1998; GenBank #) was derived from nucleotides 1342 to 2140, for Homer 1b/c (Xiao, 1998; GenBank # ) from nucleotides 785 to 1396, for Homer-2a/b (Xiao, 1998; GenBank #) from nucleotides 486 to 1561, and for Homer-3 (GenBank #) from nucleotides 371 to 2123. Probe (about 10⁶ cpm in 75 µl hybridization buffer) was applied to each slide. Coverslipped slides were then incubated in humidified chambers overnight at 56°C. Following completion of wash steps, slides were air dried and exposed to Kodak Biomax MR film for 2-3 days.

The anatomic distribution in unstimulated animals reveals that expression of Homer 1a is similar to the expression of Homer 1b and Homer 1c. High levels of expression of Homer 1a are observed in the hippocampus, striatum and cortex. In the cortex, there is laminar expression with the highest levels in the superficial and deep layers. Expression of Homer 2a and 2b is enriched in the thalamus, olfactory bulb and principle neurons of the hippocampus in contrast to the cortex where low levels of expression of Homer 2a and 2b are observed. Homer 3 is expressed primarily in the cerebellum and hippocampus.

In situ hybridization studies demonstrate the dramatic induction of Homer 1a in response to MECS. In the hippocampus, induction of expression is estimated to be greater than 20-fold compared to hippocampus from unstimulated animals. MECS induced an increase in Homer 1b and 1c expression of about 1.5 fold as determined by blot analysis. Expression of Homer 2 and Homer 3 is not altered in response to MECS.

### EXAMPLE 9

### Formation of Multimeric Complexes of Homer Proteins.

The CC secondary structure is implicated in protein-protein interactions (Lupas, 1996 *supra*). Therefore, the possibility that this domain might confer the ability to form homo- or hetero-multimers between Homer family members was examined. For examining the coiled-coil interaction of Homer family members, myc-tagged Homer-1c and Homer-2b were transfected into HEK293 cells and cell extracts were made 2-3 days post-transfection. Cell lysates were treated as described above.

First, the ability of full length, bacterially-expressed GST fusion proteins of Homer to bind full length myc-tagged Homer proteins expressed in HEK293 cells was tested. myc Homer 1c bound Homer 1b, Homer 2b, Homer 3, Homer 1b and Homer 2b carboxy-terminal CC domain, but not Homer 1a or Homer 2-amino-terminus. This is consistent with the notion that the CC domain is important in the interaction, since Homer 1a and Homer 2-amino-terminus doe not encode the CC domain. To test the specificity of the CC domain interactions, GST fusions of dynein IC-1a and dynein IC-2c were generated. The CC domains of these proteins show modest sequence to Homer family CC domains and bind to the CC domain of dynactin (Gill, 1991 *supra*). None of the myc-tagged Homer family members bound to either dynein IC-1a or dynein IC-2c.

To determine whether Homer family members naturally form multimers in brain, immunoprecipitates of cerebellum were examined. Extracts immunoprecipitated with Homer 1b/c antibody contained Homer 3, while extracts immunoprecipitated with Homer 3 contained Homer 1b/c. While it is possible that these co-immunoprecipitated Homer family members are associated by means other than their CC domains, the fact the amino-terminus of Homer is monovalent and cannot form extended concatomers supports a model of multimerization mediated by the CC domains. Homer 2 was not detected as a multimer with either Homer 1 or Homer 3 in these immunoprecipitation experiments.

### EXAMPLE 10

### Homer Family Proteins are Enriched at Brain Synaptic Fractions and are Expressed in Certain Peripheral Tissues

The distribution and localization of Homer family proteins was examined at the using immunochemical methods. Tissue extracts were assayed using immunoblot analysis and tissue localization was examined using immunohistochemistry at the light and ultrastructural levels.

**(a) Immunoblot Analysis** Immunoblot staining of SDS (2%) extracts of various brain regions were examined to assess the distribution of Homer proteins in the brain. Homer 1b/c antibody detected a single band of about 47 kDa in cortex, hippocampus and cerebellum. These regions have similar levels of expression. The Homer 2a/b antibody detected a single major band in each of cortex, hippocampus and cerebellum. Less intense, higher apparent molecular mass bands were detected at about 60 and about 80 kDa. Homer 3 immunoblots showed low level expression in cortex and hippocampus and intense staining of a single band in cerebellum (47 kDa). Immmunostaining was completely blocked by preincubating the antibody with 10µg/ml of the relevant peptide antigen.

### (b) Immunohistochemistry

For light microscopy, rats were deeply anesthetized with sevoflurane and perfused through the aorta with 250 ml of saline followed by 400 cc each of 4% paraformaldehyde in 0.1 % phosphate buffer (pH 6.5) and 4% paraformaldehyde in 0.1% phosphate buffer (pH 8.5). The rat was allowed to postfix for 1 hr. at room temperature and then prefused with 15% sucrose in 0.1 % phosphate buffer (pH 7.4). The brain was removed and sectioned at 40 µm on a freezing sliding block microtome and collected in PBS. Tissue was stained with an immunoperoxidase technique, as follows. Brain sections were incubated in PBS containing 0.3% H₂O₂ and 0.25% Triton X-100 for 30 m and then washed 3x5 m in PBS. Sections were incubated in a buffer "PGT" containing 3% normal goat serum (Colorado Serum Co.) and 0.25% Triton X-100 in PBS for 1 hr. and then transferred to the primary antiserum diluted 1:750 in the same PGT buffer. Sections were gently shaken for 48 h at 4°C, washed 4x5 m in PBS and then incubated for 1 hr. at room temperature in a goat anti-rabbit IgG conjugated to horseradish peroxidase (Biosource International) diluted 1:100 in PGT. Sections were washed 4x5 m in PBS and incubated for 6 m at room temperature in 0.05% diaminobenzidine dihydrochloride (DAB:Sigma) and 0.01 % H₂O₂ in 0.1 M phosphate buffer. Sections were washed in PBS, mounted onto gelatin chrome-alum subbed slides, dehydrated in a series of graded ethanol, cleared in xylene and coverslipped with DPX (BDH Limited).

Immunohistochemistry was performed to determine the cellular localization of Homer 1b/c and Homer 2a/b and Homer 3 in rat brain. Light microscopic examinations indicated that all three Homer proteins are enriched in Purkinje neurons. Immunoreactivity is present in the cytoplasmic region of the soma and extends prominently into the dendritic arbor. The nucleus is not stained. Little or no staining is detected in the contiguous granule cell layer. A similar light microscopic pattern of cellular localization was detected for Homer 3. Homer 2 immunostaining in cerebellum also showed staining in Purkinje neurons, but appeared technically less differentiated.

### (c) Electron Microscopy

For EM, a postembedding immunogold method as described previously (Wang, *et al., JNeurosci* 18:1148 1998) was used and modified from the method of (Matsubara, *et al., J Neurosci* 16:4457 1996). Briefly, male Sprague-Dawley rats were perfused with 4% paraformaldehyde plus 0.5% glutaraldehyde in 0.1 M phosphate buffer. Two hundred micrometer parasagittal sections of the rostral cerebellum (folia III-V) were cryoprotected in 30% glycerol and frozen in liquid propane in a Leica EM CPC. Frozen sections were immersed in 1.5% uranyl acetate in methanol at -90°C in a Leica AFS freeze-substitution instrument, infiltrated with Lowicryl HM 20 resin at -45°C, and polymerized with UV light. Thin sections were incubated in 0.1% sodium borohydride plus 50 mM glycine in Tris-buffered saline/0.1% Triton X-100 (TBST), followed by 10% normal goat serum (NGS) in TBST, primary antibody in 1% NGS/TBST, 10 nm immunogold (Amersham) in 1% NGS/TBST plus 0.5% polyethylene glycol, and finally staining in uranyl acetate and lead citrate. Primary antibodies were used at dilutions of 1:500 for Homer 1b and 1:100-1:400 for Homer 3.

Immunogold EM of Purkinje neurons of the cerebellum was performed to determine whether Homer family proteins are associated with synaptic structures. Homer 1b/c showed striking localization to the region of the postsynapic spine. Gold particles are densely concentrated in the region of the postsynaptic density (PSD). A very similar distribution is noted for Homer 3 immunoreactivity. It is noted that rather than being concentrated directed over the PSD or the contiguous plasma membrane, the majority of the gold particles appear to be present in the cytoplasm immediately subjacent to these structures.

**Peripheral Tissues** Homer proteins are expressed in peripheral tissues. In detergent extracts of heart and kidney, a single band at 47 kDa immunoreactive to Homer 1b and 1c is detected. In extracts of liver, a complex of three bands ranging from about 44 to 47 kDa is detected. In heart, liver, skeletal muscle and intestine, bands immunoreactive to Homer 2a and 2b are detected. Homer 3 immunoreactive bands are detected in extracts of lung and thymus.

**Subcellular Distribution** To examine the subcellular distribution of Homer proteins, a biochemical fractionation of rat forebrain was performed and fractions were analyzed by Western blotting with Homer antibodies. Fractions were blotted for mGluR5, BIP and synaptophysin to monitor anticipated enrichment of fractions. Homer 1b/c, 2a/b and 3 were present in the crude nuclear pellet (P1), the medium spin crude synaptosomal pellet (P2), and the high speed microsomal pellet (P3). BIP is a 78 kDa ER resident protein (Munro and Pelham, *Cell* 48:899 (1987)). and was enriched in both the P3 and the S3 fractions. While Homer 1b/c and Homer 3 were not abundant in the soluble (S3) fraction, Homer 2 was enriched in the S3 fraction. The P2 fraction was subfractionated after hypotonic lysis. The 25,000 x g pellet (LP1), which is enriched in PSDs (Huttner *et al., J Cell Biol* 96:1374 (1983)), showed enriched presence of mGluR5. The high speed pellet (165,000 x g; LP2) showed the anticipated enrichment in the synaptic vesicle protein synaptophysin (P38). Each of the Homer proteins was enriched in the LP1 fraction relative to LP2. the final soluble fraction (LS2) was uniquely enriched in Homer 2.

### EXAMPLE 11

### Transgenic Mouse Model Demonstrates that Expression of Homer 1a Selectively Blocks Binding of Homer 1b/c to mGluR5 In Vivo

N-terminal myc-tagged full-length Homer 1a ORF was cloned into the expression vector pT2 (Gordon, *et al., Cell* 50:445 1987; Aigner, *et al*., *Cell* 83:269 1995). Transgenic mice were generated at the University of Alabama Transgenic Facility. Expression of the transgene protein was assayed by western blot with rabbit polyclonal antisera that recognizes all Homer 1 isoforms (pan-Homer 1 antibody) and myc antibody.

Homer 1a is unique within the family of Homer related proteins in that it is dynamically regulated and it lacks the CC domain. Accordingly, it was hypothesized that the IEG would bind to group 1 metabotropic receptors and disrupt the formation of multivalent complexes of Homer and mGluR. To examine this hypothesis, a transgenic mouse was generated that expresses Homer 1a under the control of a modified Thy-1 promoter (Gordon *et al*., 1987, *supra*), which drives neuron-specific expression in postnatal brain (Aigner *et al*., 1995, *supra*). Transgenic mice expressed Homer 1a at high levels in cortex, hippocampus, cerebellum and thalamus/brainstem relative to levels in wild type litter mate controls. The pattern of Homer 1a transgene expression is consistent with the previously reported activity of this promoter (Gordon *et al,* 1987, *supra).* As expected, antibodies for both Homer 1b/c and Homer 2a/b co-immunoprecipitated mGluR5 from detergent extracts of wild type forebrain. By contrast, Homer 1b/c antibody did not co-immunoprecipitate mGluR5 from transgenic mice. The effect of Homer 1a transgene expression was selective in that it did not disrupt the co-immunoprecipitation of Homer 3 with Homer 1b/c. The latter observation is consistent with the notion that the Homer 1b/c-Homer 3 interaction is mediated by the CC domain and is predicted not to be altered by Homer 1a expression. Homer 1a was not part of the complex co-immunoprecipitated with Homer 1b/c, consistent with the notion that the CC is necessary for association with the complex. The effect of the Homer 1a transgene in blocking the in vivo coupling of mGluR5 and Homer 1b/c was additionally selective in that Homer 2 antibody co-immunoprecipitated mGluR5 similarly from extracts of wild type and transgenic mice. Thus Homer 1a appears to selectively disrupt the interaction of Homer 1b/c with mGluR5 but not Homer 2 with mGluR5. Homer 3 is less highly expressed in forebrain than Homer 1b/c or Homer 2a/b and co-immunoprecipitates of mGluR5 with Homer 3 antibody were less clean. Accordingly, it could not be determined in these experiments whether Homer 1a also competes with Homer 3. Identical results were obtained in tow independent mouse lines that express Homer 1a transgene. The Homer 1a expressing transgenic mice have not been behaviorally characterized but appear normal in size and gross motor activity.

### EXAMPLE 12

### Yeast Two-Hybrid Screen

To examine the physiological functions of Homer, a novel family of proteins was identified based on its ability to interact with Homer family proteins in a yeast two-hybrid screen of a brain cDNA library. Homer 1a was subcloned into pPC97 (Chevray and Nathans, *Proc. Natl. Acad. Sci. U.S.A.,* 89:5789 (1992)) and used to screen a random primed cDNA library prepared from seizure-stimulated rat hippocampus and cortex cloned in pPC86 (Chevray and Nathans, 1992, *id*.) as described previously (Brakeman *et al., Nature, 386:284* (1997)). The same library was rescreened using the PDZ domain of Shank 3 (amino acid residues 559-673) cloned into pPC86. The Shank 3 PDZ domain was also tested for interaction with mGluR constructs in pPC86. mGluR5 constructs included a wild type C-terminal 241 amino acid fragment and a four amino acid carboxy-terminal deletion of the same fragment.

Using Homer as "bait" in a yeast two-hybrid screen of a rat cortex and hippocampus cDNA library, multiple cDNA isolates of two novel genes were obtained. Sequencing and full length cloning identified these as distinct members of a gene family, termed Shank 1 and 3 (Naisbitt *et al*., in press). Shank family proteins are closely related to a previously described protein, termed Cortactin Binding protein (CortBP-1; *Du et al., Mol. Cell. Biol*., 18:5838 (1998)).

### EXAMPLE 13

### Interactions Between Homer Proteins and Shank Proteins In Vitro and In Vivo

To characterize the interaction between Homer proteins and Shank proteins, the Shank cDNAs isolated from the yeast two-hybrid screen (Example 10)) were expressed in HEK293 for GST pulldown assays with GST-Homer 1a. The interaction between Homer and Shank proteins was further characterized by co-immunoprecipitation assays.

### (a) Expression Constructs

Shank expression constructs were prepared as described (Naisbitt *et al*., in press). Site directed point mutants of Shank were generated using Quik Change (Stratagene). GST fusion constructs were prepared by polymerase chain reaction (PCR) using Pfu Polymerase (Stratagene) with specific primers that included SalI and NotI sequences. After digestion with SaII/NotI, PCR products were subcloned into pGEX4T-2 vector (Pharmacia Biotech, Uppsala, Sweden) or N-myc-tagged pRK 5 vector (modified from Genentech). All constructs were confirmed by sequencing. GST-fusion proteins were expressed in BL21 E. coli strains (GIBCO, BRL). Bacteria were harvested and lysed in PBS, 1% Triton X-100, 2 mM phenylmethylsulfonyl fluoride (PMSF) and pelleted at 13,000 rpm (Sorvall SS-34) at 4°C for 5 m. Proteins were purified by incubating 1 ml bed volume glutathione-sepharose beads (Sigma) with bacterial supernatant at 4 °C for 10 m. and washed twice with PBS and PBS plus 1% Triton X-100. Bound proteins were eluted with 10 mM glutathione and dialyzed against PBS at 4°C. Protein concentrations were measured by BCA (Pierce, Illinois). mGluR5 constructs and mutants are described in Tu *et al., Neuron* 21:717 (1998).

### (b) GST pulldown and Co-immunoprecipitation Assays

Expression constructs were transiently transfected into HEK293 cells using the calcium phosphate method. Cells were lysed 24-48 h post-transfection with PBS plus 1 % Triton X-100. GST pull down assays were performed by mixing 100 µl cell lysates with beads charged with GST fusion proteins (1-3 µg/50 µl bed vol.) at 4°C for 2 h followed by washing once with PBS, once with PBS plus 1% Triton X-100. Bound proteins were eluted with 100 µl 2 x SDS loading buffer and detected by SDS-PAGE and immunoblotting using ECL reagents (Amersham). GST pull down assays of mGluRla and mGluR5 from brain lysates were performed by sonicating rat cerebellum or cortex in 50mM Tris, 1mM EDTA, 1% CHAPS (Sigma), 0.5% deoxycholic acid (Sigma) and proteinase inhibitors with GST-proteins and these tissue extracts were then processed as above. For immunoprecipitation from COS7 cells, transfected cells were extracted in RIPA (see Naisbitt *et al*., in press). Soluble extracts were precipitated with 2 µg control non-immune IgG, Myc or Shank 1 (56/e) antibodies (Naisbitt *et al*., in press).

Extracts of forebrain crude synaptosomes for immunoprecipitation were prepared using deoxycholic acid as described previously (Dunah *et al., Mol. Pharmacol*. 53429 (1998)). Forebrain P2 fraction was extracted in 1% deoxycholic acid, dialyzed over night into 0.1% Triton X-100, 50 mM Tris, pH 7.4. Concurrently, 5 g of each antibody was pre-incubated overnight with 10 µl bed volume protein A-sepharose. After centrifugation at 100,000g for 1 h, 50 µg of extract was incubated with antibody-protein A in 100 µl 0.1 % Triton X-100, 50 mM Tris, pH 7.4 for 2 h at 4°C. Pellets were washed 4 times with 1 ml incubation buffer, and bound proteins were analyzed by immunoblotting.

**Antibodies** Shank antibodies were raised in rabbits immunized with GST-fusions of Shank 3 residues 1379-1740 and 1379-1675 (Covance, Denver, PA). Similar bands were seen on rat brain immunoblots with both antisera. GKAP, PSD 95 and Shank 1 (56/e) antibodies are described in (Naisbitt *et al.,* in press). Homer antibodies are described above. Anti-mGluR 1a monoclonal antibody is from Pharmingen and rabbit polyclonal mGluR5 antiserum was obtained from Dr Richard Huganir (Johns Hopkins University).

Shank cDNAs derived from the yeast two-hybrid screen were expressed in HEK293 cells for GST pulldown assays with GST-Homer 1a. Each of the Shank polypeptides specifically bound Homer 1a. Based on the finding that the Homer EVH1 domain binds a specific proline-rich motif, three potential Homer binding sites (or Homer "ligands") that are conserved in Shank 1, 2, 3 and CortBP-1 were identified.(Naisbitt *et al*., in press). To define the Homer binding site on Shank family proteins, three deletion fragments of Shank 3 that included, respectively, amino acid residues 559-908, amino acid residues 1143-1408, and amino acid residues 1379-1740 were testing for their ability to bind to Homer 1b, Homer 1c, Homer 2 and Homer 3 in GST pulldown assays. Similar binding specificity was detected with each of the Homer proteins. Only Shank3 fragment 1143-1408 bound to Homer. This region contains the amino acid sequence that most closely resembles the Homer ligand peptide consensus (LVP**PP**EE**F**AN; residues 1307-1316). A similar sequence is present in Shank1 (PLP**PP**LE**F**SN 1563-1572; see Naisbitt *et al*., in press). CortBP possesses two similar sites; (PLP**PP**LE**F**AN; residues 813-822) and (FLP**PP**ES**F**DA residues 878-887). Fragments of Shank3 containing amino acid residues located nearer the amino-terminal of the protein such as Shank 3 fragment 559-908 (which includes the PDZ domain and the first proline-rich motif) did not bind to Homer, but did bind to GKAP (Naisbitt *et al*., in press). Similarly, Shank3 fragment 1379-1740, which includes the carboxy-terminal proline-rich sequence and the SAM domain, did not bind to Homer, though it is capable of binding itself and cortactin (Naisbitt *et al*., in press). These studies identify the Homer binding site as being distinct from either the PDZ domain that binds GKAP, or the proline-rich binding site that binds cortactin and which is located nearer to the carboxy-terminal (Naisbitt *et al*., in press).

To confirm the site of Homer interaction, site directed point mutants of the putative Homer ligand in Shank3 were assessed for their ability to bind to GST-Homer 1c. Full length wild type Shank 3, Shank3(P1311L), and Shank3(F1314C) were expressed in HEK293 cells and assayed for binding to GST-Homer 1c. Compared to wild type Shank 3, both point mutants showed dramatically reduced binding to Homer., These experiments provide further confirmation that the Homer ligand in Shank3 is the principle site of interaction.

It has been previously demonstrated that amino acids 1-110 of the Homer EVH1 domain are necessary and sufficient for binding to Homer ligands (Brakeman *et al.,* 1997, *supra;* Tu *et al.,* 1998, *supra*)*.* To confirm that the EVH1 domain of Homer mediates interactions with Shank, a series of point mutants of the Homer 1 EVH1 domain were generated. Mutations that disrupted binding to mGluR5 disrupted binding to Shank 3 in an identical manner, indicating Homer binds both proteins via a similar EVH1-dependent mechanism (Beneken *et al*., in press).

To confirm the interaction between Homer and Shank in a mammalian cell context, co-immunoprecipitation experiments were performed in heterologous cells. COS7 cell were transfected with Myc tagged-Homer 1b, Shank 1, or Shank 1 plus myc-Homer 1b. Detergent extracts of cells were subjected to immunoprecipitation and blotted with myc, shank, or control (non-immune IgG) antibodies. Homer 1b was used in these experiments because it expresses more efficiently in mammalian cells than Homer 1a. There is co- immunoprecipitation of Homer with Shank antibody and of Shank with myc antibody only from cells expressing *both* Shank and myc-Homer 1b.

To demonstrate the in vivo relevance of the Homer-Shank interaction, co-immunoprecipitation experiments were performed using detergent extracts of rat brain. Detergent extracts of rat forebrain fractions were immunoprecipitated with Shank and control (non-immune) antisera. Immunoprecipitates were blotted for Homer, Shank and GRIP antibodies. Antibodies raised against a fusion protein of Shank 1 immunoprecipitated Homer 1b and 1c proteins as well as Shank from rat forebrain. GRIP was not co-immunoprecipitated with Shank and neither Shank or Homer were precipitated by non-immune IgGs. Furthermore, another Shank antibody, generated against Shank 3 fragment 1379-1675, co-immunoprecipitated Homer 1b and 1c extracted from both cerebellum and cortex.

### EXAMPLE 14

### Homer and Shank Mediate Clustering of Cell-Surface Receptors

Shank proteins may link Homer proteins with components of a cell-surface clustering complex, such as the NMDA clustering complex.

COS7 cells were transfected using the Lipofectamine method (GIBCO-BRL) on poly-lysine coated coverslips for clustering experiments, as described in Naisbitt *et al*. (in press) and Kim *et al*. (*Neuron* 17:103 1996). Primary antibodies were used as follows: GKAP C9589, 1 µg/ml (Naisbitt *et al*., in press); Shank 56/e 0.5 µg/ml (Naisbitt *et al*., in press), PSD-95, 1:1000 diluted guinea pig serum (Kim *et al*., *Neuron* 378:85 1995). Cy3 and (fluoroscein isothiocyanate conjugate (FITC)-conjugated secondary antibodies (Jackson Immunoresearch) were used at dilutions of 1:500 and 1:100 respectively.

Yeast two-hybrid screens were performed as described in Example 10.

A yeast two-hybrid screen of the same rat brain cDNA library was performed using the PDZ domain of Shank3 as bait. From this screen, two identical clones of the carboxy-terminus of GKAP-3/SAPAP3 were isolated. In a reciprocal screen, Naisbitt *et al*. (in press) isolated multiple clones of Shank1, 2 and 3 using GKAP as bait. This result provides independent confirmation of the specificity of the interaction between the Shank and GKAP/SAPAP families of proteins.

The cDNA from the yeast two-hybrid screen encoding the carboxy-terminal 347 amino acids of GKAP-3 was expressed with an amino-terminal *myc* tag in HEK293 cells and tested for binding to GST fusion constructs of Shank3 and other PDZ containing proteins. The GST fusion of Shank3 fragments containing just the PDZ domain (residues 559-673) was sufficient to bind GKAP3, while a Shank3 construct lacking the PDZ domain (residues 665-908) failed to bind. Additionally, PDZ domains of GRIP and SAP 102 failed to pull down GKAP3, demonstrating the specificity of the Shank-GKAP interaction.

The above findings suggest that Homer, Shank and GKAP may assemble into a ternary complex. To explore this further, GST pull-down assays were performed using rat brain extracts. The carboxy-terminal 76 amino acids of GKAP1a, containing the Shank PDZ-binding sequence -QTRL, was fused to GST [GST-GKAP(carboxy-terminal)]. GST-GKAP(carboxy-terminal) specifically pulled down both Shank and Homer 1b and 1c, but not GKAP1 or several other proteins (Naisbitt *et al*., in press). Since GKAP binds directly to Shank but not to Homer (Naisbitt *et al., in press*), the results suggest that the GKAP pulldown of Homer is mediated by Shank. These findings corroborate the co-immunoprecipitation experiments of Shank and Homer from brain extracts and confirm that Homer is associated with Shank in a native complex.

Since Shank proteins may link Homer proteins with components of the NMDA clustering complex, co-clustering of these proteins in transfected COS cells was assessed. In cells co-expressing Homer 1b and PSD-95, both proteins showed a diffuse distribution in the cytoplasm. This is not surprising, since Homer and PSD-95 do not interact directly. When cells were transfected with Shank1 and GKAP in addition to Homer and PSD-95, Homer and PSD-95 redistributed into plaque-like clusters in which both proteins were exactly co-localized. By contrast, co-clustering of Homer and PSD-95 was not observed following co-transfection of Homer and PSD-95 with either Shank1 or GKAP alone. Thus, Homer and PSD-95 co-cluster only upon co-expression of Shank and GKAP. Therefore, Shank and GKAP may mediate the formation of a quaternary protein complex containing PSD-95 and Homer (see also Naisbitt *et al*., in press). Other types of macromolecular complexes may also form when Homer and Shank proteins interact. Cells expressing Homer 1b and Shank 1 (without GKAP or PSD-95) exhibited a redistribution of Homer 1b into a reticular filamentous pattern, as well as into clusters; in both kinds of structures Shank and Homer immunoreactivities were co-localized. These findings provide further evidence for an interaction between Homer and Shank, and suggest that Homer 1b and Shank can co-assemble into higher order macrocomplexes. This result is consistent with the biochemical properties of Shank that include its ability to self-multimerize and bind cortactin (Naisbitt *et al*., in press). Since Shank, GKAP, and PSD-95 are components of NMDA receptor-associated complex (Naisbitt *et al., in press*)*,* the identification of Homer as a Shank-binding protein invokes a molecular link between the NMDA receptor complex and Homer-associated synaptic proteins such as mGluR1a and 5 and the inositol trisphosphate receptor.

**Group 1 Metabotropic Receptors** Based on the observations in heterologous cells that Shank clusters with Homer 1b and that Shank together with GKAP can mediate the co-clustering of Homer and PSD-95 Shank may mediate clustering of group 1 metabotropic glutamate receptors (mGluRs). Co-expression of Shank1 and mGluR5 in COS cells did not result in obvious clustering of either protein. Similarly, Homer and mGluR5 do not form co-clusters. Co-expression of the three proteins Homer, Shank 1, and mGluR5, however, resulted in conspicuous co-clustering of mGluR5 with Shank 1. Clustering of mGluR5 in these triply transfected cells was dependent on the ability of Homer to bind the receptor since a point mutant of mGluR5 that does not interact with Homer failed to co-cluster with Shank. Thus, both Homer and Shank are required to mediate the clustering of mGluR5.

### EXAMPLE 15

### The Shank 3 PDZ Domain Binds the Carboxy-Terminus of Group 1 Metabotropic Receptors Directly at a Site Distinct from the Homer Binding Site

The Shank PDZ domain shows selective binding to the GKAP carboxy-terminus (Naisbitt *et al., in press*)*.* The carboxy-terminal sequence of GKAP (-QTRL) finds similarities with that of the group 1 mGluRs (mGluRla -SSSL; mGluR5 -SSTL) and therefore it was determined whether the PDZ domain of Shank can directly bind the carboxy-terminus of group 1 mGluRs. GST-pulldown assays were performed using extracts from heterologous cells expressing a recombinant mGluR5 carboxy-terminal 241 amino acid peptide. The mGluR5 carboxy-terminal tail bound two partially overlapping constructs of Shank 3 that included the PDZ domain (559-908; and 559-673), but not a construct from which the PDZ domain was deleted (amino acids 665-908). Binding of mGluR to the Shank3 PDZ domain was qualitatively similar to mGluR5 binding to Homer 1c and Homer 2. Negative controls included absence of binding of mGluR to SAP102 PDZI-3 and GRIP PDZ 4-6. Furthermore, a deletion mutant of the mGluR5 polypeptide that lacked the carboxy-terminal four amino acids failed to bind to the PDZ domain of Shank3. Identical interactions between Shank PDZ and mGluR5 C-terminal tail were detected in a yeast two-hybrid analysis. These studies indicate that the PDZ domain of Shank 3 can bind the carboxy-terminus of group 1 metabotropic receptors via a PDZ-mediated interaction with the carboxy-terminal sequence --S S/T L.

To confirm that Shank3 PDZ domain can bind full length native mGluRs, GST pull down assays were performed with detergent extracts of forebrain or cerebellum. The PDZ domain of Shank 3 bound specifically to mGluR1a and mGluR5 from cerebellum and forebrain, respectively. (Cerebellum predominantly expresses mGluR1, while forebrain expresses predominantly mGluR5.) While it is possible that the Shank3 PDZ pulldown of mGluRs from brain extracts is indirect, via Shank PDZ pulling down a GKAP-Shank-Homer-mGluR complex, this extended complex is unlikely given the more modest ability of GST-GKAP to pull down Homer.

These studies suggest that Shank may interact with the cytoplasmic tail of mGluR1a/5 both directly, via its PDZ domain, and indirectly, via Homer. The inability of Shank 1 to cluster mGluR5 in the absence of Homer indicates that the direct PDZ-dependent Shank-mGluR interaction is contingent upon a co-incident Homer interaction. Both modes of interaction with mGluR may be involved in mGluR clustering by Shank and contribute to physiological regulation.

### EXAMPLE 16

### Shank and Homer Co-Localization at Specific Post Synaptic Densities

**Immuno Electron Microscopy A** postembedding immunogold method (Petralia *et al., Nature Neurosci* 2:31 1999; Zhao *et al., J Neurosci* 18:5517 1998) was used. Male Sprague-Dawley rats was perfused with 4% paraformaldehyde plus 0.5% glutaraldehyde in 0.1 M phosphate buffer (PBS). Parasagittal sections (250 µm) of the hippocampus were cryoprotected in 30% glycerol and frozen in liquid propane in a Leica EM CPC. Frozen sections were immersed in 1.5% uranyl acetate in methanol at -90 °C in a Leica AFS freeze-substitution instrument, infiltrated with Lowicryl HM 20 resin at -45 °C, and polymerized with UV light. Thin sections were incubated in 0.1 % sodium borohydride plus 50 mM glycine in Tris-buffered saline/0.1% Triton X-100 (TBST), followed by incubations in 10% normal goat serum (NGS) in TBST, primary antibody in 1% NGS/TBST, 10 nm immunogold (Amersham) in 1% NGS/TBST plus 0.5% polyethylene glycol, and finally staining with uranyl acetate and lead citrate. For double labeling, the first primary antibody (*e*.*g*., Shank; Shank3 1379-1675 antigen) and corresponding immunogold-conjugated antibody (10 nm gold) were applied, sections were exposed to paraformaldehyde vapors at 80°C for one hour, and the second primary (Homer 1b and 1c) and secondary (20 nm gold; Ted Pella/BBI International) antibodies were applied the following day. Controls (showing little or no gold labeling) included absence of the primary antibody for single labeling and absence of the second primary antibody for double labeling. Primary antibodies were used at dilutions of 1:100-1:300 for Shank and 1:400 for Homer 1b and 1c.

An antibody generated against a carboxy-terminal region of Shank 3 (amino acids 1379-1675) was used to examine the ultrastructural distribution of the Shank proteins in brain. This antibody recognizes multiple bands on brain immunoblots, including major bands of ~160-180 kD and ~210 kD in forebrain and cerebellum, similar to those seen with other Shank antibodies (see Naisbitt *et al., in press*)*.* The different size bands presumably derive from the multiple Shank genes and splice variants. All Shank immunoreactivity is blocked by incubation of the Shank antibody with the Shank fusion protein antigen.

Immunogold electron microscopy revealed intense Shank immunoreactivity at the PSD of CA1 pyramidal neurons. Gold particles were distributed over the entire region of the PSD. In the same preparations, Homer 1b/1c was found to co-distribute with Shank. In all profiles with immunostaining for both Shank and Homer, gold particles were present over the PSD but also extended into the region subjacent to the PSD. This distribution is similar to the distribution of NMDA receptors associated with the postsynaptic membrane (Petralia *et al*., 1999, *supra*) and distinct from the distribution of mGluR5 which are most prevalent in the perisynaptic membrane region just outside the PSD (Lujan *et al., Eur J Neurosci* 8:1488 1996). This spatial localization is consistent with the idea that Shank 3 and Homer interact with components of both the NMDA receptor and metabotropic receptor signaling complexes.

This family of proteins that interact with Homer are identical to the Shank family of postsynaptic density (PSD) proteins that interact with GKAP and PSD-95 complex (Naisbitt *et al*., in press). Shank uses distinct domains to bind to GKAP and to Homer, and thus can form a bridge between proteins of this family. Shank/GKAP is also associated with NMDA receptors through the PSD-complex (Naisbitt *et al*., in press) and thus the Homer-Shank interaction indicates a molecular link between NMDA receptors and Homer-associated proteins such as mGlu receptors and inositol trisphosphate receptors. This linkage has important implications for the coupling of NMDA receptors to intracellular calcium release pools and for excitatory synapse assembly in general.

### REFERENCES CITED

Allen, K. M., Gleeson, J. G., Bagrodia, S., Partington, M. W., MacMillan, J. C., Cerione, R. A., Mulley, J. C., and Walsh, C. A. (1998). PAK3 mutation in nonsyndromic X-linked mental retardation. Nat Genet *20,* 25-30.
Bagrodia, S., Taylor, S. J., Jordon, K. A., Van Aelst, L., and Cerione, R. A. (1998). A novel regulator of p21-activated kinases. J Biol Chem *273,* 23633-6.
Biesova, Z., Piccoli, C., and Wong, W. T. (1997). Isolation and characterization of e3B1, an eps8 binding protein that regulates cell growth. Oncogene *14*, 233-41.
Yang, W., Lin, Q., Guan, J. L., and Cerione, R. A. (1999). Activation of the Cdc42-associated tyrosine kinase-2 (ACK-2) by cell adhesion via integrin betal. J Biol Chem *274*, 8524-30.
Ziemnicka-Kotula, D., Xu, J., Gu, H., Potempska, A., Kim, K. S., Jenkins, E. C., Trenkner, E., and Kotula, L. (1998). Identification of a candidate human spectrin Src homology 3 domain- binding protein suggests a general mechanism of association of tyrosine kinases with the spectrin-based membrane skeleton. J Biol Chem *273*, 13681-92.
Abel, T., Nguyen, P. V., Barad, M., Deuel, T. A., Kandel, E. R., and Bourtchouladze, R. (1997). Genetic demonstration of a role for PKA in the late phase of LTP and in hippocampus-based long-term memory. Cell *88*, 615-26.
Aiba, A., Chen, C., Herrup, K., Rosenmund, C., Stevens, C. F., and Tonegawa, S. (1994). Reduced hippocampal long-term potentiation and context-specific deficit in associative learning in mGluR1 mutant mice. Cell *79*, 365-75.
Aigner, L., Arber, S., Kapfhammer, J. P., Laux, T., Schneider, C., Botteri, F., Brenner, H. R., and Caroni, P. (1995). Overexpression of the neural growth-associated protein GAP-43 induces nerve sprouting in the adult nervous system of transgenic mice. Cell *83,* 269-78.
Allen, K. M., Gleeson, J. G., Bagrodia, S., Partington, M. W., MacMillan, J. C., Cerione, R. A., Mulley, J. C., and Walsh, C. A. (1998). PAK3 mutation in nonsyndromic X-linked mental retardation. Nat Genet *20*, 25-30.
Aniksztejn, L., Bregestovski, P., and Ben-Ari, Y. (1991). Selective activation of quisqualate metabotropic receptor potentiates NMDA but not AMPA responses. Eur J Pharmacol *205*, 327-8.
Arai, I., Shimazoe, T., Shibata, S., Inoue, H., Yoshimatsu, A., and Watanabe, S. (1996). Enhancement of dopamine release from the striatum through metabotropic glutamate receptor activation in methamphetamine sensitized rats. Brain Res *729* , 277-80.
Arai, I., Shimazoe, T., Shibata, S., Inoue, H., Yoshimatsu, A., and Watanabe, S. (1997). Methamphetamine-induced sensitization of dopamine release via a metabotropic glutamate receptor mediated pathway in rat striatal slices. Jpn J Pharmacol *73* , 243-6.
Bagrodia, S., Taylor, S. J., Jordon, K. A., Van Aelst, L., and Cerione, R. A. (1998). A novel regulator of p21-activated kinases. J Biol Chem *273*, 23633-6.
Banno, T., and Kohno, K. (1998). Conformational changes of the smooth endoplasmic reticulum are facilitated by L-glutamate and its receptors in rat Purkinje cells. J Comp Neurol *402*, 252-63.
Barnes, C. A., Jung, M. W., McNaughton, B. L., Korol, D. K., Andreasson, K., and Worley, P. F. (1994). LTP saturation and spatial learning disruption: Effects of task variables and saturation levels. Journal of Neuroscience *14*, 5793-5806.
Bashir, Z. I., Bortolotto, Z. A., Davies, C. H., Berretta, N., Irving, A. J., Seal, A. J., Henley, J. M., Jane, D. E., Watkins, J. C., and Collingridge, G. L. (1993). Induction of LTP in the hippocampus needs synaptic activation of glutamate metabotropic receptors. Nature *363* , 347-50.
Baude, A., Nusser, Z., Roberts, J. D., Mulvihill, E., McIlhinney, R. A., and Somogyi, P. (1993). The metabotropic glutamate receptor (mGluR1 alpha) is concentrated at perisynaptic membrane of neuronal subpopulations as detected by immunogold reaction. Neuron *11*, 771-87.
Ben-Ari, Y., Aniksztejn, L., and Bregestovski, P. (1992). Protein kinase C modulation of NMDA currents: an important link for LTP induction. Trends Neurosci *15*, 333-9.
Beneken, J., Tu, J. C., Xiao, B., Yuan, J. P., Worley, P. F., and Leahy, D. J. (submitted). Crystal structure of the Homer EVH1 Domain: A versitile binding module with unexpected homology to PH domains. Neuron.
Berke, J. D., Paletzki, R. F., Aronson, G. J., Hyman, S. E., and Gerfen, C. R. (1998). A complex program of striatal gene expression induced by dopaminergic stimulation. J Neurosci *18*, 5301-10.
Berridge, M. J. (1998). Neuronal Calcium Signaling. Neuron *21*, 13-26.
Bhat, M. A., Izaddoost, S., Lu, Y., Cho, K. O., Choi, K. W., and Bellen, H. J. (1999). Discs Lost, a novel multi-PDZ domain protein, establishes and maintains epithelial polarity. Cell *96*, 833-45.
Blue, M. E., and Parnavelas, J. G. (1983). The formation and maturation of synapses in the visual cortex of the rat. I. Qualitative analysis. J Neurocytol *12*, 599-616.
Bonci, A., and Williams, J. T. (1996). A common mechanism mediates long-term changes in synaptic transmission after chronic cocaine and morphine. Neuron *16*, 631-9.
Bootman, M. D., Berridge, M. J., and Lipp, P. (1997). Cooking with calcium: the recipes for composing global signals from elementary events. Cell *91*, 367-73.
Bortolotto, Z. A., Bashir, Z. I., Davies, C. H., and Collingridge, G. L. (1994). A molecular switch activated by metabotropic glutamate receptors regulates induction of long-term potentiation. Nature *368*, 740-3.
Brakeman, P. R., Lanahan, A. A., O'Brien, R., Roche, K., Barnes, C. A., Huganir, R. L., and Worley, P. F. (1997). Homer: a protein that selectively binds metabotropic glutamate receptors. Nature *386,* 284-8.
Carlezon, W. A., Jr., Thome, J., Olson, V. G., Lane-Ladd, S. B., Brodkin, E. S., Hiroi, N., Duman, R. S., Neve, R. L., and Nestler, E. J. (1998). Regulation of cocaine reward by CREB. Science *282*, 2272-5.
Chevray, P. M., and Nathans, D. (1992). Protein interaction cloning in yeast: identification of mammalian proteins that react with the leucine zipper of Jun. Proc Natl Acad Sci U S A *89*, 5789-93.
Cole, A., Saffen, D., Baraban, J., and Worley, P. (1989). Rapid increase of an immediate early gene mRNA in hippocampal neurons by synaptic NMDA receptor activation. Nature *340*, 474-476.
Conquet, F., Bashir, Z. I., Davies, C. H., Daniel, H., Ferraguti, F., Bordi, F., Franz-Bacon, K., Reggiani, A., Matarese, V., Conde, F., and et, a. (1994). Motor deficit and impairment of synaptic plasticity in mice lacking mGluR1. Nature *372* , 237-43.
Cull-Candy, S. G., Brickley, S. G., Misra, C., Feldmeyer, D., Momiyama, A., and Farrant, M. (1998). NMDA receptor diversity in the cerebellum: identification of subunits contributing to functional receptors. Neuropharmacology *37*, 1369-80.
Daniels, R. H., Zenke, F. T., and Bokoch, G. M. (1999). alphaPix stimulates p21-activated kinase activity through exchange factor-dependent and -independent mechanisms. J Biol Chem *274*, 6047-50.
Dong, H., O'Brien, R J., Fung, E. T., Lanahan, A. A., Worley, P. F., and Huganir, R L. (1997). GRIP: a synaptic PDZ domain-containing protein that interacts with AMPA receptors [see comments]. Nature *386*, 279-84.
DuBois, R. N., and Smalley, W. E. (1996). Cyclooxygenase, NSAIDs, and colorectal cancer. J Gastroenterol *31*, 898-906.
Dudek, S. M., and Bear, M. F. (1989). A biochemical correlate of the critical period for synaptic modification in the visual cortex. Science *246*, 673-5.
Dunah, A. W., Luo, J., Wang, Y. H., Yasuda, R. P., and Wolfe, B. B. (1998). Subunit composition of N-methyl-D-aspartate receptors in the central nervous system that contain the NR2D subunit. Mol Pharmacol *53*, 429-37.
Eck, M. J., Dhe-Paganon, S., Trub, T., Nolte, R. T., and Shoelson, S. E. (1996). Structure of the IRS-1 PTB domain bound to the juxtamembrane region of the insulin receptor. Cell *85*, 695-705.
Elmer, G. I., Gorelick, D. A., Goldberg, S. R., and Rothman, R. B. (1996). Acute sensitivity vs. context-specific sensitization to cocaine as a function of genotype. Pharmacol Biochem Behav *53*, 623-8.
Emptage, N., Bliss, T. V., and Fine, A. (1999). Single synaptic events evoke NMDA receptor-mediated release of calcium from internal stores in hippocampal dendritic spines [In Process Citation]. Neuron *22*, 115-24.
Fields, S., and Song, O. (1989). A novel genetic system to detect protein-protein interactions. Nature *340,* 245-6.
Fiorillo, C. D., and Williams, J. T. (1998). Glutamate mediates an inhibitory postsynaptic potential in dopamine neurons [In Process Citation]. Nature *394*, 78-82.
Fitzgerald, L. W., Ortiz, J., Hamedani, A. G., and Nestler, E. J. (1996). Drugs of abuse and stress increase the expression of GluR1 and NMDAR1 glutamate receptor subunits in the rat ventral tegmental area: common adaptations among cross-sensitizing agents. J Neurosci *16,* 274-82.
Fosnaugh, J. S., Bhat, R. V., Yamagata, K., Worley, P. F., and Baraban, J. M. (1995). Activation of Arc, a putative "effector" immediate early gene, by cocaine in rat brain. J. Neurochemistry *64*, 2377-2380.
Gertler, F. B., Niebuhr, K., Reinhard, M., Wehland, J., and Soriano, P. (1996). Mena, a relative of VASP and Drosophila Enabled, is implicated in the control of microfilament dynamics. Cell *87*, 227-39.
Goebel, D. J., and Poosch, M. S. (1999). NMDA receptor subunit gene expression in the rat brain: a quantitative analysis of endogenous mRNA levels of NR1Com, NR2A, NR2B, NR2C, NR2D and NR3A [In Process Citation]. Brain Res Mol Brain Res *69,* 164-70.
Golshani, P., Warren, R. A., and Jones, E. G. (1998). Progression of change in NMDA, non-NMDA, and metabotropic glutamate receptor function at the developing corticothalamic synapse. J Neurophysiol *80*, 143-54.
Haffner, C., Jarchau, T., Reinhard, M., Hoppe, J., Lohmann, S. M., and Walter, U. (1995). Molecular cloning, structural analysis and functional expression of the proline-rich focal adhesion and microfilament-associated protein VASP. Embo J *14*, 19-27.
Harris, K. M., and Stevens, J. K. (1989). Dendritic spines of CA 1 pyramidal cells in the rat hippocampus: serial electron microscopy with reference to their biophysical characteristics. J Neurosci *9*, 2982-97.
Harris, K. M., and Stevens, J. K. (1988). Dendritic spines of rat cerebellar Purkinje cells: serial electron microscopy with reference to their biophysical characteristics. J Neurosci *8*, 4455-69.
Henry, D. J., and White, F. J. (1991). Repeated cocaine administration causes persistent enhancement of D1 dopamine receptor sensitivity within the rat nucleus accumbens. J. Pharmacol. Exp. Ther. *258*, 882-890.
Herschman, H. R. (1994). Regulation of prostaglandin synthase-1 and prostaglandin synthase-2. Cancer and Metastasis Reviews *13*, 241-256.
Hope, B. T., Kosofsky, B., Hyman, S. E., and Nestler, E. J. (1992). Regulation of IEG expression and AP-1 binding by chronic cocaine in the rat nucleus accumbens. Proc. Natl. Acad. Sci. USA *89*, 5764-5768.
Hsueh, Y. P., and Sheng, M. (1998). Anchoring of glutamate receptors at the synapse. Prog Brain Res *116,* 123-31.
Hyman, S. E. (1996). Addiction to cocaine and amphetamine. Neuron *16*, 901-4.
Hyvönen, M., Macias, M. J., Nilges, M., Oschkinat, H., Sarastre, M., and Wilmanns, M. (1995). Structure of the binding site for inositol phosphates in a PH domain. EMBO J. *14*, 4676-4685.
Ikeda, S. R., Lovinger, D. M., McCool, B. A., and Lewis, D. L. (1995). Heterologous expression of metabotropic glutamate receptors in adult rat sympathetic neurons: subtype-specific coupling to ion channels. Neuron *14*, 1029-38.
Ingi, T., Krumins, A. M., Chidiac, P., Brothers, G. M., Chung, S., Snow, B. E., Barnes, C. A., Lanahan, A. A., Siderovski, D. P., Ross, E. M., Gilman, A. G., and Worley, P. F. (1998). Dynamic Regulation of RGS2 Suggests a Novel Mechanism in G-Protein Signaling and Neuronal Plasticity. J. Neurosci. *18*, 7178-7188.
Kammermeier, P., Xiao, B., Tu, J., Worley, P., and Ikeda, S. (submitted). A functional role for the interaction of Homer proteins with group 1 mGluRs. J. Neuroscience.
Kaufmann, W. E., Worley, P. F., Pegg, J., Bremer, M., and Isakson, P. (1996). COX-2, a synaptically induced enzyme, is expressed by excitatory neurons at postsynaptic sites in rat cerebral cortex. Proc Natl Acad Sci U S A *93* , 2317-21.
Kim, E., Naisbitt, S., Hsueh, Y. P., Rao, A., Rothschild, A., Craig, A. M., and Sheng, M. (1997). GKAP, a novel synaptic protein that interacts with the guanylate kinase-like domain of the PSD-95/SAP90 family of channel clustering molecules. J Cell Biol *136*, 669-78.
Kim, J. H., and Vezina, P. (1998). Metabotropic glutamate receptors are necessary for sensitization by amphetamine. Neuroreport *9*, 403-6.
Koff, J. M., Shuster, L., and Miller, L. G. (1994). Chronic cocaine administration is associated with behavioral sensitization and time-dependent changes in striatal dopamine transporter binding. J Pharmacol Exp Ther *268,* 277-82.
Kombian, S. B., and Malenka, R. C. (1994). Simultaneous LTP of non-NMDA- and LTD of NMDA-receptor-mediated responses in the nucleus accumbens. Nature *368,* 242-6.
Kornau, H. C., Schenker, L. T., Kennedy, M. B., and Seeburg, P. H. (1995). Domain interaction between NMDA receptor subunits and the postsynaptic density protein PSD-95. Science *269*, 1737-40.
Kraulis, P. J. (1991). MOLSCRIPT: a program to produce both detailed and schematic plots of protein structures. J. Appl. Cryst. *24,* 946-950
Lanahan, A., Lyford, G., Stevenson, G. S., Worley, P. F., and Barnes, C. A. (1997). Selective alteration of long-term potentiation-induced transcriptional response in hippocampus of aged, memory-impaired rats. Journal of Neuroscience *17*, 2876-2885.
Lanahan, A., and Worley, P. (1998). Immediate-Early Genes and Synaptic Function. Neurobiol Learn Mem *70*, 37-43.
Lau, L. F., and Nathans, D. (1991). Genes induced by serum growth factors. In The Hormonal Control of Gene Transcription. Vol. 6. Molecular Aspects, P. Cohen and J. G. Foulkes, eds. (Amsterdam: Elsevier Science Publishers B.V.), pp. 257-293.
Le Moal, M. (1995). Mesocorticolimbic Dopaminergic Neurons; Functional and Regulatory Roles. In Psychopharmacology: The Fourth Generation of Progress, F. B. a. D. Kupfer, ed. (New York: Raven Press Ltd.), pp. 283-294.
Leanna, C., and Hannink, M. (1996). The reverse two-hybrid system: a genetic scheme for selection against specific protein/protein interactions. Nucleic Acids Res. *24*, 3341-3347.
Leong, P., and MacLennan, D. H. (1998). Complex interactions between skeletal muscle ryanodine receptor and dihydropyridine receptor proteins [In Process Citation]. Biochem Cell Biol *76*, 681-94.
Leong, P., and MacLennan, D. H. (1998). The cytoplasmic loops between domains II and III and domains III and IV in the skeletal muscle dihydropyridine receptor bind to a contiguous site in the skeletal muscle ryanodine receptor. J Biol Chem *273*, 29958-64.
Lester, L. B., and Scott, J. D. (1997). Anchoring and scaffold proteins for kinases and phosphatases. Recent Prog Horm Res *52*, 409-29; discussion 429-30.
Linden, D. J. (1999). The return of the spike: postsynaptic action potentials and the induction of LTP and LTD. Neuron *22*, 661-6.
Lu, Y. M., Jia, Z., Janus, C., Henderson, J. T., Gerlai, R., Wojtowicz, J. M., and Roder, J. C. (1997). Mice lacking metabotropic glutamate receptor 5 show impaired learning and reduced CA1 long-term potentiation (LTP) but normal CA3 LTP. J Neurosci *17,* 5196-205.
Lujan, R., Nusser, Z., Roberts, J. D., Shigemoto, R., and Somogyi, P. (1996). Perisynaptic location of metabotropic glutamate receptors mGluR1 and mGluR5 on dendrites and dendritic spines in the rat hippocampus. Eur J Neurosci *8,* 1488-500.
Lujan, R., Roberts, J. D., Shigemoto, R., Ohishi, H., and Somogyi, P. (1997). Differential plasma membrane distribution of metabotropic glutamate receptors mGluR1 alpha, mGluR2 and mGluR5, relative to neurotransmitter release sites. J Chem Neuroanat *13*, 219-41.
Lyford, G., Yamagata, K., Kaufmann, W. E., Barnes, C. A., Sanders, L. K., Copeland, N. G., Gilbert, D. J., Jenkins, N. A., Lanahan, A. A., and Worley, P. F. (1995). *Arc,* a growth factor and activity-regulated gene encodes a novel cytoskeleton-associated protein that is enriched in neuronal dendrites. Neuron *14*, 433-445.
Lyford, G. L., Yamagata, K., Kaufmann, W. E., Barnes, C. A., Sanders, L. K., Copeland, N. G., Gilbert, D. J., Jenkins, N. A., Lanahan, A. A., and Worley, P. F. (1995). Arc, a growth factor and activity-regulated gene, encodes a novel cytoskeleton-associated protein that is enriched in neuronal dendrites. Neuron *14,* 433-45.
Manser, E., Loo, T. H., Koh, C. G., Zhao, Z. S., Chen, X. Q., Tan, L., Tan, I., Leung, T., and Lim, L. (1998). PAK kinases are directly coupled to the PIX family of nucleotide exchange factors. Mol Cell *1*, 183-92.
Martin, L. J., Blackstone, C. D., Huganir, R. L., and Price, D. L. (1992). Cellular localization of a metabotropic glutamate receptor in rat brain. Neuron *9*, 259-70.
Marx, S. O., Ondrias, K., and Marks, A. R. (1998). Coupled gating between individual skeletal muscle Ca2+ release channels (Ryanodine receptors) [In Process Citation]. Science *281*, 818-21.
Matsui, T., Maeda, M., Doi, Y., Yonemura, S., Amano, M., Kaibuchi, K., Tsukita, S., and Tsukita, S. (1998). Rho-kinase phosphorylates COOH-terminal threonines of ezrin/radixin/moesin (ERM) proteins and regulates their head-to-tail association. J Cell Biol *140*, 647-57.
Miserendino, M., Guitart, X., Terwilliger, R., Chi, S., and Nestler, E. J. (1993). Individual differences in locomotor activity are associated with levels of tyrosine hydroxylase and neurofilament proteins in the ventral tegmental area of Sprague Dawley rats. Mol. Cell. Neurosci. *4*, 440-448.
Moratalla, R., Elibol, B., Vallejo, M., and Graybiel, A. M. (1996). Network-level changes in expression of inducible Fos-Jun proteins in the striatum during chronic cocaine treatment and withdrawal. Neuron *17*, 147-56.
Morgan, J. I., and Curran, T. (1991). Stimulus-transcription coupling in the nervous system. Ann. Rev. Neurosci. *14*, 421-452.
Naisbitt, S., Kim, E., Tu, J., Xiao, B., Sala, C., Valtschanoff, J., Weinberg, R, Worley, P., and Sheng, M. (1999). Shank, a novel family of postsynaptic density proteins that binds to the NMDA receptor/PSD-95/GKAP complex and cortactin. Neuron *23*, 569-582.
Naisbitt, S., Kim, E., Weinberg, R. J., Rao, A., Yang, F. C., Craig, A. M., and Sheng, M. (1997). Characterization of guanylate kinase-associated protein, a postsynaptic density protein at excitatory synapses that interacts directly with postsynaptic density-95/synapse-associated protein 90. J Neurosci *17*, 5687-96.
Nakanishi, S., Masu, M., Bessho, Y., Nakajima, Y., Hayashi, Y., and Shigemoto, R (1994). Molecular diversity of glutamate receptors and their physiological functions. [Review]. Exs *71*, 71-80.
Narasimhan, K., Pessah, I. N., and Linden, D. J. (1998). Inositol-1,4,5-trisphosphate receptor-mediated Ca mobilization is not required for cerebellar long-term depression in reduced preparations. J Neurophysiol *80*, 2963-74.
Nestler, E. J., and Aghajanian, G. K. (1997). Molecular and cellular basis of addiction. Science *278*, 58-63.
Nestler, E. J., Hope, B. T., and Widnell, K. L. (1993). Drug addiction: a model for the molecular basis of neural plasticity. Neuron *11*, 995-1006.
Nestler, E. J., Terwilliger, R Z., Walker, J. R., Servarino, K. A., and Duman, R. S. (1990). Chronic cocaine treatment decreases levels of the G-protein subunits Gia and Goa in discrete regions of rat brain. J. Neurochem. *55*, 1079-1082.
Niebuhr, K., Ebel, F., Frank, R., Reinhard, M., Domann, E., Carl, U. D., Walter, U., Gertler, F. B., Wehland, J., and Chakraborty, T. (1997). A novel proline-rich motif present in ActA of Listeria monocytogenes and cytoskeletal proteins is the ligand for the EVH1 domain, a protein module present in the Ena/VASP family. EMBO J *16,* 5433-44.
Nusser, Z., Mulvihill, E., Streit, P., and Somogyi, P. (1994). Subsynaptic segregation of metabotropic and ionotropic glutamate receptors as revealed by immunogold localization. Neuroscience *61*, 421-7.
O'Brien, R. J., Xu, D., Petralia, R. S., Steward, O., Huganir, R L., and Worley, P. F. (1999). Synaptic clustering of AMPA receptors by the extracellular immediate-early gene product Narp. Neuron *23*, 309-323.
O'Rourke, B., Kass, D. A., Tomaselli, G. F., Kaab, S., Tunin, R., and Marban, E. (1999). Mechanisms of altered excitation-contraction coupling in canine tachycardia-induced heart failure, I: experimental studies. Circ Res *84*, 562-70.
Okabe, S., Collin, C., Auerbach, J. M., Meiri, N., Bengzon, J., Kennedy, M. B., Segal, M., and McKay, R. D. (1998). Hippocampal synaptic plasticity in mice overexpressing an embryonic subunit of the NMDA receptor. J Neurosci *18*, 4177-88.
Otani, S., and Connor, J. A. (1998). Requirement of rapid Ca2+ entry and synaptic activation of metabotropic glutamate receptors for the induction of long-term depression in adult rat hippocampus. J Physiol (Lond) *511*, 761-70.
Petralia, R., Esteban, J., Wang, Y.-X., Partridge, J., Zhao, H.-M., Wenthold, R., and Malinow, R. (1999). Selective acquisition of AMPA receptors over postnatal development suggests a molecular basis for silent synapses. Nature Neurosci. *2*, 31-36.
Philipp, S., and Flockerzi, V. (1997). Molecular characterization of a novel human PDZ domain protein with homology to INAD from Drosophila melanogaster. FEBS Lett *413,* 243-8.
Piazza, P. V., Deminiere, J.-M., LeMoal, M., and Simon, H. (1989). Factors that predict individual vulnerability to amphetamine self-administration. Science *245,* 1511-1513.
Pin, J. P., and Duvoisin, R (1995). The metabotropic glutamate receptors: structure and functions. [Review]. Neuropharmacology *34*, 1-26.
Pisabarro, M. T., Serrano, L., and Wilmanns, M. (1998). Crystal Structure of the Abl-SH3 Domain Complexed with a Designed High- affinity Peptide Ligand: Implications for SH3-Ligand Interactions. J Mol Biol *281*, 513-21.
Ponting, C. P., and Phillips, C. (1997). Identification of homer as a homologue of the Wiskott-Aldrich syndrome protein suggests a receptor-binding function for WH1 domains. J Mol Med *75,* 769-71.
Prehoda, K. E., Lee, D. J., and Lim, W. A. (1999). Strucute of the Enabled/VASP Homology 1 domain-peptide complex: A key component in the spatial control of actin assembly. Cell *97,* 471-480.
Qian, Z., Gilbert, M. E., Colicos, M. A., Kandel, E. R., and Kuhl, D. (1993). Tissue-plasminogen activator is induced as an immediate-early gene during seizure, kindling and long-term potentiation. Nature *361*, 453-457.
Riedel, G. (1996). Function of metabotropic glutamate receptors in learning and memory. Trends in Neurosciences *19*, 219-224.
Robinson, T. E., and Kolb, B. (1999). Alterations in the morphology of dendrites and dendritic sp ines in the nucleus accumbens and prefrontal cortex following repeated treatment with amphetamine or cocaine. Eur J Neurosci *11*, 1598-604.
Roche, K., Tu, J., Petralia, R., Xiao, B., Wenthold, R., and Worley, P. (accepted). Homer 1b regulates the surface expression of type I metabotropic glutamate receptors. J. Biol. Chem.
Romano, C., Sesma, M. A., McDonald, C. T., O'Malley, K., Van den Pol, A. N., and Olney, J. W. (1995). Distribution of metabotropic glutamate receptor mGluR5 immunoreactivity in rat brain. J Comp Neurol *355*, 455-69.
Ross, C. A., Meldolesi, J., Milner, T. A., Satoh, T., Supattapone, S., and Snyder, S. H. (1989). Inositol 1,4,5-trisphosphate receptor localized to endoplasmic reticulum in cerebellar Purkinje neurons. Nature *339,* 468-70.
Saffen, D. W., Cole, A. J., Worley, P. F., Christy, B. A., Ryder, K., and Baraban, J. M. (1988). Convulsant-induced increase in transcription factor messenger RNAs in rat brain. Proceedings of the National Academy of Sciences (USA) *85*, 7795-7799.
Saiki, Y., El-Hayek, R., and Ikemoto, N. (1999). Involvement of the Glu724-Pro760 region of the dihydropyridine receptor II-III loop in skeletal muscle-type excitation-contraction coupling. J Biol Chem *274*, 7825-32.
Satoh, T., Ross, C. A., Villa, A., Supattapone, S., Pozzan, T., Snyder, S. H., and Meldolesi, J. (1990). The inositol 1,4,5,-trisphosphate receptor in cerebellar Purkinje cells: quantitative immunogold labeling reveals concentration in an ER subcompartment. J Cell Biol *111*, 615-24.
Seibert, K., Zhang, Y., Leahy, K., Hauser, S., Masferrer, J., Perkins, W., Lee, L., and Isakson, P. (1994). Pharmacological and biochemical demonstration of the role of cyclooxygenase 2 in inflammation and pain. Proc Natl Acad Sci U S A *91*, 12013-7.
Selig, D. K., Lee, H. K., Bear, M. F., and Malenka, R. C. (1995). Reexamination of the effects of MCPG on hippocampal LTP, LTD, and depotentiation. J Neurophysiol *74,* 1075-82.
Shatz, C. J. (1990). Impulse activity and the patterning of connections during CNS development. Neuron *5*, 745-756.
Shaw, R. J., Henry, M., Solomon, F., and Jacks, T. (1998). RhoA-dependent phosphorylation and relocalization of ERM proteins into apical membrane/actin protrusions in fibroblasts. Mol Biol Cell *9*, 403-19.
Sheng, M., and Wyszynski, M. (1997). Ion channel targeting in neurons. Bioessays *19*, 847-53.
Shih, H., Goldman, P., DeMaggio, A., Hollenberg, S., Goodman, R., and Hoekstra, M. (1996). A positive genetic selection for disrupting protein-protein interactions:Identification of CREB mutations that prevent association with the coactivator CBP. Proc. Natl. Acad. Sci. USA 93, 13896-13901.
Spacek, J., and Harris, K. M. (1997). Three-dimensional organization of smooth endoplasmic reticulum in hippocampal CA1 dendrites and dendritic spines of the immature and mature rat. J Neurosci *17*, 190-203.
Steward, O., Wallace, C. S., Lyford, G. L., and Worley, P. F. (1998). Synaptic activation causes the mRNA for the immediate early gene Arc to localize selectively near activated postsynaptic sites on neuronal dendrites. Neuron *21*, 741-751.
Stewart, W. F., Kawas, C., Corrada, M., and Metter, E. J. (1997). Risk of Alzheimer's disease and duration of NSAID use [see comments]. Neurology *48*, 626-32.
Storck, T., Kruth, U., Kolhekar, R., Sprengel, R., and Seeburg, P. H. (1996). Rapid construction in yeast of complex targeting vectors for gene manipulation in the mouse. Nucleic Acids Res 24, 4594-6.
Svoboda, K., and Mainen, Z. F. (1999). Synaptic [Ca2+]: intracellular stores spill their guts. Neuron 22, 427-30.
Symons, M., Derry, J. M., Karlak, B., Jiang, S., Lemahieu, V., McCormick, F., Francke, U., and Abo, A. (1996). Wiskott-Aldrich syndrome protein, a novel effector for the GTPase CDC42Hs, is implicated in actin polymerization. Cell *84*, 723-34.
Taber, M. T., and Fibiger, H. C. (1995). Electrical stimulation of the prefrontal cortex increases dopamine release in the nucleus accumbens of the rat: modulation by metabotropic glutamate receptors. J Neurosci *15*, 3896-904.
Takei, K., Mignery, G. A., Mugnaini, E., Sudhof, T. C., and De Camilli, P. (1994). Inositol 1,4,5-trisphosphate receptor causes formation of ER cistemal stacks in transfected fibroblasts and in cerebellar Purkinje cells. Neuron *12*, 327-42.
Takeuchi, M., Hata, Y., Hirao, K., Toyoda, A., Irie, M., and Takai, Y. (1997). SAPAPs. A family of PSD-95/SAP90-associated proteins localized at postsynaptic density. J Biol Chem *272,* 11943-51.
Testa, C. M., Standaert, D. G., Landwehrmeyer, G. B., Penney, J., Jr., and Young, A. B. (1995). Differential expression of mGluR5 metabotropic glutamate receptor mRNA by rat striatal neurons. J Comp Neurol *354*, 241-52.
Tolliver, B. K., and Carney, J. M. (1994). Comparison of cocaine and GBR 12935: effects on locomotor activity and stereotypy in two inbred mouse strains. Pharmacol Biochem Behav *48*, 733-9.
Tsui, C. C., Copeland, N. G., Gilbert, D. J., Jenkins, N. A., Barnes, C., and Worley, P. F. (1996). Narp, a novel member of the pentraxin family, promotes neuite outgrowth and is dynamically regulated by neuronal activity. Journal of Neuroscience *16*, 2463-2478.
Tsunoda, S., Sierralta, J., Sun, Y., Bodner, R., Suzuki, E., Becker, A., Socolich, M., and Zuker, C. S. (1997). A multivalent PDZ-domain protein assembles signalling complexes in a G- protein-coupled cascade. Nature *388*, 243-9.
Tu, J. C., Bo Xiao, B., Naisbitt, S., Yuan, J. P., Petralia, R S., Brakeman, P. R., Aakalu, V. K., Lanahan, A. A., Sheng, M., and Worley, P. (1999). mGluR/Homer and PSD-95 Complexes Are Linked by the Shank Family of Postsynaptic Density Proteins. Neuron *23*, 583-592.
Tu, J. C., Xiao, B., Yuan, J., Lanahan, A., Leoffert, K., Li, M., Linden, D., and Worley, P. F. (1998). Homer binds a novel proline rich motif and links group1 metabotropic glutamate receptors with IP3 receptors. Neuron *21,* 717-726.
Ujike, H:, Okumura, K., Zushi, Y., Akiyama, K., and Otsuki, S. (1992). Persistent supersensitivity of sigma receptors develops during repeated methamphetamine treatment. Eur J Pharmacol *211,* 323-8.
Vicini, S., Wang, J. F., Li, J. H., Zhu, W. J., Wang, Y. H., Luo, J. H., Wolfe, B. B., and Grayson, D. R. (1998). Functional and pharmacological differences between recombinant N-methyl- D-aspartate receptors. J Neurophysiol *79* , 555-66.
Vidal, M., Brachmann, R., Fattaey, A., Harlow, E., and Boeke, J. (1996). Reverse two-hybrid and one-hybrid systems to detect dissociation of protein-protein and DNA-protein interactions. Proc. Natl. Acad. Sci. USA *93*, 10315-10320.
Vidal, M., Pascal, B., Chen, E., Boeke, J., and Harlow, E. (1996). Genetic characterization of a mammalian protein-protein interaction domain by using a yeast reverse two-hybrid system. Proc. Natl. Acad. Sci. USA *93*, 10321-10326.
Villa, A., Sharp, A. H., Racchetti, G., Podini, P., Bole, D. G., Dunn, W. A., Pozzan, T., Snyder, S. H., and Meldolesi, J. (1992). The endoplasmic reticulum of Purkinje neuron body and dendrites: molecular identity and specializations for Ca2+ transport. Neuroscience *49*, 467-77.
Vrana, S. L., Vrana, K. E., Koves, T. R., Smith, J. E., and Dworkin, S. I. (1993). Chronic cocaine administration increases CNS tryosine hydroxylase enzyme activity and mRNA levels and trypophan hydroxylase enzyme activity levels. J. Neurochem. *61*, 2262-2268.
Wlodawer, A., Hodgson, K. O., and Shooter, E. M. (1975). Crystallization of nerve growth factor from mouse submaxillary glands. Proc Natl Acad Sci U S A *72*, 777-9.
Wolf, M. E. (1998). The role of excitatory amino acids in behavioral sensitization to psychomotor stimulants. Prog Neurobiol *54*, 679-720.
Worley, P. F., Baraban, J. M., Colvin, J. S., and Snyder, S. H. (1987). Inositol trisphosphate receptor localization in brain: variable stoichiometry with protein kinase C. Nature *325*, 159-161.
Worley, P. F., Baraban, J. M., Supattapone, S., Wilson, V. S., and Snyder, S. H. (1987). Characterization of inositol trisphosphate receptor binding in brain: Regulation by calcium and pH. Journal Biological Chemistry *262*, 12132-12136.
Worley, P. F., Cole, A. J., Saffen, D. W., and Baraban, J. M. (1990). Transcription factor regulation in brain: focus on activity and NMDA dependent regulation. In Molecular mechanisms of aging, K. Beyreuther and G. Schettler, eds. (Heidelberg: Springer-Verlag), pp. 62-76.
Xiao, B., Tu, J. C., Petralia, R. S., Yuan, J., Doan, A., Breder, C., Ruggiero, A., Lanahan, A. A., Wenthold, R. J., and Worley, P. F. (1998). Homer regulates the association of Group 1 metabotropic receptors with multivalent complexes of Homer-related, synaptic proteins. Neuron *21*, 707-716.
Yamagata, K., Andreasson, K. I., Kaufmann, W. E., Barnes, C. A., and Worley, P. F. (1993). Expression of a Mitogen-Inducible Cyclooxygenase in Brain Neurons: Regulation by Synaptic Activity and Glucocorticoids. Neuron *11*, 371-386.
Yamagata, K., Sanders, L. K., Kaufmann, W. E., Barnes, C. A., Nathans, D., and Worley, P. F. (1994). *Rheb,* a growth factor and synaptic activity regulated gene, encodes a novel Ras-related protein. Journal of Biological Chemistry *269,* 16333-16339.
Yee, W., and Worley, P. F. (1997). Rheb interacts with Raf-1 kinase and may function to integrate growth factor- and protein kinase A-dependent signals. Molecular and Cellular Biology *17*, 921-933.
Yu, H., Chen, J. K., Feng, S., Dalgarno, D. C., Brauer, A. W., and Schreiber, S. L. (1994). Structural basis for the binding of proline-rich peptides to SH3 domains. Cell *76*, 933-45.

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

**Table 1. Data Collection, Phase Calculation, and Refinement Statistics MAD Data Collection Statistics**

| Wavelength (λ) | 09879 | 09793 | 09790 | 09611 |
|---|---|---|---|---|
| Unique reflections | 24051 | 24179 | 24226 | 24481 |
| Redundancy | 6.2 | 6.2 | 6.2 | 6.3 |
| Completeness (%) | 96.8 | 97.2 | 97.3 | 98.4 |
| Signal (<I>/σ<I>)^{a} | 21.5 (2.3) | 21.0 (2.1) | 20.9 (2.0) | 20.4 (1.9) |
| R_{sym} (%) | 8.1 | 8.7 | 9.1 | 8.8 |
| Overall figure of merit | 0.71 | | | |

| **MAD Structure Factor Ratios**^{**b**} **and Anomalous Scattering Factors**^{**c**} | | | | |
|---|---|---|---|---|
| Wavelength (λ) | 0.9879 | 0.9783 | 0.9790 | 0.9611 |
| 0.9879 | 0.033 | 0.040 | 0.032 | 0.026 |
| 0.9793 | | 0.047 | 0.029 | 0.044 |
| 0.9790 | | | 0.063 | 0.036 |
| 0.9611 | | | | 0.050 |
| f' (e) | -4.87 | -9.96 | -8.06 | -4.15 |
| f' (e) | 0.47 | 3.77 | 6.28 | 4.12 |

| Refinement Statistics | | | | |
|---|---|---|---|---|
| R_{crys}t (%) | 25.3 | | | |
| R_{free} (%) | 28.4 | | | |
| Average B (Å²) | 24.8 protein/31.7 solvent | | | |
| No. of water molecules | 88 | | | |
| RMSD bond lengths (Å) | 0.0126 | | | |
| RMSD bond angles (°) | 1.745 | | | |
| RMSD B values (Å²) | 0.837/1.487 bonds/angles main chain 1.021/1.594 bonds/angles side chains | | | |

| | | | | |
|---|---|---|---|---|
| ^{a}Values in parentheses are for the highest resolution shell (1.73-1.70Å). R_{sym} = 100 x Σ \|I - <I> \|/ΣI where I is the integrated intensity of a given reflection. | | | | |
| ^{b}RMS (A \|F\|) /RMS (\|F\|) where Δ \|F\| is the Bijvoet difference at one wavelength (values on the diagonal) or the dispersive differences between two wavelength (values off the diagonal). | | | | |
| ^{c}Anomalous components of the Se scattering factors as a function of wavelength as determined by SOLVE (Terwilliger and Eisenberg, 1983). | | | | |
| ^{d}All rounds of refinement included data for which \|F\| >2.0σ. R value = Σ\|Fₚ(obs) - Fₚ(calc) \|/ΣFₚ(obs), where Fp is the structure factor amplitude. The free R value was calculated from 10% of the data that was excluded from the refinement (Brünger, 1992). | | | | |

**Amino Acid Residues and the Homer Binding Domain**

| **Mutation** | **Expression Level (Western Blot)** | Binding^{a} |
|---|---|---|
| Homer 2 EVH WT | ++ | +- |
| F7A | - | ND |
| F7R | + | + |
| S8L | + | - |
| N23A | ++ | + |
| S28A | + | - |
| V34M | ++ | + |
| S35V | ++ | + |
| D39A | ++ | - |
| R42E | ++ | - |
| R42A | ++ | + |
| R46A | ++ | - |
| R46C | ++ | + |
| I48A | ++ | + |
| N58A | ++ | + |
| N64G | ++ | + |
| F67S | + | - |
| K69A | ++ | + |
| Q72A | ++ | + |
| F74A | ++ | + |
| F74L | ++ | + |
| F90S | ++ | + |
| E93K | + | + |
| H95A | ++ | + |
| L96S | + | + |
| F109C | ++ | + |

| | | |
|---|---|---|
| ^{a} (-) indicates substantially reduced binding relative to wild-type (+). | | |

**Table 2. WASP EVH1 Mutations Table 2A - β1 region**

| WASP Residue/Mutation | Homer Residue |
|---|---|
| Exposed | |
| L39M | Met 1 |
| C43W | Pro 5 |
| L46P | Ser 8 |
| T48I | Arg 10 |
| E133K | His 95 |

| Buried/partially buried | |
|---|---|
| T45M | Phe 7 |
| A47D | Thr 9 |
| A49E | Ala 11 |

**Table 2B - β3 region**

| WASP Residue/Mutation | Homer Residue |
|---|---|
| Exposed | |
| S82P/F | Arg 42 |

| Buried/partially buried | |
|---|---|
| F84L | Val 44 |
| R86C/H/P/L | Arg 46 |
| G89D | Ser 49 |

**Table 2C - Other mutations**

| WASP Residue/Mutation | Homer Residue |
|---|---|
| Exposed | |
| P58L | Pro 18 |
| E131K | Glu 93 |

| Buried/partially buried | |
|---|---|
| H68P | Ser 28 |
| V75M | Ser 35 |
| Y107S/C | Phe 67 |
| G125R | Gly 87 |
| F128S | Phe 90 |
| A134T/V | Leu 96 |

| Other | |
|---|---|
| A56V | - |
| W97C | - |

## Claims

1. An isolated nucleic acid encoding Homer protein 1b, wherein said nucleic acid has the nucleotide sequence set forth in SEQ ID NO:3 (Figure 8).

2. An isolated Homer protein 1b, wherein said protein has substantially the same amino acid sequence as set forth in SEQ ID NO:4 (Figure 9).

3. An expression vector encoding a polynucleotide of claim 1.

4. The expression vector of claim 3, wherein the vector is virus-derived.

5. The expression vector of claim 3, wherein the vector is a plasmid.

6. A host cell comprising a vector of claim 3.

7. The host cell of claim 6, wherein the host cell is a prokaryotic cell.

8. The host cell of claim 6, wherein the host cell is a eukaryotic cell.

9. An isolated nucleic acid encoding Homer protein 2a, wherein said nucleic acid has the nucleotide sequence set forth in SEQ ID NO:7 (Figure 10).

10. An isolated Homer protein 2a, wherein said protein has substantially the same amino acid sequence as set forth in SEQ ID NO:8 (Figure 11).

11. An expression vector comprising a polynucleotide of claim 9.

12. The expression vector of claim 11, wherein the vector is a viral vector.

13. The expression vector of claim 11, wherein the vector is a plasmid.

14. An isolated host cell comprising a vector of claim 11.

15. The host cell of claim 14, wherein the host cell is a prokaryotic cell.

16. The host cell of claim 14, wherein the host cell is a eukaryotic cell.

17. A method for identifying a compound that modulates a cellular response mediated by a cell-surface receptor comprising:
(a) incubating the compound and a cell expressing a cell-surface receptor and a Homer protein under conditions sufficient to permit the compound to interact with the cell;
(b) exposing the cell to a cell-surface receptor ligand; and
(c) comparing a cellular response to said ligand in a cell incubated with said compound with the response of a cell not incubated with said compound, thereby identifying a compound that modulates a cellular response.

18. The method of claim 17, wherein said cell-surface receptor is a glutamate receptor.

19. The method of claim 18, wherein the glutamate receptor is a Group I metabotropic glutamate receptor or ionotropic glutamate receptor.

20. The method of claim 19, wherein the metabotropic glutamate receptor is selected from the group consisting of metabotropic glutamate receptor 1 a and metabotropic glutamate receptor 5.

21. The method of claim 20, wherein the ionotropic glutamate receptor is selected from the group consisting of NMDA glutamate receptor of the class NR2B and NMDA glutamate receptor of the class NR2D.

22. The method of claim 17, wherein said Homer protein is selected from the group consisting of Homer 1a, Homer 1b, Homer 1c, Homer 2a, Homer 2b and Homer 3.

23. The method of claim 17, wherein said Homer protein is Homer 1a.

24. The method of claim 17, wherein said cellular response is an increase or decrease in calcium mobilization.

25. The method of claim 17, wherein said compound is selected from peptides, peptidomimetics, polypeptides, pharmaceuticals, chemical compounds, biological agents, antibodies, neurotropic agents, combinatorial compound libraries and anti-epileptic agents.

26. The method of claim 17, wherein said cell is selected from the group consisting of a neuronal cell, a glial cell, a cardiac cell, a bronchial cell, a uterine cell, a testicular cell, a liver cell, a renal cell, an intestinal cell, a thymus cell, a spleen cell, a placental cell, a skeletal muscle cell and a smooth muscle cell.

27. A method for identifying a compound that modulates a cellular response mediated by an intracellular protein comprising:
(a) incubating the compound and a cell expressing an intracellular protein and a Homer protein under conditions sufficient to permit the compound to interact with the cell;
(b) exposing the cell to conditions that activate said intracellular protein; and
(c) comparing a cellular response in a cell incubated with said compound with the cellular response of a cell not incubated with said compound, thereby identifying a compound that modulates a cellular response.

28. The method of claim 27, wherein said intracellular protein is selected from the group consisting of an inositol trisphosphate receptor, a ryanodine receptor, I42, 130, hInaD, and ACK-2.

29. The method of claim 27 wherein said Homer protein is selected from the group consisting of Homer 1a, Homer 1b, Homer 1c, Homer 2a, Homer 2b and Homer 3.

30. The method of claim 27, wherein said Homer protein is Homer 1a.

31. The method of claim 27, wherein said cellular response is an increase or decrease in calcium mobilization.

32. The method of claim 27, wherein the compound is selected from peptides, peptidomimetics, polypeptides, pharmaceuticals, chemical compounds, biological agents, antibodies, neurotropic agents and anti-epileptic agents.

33. A method for identifying a compound that modulates receptor-activated calcium mobilization comprising:
(a) incubating the compound and a cell expressing a Homer protein under conditions sufficient to permit the compound to interact with the cell;
(b) exposing the cell to conditions sufficient to activate calcium mobilization; and
(c) comparing the cellular response in a cell incubated with said compound with the cellular response of a cell not incubated with said compound, thereby identifying a compound that modulates receptoractivated calcium mobilization.

34. The method of claim 33, wherein said Homer protein is selected from Homer 1a, Homer 1b, Homer 1c, Homer 2a, Homer 2b, Homer 3.

35. The method of claim 33, wherein said Homer protein is Homer 1a.

36. The method of claim 33, wherein said cell response is an increase in calcium mobilization.

37. The method of claim 33, wherein said cell response is a decrease in calcium mobilization.

38. The method of claim 33, wherein the compound is selected from peptides, peptidomimetics, polypeptides, pharmaceuticals, chemical compounds, biological agents, antibodies, neurotropic agents and anti-epileptic agents.

39. A method of modulating receptor-mediated calcium mobilization comprising exposing a cell to a compound in a sufficient amount to modulate the calcium mobilization that normally occurs when a cell is exposed to an amount of ligand sufficient to activate an intracellular signaling pathway.

40. The method of claim 39, wherein the compound is selected from peptides, peptidomimetics, polypeptides, pharmaceuticals, chemical compounds, biological agents, antibodies, neurotropic agents and anti-epileptic agents.

41. The method of claim 39, wherein the ligand is an agonist or antagonist of metabotropic glutamate receptors.

42. The method of claim 39, wherein the intracellular signaling pathway is an inositol trisphosphate signaling pathway.

43. A method of identifying a compound that inhibits Homer protein activity comprising:
(a) designing a potential inhibitor for Homer protein activity that will form non-covalent bonds with amino acids in a Homer binding site based upon the crystal structure co-ordinates of Homer protein binding domain;
(b) synthesizing the inhibitor; and
(c) determining whether the inhibitor inhibits Homer protein activity.

44. The method of claim 43, wherein the crystal structure coordinates of the Homer protein binding domain are obtained from a Homer protein crystal having orthorhombic space group symmetry P2₁2₁2₁ with a = 33.79, b = 51.40, and c = 66.30 Angstroms.

45. The method of claim 44, wherein the coordinates of the Homer protein binding domain are obtained by means of computational analysis.

46. The method of claim 44, wherein the potential inhibitor is designed to form hydrogen bonds with tryptophan²⁴, phenylalanine⁷⁴, threonine⁶⁶, threonine⁶⁸, glutamine⁷⁶, alanine⁷⁸, threonine⁷⁰, and valine⁸⁵of the Homer binding domain.

47. A method for identifying a compound that affects the formation of cell surface receptors into clusters, comprising:
(a) incubating the compound and a cell expressing a Homer protein and a Shank protein under conditions sufficient to allow the compound to interact with the cell;
(b) determining the effect of the compound on the formation of cell-surface receptors into clusters; and
(c) comparing the formation of cell-surface receptors into clusters of said cell contacted with said compound with the formation of cell-surface receptors into clusters in a cell not contacted with said compounds, thereby identifying a compound that affects the formation of cell-surface receptors into clusters.

48. The method of claim 47, wherein the cell-surface receptor is a NMDA receptor.

49. The method of claim 47, wherein the cell-surface receptor is a metabotropic glutamate receptor.

50. The method of claim 49, wherein the metabotropic glutamate receptor is a group I metabotropic glutamate receptor.

51. The method of claim 50, wherein the metabotropic glutamate receptor is metabotropic glutamate receptor 1α.

52. The method of claim 50, wherein the metabotropic glutamate receptor is metabotropic glutamate receptor 5.

53. The method of claim 47, wherein said Homer protein is selected from the group consisting of Homer 1a, Homer 1b, Homer 1c, Homer 1a, Homer 2b and Homer 3.

54. The method of claim 47, wherein the compound is selected from peptides, peptidomimetics, polypeptides, pharmaceuticals, chemical compounds, biological agents, antibodies, neurotropic agents and anti-epileptic agents.

55. The method of claim 47, wherein said Shank protein is selected from the group consisting of Shank 1a, Shank 1b and Shank 3, and cortactin binding protein.

56. The method of claim 47, wherein the effect is inhibition of the recruitment of cell-surface receptors into clusters.

57. The method of claim 47, wherein the effect is stimulation of the formation of cell-surface receptors into clusters.

58. The method of claim 47, wherein said cell is selected from the group consisting of a neuronal cell, a glial cell, a cardiac cell, a bronchial cell, a uterine cell, a testicular cell, a liver cell, a renal cell, an intestinal cell, a thymus cell, a spleen cell, a placental cell, a skeletal muscle cell and a smooth muscle cell.

59. The method of claim 47, wherein said compound is selected from peptides, peptidomimetics, polypeptides, pharmaceuticals, chemical compounds, biological agents, antibodies, neurotropic agents and anti-epileptic agents.

60. A method of treating a disorder associated with glutamate receptors comprising administering to a subject in need thereof a therapeutically effective amount of a compound that modulates Homer protein activity.

61. The method of claim 60, wherein said Homer protein is selected from the group consisting of Homer 1a, Homer 1b, Homer 1c, Homer 2a, Homer 2b, and Homer 3.

62. The method of claim 60, wherein said Homer protein is Homer 1a.

63. The method of claim 60, wherein said disorder is selected from epilepsy, glutamate toxicity, disorders of memory, disorders of learning, stroke, schizophrenia, Alzheimer's disease, tissue degeneration and disorders of brain development.

64. A method of treating a disorder associated with Homer protein activity comprising administering to a subject in need thereof a therapeutically effective amount of a compound that modulates Homer protein activity.

65. The method of claim 64, wherein said disorder is a cardiac disorder, a disorder of musculature, a vasculature disorder, a neurological disorder, a psychiatric disorder, a renal disorder, a uterine disorder or a disorder of bronchial tissue.

66. An isolated nucleic acid encoding Homer protein 1c, wherein said nucleic acid has the nucleotide sequence set forth in SEQ ID NO: 5.

67. An isolated Homer protein 1c, wherein said protein has substantially the same amino acid sequence as set forth in SEQ ID NO: 6.

68. An isolated nucleic acid encoding Homer protein 2b, wherein said nucleic acid has the nucleotide sequence set forth in SEQ ID NO: 9.

69. An isolated Homer protein 2b, wherein said protein has substantially the same amino acid sequence as set forth in SEQ ID NO: 10.

70. An isolated nucleic acid encoding Homer protein 3, wherein said nucleic acid has the nucleotide sequence set forth in SEQ ID NO: 11.

71. An isolated Homer protein 3, wherein said protein has substantially the same amino acid sequence as set forth in SEQ ID NO: 12.

72. An isolated peptide, wherein said peptide is set forth in SEQ ID NO: 13, wherein R can be arginine, or zero, or one other amino acid residue.

73. An isolated peptide, wherein said peptide is set forth in SEQ ID NO: 14.

74. An isolated nucleic acid encoding a Homer Interacting Protein, wherein said nucleic acid has the nucleotide sequence set forth in SEQ ID NO: 15 or SEQ. ID NO: 17.

75. An isolated Homer Interacting Protein, wherein said protein has substantially the same amino acid sequence as set forth in SEQ ID NO: 16 or 18.

76. An isolated nucleic acid encoding Homer Interacting Protein, wherein said nucleic acid is encoded by SEQ ID NO: 19.

77. An isolated Homer Interacting Protein, wherein said protein has substantially the same amino acid sequence as set forth in SEQ ID NO: 20.

78. A substantially purified polypeptide containing a proline-rich region that binds to a polypeptide of the Homer family.

79. The polypeptide of claim 78, wherein said polypeptide is a cell-surface receptor.

80. The polypeptide of claim 78, wherein said polypeptide is an intracellular receptor.

81. A transgenic non-human animal having a transgene that expresses Homer protein 1a chromosomally integrated into the germ cells of the animal.

82. The transgenic animal of claim 81, wherein the animal is murine.

83. A method for affecting the natural aging process comprising administering to a subject in need thereof, an effective amount of a compound that modulates Homer protein activity.
